# EUROPEAN PATENT SPECIFICATION

(11) **EP 2 495 326 B1**
(45) Date of publication and mention of the grant of the patent: **20.11.2019**
(21) Application number: 11189811.0
(22) Date of filing: 21.04.2006
(51) Int. Cl.: C12N 15/44, C07K 14/11, A61K 39/145

(54) **Materials and methods for respiratory disease control in canines**
Materialien und Verfahren zur Kontrolle von Atemwegserkrankungen in Hunden
Matériaux et procédés de contrôle de maladie respiratoire chez les canins

(30) Priority: 21.04.2005 US 673443 P
(43) Date of publication of application: 05.09.2012
(62) Divisional of application: 06769869.6
(73) Proprietor: University of Florida Research Foundation, Inc., Gainesville, FL 32611 (US); The Government of the United States of America as represented by the Secretary of the Department of Health and Human Services, Atlanta, Georgia 30341 (US); Cornell Research Foundation, Inc., Ithaca, NY 14850 (US)
(72) Inventor: Crawford, Patti C., Gainesville, FL 32611 (US); Gibbs, Paul J., Gainesville, FL 32611 (US); Dubovi, Edward J., Ithaca, NY 14851-5786 (US); Donis, Ruben O., Technology Transfer Office 4770 Buford Hwy (K79) Atlanta, Georgia 30341 (US); Castleman, William L., Gainesville, FL 32611 (US); Cox, Nancy J., Technology Transfer Office 4770 Buford Hwy (K79) Atlanta, Georgia 30341 (US); Katz, Jacqueline, Technology Transfer Office 4770 Buford Hwy (K79) Atlanta, Georgia 30341 (US); Klimov, Alexander I., Technology Transfer Office 4770 Buford Hwy (K79) Atlanta, Georgia 30341 (US)
(74) Representative: Roberts, Michael Austin

(56) References cited:
- WO-A2-01/60849
- WO-A2-2007/047938
- WO-A2-2007/048086
- WO-A2-2007/118206
- CRAWFORD P C ET AL: "Transmission of Equine influenza virus to dogs", SCIENCE, AMERICAN ASSOCIATION FOR THE ADVANCEMENT OF SCIENCE, WASHINGTON, DC; US, vol. 310, no. 5747, 21 October 2005 (2005-10-21), pages 482-485, XP003003531, ISSN: 0036-8075, DOI: 10.1126/SCIENCE.1117950
- YOON KYOUNG-JIN ET AL: "Influenza virus infection in racing greyhounds", EMERGING INFECTIOUS DISEASES, EID, ATLANTA, GA, US, vol. 11, no. 12, 1 December 2005 (2005-12-01), pages 1974-1976, XP008080869, ISSN: 1080-6040
- DUBOVI EJ ET AL: "ISOLATION OF EQUINE INFLUENZA VIRUS FROM RACING GREYHOUNDS WITH FATAL HEMORRHAGIC PNEUMONIA", PROCEEDINGS OF THE ANNUAL MEETING OF THE AMERICAN ASSOCIATION OFVETERINARY LABORATORY DIAGNOSTICIANS, X, XX, no. 47TH, 1 October 2004 (2004-10-01), page 158, XP009081246,
- KLIMOV A I ET AL: "Sequence changes in the live attenuated, cold-adapted variants of influenza A/Leningrad/134/57 (H2N2) virus", VIROLOGY, ELSEVIER, AMSTERDAM, NL, vol. 186, no. 2, 1 February 1992 (1992-02-01), pages 795-797, XP026760031, ISSN: 0042-6822, DOI: 10.1016/0042-6822(92)90050-Y [retrieved on 1992-02-01]

## Description

This application is a divisional application of European Application No. 06769869.6

### BACKGROUND OF THE INVENTION

"Kennel cough" or infectious tracheobronchitis (ITB) is an acute, contagious respiratory infection in dogs characterized mainly by coughing (Ford *et al,* 1998). Canine ITB is considered one of the most prevalent infectious respiratory diseases of dogs worldwide, and outbreaks can reach epidemic proportions when dogs are housed in high-density population environments such as kennels. Most outbreaks are due to direct dog-to-dog contact or aerosolization of respiratory secretions (Ford *et al,* 1998). The clinical signs are caused by infection with one or a combination of bacterial and viral agents that colonize the epithelium of the upper and lower respiratory tract. Canine parainfluenza virus (CPiV) and *Bordetella bronchiseptica* bacteria are the most common organisms isolated from affected dogs, but several other viruses such as canine distemper virus (CDV) and canine adenoviruses-1 and -2 (CAV-1, CAV-2), along with bacteria such as *Streptococcus sp., Pasteurella multicoda* and *Escherichia coli,* can influence the clinical course and outcome (Ford *et al,* 1998). While outbreaks occur most efficiently and rapidly in high-density populations with high morbidity, complicated respiratory infections and death are uncommon. Although life-threatening secondary bacterial pneumonia can develop, the majority of ITB cases are self-limiting and resolve without any treatment (Ford *et al,* 1998).

In July 1992, a respiratory infection presumed to be "kennel cough" became epidemic at several greyhound tracks in New England, Florida, West Virginia, Wisconsin, Kansas, Colorado, Oklahoma and Arizona. According to veterinarians, most of the affected dogs had a mild cough that resolved, but more than a dozen greyhounds developed an acute hemorrhagic pneumonia followed by rapid death (Greyhound Daily News, 1999).

In late 1998 to early 1999, several outbreaks of "kennel cough" occurred in racing greyhound kennels across the country, resulting in mandatory closure of tracks and quarantine of all racing greyhounds in the U.S. for several weeks (Greyhound Daily News, 1999). At one track in Florida (Palm Beach Kennel Club), coughing was recorded in nearly 40% of the dog population on a single day (Personal communication from Dr. William Duggar). Similar to the outbreak in 1992, the coughing resolved in most greyhounds, but 10 dogs in Florida died from a hemorrhagic pneumonia syndrome uncharacteristic of "kennel cough" (Putnam, 1999).

In March-April 2003, another outbreak of "kennel cough" occurred at greyhound tracks in the eastern U.S. The outbreak is believed to have originated in kennels at four tracks in Florida and caused the suspension of racing and quarantine of dogs for almost three weeks. Nearly 25% of the dogs at the track in West Palm Beach were affected, while almost 50% of the 1400 dogs at Derby Lane in St. Petersburg developed coughing. Again, most dogs recovered, but several dogs have died from the respiratory infection. The estimated economic impact of the respiratory outbreak at the Derby Lane track alone was $2 million.

There are no published reports documenting the etiology or clinicopathology of the "kennel cough" epidemics in racing greyhound kennels in 1992, 1998-1999, or 2003. The assumption has been that the infections were due to CPiV and/or *B. bronchiseptica,* the two most common causes of kennel cough. Unsubstantiated communications such as web sites have attributed the fatal hemorrhagic pneumonias reported in some of the coughing dogs to infection with β-hemolytic *Streptococcus equi* subspecies *zooepidemicus,* and refer to the syndrome as "canine streptococcal toxic shock."

Transmission of virus from one host species to another is a crucial feature of the ecology and epidemiology of influenza virus (Webster, 1998). Two basic mechanisms of interspecies transmission of influenza virus are possible (Webster *et al.,* 1992; Lipatov *et al.,* 2004). One is the direct transfer of an essentially unaltered virus from one species to another. Examples of this mechanism include the recent human infections with the H5N1 subtype of avian influenza virus (Subbarao *et al.,* 1998; Peiris *et al.,* 2004; Guan *et al.,* 2004) and possibly the pandemic of 1918, known as Spanish flu (Reid *et al.,* 2004). The second mechanism is a consequence of the segmented nature of the influenza genome. Co-infection of a host with viruses from different species can result in reassortment of the segmented viral genes and the generation of a recombinant with the ability to infect other species. For example, novel viruses generated by gene reassortment between avian and human influenza viruses resulted in human influenza pandemics in 1957 and 1968 (Webster *et al.,* 1992; Lipatov *et al.,* 2004; Kawaoka *et al.,* 1989).

Most direct transmissions of unaltered influenza viruses from the natural host species to a different species are terminal events because sustained transmission between individuals of the new species fails to occur. Multiple virus-host interactions are necessary for replication and horizontal transmission and provide a formidable barrier to perpetuation of influenza viruses in the new host (Webby *et al.,* 2004). Therefore, establishment of new host-specific lineages of influenza virus is uncommon and has only occurred in domestic poultry, pigs, horses, and humans (Webster *et al.,* 1992; Lipatov *et al.,* 2004).

Because of the serious nature of influenza virus infection, there remains a need for methods for diagnosing, preventing, and treating infection by influenza virus.

### BRIEF SUMMARY OF THE INVENTION

The subject invention pertains *inter alia* to isolated influenza A virus that is capable of infecting a canid animal, and a composition comprising an immunogen of the influenza A virus.

In a first aspect of the invention, there is provided an isolated canine influenza A virus, the influenza virus comprising a hemagglutinin (HA) polypeptide or a polynucleotide which encodes an HA polypeptide, wherein the HA polypeptide comprises the amino acid sequence shown in SEQ ID NO: 78, or a mature sequence thereof wherein the N-terminus amino acid signal sequence of a full-length sequence has been removed.

In another aspect of the invention, there is provided a polynucleotide expression construct comprising regulatory elements and a polynucleotide which comprises a canine influenza A virus polynucleotide selected from: (i) a polynucleotide which encodes the HA polypeptide comprising the amino acid sequence shown in SEQ ID NO: 78; (ii) a polynucleotide which encodes a mature sequence of the HA polypeptide comprising the amino acid sequence shown in SEQ ID NO: 78 wherein the N-terminus amino acid signal sequence of a full-length sequence has been removed; and (iii) a polynucleotide having the nucleotide sequence shown in SEQ ID NO: 77.

In a further aspect of the invention, there is provided an isolated antibody that binds specifically to a canine influenza A virus polypeptide selected from: (i) an HA polypeptide comprising the amino acid sequence shown in SEQ ID NO: 78; and (ii) a mature sequence of the HA polypeptide comprising the amino acid sequence shown in SEQ ID NO: 78 wherein the N-terminus amino acid signal sequence of a full-length sequence has been removed.

Another aspect of the invention provides an isolated, in vitro cell comprising an influenza virus of the invention or a polynucleotide which comprises a canine influenza A virus polynucleotide selected from: (i) a polynucleotide which encodes the HA polypeptide comprising the amino acid sequence shown in SEQ ID NO: 78; (ii) a polynucleotide which encodes a mature sequence of the HA polypeptide comprising the amino acid sequence shown in SEQ ID NO: 78 wherein the N-terminus amino acid signal sequence of a full-length sequence has been removed; and (iii) a polynucleotide having the nucleotide sequence shown in SEQ ID NO: 77.

Also provided according to the invention is a reassortant virus comprising a canine influenza A virus polynucleotide selected from: (i) a polynucleotide which encodes the HA polypeptide comprising the amino acid sequence shown in SEQ ID NO: 78; (ii) a polynucleotide which encodes a mature sequence of the HA polypeptide comprising the amino acid sequence shown in SEQ ID NO: 78 wherein the N-terminus amino acid signal sequence of a full-length sequence has been removed; and (iii) a polynucleotide having the nucleotide sequence shown in SEQ ID NO: 77.

The invention further provides a recombinant viral vector or recombinant polynucleotide vector comprising an isolated polynucleotide which comprises a canine influenza A virus polynucleotide selected from: (i) a polynucleotide which encodes the HA polypeptide comprising the amino acid sequence shown in SEQ ID NO: 78; (ii) a polynucleotide which encodes a mature sequence of the HA polypeptide comprising the amino acid sequence shown in SEQ ID NO: 78 wherein the N-terminus amino acid signal sequence of a full-length sequence has been removed; and (iii) a polynucleotide having the nucleotide sequence shown in SEQ ID NO: 77.

In another aspect of the invention, there is provided a composition comprising an immunogen of an influenza virus of the invention, optionally further comprising an adjuvant, wherein the immunogen is capable of inducing an immune response against an influenza virus that is capable of infecting a canid animal and wherein the immunogen comprises a canine influenza virus of the invention, or a canine influenza virus polynucleotide which comprises a canine influenza A virus polynucleotide selected from: (i) a polynucleotide which encodes the HA polypeptide comprising the amino acid sequence shown in SEQ ID NO: 78; (ii) a polynucleotide which encodes a mature sequence of the HA polypeptide comprising the amino acid sequence shown in SEQ ID NO: 78 wherein the N-terminus amino acid signal sequence of a full-length sequence has been removed; and (iii) a polynucleotide having the nucleotide sequence shown in SEQ ID NO: 77, or a polynucleotide expression construct of the invention, or a polypeptide comprising a canine influenza A virus polypeptide selected from: (i) an HA polypeptide comprising the amino acid sequence shown in SEQ ID NO: 78; and (ii) a mature sequence of the HA polypeptide comprising the amino acid sequence shown in SEQ ID NO: 78 wherein the N-terminus amino acid signal sequence of a full-length sequence has been removed, or a reassortant virus of the invention, or a vector of the invention, wherein the composition is provided in a physiologically acceptable carrier or buffer.

The invention further provides a canine influenza vaccine for use in inducing an immune response in a canid animal against a canine influenza virus, wherein the vaccine comprises an influenza virus of the invention, or a polynucleotide expression construct of the invention, or an isolated polypeptide comprising a canine influenza A virus polypeptide selected from: (i) an HA polypeptide comprising the amino acid sequence shown in SEQ ID NO: 78; and (ii) a mature sequence of the HA polypeptide comprising the amino acid sequence shown in SEQ ID NO: 78 wherein the N-terminus amino acid signal sequence of a full-length sequence has been removed, or a reassortant virus of the invention, or a vector of the invention, or a composition of the invention.

Further features of the invention are as defined in the appended claims and described below.

### BRIEF DESCRIPTION OF THE DRAWINGS

The patent or application file contains at least one drawing executed in color. Copies of this patent or patent application publication with color drawings will be provided by the Patent Office upon request and payment of the necessary fee.

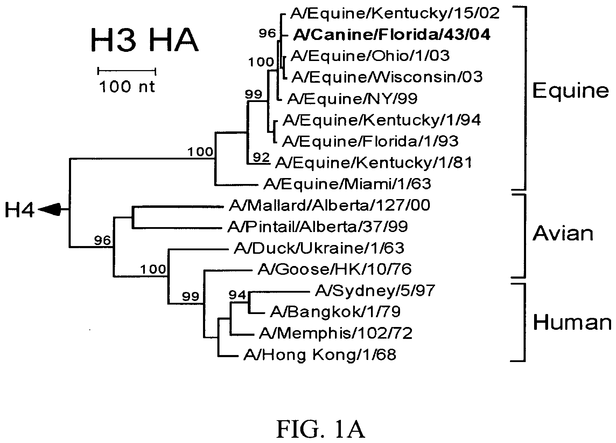

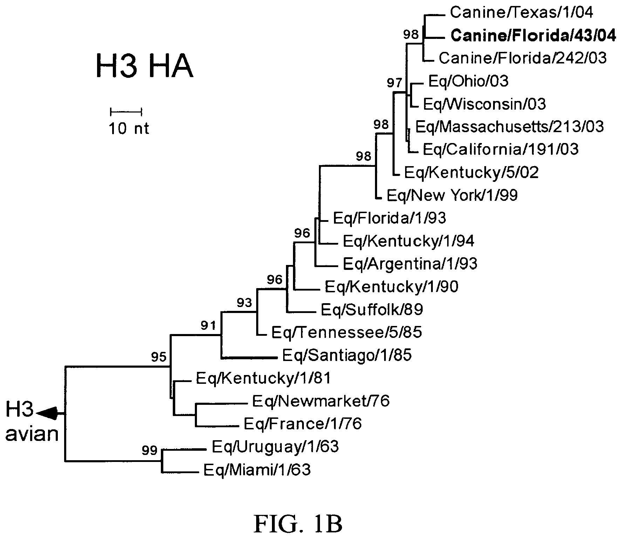

**Figures 1A-1B** show phylogenetic relationships among the hemagglutinin genes. **Figure 1A** shows a tree of HA genes from representative canine, human, avian, swine, and equine isolates, including A/Budgerigar/Hokkaido/1/77 (H4) as outgroup. **Figure 1B** shows a tree of the canine influenza virus HA genes with contemporary and older equine HA genes, using A/Duck/Ukraine/63 (H3) as outgroup. Phylogenetic trees were inferred from nucleotide sequences by the neighbor joining method and bootstrap analysis values ≥90% are shown. The bar denotes the number of nucleotide changes per unit length of the horizontal tree branches.

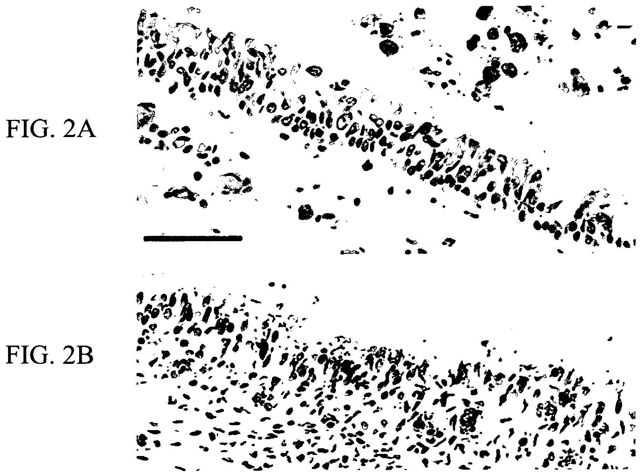

**Figures 2A-2B** show immunohistochemical detection of influenza H3 antigen in the lungs. Lung tissue sections were probed with a mouse monoclonal antibody to H3 hemagglutinin and binding was detected by immunoperoxidase reaction (brown precipitate). **Figure 2A** shows bronchial epithelium from a greyhound with spontaneous disease. Viral H3 antigen was detected in bronchial epithelial cell cytoplasm and in macrophages in airway lumens and in alveolar spaces. **Figure 2B** shows bronchial epithelium from a dog 5 days after inoculation with A/canine/Florida/43/04 (H3N8). Viral H3 antigen was detected in bronchial epithelial cell cytoplasm. Scale bar, 66 µm.

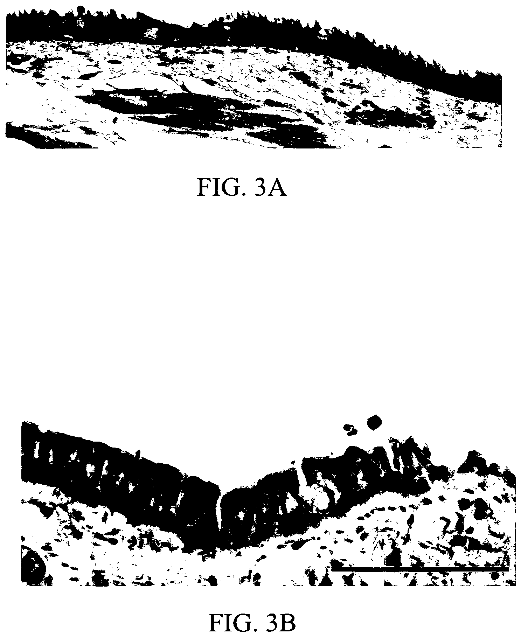

**Figure 3** shows the characteristic histological changes in the bronchi of greyhounds that died from hemorrhagic pneumonia associated with influenza virus infection. The tissues are stained with H&E. Upper panel: Normal bronchus with ciliated epithelial cells, mucous cells, and basal cells. Lower panel: Bronchus from a greyhound with spontaneous influenza. There is necrosis and erosion of the bronchial ciliated epithelial cells. Scale bar, 100 µm.

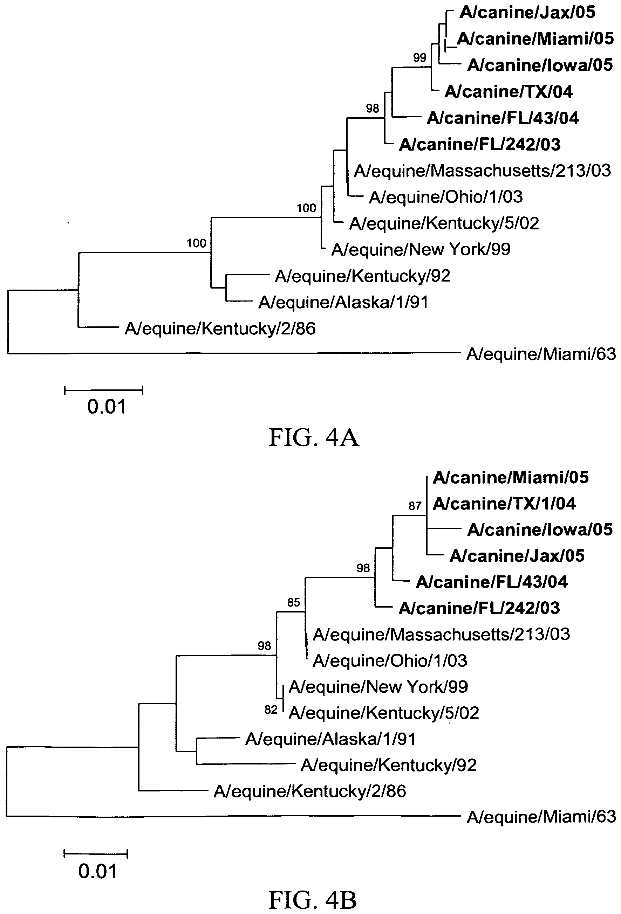

**Figures 4A-4B** shows phylogenetic relationships among the H3 hemagglutinin genes. **Figure 4A** shows a phylogenetic tree of the canine influenza virus HA genes with contemporary and older equine HA genes. **Figure 4B** shows a phylogenetic tree of the canine influenza virus HA protein with contemporary and older equine HA. Phylogenetic trees were inferred from genetic or amino acid sequences by the neighbor joining method and bootstrap analysis values ≥80% are shown. The bar denotes the number of amino acid changes per unit length of the horizontal tree branches.

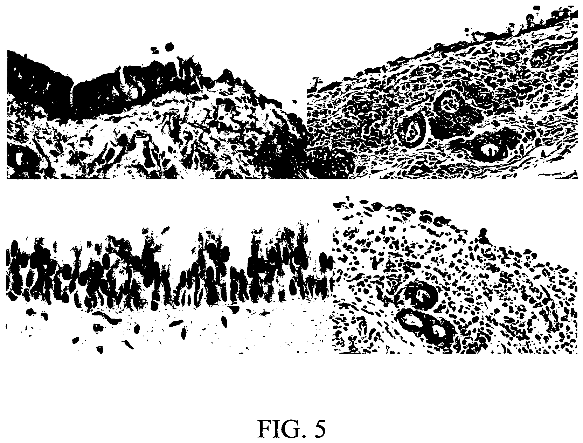

**Figure 5** shows Influenza virus H3 protein in epithelial cells of bronchi and bronchial glands in lungs of dogs that died of pneumonia associated with influenza virus infection. Upper panels: Erosion of ciliated bronchial epithelial cells in bronchi. Tissues were stained with H&E. Lower panels: Influenza virus H3 protein in the cytoplasm of bronchial (left) and bronchial gland (right) epithelial cells. Tissues were stained with a monoclonal antibody to influenza H3 detected by immunoperoxidase reaction (brown precipitate) and counterstained with hematoxylin.

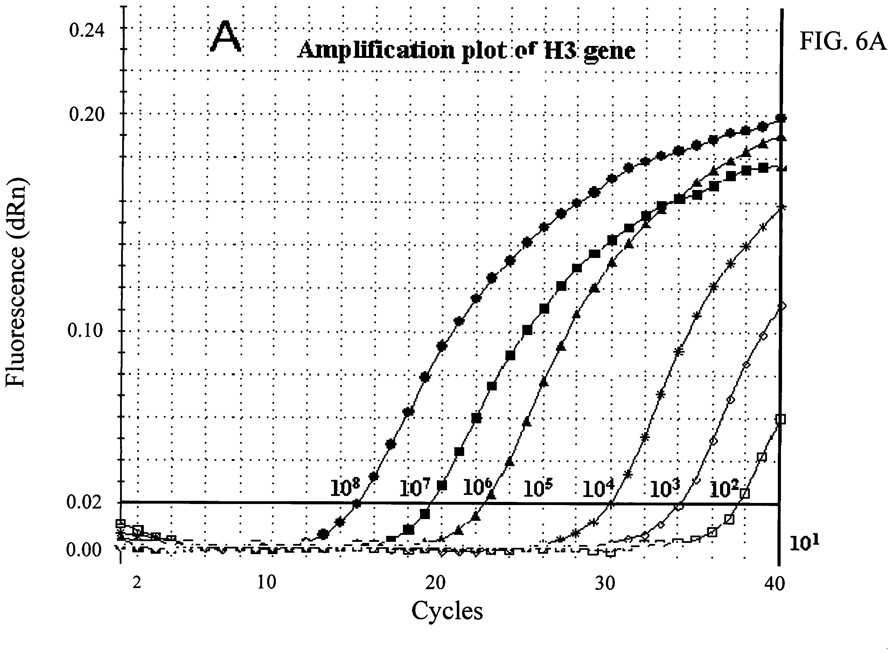

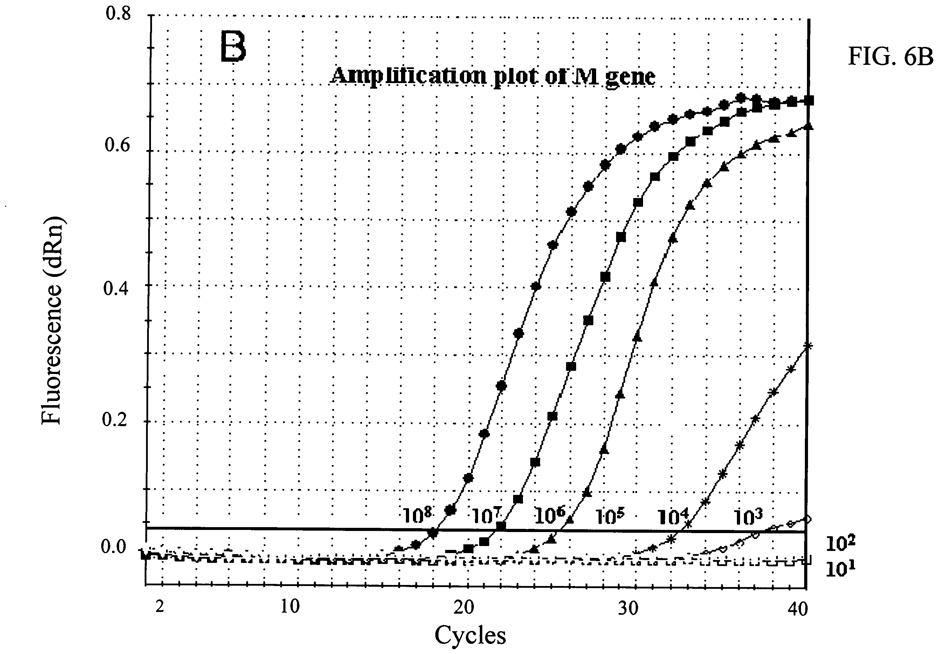

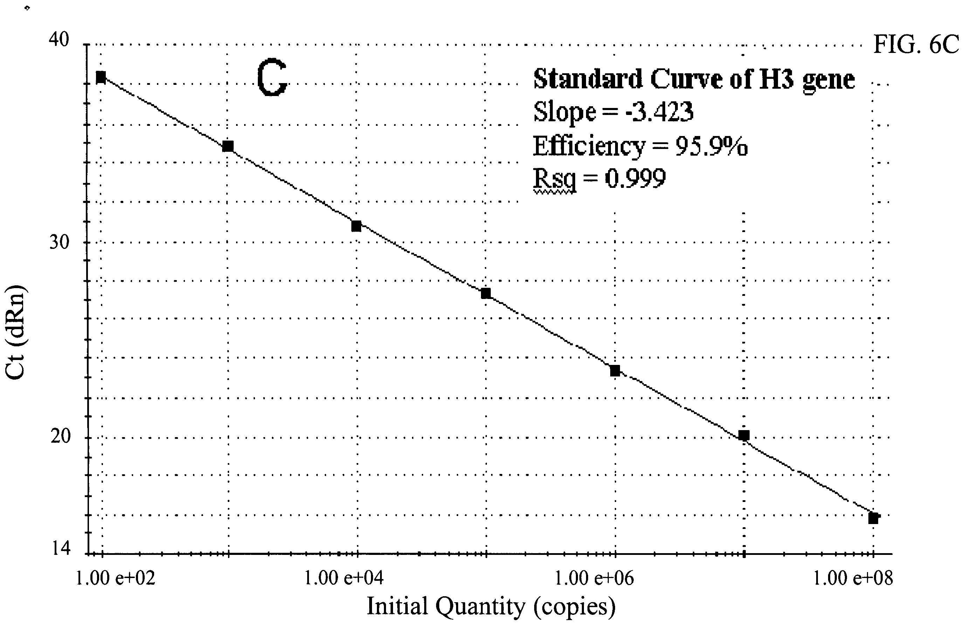

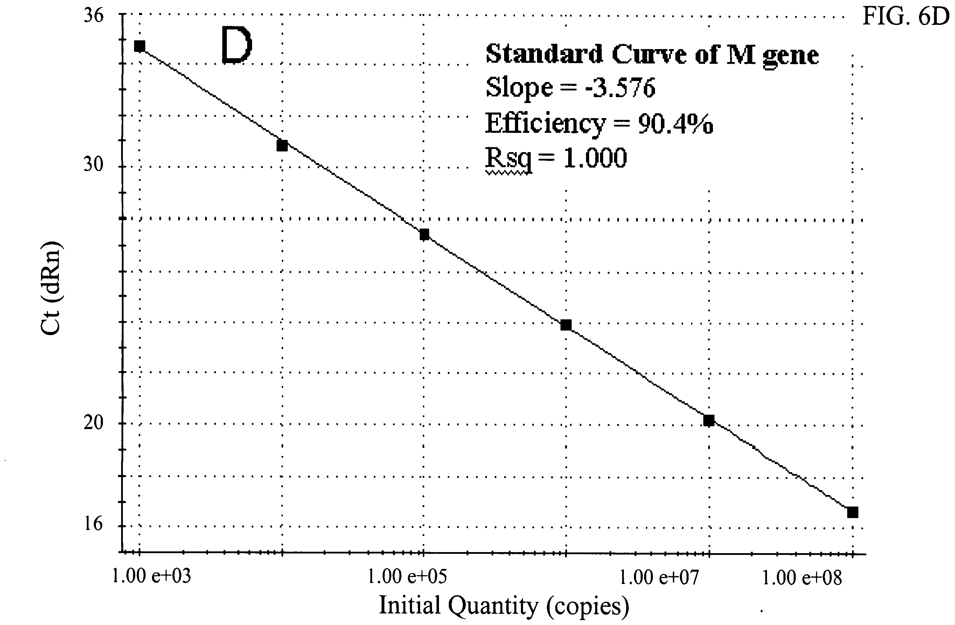

**Figures 6A-6D** show amplification plots of H3 and Matrix genes (**Figure 6A** and **Figure 6B****)** obtained from the amplification of 10-fold serially diluted *in vitro* transcribed RNA standards. Standard curves of H3 and Matrix genes (**Figure 6C** and **Figure 6D**) constructed by plotting the log of starting RNA concentrations against the threshold cycle (Ct) obtained from each dilution.

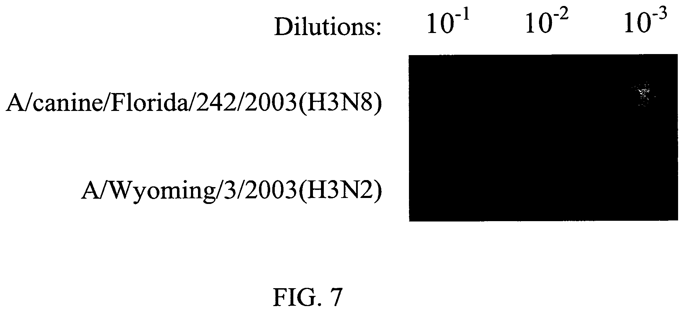

**Figure 7** shows sensitivity of Directigen Flu A was tested with 10-fold serially diluted virus stocks including A/Wyoming/3/2003 and A/canine/FL/242/2003. The purple triangle indicates positive result.

### BRIEF DESCRIPTION OF THE SEQUENCES

**SEQ ID NO: 1** is a nucleotide sequence of a canine influenza virus (Florida/43/04) encoding a PB2 protein.
**SEQ ID NO: 2** is the amino acid sequence encoded by SEQ ID NO: 1.
**SEQ ID NO: 3** is a nucleotide sequence of a canine influenza virus (Florida/43/04) encoding a PB1 protein.
**SEQ ID NO: 4** is the amino acid sequence encoded by SEQ ID NO: 3.
**SEQ ID NO: 5** is a nucleotide sequence of a canine influenza virus (Florida/43/04) encoding a PA protein.
**SEQ ID NO: 6** is the amino acid sequence encoded by SEQ ID NO: 5.
**SEQ ID NO: 7** is a nucleotide sequence of a canine influenza virus (Florida/43/04) encoding an NS protein.
**SEQ ID NO: 8** is the amino acid sequence encoded by SEQ ID NO: 7.
**SEQ ID NO: 9** is a nucleotide sequence of a canine influenza virus (Florida/43/04) encoding an NP protein.
**SEQ ID NO: 10** is the amino acid sequence encoded by SEQ ID NO: 9.
**SEQ ID NO: 11** is a nucleotide sequence of a canine influenza virus (Florida/43/04) encoding an NA protein.
**SEQ ID NO: 12** is the amino acid sequence encoded by SEQ ID NO: 11.
**SEQ ID NO: 13** is a nucleotide sequence of a canine influenza virus (Florida/43/04) encoding an MA protein.
**SEQ ID NO: 14** is the amino acid sequence encoded by SEQ ID NO: 13.
**SEQ ID NO: 15** is a nucleotide sequence of a canine influenza virus (Florida/43/04) encoding an HA protein.
**SEQ ID NO: 16** is the amino acid sequence encoded by SEQ ID NO: 15.
**SEQ ID NO: 17** is a nucleotide sequence of a canine influenza virus (FL/242/03) encoding a PB2 protein.
**SEQ ID NO: 18** is the amino acid sequence encoded by SEQ ID NO: 17.
**SEQ ID NO: 19** is a nucleotide sequence of a canine influenza virus (FL/242/03) encoding a PB1 protein.
**SEQ ID NO: 20** is the amino acid sequence encoded by SEQ ID NO: 19.
**SEQ ID NO: 21** is a nucleotide sequence of a canine influenza virus (FL/242/03) encoding a PA protein.
**SEQ ID NO: 22** is the amino acid sequence encoded by SEQ ID NO: 21.
**SEQ ID NO: 23** is a nucleotide sequence of a canine influenza virus (FL/242/03) encoding an NS protein.
**SEQ ID NO: 24** is the amino acid sequence encoded by SEQ ID NO: 23.
**SEQ ID NO: 25** is a nucleotide sequence of a canine influenza virus (FL/242/03) encoding an NP protein.
**SEQ ID NO: 26** is the amino acid sequence encoded by SEQ ID NO: 25.
**SEQ ID NO: 27** is a nucleotide sequence of a canine influenza virus (FL/242/03) encoding an NA protein.
**SEQ ID NO: 28** is the amino acid sequence encoded by SEQ ID NO: 27.
**SEQ ID NO: 29** is a nucleotide sequence of a canine influenza virus (FL/242/03) encoding an MA protein.
**SEQ ID NO: 30** is the amino acid sequence encoded by SEQ ID NO: 29.
**SEQ ID NO: 31** is a nucleotide sequence of a canine influenza virus (FL/242/03) encoding an HA protein.
**SEQ ID NO: 32** is the amino acid sequence encoded by SEQ ID NO: 31.
**SEQ ID NO: 33** is the mature form of the HA protein shown in SEQ ID NO: 16 wherein the N-terminal 16 amino acid signal sequence has been removed.
**SEQ ID NO: 34** is the mature form of the HA protein shown in SEQ ID NO: 32 wherein the N-terminal 16 amino acid signal sequence has been removed.
**SEQ ID NO: 35** is an oligonucleotide.
**SEQ ID NO: 36** is an oligonucleotide.
**SEQ ID NO: 37** is an oligonucleotide.
**SEQ ID NO: 38** is an oligonucleotide.
**SEQ ID NO: 39** is an oligonucleotide.
**SEQ ID NO: 41** is an oligonucleotide.
**SEQ ID NO: 42** is an oligonucleotide.
**SEQ ID NO: 43** is an oligonucleotide.
**SEQ ID NO: 44** is an oligonucleotide.
**SEQ ID NO: 45** is an oligonucleotide.
**SEQ ID NO: 46** is an oligonucleotide.
**SEQ ID NO: 47** is a nucleotide sequence of a canine influenza virus (Miami/2005) encoding a PB2 protein.
**SEQ ID NO: 48** is the amino acid sequence encoded by SEQ ID NO: 47.
**SEQ ID NO: 49** is a nucleotide sequence of a canine influenza virus (Miami/2005) encoding a PB1 protein.
**SEQ ID NO: 50** is the amino acid sequence encoded by SEQ ID NO: 49.
**SEQ ID NO: 51** is a nucleotide sequence of a canine influenza virus (Miami/2005) encoding a PA protein.
**SEQ ID NO: 52** is the amino acid sequence encoded by SEQ ID NO: 51.
**SEQ ID NO: 53** is a nucleotide sequence of a canine influenza virus (Miami/2005) encoding an NS protein.
**SEQ ID NO: 54** is the amino acid sequence encoded by SEQ ID NO: 53.
**SEQ ID NO: 55** is a nucleotide sequence of a canine influenza virus (Miami/2005) encoding an NP protein.
**SEQ ID NO: 56** is the amino acid sequence encoded by SEQ ID NO: 55.
**SEQ ID NO: 57** is a nucleotide sequence of a canine influenza virus (Miami/2005) encoding an NA protein.
**SEQ ID NO: 58** is the amino acid sequence encoded by SEQ ID NO: 57.
**SEQ ID NO: 59** is a nucleotide sequence of a canine influenza virus (Miami/2005) encoding an MA protein.
**SEQ ID NO: 60** is the amino acid sequence encoded by SEQ ID NO: 59.
**SEQ ID NO: 61** is a nucleotide sequence of a canine influenza virus (Miami/2005) encoding an HA protein.
**SEQ ID NO: 62** is the amino acid sequence encoded by SEQ ID NO: 61.
**SEQ ID NO: 63** is a nucleotide sequence of a canine influenza virus (Jacksonville/2005) encoding a PB2 protein.
**SEQ ID NO: 64** is the amino acid sequence encoded by SEQ ID NO: 63.
**SEQ ID NO: 65** is a nucleotide sequence of a canine influenza virus (Jacksonville/2005) encoding a PB1 protein.
**SEQ ID NO: 66** is the amino acid sequence encoded by SEQ ID NO: 65.
**SEQ ID NO: 67** is a nucleotide sequence of a canine influenza virus (Jacksonville/2005) encoding a PA protein.
**SEQ ID NO: 68** is the amino acid sequence encoded by SEQ ID NO: 67.
**SEQ ID NO: 69** is a nucleotide sequence of a canine influenza virus (Jacksonville/2005) encoding an NS protein.
**SEQ ID NO: 70** is the amino acid sequence encoded by SEQ ID NO: 69.
**SEQ ID NO: 71** is a nucleotide sequence of a canine influenza virus (Jacksonville/2005) encoding an NP protein.
**SEQ ID NO: 72** is the amino acid sequence encoded by SEQ ID NO: 71.
**SEQ ID NO: 73** is a nucleotide sequence of a canine influenza virus (Jacksonville/2005) encoding an NA protein.
**SEQ ID NO: 74** is the amino acid sequence encoded by SEQ ID NO: 73.
**SEQ ID NO: 75** is a nucleotide sequence of a canine influenza virus (Jacksonville/2005) encoding an MA protein.
**SEQ ID NO: 76** is the amino acid sequence encoded by SEQ ID NO: 75.
**SEQ ID NO: 77** is a nucleotide sequence of a canine influenza virus (Jacksonville/2005) encoding an HA protein.
**SEQ ID NO: 78** is the amino acid sequence encoded by SEQ ID NO: 77.
**SEQ ID NO: 79** is an oligonucleotide.
**SEQ ID NO: 80** is an oligonucleotide.
**SEQ ID NO: 81** is an oligonucleotide.
**SEQ ID NO: 82** is an oligonucleotide.
**SEQ ID NO: 83** is an oligonucleotide.
**SEQ ID NO: 84** is an oligonucleotide.
**SEQ ID NO: 85** is an oligonucleotide.
**SEQ ID NO: 86** is an oligonucleotide.
**SEQ ID NO: 87** is an oligonucleotide.
**SEQ ID NO: 88** is an oligonucleotide.

### DETAILED DISCLOSURE

The subject disclosure (not according to the invention, unless otherwise stated) concerns isolated influenza virus that is capable of infecting canids and causing respiratory disease. An influenza virus can comprise a polynucleotide which encodes a protein having an amino acid sequence shown in any of SEQ ID NOs: 2, 4, 6, 8, 10, 12, 14 , 16, 18, 20, 22, 24, 26, 28, 30, 32, 33, 34, 48, 50, 52, 54, 56, 58, 60, 62, 64, 66, 68, 70, 72, 74, 76, or 78, or a functional and/or immunogenic fragment or variant thereof. The polynucleotide can comprise the nucleotide sequence shown in any of SEQ ID Nos: 1, 3, 5, 7, 9, 11, 13, 15, 17, 19, 21, 23, 25, 27, 29, 31, 47, 49, 51, 53, 55, 57, 59, 61, 63, 65, 67, 69, 71, 73, 75, or 77, or a fragment or variant thereof. An isolated influenza virus of the present disclosure is subtype H3. Virus can be isolated from infected dogs and cultured in cells or eggs according to methods described herein. The influenza virus is an influenza A virus.

The subject disclosure also concerns polynucleotides that comprise all or part of a gene or genes or a genomic segment of an influenza virus. A polynucleotide of the disclosure may comprise an influenza hemagglutinin (HA) gene, neuraminidase (NA) gene, nucleoprotein (NP) gene, matrix protein (MA or M) gene, polymerase basic (PB) protein gene, polymerase acidic (PA) protein gene, non-structural (NS) protein gene, or a functional fragment or variant of any of these genes. A polynucleotide of the disclosure may comprise the hemagglutinin (HA) gene, or a functional fragment or variant thereof. The HA gene may encode a hemagglutinin protein having one or more of the following: a serine at position 83; a leucine at position 222; a threonine at position 328; and/or a threonine at position 483, versus the amino acid sequence of equine H3 consensus sequence. The HA gene may encode a polypeptide having an amino acid sequence shown in SEQ ID NOs: 16, 32, 62, or 78, or a functional and/or immunogenic fragment or variant thereof. The HA gene may comprise a nucleotide sequence shown in SEQ ID NOs: 15, 31, 61, or 77.

A polynucleotide of the disclosure encodes a polypeptide having the amino acid sequence shown in any of SEQ ID NOs: 2, 4, 6, 8, 10, 12, 14, 16, 18, 20, 22, 24, 26, 28, 30, 32, 33, 34, 48, 50, 52, 54, 56, 58, 60, 62, 64, 66, 68, 70, 72, 74, 76, or 78, or a functional and/or immunogenic fragment or variant thereof. The polynucleotide encoding the amino acid sequence shown in SEQ ID NOs: 2, 4, 6, 8, 10, 12, 14, 16, 18, 20, 22, 24, 26, 28, 30, 32, 33, 34, 48, 50, 52, 54, 56, 58, 60, 62, 64, 66, 68, 70, 72, 74, 76, or 78, comprises the nucleotide sequence shown in SEQ ID NOs: 1, 3, 5, 7, 9, 11, 13, 15, 17, 19, 21, 23, 25, 27, 29, 31, 47, 49, 51, 53, 55, 57, 59, 61, 63, 65, 67, 69, 71, 73, 75, or 77, respectively, or a sequence encoding a functional and/or immunogenic fragment or variant of any of SEQ ID NOs: 2, 4, 6, 8, 10, 12, 14, 16, 18, 20, 22, 24, 26, 28, 30, 32, 33, 34, 48, 50, 52, 54, 56, 58, 60, 62, 64, 66, 68, 70, 72, 74, 76, or 78. Thus, the subject disclosure concerns polynucleotide sequences comprising the nucleotide sequence shown in any of SEQ ID NOs: 1, 3, 5, 7, 9, 11, 13, 15, 17, 19, 21, 23, 25, 27, 29, 31, 47, 49, 51, 53, 55, 57, 59, 61, 63, 65, 67, 69, 71, 73, 75, or 77, or a fragment or variant, including a degenerate variant, of any of SEQ ID NOs: 1, 3, 5, 7, 9, 11, 13, 15, 17, 19, 21, 23, 25, 27, 29, 31, 47, 49, 51, 53, 55, 57, 59, 61, 63, 65, 67, 69, 71, 73, 75, or 77. A polynucleotide of the disclosure can comprise: Nucleotides 1-2271 of SEQ ID NO: 3; Nucleotides 1-2148 of SEQ ID NO: 5; Nucleotides 1-657 of SEQ ID NO: 7; Nucleotides 1-1494 of SEQ ID NO: 9; Nucleotides 1-1410 of SEQ ID NO: 11; Nucleotides 1-756 of SEQ ID NO: 13; Nucleotides 1-1695 of SEQ ID NO: 15; Nucleotides 1-2271 of SEQ ID NO: 19; Nucleotides 1-2148 of SEQ ID NO: 21; Nucleotides 1-657 of SEQ ID NO: 23; Nucleotides 1-1494 of SEQ ID NO: 25; Nucleotides 1-756 of SEQ ID NO: 29; Nucleotides 1-1695 of SEQ ID NO: 31; Nucleotides 1-2277 of SEQ ID NO: 47; Nucleotides 1-2271 of SEQ ID NO: 49; Nucleotides 1-2148 of SEQ ID NO: 51; Nucleotides 1-690 of SEQ ID NO: 53; Nucleotides 1-1494 of SEQ ID NO: 55; Nucleotides 1-1410 of SEQ ID NO: 57; Nucleotides 1-756 of SEQ ID NO: 59; Nucleotides 1-1695 of SEQ ID NO: 61; Nucleotides 1-2277 of SEQ ID NO: 63; Nucleotides 1-2271 of SEQ ID NO: 65; Nucleotides 1-2148 of SEQ ID NO: 67; Nucleotides 1-690 of SEQ ID NO: 69; Nucleotides 1-1494 of SEQ ID NO: 71; Nucleotides 1-1410 of SEQ ID NO: 73; Nucleotides 1-756 of SEQ ID NO: 75; and Nucleotides 1-1695 of SEQ ID NO: 77. Nucleotide and amino acid sequences of viral polynucleotide and polypeptide sequences contemplated within the scope of the present disclosure have also been deposited with GenBank at accession Nos. DQ124147 through DQ124161 and DQ124190.

The subject disclosure also concerns polypeptides encoded by polynucleotides of an influenza virus. The subject disclosure also concerns functional and/or immunogenic fragments and variants of the subject polypeptides. Polypeptides contemplated include HA protein, NA protein, NS protein, nucleoprotein, polymerase basic protein, polymerase acidic protein, and matrix protein of an influenza virus of the disclosure. A polypeptide of the disclosure may have an amino acid sequence shown in any of SEQ ID NOs: 2, 4, 6, 8, 10, 12, 14, 16, 18, 20, 22, 24, 26, 28, 30, 32, 33, 34, 48, 50, 52, 54, 56, 58, 60, 62, 64, 66, 68, 70, 72, 74, 76, or 78, or a functional and/or immunogenic fragment or variant thereof.

The subject disclosure also concerns polynucleotide expression constructs comprising a polynucleotide sequence of the present disclosure. An expression construct of the disclosure may comprise a polynucleotide sequence encoding a polypeptide comprising an amino acid sequence shown in any of SEQ ID NOs: 2, 4, 6, 8, 10, 12, 14, 16, 18, 20, 22, 24, 26, 28, 30, 32, 33, 34, 48, 50, 52, 54, 56, 58, 60, 62, 64, 66, 68, 70, 72, 74, 76, or 78, or a functional and/or immunogenic fragment or variant thereof. The polynucleotide encoding the amino acid sequence may be SEQ ID NOs: 2, 4, 6, 8, 10, 12, 14, 16, 18, 20, 22, 24, 26, 28, 30, 32, 33, 34, 48, 50, 52, 54, 56, 58, 60, 62, 64, 66, 68, 70, 72, 74, 76, or 78 comprises the nucleotide sequence shown in SEQ ID NOs: 1, 3, 5, 7, 9, 11, 13, 15, 17, 19, 21, 23, 25, 27, 29, 31, 47, 49, 51, 53, 55, 57, 59, 61, 63, 65, 67, 69, 71, 73, 75, or 77, respectively, or a sequence encoding a functional and/or immunogenic fragment or variant of any of SEQ ID NOs: 2, 4, 6, 8, 10, 12, 14, 16, 18, 20, 22, 24, 26, 28, 30, 32, 33, 34, 48, 50, 52, 54, 56, 58, 60, 62, 64, 66, 68, 70, 72, 74, 76, or 78. Thus, the subject disclosure concerns expression constructs comprising a polynucleotide sequence comprising the nucleotide sequence shown in any of SEQ ID NOs: 1, 3, 5, 7, 9, 11, 13, 15, 17,19, 21, 23, 25, 27, 29, 31, 47, 49, 51, 53, 55, 57, 59, 61, 63, 65, 67, 69, 71, 73, 75, or 77, or a fragment or variant, including a degenerate variant, of any of SEQ ID NOs: 1, 3, 5, 7, 9, 11, 13, 15, 17, 19, 21, 23, 25, 27, 29, 31, 47, 49, 51, 53, 55, 57, 59, 61, 63, 65, 67, 69, 71, 73, 75, or 77. An expression construct of the present disclosure may provide for overexpression of an operably linked polynucleotide of the disclosure.

Expression constructs may generally include regulatory elements that are functional in the intended host cell in which the expression construct is to be expressed. Thus, a person of ordinary skill in the art can select regulatory elements for use in, for example, human host cells, mammalian host cells, insect host cells, yeast host cells, bacterial host cells, and plant host cells. The regulatory elements may be ones that are functional in canine cells. Regulatory elements include promoters, transcription termination sequences, translation termination sequences, enhancers, and polyadenylation elements. As used herein, the term "expression construct" refers to a combination of nucleic acid sequences that provides for transcription of an operably linked nucleic acid sequence. As used herein, the term "operably linked" refers to a juxtaposition of the components described wherein the components are in a relationship that permits them to function in their intended manner. In general, operably linked components are in contiguous relation.

An expression construct can comprise a promoter sequence operably linked to a polynucleotide sequence encoding a polypeptide. Promoters can be incorporated into a polynucleotide using standard techniques known in the art. Multiple copies of promoters or multiple promoters can be used in an expression construct. A promoter can be positioned about the same distance from the transcription start site in the expression construct as it is from the transcription start site in its natural genetic environment. Some variation in this distance is permitted without substantial decrease in promoter activity. A transcription start site is typically included in the expression construct. A promoter associated with an expression construct can provide for overexpression of an operably linked polynucleotide.

Promoters for use with an expression construct in eukaryotic cells can be of viral or cellular origin. Viral promoters include, but are not limited to, cytomegalovirus (CMV) gene promoters, SV40 early or late promoters, or Rous sarcoma virus (RSV) gene promoters. Promoters of cellular origin include, but are not limited to, desmin gene promoter and actin gene promoter Promoters suitable for use with an expression construct in yeast cells include, but are not limited to, 3-phosphoglycerate kinase promoter, glyceraldehyde-3-phosphate dehydrogenase promoter, metallothionein promoter, alcohol dehydrogenase-2 promoter, and hexokinase promoter.

If the expression construct is to be provided in or introduced into a plant cell, then plant viral promoters, such as, for example, a cauliflower mosaic virus (CaMV) 35S (including the enhanced CaMV 35S promoter (see, for example U.S. Patent No. 5,106,739 and An, 1997)) or a CaMV 19S promoter can be used. Other promoters that can be used for expression constructs in plants include, for example, prolifera promoter, Ap3 promoter, heat shock promoters, T-DNA 1'- or 2'-promoter of A. *tumefaciens,* polygalacturonase promoter, chalcone synthase A (CHS-A) promoter from petunia, tobacco PR-1a promoter, ubiquitin promoter, actin promoter, alcA gene promoter, pin2 promoter (Xu *et al.,* 1993), maize WipI promoter, maize trpA gene promoter (U.S. Patent No. 5,625,136), maize CDPK gene promoter, and RUBISCO SSU promoter (U.S. Patent No. 5,034,322) can also be used. Root-specific promoters, such as any of the promoter sequences described in U.S. Patent No. 6,455,760 or U.S. Patent No. 6,696,623, or in published U.S. patent application Nos. 20040078841; 20040067506; 20040019934; 20030177536; 20030084486; or 20040123349, can be used with an expression construct of the disclosure. Constitutive promoters (such as the CaMV, ubiquitin, actin, or NOS promoter), developmentally-regulated promoters, and inducible promoters (such as those promoters than can be induced by heat, light, hormones, or chemicals) can also be used. Tissue-specific promoters, for example fruit-specific promoters, such as the E8 promoter of tomato (accession number: AF515784; Good *et al.* (1994)) can also be used. Seed-specific promoters such as the promoter from a β-phaseolin gene (for example, of kidney bean) or a glycinin gene (for example, of soybean), and others, can also be used.

For expression in prokaryotic systems, an expression construct can comprise promoters such as, for example, alkaline phosphatase promoter, tryptophan (trp) promoter, lambda P_{L} promoter, β-lactamase promoter, lactose promoter, phoA promoter, T3 promoter, T7 promoter, or tac promoter (de Boer *et al.,* 1983).

Expression constructs may optionally contain a transcription termination sequence, a translation termination sequence, a sequence encoding a signal peptide, and/or enhancer elements. Transcription termination regions can typically be obtained from the 3' untranslated region of a eukaryotic or viral gene sequence. Transcription termination sequences can be positioned downstream of a coding sequence to provide for efficient termination. A signal peptide sequence is a short amino acid sequence typically present at the amino terminus of a protein that is responsible for the relocation of an operably linked mature polypeptide to a wide range of post-translational cellular destinations, ranging from a specific organelle compartment to sites of protein action and the extracellular environment. Targeting gene products to an intended cellular and/or extracellular destination can be achieved through the use of an operably linked signal peptide sequence. Classical enhancers are cis-acting elements that increase gene transcription and can also be included in the expression construct. Classical enhancer elements are known in the art, and include, but are not limited to, the CaMV 35S enhancer element, cytomegalovirus (CMV) early promoter enhancer element, and the SV40 enhancer element. Intron-mediated enhancer elements that enhance gene expression are also known in the art. These elements must be present within the transcribed region and are orientation dependent.

DNA sequences which direct polyadenylation of mRNA transcribed from the expression construct can also be included in the expression construct, and include, but are not limited to, an octopine synthase or nopaline synthase signal.

Expression constructs can also include one or more dominant selectable marker genes, including, for example, genes encoding antibiotic resistance and/or herbicide-resistance for selecting transformed cells. Antibiotic-resistance genes can provide for resistance to one or more of the following antibiotics: hygromycin, kanamycin, bleomycin, G418, streptomycin, paromomycin, neomycin, and spectinomycin. Kanamycin resistance can be provided by neomycin phosphotransferase (NPT II). Herbicide-resistance genes can provide for resistance to phosphinothricin acetyltransferase or glyphosate. Other markers used for cell transformation screening include, but are not limited to, genes encoding β-glucuronidase (GUS), β-galactosidase, luciferase, nopaline synthase, chloramphenicol acetyltransferase (CAT), green fluorescence protein (GFP), or enhanced GFP (Yang *et al.,* 1996).

The subject disclosure also concerns polynucleotide vectors comprising a polynucleotide sequence that encodes a polypeptide. Unique restriction enzyme sites can be included at the 5' and 3' ends of an expression construct or polynucleotide to allow for insertion into a polynucleotide vector. As used herein, the term "vector" refers to any genetic element, including for example, plasmids, cosmids, chromosomes, phage, virus, and the like, which is capable of replication when associated with proper control elements and which can transfer polynucleotide sequences between cells. Vectors contain a nucleotide sequence that permits the vector to replicate in a selected host cell. A number of vectors are available for expression and/or cloning, and include, but are not limited to, pBR322, pUC series, M13 series, pGEM series, and pBLUESCRIPT vectors (Stratagene, La Jolla, CA and Promega, Madison, WI).

The subject disclosure also concerns oligonucleotide probes and primers, such as polymerase chain reaction (PCR) primers, that can hybridize to a coding or non-coding sequence of a polynucleotide. Oligonucleotide probes can be used in methods for detecting influenza virus nucleic acid sequences. Oligonucleotide primers can be used in PCR methods and other methods involving nucleic acid amplification. A probe or primer of the disclosure can hybridize to a polynucleotide of the disclosure under stringent conditions. Probes and primers can optionally comprise a detectable label or reporter molecule, such as fluorescent molecules, enzymes, radioactive moiety, and the like. Probes and primers can be of any suitable length for the method or assay in which they are being employed. Typically, probes and primers can be 10 to 500 or more nucleotides in length. Probes and primers that are 10 to 20, 21 to 30, 31 to 40, 41 to 50, 51 to 60, 61 to 70, 71 to 80, 81 to 90, 91 to 100, or 101 or more nucleotides in length are contemplated. Probes and primers are any of 9, 10, 11, 12, 13, 14, 15, 16, 17, 18, 19, 20, 21, 22, 23, 24, 25, 26, 27, 28, 29, or 30 nucleotides in length. Probes and primers can have complete (100%) nucleotide sequence identity with the polynucleotide sequence, or the sequence identity can be less than 100%. For example, sequence identity between a probe or primer and a sequence can be 99%, 98%, 97%, 96%, 95%, 90%, 85%, 80%, 75%, 70% or any other percentage sequence identity so long as the probe or primer can hybridize under stringent conditions to a nucleotide sequence of a polynucleotide. Exemplified probes and primers of the disclosure include those having the nucleotide sequence shown in any of SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, and SEQ ID NO: 46, or a functional fragment or variant of any of the SEQ ID NOs: 35-46.

As used herein, the terms "nucleic acid," "polynucleotide," and "oligonucleotide" refer to a deoxyribonucleotide, ribonucleotide, or a mixed deoxyribonucleotide and ribonucleotide polymer in either single- or double-stranded form, and unless otherwise limited, would encompass known analogs of natural nucleotides that can function in a similar manner as naturally-occurring nucleotides. Polynucleotide sequences include the DNA strand sequence that can be transcribed into RNA and the RNA strand that can be translated into protein. The complementary sequence of any nucleic acid, polynucleotide, or oligonucleotide of the present disclosure is also contemplated within the scope of the disclosure. Polynucleotide sequences also include both full-length sequences as well as shorter sequences derived from the full-length sequences. The subject disclosure also encompasses those polynucleotides that are complementary in sequence to the polynucleotides disclosed herein. Polynucleotides and polypeptides of the disclosure can be provided in purified or isolated form.

Because of the degeneracy of the genetic code, a variety of different polynucleotide sequences can encode a polypeptide. A table showing all possible triplet codons (and where U also stands for T) and the amino acid encoded by each codon is described in Lewin (1985). In addition, it is well within the skill of a person trained in the art to create alternative polynucleotide sequences encoding the same, or essentially the same, polypeptides. These degenerate variant and alternative polynucleotide sequences are within the scope of the subject disclosure. As used herein, references to "essentially the same" sequence refers to sequences which encode amino acid substitutions, deletions, additions, or insertions which do not materially alter the functional and/or immunogenic activity of the polypeptide encoded by the polynucleotides of the present disclosure.

The subject disclosure also concerns variants of the polynucleotides of the present disclosure that encode polypeptides of the disclosure. Variant sequences include those sequences wherein one or more nucleotides of the sequence have been substituted, deleted, and/or inserted. The nucleotides that can be substituted for natural nucleotides of DNA have a base moiety that can include, but is not limited to, inosine, 5-fluorouracil, 5-bromouracil, hypoxanthine, 1-methylguanine, 5-methylcytosine, and tritylated bases. The sugar moiety of the nucleotide in a sequence can also be modified and includes, but is not limited to, arabinose, xylulose, and hexose. In addition, the adenine, cytosine, guanine, thymine, and uracil bases of the nucleotides can be modified with acetyl, methyl, and/or thio groups. Sequences containing nucleotide substitutions, deletions, and/or insertions can be prepared and tested using standard techniques known in the art.

Substitution of amino acids other than those specifically exemplified or naturally present in a polypeptide are also contemplated within the scope of the present disclosure. For example, non-natural amino acids can be substituted for the amino acids of a polypeptide, so long as the polypeptide having the substituted amino acids retains substantially the same functional activity as the polypeptide in which amino acids have not been substituted. Examples of non-natural amino acids include, but are not limited to, ornithine, citrulline, hydroxyproline, homoserine, phenylglycine, taurine, iodotyrosine, 2,4-diaminobutyric acid, α-amino isobutyric acid, 4-aminobutyric acid, 2-amino butyric acid, γ-amino butyric acid, ε-amino hexanoic acid, 6-amino hexanoic acid, 2-amino isobutyric acid, 3-amino propionic acid, norleucine, norvaline, sarcosine, homocitrulline, cysteic acid, τ-butylglycine, τ-butylalanine, phenylglycine, cyclohexylalanine, β-alanine, fluoro-amino acids, designer amino acids such as β-methyl amino acids, C-methyl amino acids, N-methyl amino acids, and amino acid analogues in general. Non-natural amino acids also include amino acids having derivatized side groups. Furthermore, any of the amino acids in the protein can be of the D (dextrorotary) form or L (levorotary) form. Allelic variants of a protein sequence of a polypeptide of the present disclosure are also encompassed within the scope of the disclosure.

Amino acids can be generally categorized in the following classes: non-polar, uncharged polar, basic, and acidic. Conservative substitutions whereby a polypeptide of the present disclosure having an amino acid of one class is replaced with another amino acid of the same class fall within the scope of the subject disclosure so long as the polypeptide having the substitution still retains substantially the same functional activity as the polypeptide that does not have the substitution. Polynucleotides encoding a polypeptide having one or more amino acid substitutions in the sequence are contemplated within the scope of the present disclosure. Table 11 below provides a listing of examples of amino acids belonging to each class. Single letter amino acid abbreviations are defined in Table 12.

Fragments and variants of polypeptides of influenza virus of the present disclosure can be generated using standard methods known in the art and tested for the presence of function or immunogenecity using standard techniques known in the art. For example, for testing fragments and/or variants of a neuraminidase polypeptide of the disclosure, enzymatic activity can be assayed. Thus, an ordinarily skilled artisan can readily prepare and test fragments and variants of a polypeptide and determine whether the fragment or variant retains activity relative to full-length or a non-variant polypeptide.

Polynucleotides and polypeptides contemplated within the scope of the subject disclosure can also be defined in terms of more particular identity and/or similarity ranges with those sequences of the disclosure specifically exemplified herein. The sequence identity will typically be greater than 60%, preferably greater than 75%, more preferably greater than 80%, even more preferably greater than 90%, and can be greater than 95%. The identity and/or similarity of a sequence can be 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, or 99% as compared to a sequence exemplified herein. Unless otherwise specified, as used herein percent sequence identity and/or similarity of two sequences can be determined using the algorithm of Karlin and Altschul (1990), modified as in Karlin and Altschul (1993). Such an algorithm is incorporated into the NBLAST and XBLAST programs of Altschul *et al.* (1990). BLAST searches can be performed with the NBLAST program, score = 100, wordlength = 12, to obtain sequences with the desired percent sequence identity. To obtain gapped alignments for comparison purposes, Gapped BLAST can be used as described in Altschul *et al.* (1997). When utilizing BLAST and Gapped BLAST programs, the default parameters of the respective programs (NBLAST and XBLAST) can be used. *See* NCBI/NIH website.

The subject disclosure also contemplates those polynucleotide molecules having sequences which are sufficiently homologous with the polynucleotide sequences exemplified herein so as to permit hybridization with that sequence under standard stringent conditions and standard methods (Maniatis *et al.,* 1982). As used herein, "stringent" conditions for hybridization refers to conditions wherein hybridization is typically carried out overnight at 20-25 C below the melting temperature (Tm) of the DNA hybrid in 6x SSPE, 5x Denhardt's solution, 0.1% SDS, 0.1 mg/ml denatured DNA. The melting temperature, Tm, is described by the following formula (Beltz *et al.,* 1983):
Tm=81.5 C+16.6 Log[Na+]+0.41(%G+C)-0.61(% formamide)-600/length of duplex in base pairs.

Washes are typically carried out as follows:
(1) Twice at room temperature for 15 minutes in 1x SSPE, 0.1% SDS (low stringency wash).
(2) Once at Tm-20 C for 15 minutes in 0.2x SSPE, 0.1% SDS (moderate stringency wash).

The subject disclosure also concerns viral proteins and peptides encoded by the genes of an influenza virus of the present disclosure. TThe viral protein may be a mature HA protein. The mature HA protein may comprise one or more of the following: a serine at position 82; a leucine at position 221; a threonine at position 327; and/or a threonine at position 482. The mature HA protein may have an amino acid sequence shown in SEQ ID NO: 33 or SEQ ID NO: 34, or a functional and/or immunogenic fragment or variant of SEQ ID NO: 33 or SEQ ID NO: 34. The viral protein may be an NA protein, NS protein, PB protein, PA protein, or MA protein. Viral proteins and peptides of the disclosure can be used to generate antibodies that bind specifically to the protein or peptide. Viral proteins and peptides of the present disclosure can also be used as immunogens and in vaccine compositions.

The subject disclosure also concerns compositions and methods for inducing an immune response against an influenza virus that is capable of infecting a susceptible host animal and causing respiratory disease. The disclosure can be used to induce an immune response against an influenza virus of any subtype in a susceptible host animal. For example, the influenza virus can be an HA subtype of H1, H2, H3, H4, H5, H6, H7, H8, H9, H10, H11, H12, H13, H14, H15, or H16, and an NA subtype of N1, N2, N3, N4, N5, N6, N7, N8, or N9. The HA subtype may be H3 or H5. The NA subtype may be N7 or N8. An immune response may be induced against an influenza virus of subtype H3N8. The host animal may be a canid. Canines include wild, zoo, and domestic canines, such as wolves, coyotes, and foxes. Canines also include dogs, particularly domestic dogs, such as, for example, pure-bred and/or mongrel companion dogs, show dogs, working dogs, herding dogs, hunting dogs, guard dogs, police dogs, racing dogs, and/or laboratory dogs. The host animal may be a domesticated dog, such as a greyhound. An animal may be administered an effective amount of an immunogenic composition of the present disclosure sufficient to induce an immune response against an influenza virus of the disclosure. The immune response can be a humoral and/or cellular immune response. The immune response may be a protective immune response that is capable of preventing or minimizing viral infection in the immunized host animal for some period of time subsequent to the immunization. Thus, the subject disclosure also concerns vaccine compositions and methods that can provide a vaccinated animal with a protective immune response to a virus of the present disclosure.

As described herein, the vaccine or immunogenic compositions of the subject disclosure may comprise cell-free whole virus, including attenuated or inactivated virus, or portions of the virus, including subvirion particles (including "split vaccine" wherein a virion is treated to remove some or all viral lipids), viral proteins (including individual proteins and macromolecular complexes of multiple proteins), polypeptides, and peptides, as well as virus-infected cell lines, or a combination of any of these. Vaccine or immunogenic compositions comprising virus-infected cell lines may comprise multiple cell lines, each infected with a different viral strain.

The vaccine or immunogenic compositions of the subject disclosure also encompass recombinant viral vector-based constructs that may comprise, for example, genes encoding HA protein, NA protein, nucleoprotein, polymerase basic protein, polymerase acidic protein, and/or matrix protein of an influenza virus of the present disclosure. Any suitable viral vector that can be used to prepare a recombinant vector/virus construct is contemplated for use with the subject disclosure. For example, viral vectors derived from adenovirus, avipox, herpesvirus, vaccinia, canarypox, entomopox, swinepox, West Nile virus and others known in the art can be used with the compositions and methods of the present disclosure. Recombinant polynucleotide vectors that encode and express components can be constructed using standard genetic engineering techniques known in the art. In addition, the various vaccine compositions described herein can be used separately and in combination with each other. For example, primary immunizations of an animal may use recombinant vector-based constructs, having single or multiple strain components, followed by secondary boosts with vaccine compositions comprising inactivated virus or inactivated virus-infected cell lines. Other immunization protocols with the vaccine compositions of the disclosure are apparent to persons skilled in the art and are contemplated within the scope of the present disclosure.

The subject disclosure also concerns reassortant virus comprising at least one gene or genomic segment of an influenza virus of the present disclosure and the remainder of viral genes or genomic segments from a different influenza virus of the disclosure or from an influenza virus other than a virus of the present disclosure. Reassortant virus can be produced by genetic reassortant of nucleic acid of a donor influenza virus of the present disclosure with nucleic acid of a recipient influenza virus and then selecting for reassortant virus that comprises the nucleic acid of the donor virus. Methods to produce and isolate reassortant virus are well known in the art (Fields *et al.,* 1996). A reassortant virus of the disclosure may comprise genes or genomic segments of a human, avian, swine, or equine influenza virus. A reassortant virus of the present disclosure can include any combination of nucleic acid from donor and recipient influenza virus so long as the reassortant virus comprises at least one gene or genomic segment from a donor influenza virus of the present disclosure. A recipient influenza virus can be an equine influenza virus.

Natural, recombinant or synthetic polypeptides of viral proteins, and peptide fragments thereof, can also be used as vaccine compositions according to the subject methods. Viral polypeptides derived from multiple strains can be combined in a vaccine composition and are used to vaccinate a host animal. For example, polypeptides based on the viral HA protein from at least two different strains of influenza virus of the disclosure can be combined in the vaccine. The polypeptides may be homologous to one strain or may comprise hybrid or chimeric polypeptides whose amino acid sequence is derived from joining or linking polypeptides from at least two distinct strains. Procedures for preparing viral polypeptides are well known in the art. For example, viral polypeptides and peptides can be synthesized using solid-phase synthesis methods (Merrifield, 1963). Viral polypeptides and peptides can also be produced using recombinant DNA techniques wherein a polynucleotide molecule encoding an viral protein or peptide is expressed in a host cell, such as bacteria, yeast, or mammalian cell lines, and the expressed protein purified using standard techniques of the art.

Vaccine compositions of the present disclosure also include naked nucleic acid compositions. A nucleic acid may comprise a nucleotide sequence encoding an HA and/or an NA protein of an influenza virus of the present disclosure. Methods for nucleic acid vaccination are known in the art and are described, for example, in U.S. Patent Nos. 6,063,385 and 6,472,375. The nucleic acid can be in the form of a plasmid or a gene expression cassette. The nucleic acid may be provided encapsulated in a liposome which is administered to an animal.

Vaccine compositions and immunogens, such as polypeptides and nucleic acids, that can be used in accordance with the present disclosure can be provided with a pharmaceutically-acceptable carrier or diluent. Compounds and compositions useful in the subject disclosure can be formulated according to known methods for preparing pharmaceutically useful compositions. Formulations are described in detail in a number of sources which are well known and readily available to those skilled in the art. For example, Remington's Pharmaceutical Science by E.W. Martin, Easton Pennsylvania, Mack Publishing Company, 19th ed., 1995, describes formulations which can be used in connection with the subject disclosure. In general, the compositions of the subject disclosure will be formulated such that an effective amount of an immunogen is combined with a suitable carrier in order to facilitate effective administration of the composition. The compositions used in the present methods can also be in a variety of forms. These include, for example, solid, semi-solid, and liquid dosage forms, such as tablets, pills, powders, liquid solutions or suspension, suppositories, injectable and infusible solutions, and sprays. The preferred form depends on the intended mode of administration and therapeutic application. The compositions also preferably include conventional pharmaceutically acceptable carriers and diluents which are known to those skilled in the art. Examples of carriers or diluents for use with the subject peptidomimetics include, but are not limited to, water, saline, oils including mineral oil, ethanol, dimethyl sulfoxide, gelatin, cyclodextrans, magnesium stearate, dextrose, cellulose, sugars, calcium carbonate, glycerol, alumina, starch, and equivalent carriers and diluents, or mixtures of any of these. Formulations of an immunogen of the disclosure can also comprise suspension agents, protectants, lubricants, buffers, preservatives, and stabilizers. To provide for the administration of such dosages for the desired therapeutic treatment, pharmaceutical compositions of the disclosure will advantageously comprise between about 0.1% and 45%, and especially, 1 and 15% by weight of the immunogen or immunogens based on the weight of the total composition including carrier or diluent.

The vaccine and immunogenic compositions can be prepared by procedures well known in the art. For example, the vaccine or immunogens are typically prepared as injectables, e.g., liquid solutions or suspensions. The vaccine or immunogens are administered in a manner that is compatible with dosage formulation, and in such amount as will be therapeutically effective and immunogenic in the recipient. The optimal dosages and administration patterns for a particular vaccine or immunogens formulation can be readily determined by a person skilled in the art.

Peptides and/or polypeptides can also be provided in the form of a multiple antigenic peptide (MAP) construct. The preparation of MAP constructs has been described in Tam (1988). MAP constructs utilize a core matrix of lysine residues onto which multiple copies of an immunogen are synthesized (Posnett *et al.,* 1988). Multiple MAP constructs, each containing the same or different immunogens, can be prepared and administered in a vaccine composition. A MAP construct may be provided with and/or administered with one or more adjuvants. Influenza polypeptides can also be produced and administered as macromolecular protein structures comprising one or more polypeptides. Published U.S. Patent Application US2005/0009008 describes methods for producing virus-like particles as a vaccine for influenza virus.

According to the methods of the subject disclosure, the vaccine and immunogenic compositions described herein are administered to susceptible hosts, typically canids, and more typically domesticated dogs, in an effective amount and manner to induce protective immunity against subsequent challenge or infection of the host by virus. The host animal may be a canid. Canines include wild, zoo, and domestic canines, such as wolves, coyotes, and foxes. Canines also include dogs, particularly domestic dogs, such as, for example, pure-bred and/or mongrel companion dogs, show dogs, working dogs, herding dogs, hunting dogs, guard dogs, police dogs, racing dogs, and/or laboratory dogs. The host animal may be a domesticated dog, such as a greyhound. The vaccines or immunogens are typically administered parenterally, by injection, for example, either subcutaneously, intraperitoneally, or intramuscularly. Other suitable modes of administration include oral or nasal administration. Usually, the vaccines or immunogens are administered to an animal at least two times, with an interval of one or more weeks between each administration. However, other regimens for the initial and booster administrations of the vaccine or immunogens are contemplated, and may depend on the judgment of the practitioner and the particular host animal being treated.

Virus and virus-infected cells in a vaccine formulation may be inactivated or attenuated using methods known in the art. For example, whole virus and infected cells can be inactivated or attenuated by exposure to paraformaldehyde, formalin, phenol, UV light, elevated temperature and the like. The amount of cell-free whole virus in a vaccine dose will usually be in the range from about 0.1 mg to about 5 mg, and more usually being from about 0.2 mg to about 2 mg. The dosage for vaccine formulations comprising virus-infected cell lines will usually contain from about 10⁶ to about 10⁸ cells per dose, and more usually from about 5 x 10⁶ to about 7.5 x 10⁷ cells per dose. The amount of protein or peptide immunogen in a dose for an animal can vary from about 0.1 µg to 10000 µg, or about 1 µg to 5000 µg, or about 10 µg to 1000 µg, or about 25 µg to 750 µg, or about 50 µg to 500 µg, or 100 µg to 250 µg, depending upon the size, age, *etc.,* of the animal receiving the dose.

An immunogenic or vaccine composition, such as virus or virus-infected cells or viral proteins or peptides, can be combined with an adjuvant, typically just prior to administration. Adjuvants contemplated for use in the vaccine formulations include threonyl muramyl dipeptide (MDP) (Byars *et al.,* 1987), saponin, *Cornebacterium parvum,* Freund's complete and Fruend's incomplete adjuvants, aluminum, or a mixture of any of these. A variety of other adjuvants suitable for use with the methods and vaccines, such as alum, are well known in the art and are contemplated for use with the subject disclosure.

The subject disclosure also concerns antibodies that bind specifically to a protein or a peptide of the present disclosure. Antibodies may include monoclonal and polyclonal antibody compositions. The antibodies may be monoclonal antibodies. Whole antibodies and antigen binding fragments thereof are contemplated in the present disclosure. Thus, for example, suitable antigen binding fragments include Fab₂, Fab and Fv antibody fragments. Antibodies can be labeled with a detectable moiety, such as a fluorescent molecule (*e.g.,* fluorescein or an enzyme).

The subject disclosure also concerns methods and compositions for detection and identification of an influenza virus of the disclosure and for diagnosis of infection of an animal with an influenza virus of the present disclosure. The methods of the disclosure include detection of the presence of canine influenza, in a biological sample from an animal. The detection of canine influenza in a sample, is useful to diagnose canine influenza in an animal. In turn, this information can provide the ability to determine the prognosis of an animal based on distinguishing levels of canine influenza present over time, and can assist in selection of therapeutic agents and treatments for the animal, and assist in monitoring therapy. The method also provides the ability to establish the absence of canine influenza in an animal tested.

The ability to detect canine influenza in an animal permits assessment of outbreaks of canine influenza in different geographical locations. This information also permits early detection so that infected animals can be isolated, to limit the spread of disease, and allows early intervention for treatment options. In addition, having this information available can provide direction to medical personnel for preparing to treat large numbers of ill animals, including assembling medical supplies, and, if available, vaccines.

A method of the present disclosure involves the collection of a biological sample from a test animal, such as a canine. The biological sample may be any biological material, including, cells, tissue, hair, whole blood, serum, plasma, nipple aspirate, lung lavage, cerebrospinal fluid, saliva, sweat and tears.

The animal test sample may come from an animal suspected of having canine influenza virus, whether or not the animal exhibits symptoms of the disease. Control samples can also be provided or collected from animals known to be free of canine influenza. Additional controls may be provided, *e.g.,* to reduce false positive and false negative results, and verify that the reagents in the assay are actively detecting canine influenza A virus.

In addition to detecting the presence or absence of canine influenza in a biological sample, the methods of detection used in the disclosure can detect mutations in canine influenza virus, such as changes in nucleic acid sequence, that may result from the environment, drug treatment, genetic manipulations or mutations, injury, change in diet, aging, or any other characteristic(s) of an animal. Mutations may also cause canine influenza A to become resistant to a drug that was formerly effective, or to enable the virus to infect and propagate in a different species of animal, or human. For example, avian influenza A virus has been shown to infect other animals and humans.

Detecting an influenza virus in an animal, diagnosis may be facilitated by the collection of high-quality specimens, their rapid transport to a testing facility, and appropriate storage, before laboratory testing. Virus is best detected in specimens containing infected cells and secretions. Specimens for the direct detection of viral antigens and/or for nucleic acids and/or virus isolation in cell cultures may be taken during the first 3 days after onset of clinical symptoms. A number of types of specimens are suitable to diagnose virus infections of the upper respiratory tract, including, but not limited to, nasal swab, nasopharyngeal swab, nasopharyngeal aspirate, nasal wash and throat swabs. In addition to swabs, samples of tissue or serum may be taken, and invasive procedures can also be performed.

Respiratory specimens may be collected and transported in 1-5 ml of virus transport media. A number of media that are satisfactory for the recovery of a wide variety of viruses are commercially available. Clinical specimens are added to transport medium. Nasal or nasopharyngeal swabs can also be transported in the virus transport medium. One example of a transport medium is 10 gm of veal infusion broth and 2 gm of bovine albumin fraction V, added to sterile distilled water to 400 m. Antibiotics such as 0.8 ml gentamicin sulfate solution (50 mg/ml) and 3.2 ml amphotericin B (250 µg/ml) can also be added. The medium is preferably sterilized by filtration. Nasal washes, such as sterile saline (0.85% NaCl), can also be used to collect specimens of respiratory viruses.

Sera may be collected in an amount of from 1-5 ml of whole blood from an acute-phase animal, soon after the onset of clinical symptoms, and preferably not later than 7 days. A convalescent-phase serum specimen can also be collected, for example at about 14 days after onset of symptoms. Serum specimens can be useful for detecting antibodies against respiratory viruses in a neutralization test.

In some instances, samples may be collected from individual animals over a period of time (*e.g.,* once a day, once a week, once a month, biannually or annually). Obtaining numerous samples from an individual animal, over a period of time, can be used to verify results from earlier detections, and/or to identify response or resistance to a specific treatment, e.g., a selected therapeutic drug.

The methods of the present disclosure can be used to detect the presence of one or more pathological agents in a test sample from an animal, and the level of each pathological agent. Any method for detecting the pathological agent can be used, including, but not limited to, antibody assays including enzyme-linked immunosorbent assays (ELISAs), indirect fluorescent antibody (IFA) tests, hemagglutinating, and inhibition of hemagglutination (HI) assays, and Western Blot. Known cell-culture methods can also be used. Positive cultures can be further identified using immunofluorescence of cell cultures or HI assay of the cell culture medium (supernatant).

In addition, methods for detecting nucleic acid (DNA or RNA) or protein can be used. Such methods include, but are not limited to, polymerase chain reaction (PCR), and reverse transcriptase (RT) PCR tests and real time tests, and quantitative nuclease protection assays. There are commercially available test kits available to perform these assays. For example, QIAGEN (Valencia, CA) sells a one step RT-PCR kit, and viral RNA extraction kit.

The method may utilize an antibody specific for a virus or viral protein of the disclosure. An antibody specific for an HA protein of a virus of the disclosure may be utilized. An antibody specific for an NP protein of a virus of the disclosure may be used. A suitable sample, such as from the nasal or nasopharyngeal region, is obtained from an animal and virus or viral protein is isolated therefrom. The viral components are then screened for binding of an antibody specific to a protein, such as HA or NP, of a virus of the disclosure. A serum sample (or other antibody containing sample) may be obtained from an animal and the serum screened for the presence of antibody that binds to a protein of a virus of the disclosure. For example, an ELISA assay can be performed where the plate walls have HA and/or NP protein, or a peptide fragment thereof, bound to the wall. The plate wall is then contacted with serum or antibody from a test animal. The presence of antibody in the animal that binds specifically to the HA and/or NP protein is indicative that the test animal is infected or has been infected with an influenza virus of the present disclosure.

The presence of a pathological agent may be detected by determining the presence or absence of antibodies against the agent, in a biological sample. It can take some time (e.g. months) after an animal is infected before antibodies can be detected in a blood test. Once formed, antibodies usually persist for many years, even after successful treatment of the disease. Finding antibodies to canine influenza A may not indicate whether the infection was recent, or sometime in the past.

Antibody testing can also be done on fluid(s). Antibody assays include enzyme-linked immunosorbent assays (ELISAs), indirect fluorescent antibody (IFA) assays, and Western Blot. Preferably, antibody testing is done using multiple assays, for example ELISA or IFA followed by Western blot. Antibody assays can be done in a two-step process, using either an ELISA or IFA assay, followed by a Western blot assay. ELISA is considered a more reliable and accurate assay than IFA, but IFA may be used if ELISA is not available. The Western blot test (which is a more specific test) can also be done in all animals, particularly those that have tested positive or borderline positive (equivocal) in an ELISA or IFA assay.

Other antibody-based tests that can be used for detection of influenza virus include hemagglutination inhibition assays. Hemagglutination activity can be detected in a biological sample from an animal, using chicken or turkey red blood cells as described (Burleson *et al.,* 1992) and Kendal *et al.,* 1982). An influenza or an HA protein or peptide of the disclosure may be contacted with a test sample containing serum or antibody. Red blood cells (RBC) from an animal, such as a bird, are then added. If antibody to HA is present, then the RBC will not agglutinate. If antibody to HA is not present, the RBC will agglutinate in the presence of HA. Variations and modifications to standard hemagglutination inhibition assays are known in the art and contemplated within the scope of the present disclosure.

Infection of an animal can also be determined by isolation of the virus from a sample, such as a nasal or nasopharyngeal swab. Viral isolation can be performed using standard methods, including cell culture and egg inoculation.

A nucleic acid-based assay can be used for detection of a virus of the present disclosure. A nucleic acid sample may be obtained from an animal and the nucleic acid subjected to PCR using primers that will generate an amplification product if the nucleic acid contains a sequence specific to an influenza virus of the present disclosure. RT-PCR can be used in an assay for the subject virus. Real-time RT-PCR can be used to assay for an influenza virus of the disclosure. PCR, RT-PCR and real-time PCR methods are known in the art and have been described in U.S. Patent Nos. 4,683,202; 4,683,195; 4,800,159; 4,965,188; 5,994,056; 6,814,934; and in Saiki *et al.* (1985); Sambrook *et al.* (1989); Lee *et al.* (1993); and Livak *et al.* (1995). The PCR assay may use oligonucleotides specific for an influenza matrix (MA) gene and/or HA gene. The amplification product can also be sequenced to determine if the product has a sequence of an influenza virus of the present disclosure. Other nucleic acid-based assays can be used for detection and diagnosis of viral infection by a virus of the disclosure and such assays are contemplated within the scope of the present disclosure. A sample containing a nucleic acid can be subjected to a PCR-based amplification using forward and reverse primers where the primers are specific for a viral polynucleotide or gene sequence. If the nucleic acid in the sample is RNA, then RT-PCR can be performed. For real-time PCR, a detectable probe is utilized with the primers.

Primer sets specific for the hemagglutinin (HA) gene of many of the circulating influenza viruses are known, and are continually being developed. The influenza virus genome is single-stranded RNA, and a DNA copy (cDNA) must be made using a reverse transcriptase (RT) polymerase. The amplification of the RNA genome, for example using RT-PCR, requires a pair of oligonucleotide primers, typically designed on the basis of the known HA sequence of influenza A subtypes and of neuraminadase (NM)-1. The primers can be selected such that they will specifically amplify RNA of only one virus subtype. DNAs generated by using subtype-specific primers can be further analyzed by molecular genetic techniques such as sequencing. The test is preferably run with a positive control, or products are confirmed by sequencing and comparison with known sequences. The absence of the target PCR products (*i.e,* a "negative" result) may not rule out the presence of the virus. Results can then be made available within a few hours from either clinical swabs or infected cell cultures. PCR and RT-PCR tests for influenza A virus are described by Fouchier *et al.,* 2000 and Maertzdorf *et al.,* 2004.

The subject disclosure also concerns methods for screening for compounds or drugs that have antiviral activity against a virus of the present disclosure. Cells infected with a virus of the disclosure are contacted with a test compound or drug. The amount of virus or viral activity following contact can then be determined. Those compounds or drugs that exhibit antiviral activity can be selected for further evaluation.

The subject disclosure also concerns isolated cells infected with an influenza virus of the present disclosure. The cell may be a canine cell, such as canine kidney epithelial cells.

The subject disclosure also concerns cells transformed with a polynucleotide of the present disclosure encoding a polypeptide of the disclosure. Preferably, the polynucleotide sequence is provided in an expression construct of the disclosure. More preferably, the expression construct provides for overexpression in the cell of an operably linked polynucleotide of the disclosure. The cell may be transformed with a polynucleotide sequence comprising a sequence encoding the amino acid sequence shown in any of SEQ ID NOs: 2, 4, 6, 8, 10, 12, 14, 16, 18, 20, 22, 24, 26, 28, 30, 32, 33, 34, 48, 50, 52, 54, 56, 58, 60, 62, 64, 66, 68, 70, 72, 74, 76, or 78, or a functional fragment or variant thereof. The cell may be transformed with a polynucleotide encoding the amino acid sequence shown in SEQ ID NOs: 2, 4, 6, 8, 10, 12, 14, 16, 18, 20, 22, 24, 26, 28, 30, 32, 33, 34, 48, 50, 52, 54, 56, 58, 60, 62, 64, 66, 68, 70, 72, 74, 76, or 78 comprises the nucleotide sequence shown in SEQ ID NOs: 1, 3, 5, 7, 9, 11, 13, 15, 17, 19, 21, 23, 25, 27, 29, 31, 47, 49, 51, 53, 55, 57, 59, 61, 63, 65, 67, 69, 71, 73, 75, or 77, respectively, or a sequence encoding a functional fragment or variant of any of SEQ ID NOs: 2, 4, 6, 8, 10, 12, 14, 16, 18, 20, 22, 24, 26, 28, 30, 32, 33, 34, 48, 50, 52, 54, 56, 58, 60, 62, 64, 66, 68, 70, 72, 74, 76, or 78. Thus, the subject disclosure concerns cells transformed with a polynucleotide sequence comprising the nucleotide sequence shown in any of SEQ ID NOs: 1, 3, 5, 7, 9, 11, 13, 15, 17, 19, 21, 23, 25, 27, 29, 31, 47, 49, 51, 53, 55, 57, 59, 61, 63, 65, 67, 69, 71, 73, 75, or 77, or a fragment or variant, including a degenerate variant, of any of SEQ ID NOs: 1, 3, 5, 7, 9, 11, 13, 15, 17, 19, 21, 23, 25, 27, 29, 31, 47, 49, 51, 53, 55, 57, 59, 61, 63, 65, 67, 69, 71, 73, 75, or 77.

The transformed cell can be a eukaryotic cell, for example, a plant cell, including protoplasts, or the transformed cell can be a prokaryotic cell, for example, a bacterial cell such as *E. coli* or *B. subtilis.* Animal cells include human cells, mammalian cells, partially canine cells, avian cells, and insect cells. Plant cells include, but are not limited to, dicotyledonous, monocotyledonous, and conifer cells.

The subject disclosure also concerns plants, including transgenic plants that express and produce a viral protein or polypeptide of the present disclosure. Plants, plant tissues, and plant cells transformed with or bred to contain a polynucleotide of the disclosure are contemplated by the present disclosure. Preferably, the polynucleotide of the disclosure is overexpressed in the plant, plant tissue, or plant cell. Plants can be used to produce influenza vaccine compositions of the present disclosure and the vaccines can be administered through consumption of the plant (see, for example, U.S. Patent Nos. 5,484,719 and 6,136,320).

The subject disclosure also concerns kits for detecting a virus or diagnosing an infection by a virus of the present disclosure, a kit comprises an antibody of the disclosure that specifically binds to an influenza virus of the present disclosure, or an antigenic portion thereof. A kit may comprise one or more polypeptides or peptides of the present disclosure. The polypeptides may have an amino acid sequence shown in any of SEQ ID NOs. 2, 4, 6, 8, 10, 12, 14, 16, 18, 20, 22, 24, 26, 28, 30, 32, 33, 34, 48, 50, 52, 54, 56, 58, 60, 62, 64, 66, 68, 70, 72, 74, 76, or 78, or a functional and/or immunogenic fragment or variant thereof. A kit may comprise one or more polynucleotides or oligonucleotides of the present disclosure. tTe polynucleotides may have a nucleotide sequence shown in any of SEQ ID NOs. 1, 3, 5, 7, 9, 11, 13, 15, 17, 19, 21, 23, 25, 27, 29, 31, 47, 49, 51, 53, 55, 57, 59, 61, 63, 65, 67, 69, 71, 73, 75, or 77, or a fragment or variant thereof. A kit may optionally comprise one or more control antibody, control polypeptide or peptide, and/or control polynucleotide or oligonucleotide. The antibody, polypeptides, peptides, polynucleotides, and/or oligonucleotides of the kit can be provided in a suitable container or package.

The subject application also concerns the use of mongrel dogs as a model for infection and pathogenesis of influenza virus. A mongrel dog may be inoculated with an influenza virus, such as a canine influenza virus of the present disclosure. Optionally, the dog can be administered therapeutic agents subsequent to inoculation. The dog can also have been administered a composition for generating an immune response against an influenza virus prior to inoculation with virus. Tissue, blood, serum, and other biological samples can be obtained before and/or after inoculation and examined for the presence of virus and pathogenesis of tissue using methods known in the art including, but not limited to, PCR, RT-PCR, nucleic acid sequencing, and immunohistochemistry.

### MATERIALS AND METHODS FOR EXAMPLES 1-6 (REFERENCE)

### Blood and Nasal Swab Collection from Greyhounds.

Acute and convalescent blood samples were collected by jugular venipuncture from clinically diseased or normal greyhounds in racing kennels experiencing outbreaks of respiratory disease. Convalescent samples were collected 4 to 12 weeks after the acute sample. Serum was harvested and stored at -80°C. Nasal swabs were collected and placed in Amies transport medium with charcoal (Becton Dickinson Biosciences) pending submission for bacterial isolation.

### Postmortem examination of greyhounds.

Complete postmortem examinations were performed by the Anatomic Pathology Service at the University of Florida College of Veterinary Medicine (UF CVM) on 5 of the 8 greyhounds that died in the January 2004 outbreak at a Florida track. Postmortem examination of another dog was performed at a private veterinary clinic with submission of tissues to the UF CVM for histopathologic diagnosis. Tissues were fixed in 10% neutral buffered formalin, embedded in paraffin, and 5-µm sections were either stained with hematoxylin and eosin for histopathologic diagnosis or processed for immunohistochemistry as described below. Unfixed tissues were submitted for bacterial culture and also stored at -80°C.

### Serological tests for canine viral respiratory pathogens.

Paired acute and convalescent serum samples were submitted to the Animal Health Diagnostic Laboratory (AHDL) at the Cornell University College of Veterinary Medicine for serum neutralization assays against canine distemper virus, adenovirus type 2, and parainfluenza virus. Antibody titers were expressed as the last dilution of serum that inhibited viral infection of cell cultures. Seroconversion, defined as a ≥ 4-fold increase in antibody titer between the acute and convalescent sample, indicated viral infection. No seroconversions to these viral pathogens were detected.

### Microbial tests for canine bacterial respiratory pathogens.

Paired nasal swabs and postmortem tissues were submitted to the Diagnostic Clinical Microbiology/Parasitology/Serology Service at the UF CVM for bacterial isolation and identification. The samples were cultured on nonselective media as well as media selective for *Bordetella* species (Regan-Lowe; Remel) and *Mycoplasma* species (Remel). All cultures were held for 21 days before reporting no growth. Nasal swabs from some of the greyhounds were also submitted to the Department of Diagnostic Medicine/Pathobiology at the Kansas State University College of Veterinary Medicine for bacterial culture. Of 70 clinically diseased dogs tested, *Bordetella bronchiseptica* was isolated from the nasal cavity of 1 dog, while *Mycoplasma spp.* were recovered from the nasal cavity of 33 dogs. *Pasteurella multocida* was commonly recovered from the nasal cavity of dogs with purulent nasal discharges. Two of the dogs that died in the January 2004 outbreak had scant growth of *Escherichia coli* in the lungs postmortem, one dog had scant growth of *E. coli* and *Streptococcus canis,* and another had scant growth of *Pseudomonas aeruginosa* and a yeast. Neither *Bordetella bronchiseptica* nor *Mycoplasma* was isolated from the trachea or lungs of dogs that died.

### Virus isolation from postmortem tissues.

Frozen tissues were thawed and homogenized in 10 volumes of minimum essential medium (MEM) supplemented with 0.5% bovine serum albumin (BSA) and antibiotics. Solid debris was removed by centrifugation and supernatants were inoculated onto cultured cells or into 10-day old embryonated chicken eggs. Tissue homogenates from greyhounds that died were inoculated into diverse cell cultures that supported the replication of a broad range of viral pathogens. The cell cultures included Vero (African green monkey kidney epithelial cells, ATCC No. CCL-81), A-72 (canine tumor fibroblasts, CRL-1542), HRT-18 (human rectal epithelial cells, CRL-11663), MDCK (canine kidney epithelial cells, CCL-34), primary canine kidney epithelial cells (AHDL, Cornell University), primary canine lung epithelial cells (AHDL), and primary bovine testicular cells (AHDL). MDCK and HRT cells were cultured in MEM supplemented with 2.5 ug/mL TPCK-treated trypsin (Sigma); the remaining cell lines were cultured in MEM supplemented with 10% fetal calf serum and antibiotics. Cells were grown in 25 cm² flasks at 37°C in a humidified atmosphere containing 5% CO₂. A control culture was inoculated with the supplemented MEM. The cultures were observed daily for morphologic changes and harvested at 5 days post inoculation. The harvested fluids and cells were clarified by centrifugation and inoculated onto fresh cells as described for the initial inoculation; two blind passages were performed. Hemagglutination activity in the clarified supernatants was determined using chicken or turkey red blood cells as described (Burleson *et al.,* 1992; Kendal *et al.,* 1982). For virus isolation in chicken embryos, 0.1 mL of tissue homogenate was inoculated into the allantoic sac and incubated for 48 hours at 35°C. After two blind passages, the hemagglutination activity in the allantoic fluids was determined as described (Burleson *et al.,* 1992; Kendal *et al.,* 1982).

### RT-PCR, nucleotide sequencing, and phylogenetic analyses.

Total RNA was extracted from tissue culture supernatant or allantoic fluid using the RNeasy kit (Qiagen, Valencia, CA) according to manufacturer's instructions. The total RNA (10 ng) was reverse transcribed to cDNA using a one-step RT-PCR Kit (Qiagen, Valencia, CA) according to manufacturer's instructions. PCR amplification of the coding region of the 8 influenza viral genes in the cDNA was performed as previously described (Klimov *et al.,* 1992a), using universal gene-specific primer sets. The resulting DNA amplicons were used as templates for automated sequencing on an Applied Biosystems 3100 automated DNA sequencer using cycle sequencing dye terminator chemistry (ABI). Nucleotide sequences were analyzed using the GCG Package©, Version 10.0 (Accelyrs) (Womble, 2000). The Phylogeny Inference Package© Version 3.5 was used to estimate phylogenies and calculate bootstrap values from the nucleotide sequences (Felsenstein, 1989). Phylogenetic trees were compared to those generated by neighbor-joining analysis with the Tamura-Nei gamma model implemented in the MEGA© program (Kumar *et al.,* 2004) and confirmed by the PAUP© 4.0 Beta program (Sinauer Associates).

### Experimental inoculation of dogs.

Four 6-month old specific pathogen-free beagles [(2 males and 2 females (Liberty Research)] were used. Physical examination and baseline blood tests including complete blood cell count/differential, serum chemistry panel, and urinalysis determined that the animals were healthy. They were housed together in a BSL 2-enhanced facility accredited by the Association for Assessment and Accreditation of Laboratory Animal Care. Baseline rectal temperatures were recorded twice daily for 7 days. The dogs were anesthetized by intravenous injection of propofol (Diprivan®, Zeneca Pharmaceuticals, 0.4 mg/kg body weight to effect) for intubation with endotracheal tubes. Each dog was inoculated with a total dose of 10^{6.6} median tissue culture infectious doses (TCID₅₀) of A/Canine/Florida/43/2004 (Canine/FL/04) (H3N8) virus with half the dose administered into the distal trachea through the endotracheal tube and the other half administered into the deep nasal passage through a catheter. Physical examinations and rectal temperature recordings were performed twice daily for 14 days post inoculation (p.i.). Blood samples (4 mL) were collected by jugular venipuncture on days 0, 3, 5, 7, 10, and 14 p.i. Nasal and oropharyngeal specimens were collected with polyester swabs (Fisher Scientific) from each dog on days 0 to 5, 7, 10, and 14 p.i. The swabs were placed in viral transport medium (Remel) and stored at -80°C. Two dogs (1 male and 1 female) were euthanatized by intravenous inoculation of Beuthanasia-D® solution (1 mL/5 kg body weight; Schering-Plough Animal Health Corp) on day 5 p.i. and the remaining 2 dogs on day 14 for postmortem examination. Tissues for histological analysis were processed as described. Tissues for virus culture were stored at -80°C. This study was approved by the University of Florida Institutional Animal Care and Use Committee.

### Virus shedding from experimentally inoculated dogs.

Serial dilutions of lung homogenates and swab extracts, prepared by clarification of the swab transport media by centrifugation, were set up in MEM supplemented with 0.5% BSA and antibiotics. Plaque assays were performed as described (Burleson *et al.,* 1992) using monolayers of MDCK cells in 6-well tissue culture plates. Inoculated cell monolayers were overlaid with supplemented MEM containing 0.8% agarose and 1.5 ug/mL of TPCK-trypsin. Cells were cultured for 72 hours at 37°C in a humidified atmosphere containing 5% CO₂ prior to fixation and staining with crystal violet. Virus concentration was expressed as plaque forming units (PFU) per gram of tissue or per swab.

### Immunohistochemistry.

Deparaffinized and rehydrated 5-µm lung tissue sections from the greyhounds and beagles were mounted on Bond-Rite™ slides (Richard-Allan Scientific, Kalamazoo, MI) and subsequently treated with proteinase K (DakoCytomation, Carpenteria, CA) followed by peroxidase blocking reagent (Dako® EnVision™ Peroxidase Kit, Dako Corp.). The sections were incubated with 1:500 dilutions of monoclonal antibodies to canine distemper virus (VMRD, Inc.), canine adenovirus type 2 (VMRD, Inc.), canine parainfluenza virus (VMRD, Inc.), or influenza A H3 (Chemicon International, Inc.) for 2 hours at room temperature. Controls included incubation of the same sections with mouse IgG (1 mg/mL, Serotec, Inc.), and incubation of the monoclonal antibodies with normal canine lung sections. Following treatment with the primary antibodies, the sections were incubated with secondary immunoperoxidase and peroxidase substrate reagents (Dako® EnVision™ Peroxidase Kit, Dako Corp.) according to the manufacturer's instructions. The sections were counterstained with hematoxylin, treated with Clarifier #2 and Bluing Reagent (Richard-Allan Scientific, Kalamazoo, MI), dehydrated, and coverslips applied with Permount (ProSciTech).

### Hemagglutination inhibition (HI) assay.

Serum samples were incubated with receptor destroying enzyme (RDE, Denka) (1 part serum: 3 parts RDE) for 16 hours at 37°C prior to heat inactivation for 60 minutes at 56°C. Influenza A/Canine/FL/04 (H3N8) virus was grown in MDCK cells for 36-48 hr at 37°C. Virus culture supernatants were harvested, clarified by centrifugation, and stored at -80°C. The HI assay was performed as described previously (Kendal *et al.,* 1982). Briefly, 4 hemagglutinating units of virus in 25µl were added to an equal volume of serially diluted serum in microtiter wells and incubated at room temperature for 30 minutes. An equal volume of 0.5% v/v turkey erythrocytes was added and the hemagglutination titers were estimated visually after 30 minutes. The endpoint HI titer was defined as the last dilution of serum that completely inhibited hemagglutination. Seroconversion was defined as ≥ 4-fold increase in HI titer between paired acute and convalescent samples. Seropositivity of a single sample was defined as a HI antibody titer ≥1:32.

### Microneutralization (MN) assay.

Neutralizing serum antibody responses to A/Canine/FL/04 (H3N8) were detected by a MN assay as described previously (Rowe *et al.,* 1999) except that canine sera were RDE-treated as described above prior to the assay. The endpoint titer was defined as the highest dilution of serum that gave 50% neutralization of 100 TCID₅₀ of virus. Seroconversion was defined as ≥ 4-fold increase in MN titer between paired acute and convalescent samples. Seropositivity of a single sample was defined as a MN titer ≥1:80.

Following are examples and reference examples which illustrate procedures of the invention and disclosure. These reference examples should not be construed as limiting. All percentages are by weight and all solvent mixture proportions are by volume unless otherwise noted.

### EXAMPLE 1 (REFERENCE)

In January 2004, an outbreak of respiratory disease occurred in 22 racing greyhounds housed in 2 kennels at a Florida track and the local farm that supplied dogs to these kennels. There were approximately 60 dogs in each kennel building and 300 dogs at the farm. The outbreak occurred over a 6-day period after which no new cases were identified. Fourteen of the 22 dogs had fevers of 39.5 to 41.5°C, a soft, gagging cough for 10 to 14 days, and eventual recovery. Of the remaining 8 dogs, 6 apparently healthy dogs died unexpectedly with hemorrhage from the mouth and nose. Two other dogs were euthanatized within 24 hours of onset of hemorrhage from the mouth and nose due to rapid deterioration. Both of these dogs had fevers of 41°C. Four of the 8 deaths occurred in the kennel buildings and 4 occurred at the farm. Fifty percent of the deaths occurred on day 3 of the outbreak. The 22 dogs ranged in age from 17 months to 4 years, but 73% were 17 to 33 months old.

Two clinical syndromes were evident: a milder illness characterized by initial fever and then cough for 10-14 days (14 dogs) with subsequent recovery, or a peracute death associated with hemorrhage in the respiratory tract (8 dogs for a mortality rate of 36%). Postmortem examinations were performed on 6 of the 8 fatal cases. All dogs had extensive hemorrhage in the lungs, mediastinum, and pleural cavity. Histological examination of the respiratory tract revealed that in addition to pulmonary hemorrhage, all dogs had tracheitis, bronchitis, bronchiolitis, and suppurative bronchopneumonia (Figure 3). The epithelial lining and airway lumens in these tissues were infiltrated by neutrophils and macrophages. Lung homogenates prepared from these dogs were inoculated into a variety of monkey, human, bovine, and canine cell lines for virus culture. The lung homogenate from one dog caused cytopathic effects in Madin-Darby canine kidney epithelial cells (MDCK) cultured in the presence of trypsin, and the cell culture supernatant agglutinated chicken red blood cells. Preliminary evidence of an influenza type A virus was provided by a commercial ELISA for detection of the nucleoprotein of influenza A and B viruses, and by PCR analysis using primers specific for the matrix gene of influenza A viruses. In addition, the hemagglutinating activity was inhibited by reference antisera to the equine influenza A H3 subtype, but not by antisera specific for human influenza A subtypes H1-H11 and H13 (Table 3). To characterize the molecular properties of the virus, we determined the nucleotide sequences of the 8 RNA segments of the viral genome. Sequence comparisons with known influenza virus genes and phylogenetic analyses indicated that the 8 genes of the canine isolate were most similar to those from contemporary equine influenza A (H3N8) viruses, with which they shared ≥96-97% sequence identity (Figure 1A, Table 4). In contrast, representative genes from avian, swine, and human influenza A isolates had ≤94% identity with the canine isolate (Table 4). These data identified the canine isolate A/Canine/Florida/43/04 (Canine/FL/04) as an influenza A H3N8 virus closely related to contemporary lineages of equine influenza viruses. Since all genes of the canine isolate were of equine influenza virus origin, we concluded that the entire genome of an equine influenza virus had been transmitted to the dog.

### EXAMPLE 2 (REFERENCE)

To investigate the role of the Canine/FL/04 virus in the clinical and pathological observations in the greyhounds, we performed immunohistochemical staining (IHC) on lung tissues using a monoclonal antibody to influenza A H3. Viral H3 antigen was consistently detected in the cytoplasm of bronchial and bronchiolar epithelial cells, bronchial gland epithelial cells, and macrophages in airway lumens and alveolar spaces (Figure 2A). These data support a diagnosis of pulmonary infection with influenza virus of the H3 subtype in multiple dogs.

### EXAMPLE 3 (REFERENCE)

To determine involvement of a Canine/FL/04-like virus in the etiology of the respiratory disease outbreak, we analyzed paired acute and convalescent sera from 11 sick dogs and 16 asymptomatic contacts by hemagglutination inhibition (HI) and microneutralization (MN). Seroconversion, defined as a ≥ 4-fold rise in antibody titer to Canine/FL/04 from the acute to convalescent phase, occurred in 8 of 11 (73%) sick dogs in both assays (Table 1). Seroconversion occurred in 6 of 16 (38%) asymptomatic contacts in the HI assay, while 8 of 16 (50%) seroconverted in the MN assay (Table 1). The seroconversion data demonstrated infection of the dogs with a Canine/FL/04-like virus which coincided temporally with the onset of respiratory disease in most animals.

Single serum samples were collected 3 months after the outbreak from an additional 46 asymptomatic dogs housed with the sick dogs. Of these, 43 (93%) were seropositive in both assays. For the total population of 73 dogs tested, 93% were seropositive in both assays, including 82% (9/11) of the sick dogs and 95% (59/62) of the healthy contacts. The high seroprevalence in dogs with no history of respiratory disease indicates that most infections with canine influenza virus are subclinical and suggest efficient spread of the virus among dogs. It is not known if subclinical infections contribute to the spread of the virus.

### EXAMPLE 4 (REFERENCE)

To better understand the capacity of the Canine/FL/04 virus to infect dogs, four 6-month old purpose-bred beagles were each inoculated with 10^{6.6} median tissue culture infectious doses (TCID₅₀) by the intratracheal and intranasal routes. All dogs developed a fever (rectal temperature ≥39°C) for the first 2 days postinoculation (p.i.), but none exhibited respiratory symptoms such as cough or nasal discharge over a 14 day observation period. Virus shedding was examined by quantification of virus in nasal and oropharyngeal swabs. Only 2 of the 4 dogs shed detectable amounts of virus. One dog shed virus on days 1 and 2 p.i. (1.0-2.5 log₁₀ PFU per swab), whereas the other dog shed virus for 4 consecutive days after inoculation (1.4-4.5 log₁₀ PFU per swab). Postmortem examination of 2 dogs on day 5 p.i. revealed necrotizing and hyperplastic tracheitis, bronchitis, and bronchiolitis similar to that found in the spontaneous disease in greyhounds, but there was no pulmonary hemorrhage or bronchopneumonia. Viral H3 antigen was detected in the cytoplasm of epithelial cells of bronchi, bronchioles, and bronchial glands by IHC (Figure 2B). Infectious virus was recovered from the lung tissue of one of the dogs. Postmortem examination of the remaining 2 dogs on day 14 p.i. showed minimal histological changes in respiratory tissues, no viral H3 antigen by IHC, and no recovery of virus from lung homogenates. Seroconversion in these latter 2 dogs was detected in MN assays by day 7 p.i., with a further 2-to 3-fold increase in antibody titers by day 14. These results established the susceptibility of dogs to infection with Canine/FL/04, as evidenced by the febrile response, presence of viral antigen and infectious virus in the lung parenchyma, histopathological findings typical for influenza, and seroconversion. The failure to reproduce severe disease and death in the experimentally inoculated beagles is not surprising since a large proportion of the naturally infected greyhounds were asymptomatic.

### EXAMPLE 5 (REFERENCE)

To investigate whether a Canine/FL/04-like influenza virus had circulated among greyhound populations in Florida prior to the January 2004 outbreak, archival sera from 65 racing greyhounds were tested for the presence of antibodies to Canine/FL/04 using the HI and MN assays. There were no detectable antibodies in 33 dogs sampled from 1996 to 1999. Of 32 dogs sampled between 2000 and 2003, 9 were seropositive in both assays - 1 in 2000, 2 in 2002, and 6 in 2003 (Table 5). The seropositive dogs were located at Florida tracks involved in outbreaks of respiratory disease of unknown etiology from 1999 to 2003, suggesting that a Canine/FL/04-like virus may have been the causative agent of those outbreaks. To investigate this possibility further, we examined archival tissues from greyhounds that died from hemorrhagic bronchopneumonia in March 2003. Lung homogenates inoculated into MDCK cells and chicken embryos from one dog yielded H3N8 influenza virus, termed A/Canine/Florida/242/2003 (Canine/FL/03). Sequence analysis of the complete genome of Canine/FL/03 revealed >99% identity to Canine/FL/04 (Table 4), indicating that Canine/FL/04-like viruses had infected greyhounds prior to 2004.

### EXAMPLE 6 (REFERENCE)

From June to August 2004, respiratory disease outbreaks occurred in thousands of racing greyhounds at 14 tracks in Florida, Texas, Alabama, Arkansas, West Virginia, and Kansas.

Officials at some of these tracks estimated that at least 80% of their dog population had clinical disease. Most of the dogs had clinical signs of fever (≥ 39°C) and cough similar to the dogs in the January 2004 outbreak, but many dogs also had a mucopurulent nasal discharge. Multiple deaths were reported but an accurate mortality rate could not be determined.

We collected paired acute and convalescent sera from 94 dogs located at 4 Florida tracks: 56% of these dogs had ≥4-fold rises in antibody titers to Canine/FL/04, and 100% were seropositive (Table 6). Convalescent sera from 29 dogs in West Virginia and Kansas also had antibodies to Canine/FL/04. We isolated influenza A (H3N8) virus from the lungs of a greyhound that died of hemorrhagic bronchopneumonia at a track in Texas. Sequence analysis of the entire genome of this isolate, named A/Canine/Texas/1/2004 (Canine/TX/04), revealed ≥99% identity to Canine/FL/04 (Table 4). The isolation of three closely related influenza viruses from fatal canine cases over a 13-month period and from different geographic locations, together with the substantial serological evidence of widespread infection among racing greyhounds, suggested sustained circulation of a Canine/FL/04-like virus in the dog population.

Phylogenetic analysis of the HA genes of Canine/FL/03, Canine/FL/04, and Canine/TX/04 showed that they constitute a monophyletic group with robust bootstrap support that was clearly distinct from contemporary H3 genes of equine viruses isolated in 2002 and 2003 (Figure 1B). Phylogentic analysis and pairwise nucleotide sequence comparisons of the other 7 genomic segments supported the segregation of the canine genes as a distinct sub-lineage most closely related to the equine virus lineage (data not shown, and Table 4). The clustering of the canine influenza genes as a monophyletic group separate from equine influenza is also supported by the presence of 4 signature amino acid changes in the HA (Table 2). Together with the serological results from 2003 and 2004, these data are consistent with a single virus transmission event from horses to dogs with subsequent horizontal spread of the virus in the greyhound population. However, repeated introductions of this unique lineage of influenza virus from an unidentified reservoir species can not be formally excluded, unlikely as it may be.

The viral HA is a critical determinant of host species specificity of influenza virus (Suzuki *et al.,* 2000). To identify residues within HA that may be associated with adaptation to the canine host, we compared the deduced amino acid sequence of canine HAs to those of contemporary equine viruses. Four amino acid changes differentiate the equine and canine mature HA consensus amino acid sequences: N83S, W222L, I328T, and N483T (see Table 2). The canine viruses have an amino acid deletion when compared to the consensus equine sequences. Therefore, amino acid position 7 in the HA equine sequence is position 6 in the HA canine sequence, amino acid position 29 in the HA equine sequence is position 28 in the HA canine sequence, amino acid position 83 in the HA equine sequence is position 82 in the HA canine sequence, *etc.* Thus, the four substituted amino acids are at position 82, 221, 327, and 482 of the amino acid sequence shown in SEQ ID NO: 33 and SEQ ID NO: 34. The substitution of serine for asparagine at consensus sequence position 83 is a change of unknown functional significance since various polar residues are found in H3 molecules from other species. The strictly conserved isoleucine at consensus sequence position 328 near the cleavage site of the H3 HA has been replaced by threonine. The pivotal role of HA cleavage by host proteases in pathogenesis suggests that this change merits further study. The substitution of leucine for tryptophan at consensus sequence position 222 is quite remarkable because it represents a non-conservative change adjacent to the sialic acid binding pocket which could modulate receptor function (Weis *et al.,* 1988). Interestingly, leucine at position 222 is not unique to canine H3 HA since it is typically found in the H4, H8, H9, and H12 HA subtypes (Nobusawa *et al.,* 1991; Kovacova *et al.,* 2002). The leucine substitution may be more compatible with virus specificity for mammalian hosts since infections of swine with subtype H4 (Karasin *et al.,* 2000) and humans and swine with subtype H9 (Peiris *et al.,* 1999) viruses have been reported. The substitution of asparagine with threonine at consensus sequence position 483 resulted in the loss of a glycosylation site in the HA2 subunit that is conserved in all HA subtypes (Wagner *et al.,* 2002). Although the importance of these amino acid changes in the HA for adaptation of an equine virus to dogs remains to be determined, similar amino acid changes have been observed previously in association with interspecies transfer (Vines *et al.,* 1998; Matrosovich *et al.,* 2000). Amino acid differences between other influenza viral proteins of the disclosure and equine consensus sequence are shown in Tables 19 to 25.

The source of the equine influenza virus that initially infected racing greyhounds remains speculative. Kennels at greyhound racetracks are not located near horses or horse racetracks, suggesting that contact between greyhounds and shedding horses is not a sufficient explanation for the multiple outbreaks in different states in 2004. A potential source of exposure to the equine virus is the feeding of horsemeat to greyhounds, whose diet is supplemented with raw meat supplied by packing houses that render carcasses, including horses which could carry influenza. Precedents for this mode of infection include reports of interspecies transmission of H5N1 avian influenza virus to pigs and zoo felids fed infected chicken carcasses (Webster, 1998; Keawcharoen *et al.,* 2004; Kuiken *et al.,* 2004). Although this is a plausible route for the initial introduction of equine influenza into dogs, it does not explain the recent multiple influenza outbreaks in thousands of dogs in different states. Our experimental inoculation study demonstrated the presence of virus in the nasal passages and oropharynx of dogs, albeit at modest titers. Nevertheless, these results indicate that shedding is possible, and that dog-to-dog transmission of virus by large droplet aerosols, fomites, or direct mucosal contact could play a role in the epizootiology of the disease.

The interspecies transfer of a whole mammalian influenza virus to an unrelated mammal species is a rare event. Previous studies have provided limited serological or virological evidence, but not both, of transient infection of dogs with human influenza A (H3N2) viruses (Nikitin *et al.,* 1972, Kilbourne, *et al.,* 1975; Chang *et al.,* 1976; Houser *et al.,* 1980). However, there was no evidence of sustained circulation in the canine host. Although direct transfer of swine influenza viruses from pigs to people is well-documented (Dacso *et al.,* 1984; Kimura *et al.,* 1998; Patriarca *et al.,* 1984; Top *et al.,* 1977), there is no evidence for adaptation of the swine viruses in human hosts. In this report, we provide virological, serological, and molecular evidence for interspecies transmission of an entire equine influenza A (H3N8) virus to another mammalian species, the dog. Unique amino acid substitutions in the canine virus HA, coupled with serological confirmation of infection of dogs in multiple states in the U.S., suggest adaptation of the virus to the canine host. Since dogs are a primary companion animal for humans, these findings have implications for public health; dogs may provide a new source for transmission of novel influenza A viruses to humans.

| **Table 1.** Antibody response to A/Canine/Florida/43/04 (H3N8). | | | | |
|---|---|---|---|---|
| | **Sick Dogs (11)^{a}** | | **Healthy Contacts (16)^{b}** | |
| Response | HI^{c} | SN^{d} | HI | SN |
| Seroconversion (%)^{e} | 73 | 73 | 38 | 50 |
| Seropositive (%)^{f} | 82 | 82 | 100 | 100 |
| Geometric mean titer^{g} | 329 | 424 | 268 | 431 |

| | | | | |
|---|---|---|---|---|
| ^{a} Number of dogs with clinical signs of disease. ^{b} Number of asymptomatic dogs housed in contact with clinically diseased dogs. ^{c} Hemagglutination-inhibition (HI) assay using A/Canine/Florida/43/04 virus. ^{d} Microneutralization (MN) assay using A/Canine/Florida/43/04 virus. ^{e} Percentage of dogs with at least a 4-fold increase in antibody titer in paired acute and convalescent sera. ^{f} Percentage of dogs with a positive antibody titer (HI titer ≥32: MN titer ≥80) in the convalescent sera. ^{g} Geometric mean antibody titer for the convalescent sera. | | | | |

| **Table 2.** Amino acid differences between the canine and equine H3 hemagglutinins. | | | | |
|---|---|---|---|---|
| **Equine H3 consensus** | **Can/FL/03** | **Can/FL/04** | **Can/TX/04** | **Potential functional significance** |
| G7* | D | - ^{†} | - | D also found in duck and human H3 HA |
| 129 | - | M | M | I is conserved in H3 HAs from all species |
| N83 | S | S | S | Various polar amino acids present at this position in H3 HAs of other species |
| S92 | - | N | - | N is present in some duck H3 HAs |
| L118 | - | - | V | L is conserved in all H3 HAs |
| W222 | L | L | L | W is conserved in most H3 HAs of all species; located near the receptor binding site |
| A272 | V | A | V | V is present in some recent equine isolates |
| I328 | T | T | T | T is strictly conserved in all avian, swine or humans H3 HAs |
| N483 | T | T | T | N occurs in all H3 and other HA subtypes. Replacement results in loss of a glycosylation site. |
| K541 | - | R | - | Basic amino acid conservative change |

| | | | | |
|---|---|---|---|---|
| * Amino acid residue (single letter code) and position in the mature H3 HA. The amino acid code is: A=alanine, D=aspartic acid, G=glycine, I=isoleucine, K=lysine, L=leucine, M=methionine, N=asparagine, R=arginine, S=serine, T=threonine, V=valine, W=tryptophan . ^{†} Denotes no change from the consensus equine H3 HAs. | | | | |

| **Table 3.** Hemagglutination inhibition of a virus isolate by reference antisera to different HA subtypes. | | |
|---|---|---|
| **Reference Antisera** | **HA Specificity** | **HI titer^{a}** |
| Puerto Rico/8/34 | H1 | 5 |
| Swine/Iowa15/30 | H1 | 5 |
| Singapore/01/57 | H2 | 5 |
| Shanghai/11/87 | H3^{b} | 5 |
| Equine/Miami/1/63 | H3 | 160 |
| Duck/Czechoslovakia/56 | H4 | 5 |
| Tern/South Africa/61 | H5 | 5 |
| Turkey/Mas sachussetts/65 | H6 | 5 |
| Fowl Plague/Dutch/27 | H7 | 5 |
| Fowl Plague/Rostock/34 | H7 | 5 |
| Equine/Prague/1/56 | H7 | 5 |
| Turkey/Ontario/6118/68 | H8 | 5 |
| Quail/Hong Kong/G1/97 | H9^{b} | 5 |
| Chicken/Hong Kong/G9/97 | H9^{b} | 5 |
| Chicken/Germany/49 | H10 | 5 |
| Duck/England/56 | H11 | 5 |
| Gull/Maryland/704/77 | H13 | 5 |
| Normal sheep serum | - | 5 |
| Normal ferret serum | - | 5 |

| | | |
|---|---|---|
| ^{a} Hemagglutination inhibition titer to virus isolate from dog # 43. ^{b} Polyclonal antisera were produced in ferrets, whereas all other antisera were produced in sheep or goats. | | |

| **Table 4.** Sequence homology of A/Canine/Florida/43/04 (H3N8) genes to equine, avian, swine, and human strains of influenza A. | | | | |
|---|---|---|---|---|
| **Gene** | **Equine** | **Avian** | **Swine** | **Human** |
| PB2 DQ124147 | 96.9 (98.7)^{a} Eq/Kentucky/2/8 M73526 | 88.6 (96.8) Mall/Alberta/98/85 A Y633315 | 87.9 (96.8) Sw/Ontario/ 01911-1/99 AF285892 | 86.2(96.4) PR/8/34 (HK/213/03) AF389115 (AY576381) |
| PB1 DQ124148 | 97.1 (98.8) Eq/Tennessee/5/86 M25929 | 83.9 (97.1) Ck/BritishColumbia/04 (Gull/Md/704/77) AY61675 (M25933) | 83.9 (97.1) Sw/Korea/S109/04 (Sw/Saskatch/ 18789/02) AY790287 (AY619955) | 83.9 (97.1) WSN/33 (Sing/1/57) J02178 (M25924) |
| PA DQ124149 | 96.3 (97.5) M26082 Eq/Tennesee/5/86 | 87.0(94.3) Ck/Chile/4591/02 (Ostrich/SA/08103/95) AY303660(AF508662) | 84.3 (94.6) Sw/Hong Kong/ 126/02 M26081 | 83.8 (93.4) Taiwan/2/70 (Viet Nam/ 1203/04) AY210199 (AY818132) |
| HA (H3) DQ124190 | 97.4 (97.1) Eq/FL/1/93 L39916 | 80.7 (89.0) Dk/Norway/1/03 AJ841293 | 80.0 (87.7) Sw/Ontario/42729a/01 AY619977 | 81.8 (87.9) HK/1/68 AF348176 |
| NP DQ124150 | 96.6 (97.9) Eq/Tennesee/5/86 M30758 | 87.9 (95.1) Ck/Chile/176822/02 AY303658 | 85.4 (93.5) Sw/Ontario/42729a/01 (Sw/Fujian/1/2003) AY619974 (AY747611) | 84.7 (93.0) HK/1073/99 (Hong Kong /538/97) AF255742 (AF255751) |
| NA (N8) DQ124151 | 96.8 (97.0) Eq/Tennesee/5/86 L06583 | 84.0 (85.2) Dk/NJ/2000 L06583 | na^{b} | na^{b} |
| M DQ124152 | 97.9 (95.7) Eq/Tennesee/5/86 (Eq/Kentucky/92) M63529 (AF001683) | 94.1 (94.0) Tky/Mn/833/80 AF001683 | 93.7 (93.5) Sw/Saskatchewan/ 18789/02 M63527 | 91.2 (95.4) WSN/33 (Hong Kong/ 1073/99) J02177 (AJ278646) |
| NS DQ124153 | 97.5 (95.7) Eq/Tn/5/86 (Eq/Kentucky/92) M80973 (AF001671) | 92.0 (90.4) Mal/NY/6750/78 M80945 | 91.1 (89.1) Sw/China/8/78 (Sw/Korea/s452/04) M80968 (AY790309) | 91.4 (90.0) Brevig Mission/1/18 AF333238 |

| | | | | |
|---|---|---|---|---|
| ^{a} Percent nucleotide and amino acid (in parentheses) sequence identity of A/Canine/Florida/43/04 (H3N8) genes to the most homologous gene of influenza virus virus isolates from the species, followed by their Genbank sequence database accession numbers. ^{b} Not applicable: N8 neuraminidase was never reported in human or swine viruses. | | | | |

| **Table 5.** Antibody titers to A/canine/Florida/43/04 (H3N8) in greyhound serum collected from 1996 to 2003. | | | | | | |
|---|---|---|---|---|---|---|
| | Year^{a} | | | | | |
| | 1996 | 1997 | 1998 | 2000 | 2002 | 2003 |
| No. of dogs tested | 8 | 6 | 19 | 4 | 6 | 22 |
| No. of seropositive dogs | 0 | 0 | 0 | 1 | 2 | 6 |
| Antibody titers^{b} | | | | 512 | 232, 524 | 280-2242 |

| | | | | | | |
|---|---|---|---|---|---|---|
| ^{a} The year of serum sample collection from racing greyhounds in Florida. ^{b} Microneutralization assay antibody titers for seropositive dogs, including the range for the six 2003 seropositive dogs. | | | | | | |

| **Table 6.** Antibody response to A/canine/Florida/43/04 (H3N8) in racing greyhounds at 4 Florida tracks in June 2004. | | | | |
|---|---|---|---|---|
| **Response** | **Track A** | **Track B** | **Track C** | **Track D** |
| Number of dogs tested^{a} | 37 | 10 | 22 | 25 |
| Seroconversion (%)^{b} | 46 | 90 | 100 | 64 |
| Seropositive (%)^{c} | 100 | 100 | 100 | 100 |
| Geometric mean titer^{d} | 401 | 512 | 290 | 446 |

| | | | | |
|---|---|---|---|---|
| ^{a} Number of clinically diseased dogs tested by HI using A/canine/Florida/43/04 (H3N8). ^{b} Percentage of dogs with ≥4-fold increase in antibody titer between acute and convalescent sera. ^{c} Percentage of dogs with a positive antibody titer (HI titer>16) in the convalescent sera. ^{d} Geometric mean antibody titer for the convalescent sera. | | | | |

### MATERIALS AND METHODS FOR EXAMPLES 7-11

### Canine tissues

Postmortem examinations were performed by the Anatomic Pathology Service at the University of Florida College of Veterinary Medicine on 6 mixed breed dogs that died in April/May 2005 during an influenza outbreak in a shelter facility in northeast Florida, and on a pet Yorkshire Terrier dog that died in May 2005 during an influenza outbreak in a veterinary clinic in southeast Florida. Tissues were fixed in 10% neutral buffered formalin, embedded in paraffin, and 5-µm sections were stained with hematoxylin and eosin for histopathologic diagnosis. Unfixed tissues were stored at -80°C pending virological analyses.

### RNA extraction from canine tissue samples

Frozen lung tissues from each of the 7 dogs were thawed and homogenized in minimum essential medium (MEM) supplemented with 0.5% bovine serum albumin (BSA) and antibiotics (gentamycin and ciprofloxacin) using a disposable tissue grinder (Kendall, Lifeline Medical Inc., Danbury, CT). Total RNA was extracted using a commercial kit (RNeasy® Mini Kit, QIAGEN Inc., Valencia, CA) according to manufacturer's instructions and eluted in a final volume of 60 µL of buffer. Total RNA was also extracted from lung tissue collected from dogs without respiratory disease.

### Real-time RT-PCR

A single-step quantitative real-time RT-PCR was performed on total RNA extracted from the canine tissue samples using the QuantiTect® Probe RT-PCR Kit containing ROX as a passive reference dye (QIAGEN Inc., Valencia, CA). Briefly, 2 primer-probe sets were used for detection of influenza A sequences in each sample (**Table 7**). One primer-probe set was selective for canine hemagglutinin (H3) gene sequences. The other primer-probe set targeted a highly conserved region of the matrix (M) gene of type A influenza virus. For each real-time RT-PCR reaction, 5 µL of extracted total RNA were added to a reaction mixture containing 12.5 µL of 2X QuantiTech® Probe RT-PCR Master Mix, 0.25 µL of QuantiTech® RT Mix, forward and reverse primers (0.4 µM final concentration for each), probe (0.1 µM final concentration) and RNase-free water in a final volume of 25 µL. The TaqMan® Ribosomal RNA Control Reagents (Applied Biosystems, Foster City, CA) were used according to manufacturer's instructions for detection of 18S rRNA as an endogenous internal control for the presence of RNA extracted from the canine tissue samples.

Quantitative one-step real-time RT-PCR was performed on the reaction mixtures in a Mx3000P® QPCR System (Stratagene, La Jolla, CA). Cycling conditions included a reverse transcription step at 50°C for 30 minutes, an initial denaturation step at 95°C for 15 minutes to activate the HotStarTaq® DNA polymerase, and amplification for 40 cycles. Each amplification cycle included denaturation at 94°C for 15 seconds followed by annealing/extension at 60°C for 1 minute. The FAM (emission wavelength 518 nm) and VIC (emission wavelength 554 nm) fluorescent signals were recorded at the end of each cycle. The threshold cycle (Ct) was determined by setting the threshold fluorescence (dR) at 1000 in each individual experiment. The Mx3000P® version 2.0 software program (Stratagene, La Jolla, CA) was used for data acquisition and analysis. Samples were considered positive for influenza A virus when the threshold cycle (Ct) for the H3 or M gene was 3 units smaller than the Ct for lung tissues from dogs without respiratory disease. The positive control consisted of amplification of RNA extracted from A/canine/FL/242/03 (H3N8) virus.

### Virus isolation in MDCK cells

Frozen lung tissues from each of the 7 dogs were thawed and homogenized in 10 volumes of Dulbecco's Modified Eagle Medium (DMEM) supplemented with 0.5% (BSA) and antibiotics (gentamycin and ciprofloxacin). Solid debris was removed by centrifugation and supernatants were inoculated onto Madin-Darby canine kidney (MDCK) cells cultured in DMEM supplemented with 1 µg/mL TPCK-treated trypsin (Sigma-Aldrich Corp., St. Louis, MO) and antibiotics (gentamycin and ciprofloxacin). Cells were grown in 25 cm² flasks at 37°C in a humidified atmosphere containing 5% CO₂. The cultures were observed daily for morphologic changes and harvested at 5 days post inoculation. The harvested cultures were clarified by centrifugation and the supernatants inoculated onto fresh MDCK cells as described for the initial inoculation; two additional passages were performed for samples that did not show evidence of influenza virus by hemagglutination or RT-PCR. Hemagglutination activity in the clarified supernatants was determined using 0.5% turkey red blood cells as previously described (Burleson, F. *et al.,* 1992; Kendal, P. *et al.,* 1982). RT-PCR was performed as described below.

### Virus isolation in embryonated chicken eggs

Homogenates were prepared from frozen lung tissues as described above for inoculation of MDCK cells. The homogenates (0.2 mL) were inoculated into the allantoic sac of 10-day old embryonated chicken eggs. After 48 hours of incubation at 35°C, the eggs were chilled at 4°C overnight before harvesting the allantoic fluid. Hemagglutination activity in the clarified supernatants was determined using 0.5% turkey red blood cells as previously described (Burleson, F. *et al.,* 1992; Kendal, P. *et al.,* 1982). RT-PCR was performed as described below. Two additional passages in embryonated eggs were performed for samples that did not show evidence of influenza virus after the initial inoculation.

### RT-PCR, nucleotide sequencing, and phylogenetic analyses

Viral RNA was extracted from MDCK supernatant or allantoic fluid using the QIAamp® Viral RNA Mini Kit (QIAGEN Inc., Valencia, CA) according to manufacturer's instructions. The viral RNA was reverse transcribed to cDNA using the QIAGEN® OneStep RT-PCR Kit (QIAGEN Inc., Valencia, CA) according to manufacturer's instructions. PCR amplification of the coding region of the 8 influenza viral genes in the cDNA was performed as previously described (Klimov, A. *et al.,* 1992b), using universal gene-specific primer sets (primer sequences available on request). The resulting DNA amplicons were used as templates for automated sequencing in the ABI PRISM® 3100 automated DNA sequencer using cycle sequencing dye terminator chemistry (Applied Biosystems, Foster City, CA). Nucleotide sequences were analyzed using the Lasergene 6 Package® (DNASTAR, Inc., Madison, WI). The PHYLIP Version 3.5® software program was used to estimate phylogenies and calculate bootstrap values from the nucleotide sequences (Felsenstein, J., 1989). Phylogenetic trees were compared to those generated by neighbor-joining analysis with the Tamura-Nei model implemented in the MEGA® program (Kumar, S. *et al.,* 2004) and confirmed by the PAUP® 4.0 Beta program (Sinauer Associates, Inc., Sunderland, MA).

### Hemagglutination inhibition (HI) assay

Serum samples were incubated with receptor destroying enzyme (RDE, DENKA SEIKEN Co., Ltd., Tokyo, Japan) (1 part serum: 3 parts RDE) for 16 hours at 37°C prior to heat inactivation for 30 minutes at 56°C. Influenza A/Canine/Jacksonville/05 (H3N8) virus was grown in MDCK cells for 72 hrs at 37°C in 5% CO₂. Virus culture supernatants were harvested, clarified by centrifugation, and stored at -80°C. All other viruses used in the HI assay were grown in 10-day old embryonated chicken eggs from which allantoic fluid was collected and stored at -80°C. The HI assay was performed as described previously (Kendal, P. *et al.,* 1982). Briefly, 4 hemagglutinating units of virus in 25µl were added to an equal volume of serially diluted serum in 96-well plastic plates and incubated at room temperature for 30 minutes. An equal volume of 0.5% turkey erythrocytes was added and the hemagglutination titers were estimated visually after 30 minutes. The endpoint HI titer was defined as the last dilution of serum that completely inhibited hemagglutination.

### EXAMPLE 7 - CLINICAL CASES

In April and May 2005, a previously described (Crawford, P.C. *et al.,* 2005) respiratory disease outbreak occurred in dogs housed in a shelter facility in northeast Florida. The outbreak involved at least 58 dogs ranging in age from 3 months to 9 years, and included purebred dogs as well as mixed breeds. The most common clinical signs were purulent nasal discharge and a cough for 7 to 21 days. Of the 43 dogs that had clinical disease for ≥7 days, 41 had HI antibody titers to canine/FL/04 (H3N8) ranging from 32 to >1024. At least 10 dogs progressed to pneumonia, of which 6 were euthanized. These 6 mixed breed dogs included 3 males and 3 females ranging in age from 4 months to 3 years. The duration of clinical signs ranged from 2 to 10 days at the time of euthanasia. On postmortem examination, these dogs had pulmonary congestion and edema. Histological examination of the respiratory tract revealed rhinitis, tracheitis, bronchitis, bronchiolitis, and suppurative bronchopneumonia. There was epithelial cell necrosis and erosion in the trachea, bronchi, bronchioles, and bronchial glands. The respiratory tissues were infiltrated by neutrophils and macrophages.

In May 2005, a respiratory disease outbreak occurred in 40 pet dogs at a veterinary clinic in southeast Florida. The most common clinical signs were purulent nasal discharge and a cough for 10 to 30 days. Of the 40 dogs, 17 were seropositive for canine/FL/04 (H3N8) with HI antibody tiers ranging from 32 to >1024. Seroconversion occurred in 10 dogs for which paired acute and convalescent sera were available. Three dogs progressed to pneumonia. One of these dogs, a 9-year old male Yorkshire Terrier, died 3 days after onset of clinical signs. This dog had tracheobronchitis, pulmonary edema and congestion, and severe bronchopneumonia. Similar to the 6 shelter dogs, there was epithelial cell necrosis and erosion of the airways and neutrophilic infiltrates in the tissues.

### EXAMPLE 8 - REAL-TIME RT-PCR AND VIRAL ISOLATION

Lung tissues from the 7 dogs were analyzed by quantitative real-time RT-PCR assays that detect the M gene of influenza type A and the H3 gene of canine H3N8 influenza A virus. The lungs from all 7 dogs were positive for both the influenza A M gene and the canine influenza H3 gene (**Table 8**). After 3 passages in MDCK cells, influenza A subtype H3N8 virus was isolated from the lungs of a shelter dog that died after 3 days of pneumonia. This virus was named A/canine/Jacksonville/05 (H3N8) (canine/Jax/05). After 2 passages in embryonated chicken eggs, influenza A subtype H3N8 virus was recovered from the lungs of the pet dog that also died after 3 days of pneumonia. This virus was named A/canine/Miami//05 (H3N8) (canine/Miami/05).

### EXAMPLE 9 - GENETIC ANALYSES OF THE CANINE INFLUENZA A H3N8 ISOLATES

Sequence analyses of canine/Jax/05 and canine/Miami/05 revealed that their hemagglutinin (HA) genes were 98% identical to the canine/FL/04, canine/TX/04, and canine/Iowa/05 isolates recovered from the lungs of racing greyhounds that died of pneumonia during influenza outbreaks at tracks in 2004 and 2005 (Crawford, P.C. *et al.,* 2005; Yoon K-Y. *et al.,* 2005). In addition, the HA genes of canine/Jax/05 and canine/Miami/05 were 98% identical to contemporary equine influenza viruses isolated after the year 2000. Phylogenetic comparisons of the HA genes showed that the canine/Jax/05 and canine/Miami/05 viruses were clustered with the canine/FL/04, canine/TX/04, and canine/Iowa/05 greyhound isolates and contemporary equine isolates, forming a distinct group from the older equine viruses isolated in the early 1990's (**Figure 4**). Furthermore, the canine/Jax/05, canine/Miami/05, and canine/Iowa/05 isolates were more closely related to canine/Tx/04 than to either canine/FL/04 or canine/FL/03. The 2005 isolates formed a subgroup that appears to branch off from the earlier 2003 and 2004 canine viruses with differences at approximately 10 parsimony-informative sites. These differences support the hypothesis that canine influenza virus is being transmitted horizontally from dog-to-dog as opposed to being reintroduced periodically from an outside source. The accumulation of mutations from 2003 to 2005 illustrates the ongoing process of adaptation that the virus must undergo after being transmitted to a new host, as is expected to have happened for the canine influenza viruses.

### EXAMPLE 10 - AMINO ACID ANALYSES OF THE CANINE INFLUENZA A H3N8 ISOLATES.

There were conserved amino acid substitutions in all 6 canine isolates that differentiated them from contemporary equine influenza viruses (**Table 9**). These conserved substitutions were I15M, N83S, W222L, I328T, and N483T. Phylogenetic comparisons of the mature HA protein showed that the canine/Jax/05, canine/Miami/05, and canine/Iowa/05 viruses formed a subgroup with the canine/TX/04 isolate (**Figure 4**). There were 3 amino acid changes (LI18V, K261N, and G479E) that differentiated this subgroup from the other canine viruses (**Table 9**). There were 2 amino acid changes (F79L and G218E) that differentiated the 2005 isolates from their canine/TX/04 root. Furthermore, the 2005 isolates from non-greyhound dogs, canine/Jax/05 and canine/Miami/05, differed from the canine/Iowa/05 greyhound isolate by one amino acid change, R492K. Finally, canine/Jax/05 differed from canine/Miami/05 at a single amino acid, S107P. In all other H3N8 equine and canine viruses, S is conserved at position 107 except for A/Equine/Jilin/1/89 which has a T (Guo Y. *et al*., 1992).

### EXAMPLE 11 - ANTIGENIC ANALYSES OF THE CANINE INFLUENZA A H3N8 ISOLATES

Hemagglutination inhibition (HI) tests were performed using an antigen panel of older and contemporary equine influenza viruses and the canine influenza viruses, and serum collected in 2005 from horses and dogs that had been infected with influenza virus (**Table 10**). Serum from ferrets immunized against canine/FL/04 was also included in the analyses. The HI antibody titers in equine serum were 8 to 16-fold higher when tested with contemporary equine viruses compared to older isolates, but decreased by at least 4-fold when tested with the canine viruses. The canine serum was nonreactive with the older equine viruses, but the antibody titers increased 4-fold when tested with contemporary equine isolates and canine isolates. This was also observed for the serum from ferrets immunized against canine influenza virus. These seroreactivity patterns demonstrated the antigenic similarity between the canine influenza viruses and contemporary equine influenza viruses and were consistent with the phylogenetic analyses. The antibody titers in equine, canine, and ferret sera to the canine/Miami/05 isolate were similar to those for the 2003 and 2004 canine isolates. However, the titers were 2 to 4-fold lower for the canine/Jax/05 isolate. This suggests that canine/Jax/05 is antigenically distinct from the other canine isolates, which may in part be related to the single amino acid change at position 107 in the mature HA.

| **Table 7.** Primers and probes for quantitative real-time RT-PCR analysis for the matrix gene of influenza A virus and the H3 gene of canine influenza A (H3N8). | | | |
|---|---|---|---|
| **Primer** | **Target** | **Sequence** | **Application** |
| Ca-H3-F387 | H3 (nt 387-406) | 5'-tatgcatcgctccgatccat-3' (SEQ ID NO: 79) | Forward primer for H3 |
| Ca-H3-R487 | H3 (nt 487-467) | 5'-gctccacttcttccgttttga-3' (SEQ ID NO: 80) | Reverse primer for H3 |
| Ca-H3-P430 | H3 (nt 430-459) | FAM-aattcacagcagagggattcacatggacag-BHQ1 (SEQ ID NO: 81) | TaqMan® probe |
| FluA-M-F151 | M (nt 151-174) | 5'-catggartggctaaagacaagacc-3' ^{a} (SEQ ID NO: 82) | Forward primer for M |
| FluA-M-R276 | M (nt 276-253) | 5'-agggcattttggacaaakcgtcta-3' (SEQ ID NO: 83) | Reverse primer for M |
| FluA-M-P218 | M (nt 218-235) | FAM-acgcTcaccgTgcccAgt-BHQ1 ^{b} (SEQ ID NO: 84) | TaqMan® probe |

| | | | |
|---|---|---|---|
| ^{a} Underlined letter r represents nucleotide a or g and underlined letter k represents nucleotide g or t. ^{b} Uppercase letters represent locked nucleic acid residues. | | | |

| **Table 8.** Quantitative real-time RT-PCR and viral isolation performed on lung tissues from dogs that died from pneumonia during respiratory disease outbreaks in a shelter and veterinary clinic in Florida. | | | | | |
|---|---|---|---|---|---|
| **Dog ID** | **Location** | **Duration of clinical disease** | **Real-time RT-PCR** | | **Virus Isolation** |
| | | | **M (Ct)** | **HA (Ct)** | |
| A/canine/FL/242/03 positive control | | | 28.15 | 27.36 | |
| 1079 | Shelter (NE FL) | 2 days | 29.81 | 28.84 | none |
| 1078 | Shelter (NE FL) | 3 days | 30.37 | 29.71 | MDCK 3^{rd} passage |
| 318 | Shelter (NE FL) | 9 days | 33.89 | 32.97 | none |
| 320 | Shelter (NE FL) | 10 days | 39.44 | 37.09 | none |
| 319 | Shelter (NE FL) | 6 days | 33.87 | 32.23 | none |
| 1080 | Shelter (NE FL) | 6 days | 38.87 | 38.23 | none |
| 374 | Veterinary clinic (SE FL) | 3 days | 24.05 | 22.65 | Egg 2^{nd} passage |

| **Table 9.** Amino acid comparison of the mature HA for canine influenza viruses and contemporary equine influenza viruses. | | | | | | | | | | | | | | | | | | |
|---|---|---|---|---|---|---|---|---|---|---|---|---|---|---|---|---|---|---|
| | **Amino Acid** | | | | | | | | | | | | | | | | | |
| | 7 | **15** | 54 | 78 | 79 | **83** | 92 | 107 | 118 | 159 | 218 | **222** | 261 | **328** | 479 | **483** | 492 | 541 |
| **A/equine/KY/5/02** | G | **I** | N | V | F | **N** | S | S | L | N | G | **w** | K | **I** | G | **N** | R | K |
| **A/equine/MA/213/03** | . | . | . | A | . | . | . | . | . | S | . | . | . | . | . | . | . | . |
| **A/equine/OH/1/03** | D | . | K | A | . | . | . | . | . | S | . | . | . | . | . | . | . | . |
| **A/canine/FL/242/03** | . | **M** | K | A | . | **S** | . | . | . | S | . | **L** | . | **T** | . | **T** | . | . |
| **A/canine/FL/43/04** | . | **M** | K | A | . | **S** | N | . | . | S | . | **L** | . | **T** | . | **T** | . | R |
| **A/canine/TX/1/04** | . | **M** | K | A | . | **S** | . | . | V | S | . | **L** | N | **T** | E | **T** | . | . |
| **A/canine/Iowa/05** | . | **M** | K | A | L | **S** | . | . | V | S | E | **L** | N | **T** | E | **T** | . | . |
| **A/canine/Miami/05** | . | **M** | K | A | L | **S** | . | . | V | S | E | **L** | N | **T** | E | **T** | K | . |
| **A/canine/Jacksonville/05** | . | **M** | K | A | L | **S** | . | P | V | S | E | **L** | N | **T** | E | **T** | K | . |

| **Table 10.** Antibody titers in equine, canine, and ferret serum to older and contemporary equine influenza viruses and canine influenza viruses. | | | |
|---|---|---|---|
| | **Serum** | **antibody** | **titers^{a}** |
| **Antigens** | **Equine** | **Canine** | **Ferret^{b}** |
| equine/Miami/63 | 40 | <10 | 16 |
| equine/Ky/86 | 40 | 40 | 32 |
| equine/KY/92 | 40 | <10 | 32 |
| equine/NY/99 | 320 | 40 | 128 |
| equine/KY/05/02 | 320 | 160 | 256 |
| equine/MA/213/03 | 640 | 160 | 512 |
| equine/OH/01/03 | 640 | 160 | 512 |
| canine/FL/03 | 160 | 160 | 512 |
| canine/FL/04 | 160 | 80 | 512 |
| canine/Tx/04 | 160 | 160 | 512 |
| canine/Miami/05 | 160 | 80 | 256 |
| canine/J ax/05 | 40 | 40 | 128 |

| | | | |
|---|---|---|---|
| ^{a} Antibody titers were determined in a hemagglutination inhibition assay performed with serial dilutions of equine, canine, or ferret serum and the viruses listed in the antigen column. ^{b} Serum from ferrets immunized with canine/FL/04 virus. | | | |

### MATERIALS AND EXAMPLES METHODS FOR EXAMPLES 12-15 (REFERENCE)

### Canine influenza virus inoculum.

The virus inoculum was prepared by inoculation of Madin-Darby canine kidney (MDCK) epithelial cells with a stock of A/canine/FL/43/04 (H3N8) representing passage 3 of the original isolate previously described (Crawford *et al*., 2005). The inoculated MDCK cells in Dulbecco's Minimal Essential Media (DMEM) supplemented with 1 µg/mL TPCK-treated trypsin (Sigma-Aldrich Corp., St. Louis, MO) and antibiotics (gentamycin and ciprofloxacin) were grown in 250 cm² flasks at 37°C in a humidified atmosphere containing 5% CO₂. The cultures were observed daily for morphologic changes and harvested at 5 days post inoculation. The harvested cultures were clarified by centrifugation and the supernatants were stored at -80°C pending inoculation of dogs. An aliquot of supernatant was used for determination of virus titer by the Reed and Muench method. The titer was 10⁷ median tissue culture infectious doses (TCID₅₀) of A/canine/Florida/43/04 (canine/FL/04) per mL.

### Experimental inoculation.

Eight 4-month old colony bred mongrel dogs (Marshall BioResources, North Rose, NY) (4 males and 4 females) were used for the experimental inoculation study approved by the University of Florida Institutional Animal Care and Use Committee. The dogs' body weights ranged from 13 to 17 kg. The dogs were healthy based on physical examinations, baseline blood tests, and recording of body temperatures for 2 weeks prior to inoculation. All dogs were free from prior exposure to canine influenza virus based on serology tests performed on paired serum samples collected at the time of arrival into the facility and 2 weeks later. The dogs were anesthetized by intravenous injection of propofol (Diprivan®, Zeneca Pharmaceuticals, 0.4 mg/kg body weight to effect) for intubation with endotracheal tubes. Six dogs (3 males and 3 females) were each inoculated with 10⁷ TCID₅₀ of canine/FL/04 virus in 5 mL of sterile saline administered into the distal trachea through a small diameter rubber catheter inserted into the endotracheal tube. Two dogs (1 male and 1 female) were sham-inoculated with an equal volume of sterile saline. The sham-inoculated control dogs were housed in a different room from the virus-inoculated dogs and cared for by different personnel. Physical examinations and rectal temperature recordings were performed twice daily for 6 days post inoculation (p.i.).

### Pharyngeal and rectal swab collection.

To monitor for virus shedding, oropharyngeal specimens were collected twice daily from each dog on days 0 to 6 p.i. using polyester swabs (Fisher Scientific International Inc., Pittsburgh, PA). The swabs were placed in 1 mL of sterile phosphate-buffered saline (PBS) containing 0.5% bovine serum albumin (BSA). Rectal swabs were collected from each dog daily from days 0 to 6. Swab extracts were prepared by clarification of the swab transport media by centrifugation. An aliquot of swab extract was tested immediately for influenza A virus nucleoprotein using the Directigen™ commercial immunoassay kit (BD, Franklin Lakes, NJ) according to the manufacturer's instructions. The remaining extract was stored at -80°C pending other virological assays.

### Postmortem examinations.

On day 1 p.i., one sham-inoculated dog and one virus-inoculated dog were euthanatized by intravenous inoculation of Beuthanasia-D® solution (1 mL/5 kg body weight; Schering-Plough Animal Health Corp). One virus-inoculated dog was similarly euthanatized each day from days 2 to 5 p.i. On day 6 p.i., the remaining sham-inoculated and virus-inoculated dog were euthanatized. Complete postmortem examinations were performed by one of the investigators (WLC). Tissues were fixed in 10% neutral buffered formalin, embedded in paraffin, and 5-µm sections were either stained with hematoxylin and eosin for histopathologic diagnosis or processed for immunohistochemistry as described below. Unfixed lung tissues were submitted to the Diagnostic Clinical Microbiology/Parasitology/ Serology Service at the University of Florida College of Veterinary Medicine for bacterial isolation and identification. The samples were cultured on nonselective media as well as media selective for *Bordetella* species (Regan-Lowe; Remel, Lenexa, KS) and *Mycoplasma* species (Remel). All cultures were held for 21 days before reporting no growth. Unfixed tissues were also stored at -80°C pending virological analyses.

### Immunohistochemistry.

Deparaffinized and rehydrated 5-µm trachea and lung tissue sections were mounted on Bond-Rite™ slides (Richard-Allan Scientific, Kalamazoo, MI) and subsequently treated with proteinase K (DAKOCytomation Inc., Carpenteria, CA) followed by peroxidase blocking reagent (DAKO® EnVision™ Peroxidase Kit, DAKO Corp., Carpenteria, CA). The sections were incubated with a 1:500 dilution of monoclonal antibody to influenza A H3 (Chemicon International, Inc., Ternecula, CA) for 2 hours at room temperature. Controls included incubation of the same sections with mouse IgG (1 mg/mL, Serotec, Inc. Raleigh, NC), and incubation of the monoclonal antibody with normal canine lung sections. Following treatment with the primary antibody, the sections were incubated with secondary immunoperoxidase and peroxidase substrate reagents (Dako® EnVision™ Peroxidase Kit, Dako Corp.) according to the manufacturer's instructions. The sections were counterstained with hematoxylin, treated with Clarifier #2 and Bluing Reagent (Richard-Allan Scientific, Kalamazoo, MI), dehydrated, and coverslips applied with Permount (ProSciTech, Queensland, Australia).

### RNA extraction from swabs and tissues.

Lung and tracheal tissues from each dog were thawed and homogenized in minimum essential medium (MEM) supplemented with 0.5% bovine serum albumin (BSA) and antibiotics (gentamycin and ciprofloxacin) using a disposable tissue grinder (Kendall, Lifeline Medical Inc., Danbury, CT). Total RNA was extracted from the tissue homogenates as well as orpharyngeal and rectal swab extracts using a commercial kit (RNeasy® Mini Kit, QIAGEN Inc., Valencia, CA) according to manufacturer's instructions and eluted in a final volume of 60 µL of buffer.

### Real-time RT-PCR.

A single-step quantitative real-time RT-PCR was performed on the total RNA using the QuantiTect® Probe RT-PCR Kit containing ROX as a passive reference dye (QIAGEN Inc., Valencia, CA) and a primer-probe set that targeted a highly conserved region of the matrix (M) gene of type A influenza virus (Payungporn S. *et al*., 2006a; Payungporn S. *et al*., 2006b). For each real-time RT-PCR reaction, 5 µL of extracted total RNA were added to a reaction mixture containing 12.5 µL of 2X QuantiTech® Probe RT-PCR Master Mix, 0.25 µL of QuantiTech® RT Mix, forward and reverse primers (0.4 µM final concentration for each), probe (0.1 µM final concentration) and RNase-free water in a final volume of 25 µL. The TaqMan® GAPDH Control Reagents (Applied Biosystems, Foster City, CA) were used according to manufacturer's instructions for detection of GAPDH as an endogenous internal control for the presence of RNA extracted from the swab and tissue samples and as a normalization control.

Quantitative one-step real-time RT-PCR was performed on the reaction mixtures in a Mx3000P® QPCR System (Stratagene, La Jolla, CA). Cycling conditions included a reverse transcription step at 50°C for 30 minutes, an initial denaturation step at 95°C for 15 minutes to activate the HotStarTaq® DNA polymerase, and amplification for 40 cycles. Each amplification cycle included denaturation at 94°C for 15 seconds followed by annealing/extension at 60°C for 1 minute. The FAM (emission wavelength 518 nm) and VIC (emission wavelength 554 nm) fluorescent signals were recorded at the end of each cycle. The threshold cycle (Ct) was determined by setting the threshold fluorescence (dR) at 1000 in each individual experiment. The Mx3000P® version 2.0 software program (Stratagene, La Jolla, CA) was used for data acquisition and analysis. The positive control consisted of amplification of RNA extracted from A/canine/FL/242/03 (H3N8) virus. The results were normalized by dividing the M Ct value by the corresponding GAPDH Ct value for each sample.

### Virus re-isolation from tissues.

Frozen lung and trachea tissues from virus-inoculated dogs were thawed and homogenized in 10 volumes of DMEM supplemented with 0.5% BSA and antibiotics. Solid debris was removed by centrifugation and supernatants were inoculated onto MDCK cells cultured in DMEM supplemented with 1 µg/mL TPCK-treated trypsin (Sigma-Aldrich Corp., St. Louis, MO) and antibiotics as described above. Cells were grown in 25 cm² flasks at 37°C in a humidified atmosphere containing 5% CO₂. The cultures were observed daily for morphologic changes and harvested at 5 days post inoculation. The harvested cultures were clarified by centrifugation and the supernatants inoculated onto fresh MDCK cells as described for the initial inoculation; two additional passages were performed for samples that did not show evidence of influenza virus by hemagglutination or RT-PCR. Hemagglutination activity in the clarified supernatants was determined using 0.5% turkey red blood cells as previously described (Crawford *et al*., 2005). RT-PCR was performed as described below.

### RT-PCR, nucleotide sequencing, and phylogenetic analyses.

Viral RNA was extracted from MDCK supernatant using the QIAamp® Viral RNA Mini Kit (QIAGEN Inc., Valencia, CA) according to manufacturer's instructions. The viral RNA was reverse transcribed to cDNA using the QIAGEN® OneStep RT-PCR Kit (QIAGEN Inc., Valencia, CA) according to manufacturer's instructions. PCR amplification of the coding region of the 8 influenza viral genes in the cDNA was performed as previously described (Crawford *et al*., 2005), using universal gene-specific primer sets (primer sequences available on request). The resulting DNA amplicons were used as templates for automated sequencing in the ABI PRISM® 3100 automated DNA sequencer using cycle sequencing dye terminator chemistry (Applied Biosystems, Foster City, CA). Nucleotide sequences were analyzed using the Lasergene 6 Package® (DNASTAR, Inc., Madison, WI). The nucleotide sequences for viruses recovered from infected dogs were compared to the sequences of the virus in the inoculum to determine if any changes had occurred during replication in the respiratory tract.

### EXAMPLE 12 - CLINICAL DISEASE (REFERENCE)

All 6 virus-inoculated dogs developed a transient fever (rectal temperature ≥39°C) for the first 2 days p.i., but none exhibited respiratory signs such as cough or nasal discharge over the 6-day observation period. The sham-inoculated dogs remained clinically healthy.

### EXAMPLE 13 - VIRUS SHEDDING (REFERENCE)

Influenza A nucleoprotein was detected in the oropharyngeal swab collected from one of the virus-inoculated dogs at 24 hours p.i. The oropharyngeal swabs collected from one dog at 72, 84, and 120 hours p.i., and another dog at 108, 120, and 132 hours p.i., were positive for virus by quantitative real-time RT-PCR (**Table 11**). The absolute number of influenza M gene copies per µL of swab extract increased with time from 3 to 6 days p.i. No virus was detected in the rectal swabs.

### EXAMPLE 14 - POSTMORTEM EXAMINATIONS (REFERENCE)

In contrast to the previous experimental infection using specific pathogen-free Beagles (Crawford *et al*., 2005), the virus-inoculated mongrel dogs had pneumonia as evidenced by gross and histological analyses of the lungs from days 1 to 6 p.i. In addition to pneumonia, the dogs had rhinitis, tracheitis, bronchitis, and bronchiolitis similar to that described in naturally infected dogs (Crawford *et al*., 2005). There was epithelial necrosis and erosion of the lining of the airways and bronchial glands with neutrophil and macrophage infiltration of the submucosal tissues (**Figure 5****, upper panels**). Immunohistochemistry detected viral H3 antigen in the epithelial cells of bronchi, bronchioles, and bronchial glands (**Figure 5****, lower panels**). No bacterial superinfection was present. The respiratory tissues from the 2 sham-inoculated dogs were normal.

### EXAMPLE 15 - VIRUS REPLICATION IN TRACHEA AND LUNGS (REFERENCE)

The trachea and lungs were positive for virus by quantitative real-time RT-PCR in all dogs from 1 to 6 days p.i. (**Table 12**). The absolute number of influenza M gene copies per µL of trachea homogenate increased from 1 to 5 days p.i., then decreased on day 6. The absolute number of M gene copies per µL of lung homogenate decreased from 1 to 6 days p.i. In general, the trachea contained ≥ one log₁₀ more virus than the lung on each of the 6 days p.i.

| **Table 11.** Detection of virus shedding in the oropharynx of mongrel dogs inoculated with canine influenza virus by quantitative real-time RT-PCR. | | | |
|---|---|---|---|
| **Dog ID** | **Time p.i. (hours)^{a}** | **M/GAPDH ratio^{b}** | **Matrix gene (copies / uL)^{c}** |
| 860 | 72 | 1.20 | 1.57E+02 |
| | 84 | 1.30 | 8.25E+02 |
| | 120 | 1.23 | 1.47E+03 |
| 894 | 108 | 1.17 | 1.17E+02 |
| | 120 | 1.41 | 1.37E+02 |
| | 132 | 1.27 | 3.74E+02 |

| | | | |
|---|---|---|---|
| ^{a} Time that oropharyngeal swabs were collected from the dogs following inoculation with A/canine/FL/43/04 (H3N8) virus. ^{b} Normalization ratios were calculated by dividing the M (Ct) by the GAPDH (Ct) for each swab extract. ^{c} The absolute number of matrix gene copies per uL of swab extract. | | | |

| **Table 12.** Detection of virus replication in the trachea and lung of mongrel dogs inoculated with canine influenza virus by quantitative real-time RT-PCR. | | | | | |
|---|---|---|---|---|---|
| | | **M/GAPDH ratio^{b}** | | **Matrix gene (copies / uL)^{c}** | |
| **Dog ID** | **Time p.i. (hours)^{a}** | **Lung** | **Trachea** | **Lung** | **Trachea** |
| 797 | 24 | 1.20 | 1.43 | 8.22E+05 | 3.11E+04 |
| 801 | 48 | 1.33 | 0.99 | 1.15E+05 | 6.52E+06 |
| 789 | 72 | 1.44 | 1.12 | 2.39E+04 | 1.56E+05 |
| 819 | 96 | 1.40 | 1.27 | 3.19E+04 | 1.43E+05 |
| 860 | 120 | 1.59 | 1.04 | 3.48E+03 | 1.17E+06 |
| 894 | 144 | 1.70 | 1.15 | 4.78E+02 | 1.50E+03 |

| | | | | | |
|---|---|---|---|---|---|
| ^{a} Time that tissues were collected from the dogs following inoculation with A/canine/FL/43/04 (H3N8) virus. ^{b} Normalization ratios were calculated by dividing the M (Ct) by the GAPDH (Ct) for each tissue homogenate. ^{c} The absolute number of matrix gene copies per uL of tissue homogenate. | | | | | |

### MATERIALS AND EXAMPLES METHODS FOR EXAMPLE 16 (REFERENCE)

### Virus strains

Canine influenza virus strains as well as those of avian, equine and human origin (listed in **Table 15**) were propagated in embryonated eggs or MDCK cells and their infectivity was titrated by endpoint dilution in chicken embryos, or plaque assay. Rapid virus quantification was performed by hemagglutination assay using turkey red blood cell erythrocytes.

### Diagnostic specimens

A Total of 60 canine's lung tissues collected from suspect cases of viral respiratory disease during the year of 2005 were tested for the presence of canine influenza virus.

### RNA extraction from canine tissue samples

Blocks of lung tissue weighing between 20 and 30 mg were homogenized in a disposable tissue grinder (Kendal). Total RNA was extracted using a commercial kit (RNeasy Mini Kit, Qiagen, Valencia, CA) and eluted in a final volume of 60 µL, following the manufacturer's recommendations.

### Primers and probes design

Multiple sequence alignments of the H3 and M genes from various subtypes and from diverse species were performed using the CLUSTAL X program (Version 1.8). Matrix (M) primers and probe were selected from the conserved regions of over the known sequences corresponding to different subtypes of influenza A virus, whereas the H3 hemagglutinin gene-specific primers and probe set were selected to specifically match equine and canine influenza A virus genes and mismatch the homologous avian and human genes (**Table 13**). Primer design software (OLIGOS Version 9.1) and the web based analysis tools provided by EXIQON (http://lnatools.com) was used for Tm calculation and prediction of secondary structure as well as self hybridization. A conserved region of an 18S rRNA gene was used as endogenous internal control for the presence of RNA extracted from canine tissue sample. The Pre-Developed TaqMan® Assay Reagents for Eukaryotic 18S rRNA (VIC/TAMRA) (Applied Biosystems) was used for the real-time detection of 18S rRNA in tissue samples.

### Real-time RT-PCR condition

A single-step real-time RT-PCR was performed by using the Quantitect Probe RT-PCR Kit containing ROX as a passive reference dye (Qiagen, Valencia, CA). In each real-time RT-PCR reaction, 5 µL of RNA sample were used as a template to combine with a reaction mixture containing 10 µL of 2X QuantiTech Probe RT-PCR Master Mix, 0.2 µL of QuantiTech RT Mix, primers (0.4 µM final conc. for H3 gene or 0.6 µM final conc. for M gene), probe (0.1 µM final conc. for H3 gene or 0.2 µM final conc. for M gene) and RNase-free water in a final volume of 20 µL. One-step real-time RT-PCR was performed in the Mx3005P Real-Time QPCR System (Stratagene). Cycling conditions included a reverse transcription step at 50°C for 30 minutes. After an initial denaturation step at 95°C for 15 minutes in order to activate the HotStarTaq DNA polymerase, amplification was performed during 40 cycles including denaturation (94°C for 15 seconds) and annealing/extension (60°C for 30 seconds). The FAM (emission wavelength 516 nm for H3 and M detection) and VIC (emission wavelength 555 nm for 18S rRNA detection) fluorescent signals were obtained once per cycle at the end of the extension step. Data acquisition and analysis of the real-time PCR assay were performed using the Mx3005P software version 2.02 (Stratagene).

### Specificity of H3 primers/ probe set for canine influenza (H3N8) and universality of M primers/probe set for type A influenza virus

In order to test the specificity of each primers/probe set, RNA extracted from several known subtypes of influenza A viruses were used as a template in the real-time RT-PCR assay (**Table 15**).

### RNA standard for determination of the real-timer RT-PCR performance

The genes of canine influenza A virus (A/canine/Florida/242/2003(H3N8)) were used to generate the PCR amplicons for H3 (nt 1-487) and M (nt 1-276) by using primers linked with T7 promoter (**Table 13**). Then the purified PCR amplicons of H3 and M genes were used as templates for *in vitro* transcription by using Riboprobe *in vitro* Transcription System-T7 (Promega) following the manufacturer's recommendations. The concentration of the transcribed RNAs was calculated by measuring absorbance at 260 nm. The RNAs were then serially diluted 10-fold, ranging from 10⁸ to 10 copies/ µL to perform sensitivity tests. Moreover, a standard curve was generated by plotting the log of initial RNA template concentrations (copies/ µL) against the threshold cycle (Ct) obtained from each dilution in order to determine the overall performance of real-time RT-PCR.

### Comparative sensitivity tests between real-time RT-PCR and Directigen Flu A test kit

Stock viruses of two viral strains including A/Wyoming/3/2003 (H3N2) at 10^{6.67} EID₅₀/mL (HA=64) and A/canine/Florida/242/2003(H3N8) at 10^{7.17} EID₅₀/mL (HA=16) were used for the detection threshold assay. Logarithmic dilution of specimens in phosphate-buffered saline (PBS) (125 µL) were used in a rapid influenza A antigen detection kit, Directigen Flu A, (Becton, Dickinson and Company) following the manufacturer's instructions. Each Directigen Flu A test device has an H1N1 influenza antigen spot in the center of the membrane which develops as a purple dot and indicates the integrity of the test, which is based on a monoclonal antibody to the nucleoprotein (NP). The development of a purple triangle surrounding the dot is indicative of the presence of influenza NP in the tested specimen. The intensity of the purple signal from the triangle was scored as + (outline of triangle), ++ (lightly colored triangle), +++ (dark-purple triangle) and ++++ (very dark-purple triangle). Viral RNA was extracted 125 µL aliquots of each virus dilution by using QIAamp Viral RNA Mini Kit (Qiagen, Valencia, CA) and eluting in a final volume of 50 µL. A volume of 5 uL of the extracted viral RNAs were tested by real-time RT-PCR for comparative sensitivity test with Directigen Flu A kit.

### EXAMPLE 16 (REFERENCE)

The real-time RT-PCR assay for canine influenza relies on information from three molecular probes which target 18S rRNA from host cell was well as M and H3 from the influenza A virus genome (**Table 14**). Amplification of the host gene is a reporter of specimen quality and integrity. Clinical, necropsy or laboratory samples containing canine influenza (H3N8) virus are expected to yield amplification signal with the three probes. Specimens yielding amplification signal with M and 18S rRNA probes but negative for H3 would be indicative of an influenza virus subtype H3 from human, swine or avian origin or from non-H3 subtypes. These rare cases could be resolved by RT-PCR using HA universal primers to generate amplicon cDNA that can be analyzed by sequencing. Properly collected and handled specimens lacking influenza A virus yield 18S rRNA amplification signal only. Situations in which only the 18S rRNA probe and the H3 probes yield amplification signal are indicative of faulty technique, unless proven otherwise; either a false negative with M probes or false positive for H3 need to be demostrated. Finally, specimens failing to yield amplification signals with the three probes are indicative of defective sample collection, degradation, faulty RNA extraction or the presence of inhibitors the polymerases used in PCR.

In order to test the specificity of the H3 primers/probe set for canine influenza A virus (H3N8) and the universality of M primers/probe set for type A influenza, several subtypes of influenza A viruses were tested by real-time RT-PCR. The results show that H3 primers/probe set yielded a positive amplification signal only with canine influenza (H3N8). No significant false positive or non-specific amplification signals were observed in other subtypes or human H3 strains. The M primers/probe set yielded positive amplification signal with all of the strains tested (**Table 15**). These results indicated that H3 primers/probe specifically detects canine influenza A virus (H3N8) whereas M primers/probe detect multiple subtypes of type A influenza viruses.

The performance of real-time RT-PCR assays was evaluated by endpoint dilution of M and H3 *in vitro* transcribed RNAs. As expected, the threshold cycle (Ct) increased in direct correlation with the dilution of the RNA standards. The fluorescent signals can be detected at RNA standard dilutions of M and H3 as low as 10³ and 10² copies/µL, respectively (**Figure 6A** **and** **6B**). The standard curves of M and H3 genes were constructed by plotting the log of starting RNA concentrations against the threshold cycle (Ct) obtained from each dilution (**Figure 6C** **and** **6D**). The slope of the standard curve is used to determine the PCR reaction efficiency, which is theoretically exponential; 100% amplification efficiency would imply doubling of amplicon cocentration each cycle. The standard curves with a slope between approximately -3.1 and -3.6 are typically acceptable for most applications requiring accurate quantification (90-110 % reaction efficiency). An Rsq value is the fit of all data to the standard curve plot. If all the data lie perfectly on the line, the Rsq will be 1.00. As the data fall further from the line, the Rsq decreases. An Rsq value ≥ 0.985 is acceptable for most assays. The M standard curve revealed a slope of-3.576 (efficiency= 90.4 %) and Rsq= 1.00 whereas H3 standard curve yielded a slope of-3.423 (efficiency= 95.9%) and Rsq= 0.999. These values indicate satisfactory amplification efficiency and overall performance of the real-time RT-PCR assays. We attribute the lower efficiency and sensitivity of M primers/probe set as compared to H3 primers/probe set to the N-fold degeneracy of M primer sequences required to ensure broad coverage of M gene sequences variability across viruses of multiple subtypes, hosts and lineages.

The sensitivity of real-time RT-PCR assay was also compared with the commercial rapid antigen detection assay (Directigen Flu A). Logarithmic dilutions of A/Wyoming/3/2003 (H3N2) and A/canine/Florida/242/2003(H3N8) were analyzed with Directigen Flu A and by real-time RT-PCR. The results of Directigen Flu A showed that the sensitivities against both viral strains are approximately 100-fold dilution from the stock viruses used in these experiments (**Figure 7**). The signals (purple color) generated by the canine virus (A/canine/Florida/242/2003: 10^{6.x} PFU/ml) samples were much weaker than those found in human virus (A/Wyoming/3/2003: 10^{7.x} PFU/ml), in agreement with the lower virus concentration in these samples. Alternatively, lower signal for canine influenza could be attributed to the molecular specificity of monoclonal antibodies against the NP; *i.e.* poor conservation of the amino acids within the NP epitope of canine influenza A viruses.

Real-time RT-PCR of the M gene yielded Ct values above threshold with virus 10 and 30 PFU equivalents per reaction of A/canine/Florida/242/2003 and A/Wyoming/3/2003, respectively (**Table 16**). The differences between the sensitivity value of 2 viral strains because the differences in the original viral titers. The H3 gene detection comparison between canine and human influenza viruses was not performed because the H3 primers/probe in our realtime RT-PCR assay amplifies exclusively canine influenza A virus. RT-PCR was 10⁵ times more sensitive than the rapid antigen detection kit.

To evaluate the performance of the RT-PCR test in necropsy specimens from dogs with acute respiratory disease, sixty canine lung tissue samples submitted during the year of 2005 were tested for the presence of canine influenza A virus by real-time RT-PCR. A total of 12 out of 60 samples (20%) were positive with both M and H3 genes whereas the remaining 48 samples yielded negative result for both M and H3 gene. Virus isolation attempts were conducted by egg and MDCK cell inoculation, to evaluate the specificity of the realtime assay; 2 out 12 samples that were positive for canine influenza by RT-PCR yielded canine influenza virus (data not shown, manuscript in preparation). Although all of the tissues were collected from dogs with a history of severe respiratory disease, most of the samples yielded no canine influenza virus by either realtime PCR or conventional isolation, suggesting a high incidence of other respiratory pathogens such as *Bordetella bronchiseptica,* canine distemper or parainfluenza virus. The single step real-time RT-PCR assay herein provides a rapid, sensitive and cost-effective approach for canine influenza A virus (H3N8) detection. Rapid laboratory diagnosis of canine influenza A virus (H3N8) infections in the early stage of the disease can yield information relevant to clinical patient and facility management.

| **Table 13:** Primers and probes used for real-time RT-PCR detection and in vitro transcription | | | | |
|---|---|---|---|---|
| **Oligo name** | **Type** | **Target** | **Sequence *** | **Application** |
| Ca-H3-F387 | Forward primer | H3 (nt 387-406) | 5'-tatgcatcgctccgatccat-3' (SEQ ID NO: 79) | Real-time PCR |
| Ca-H3-R487 | Reverse primer | H3 (nt 487-467) | 5'-gctccacttcttccgttttga-3' (SEQ ID NO: 80) | |
| Ca-H3-P430 | TaqMan probe | H3 (nt 430-459) | FAM-aattcacagcagagggattcacatggacag-BHQ1 (SEQ ID NO: 81) | |
| FluA-M-F151 | Forward primer | M (nt 151-174) | 5'-catggartggctaaagacaagacc-3' (SEQ ID NO: 82) | Real-time PCR |
| FluA-M-R276 | Reverse primer | M (nt 276-253) | 5'-agggcattttggacaaakcgtcta-3' (SEQ ID NO: 83) | |
| FluA-M-P218 | LNA TaqMan probe | M (nt 218-235) | FAM-acgcTcaccgTgcccAgt-BHQ1 (SEQ ID NO: 84) | |
| H3-F1 | Forward primer | H3 (nt 1-14) | 5'-tattcgtctcagggagcaaaagcagggg-3' (SEQ ID NO: 85) | *In vitro* transcription |
| T7/H3-R490 | Reverse primer | T7 / H3 (nt 487-467) | 5' -tgtaatacgactcactatagggctccacttcttccgttttga-3' (SEQ ID NO: 86) | |
| M-F1 | Forward primer | M (nt 1-15) | 5'-gatcgctcttcagggagcaaaagcaggtag-3' (SEQ ID NO: 87) | *In vitro* transcription |
| T7/M-R276 | Reverse primer | M (nt 276-253) | 5'-tgtaatacgactcactatagggcattttggacaaagcgtc-3' (SEQ ID NO: 88) | |

| | | | | |
|---|---|---|---|---|
| * Note: Uppercases = LNA (Locked Nucleic Acid) residues, r = a or g, k= g or t, underline= T7 promoter sequence | | | | |

| **Table 14:** Interpretation of the real-time RT-PCR assay | | | |
|---|---|---|---|
| **Interpretation** | **Results** | | |
| | **M** | **H3** | **18S rRNA** |
| Positive for canine influenza A virus (H3N8) | + | + | + |
| Positive for influenza A virus (unknown subtype) | + | - | + |
| Negative for influenza A virus | - | - | + |
| Error in RNA extraction or presence of PCR inhibitor | - | - | - |

| **Table 15:** Specificity test of canine H3 primers/probe set and universality test of M primers/probe set with several subtypes of influenza A viruses | | | | |
|---|---|---|---|---|
| **Subtypes** | **Strain Name** | **Host** | **Real-time RT-PCR detection** | |
| | | | **H3 gene (Ct)** | **M gene (Ct)** |
| H1 | A/Ohio/1983 | Human | No Ct | 15.40 |
| | A/WSN/1933 | Human | No Ct | 20.09 |
| H3 | A/Wyoming/3/2003 | Human | No Ct | 28.85 |
| | A/Victoria/3/1975 | Human | No Ct | 16.62 |
| | A/canine/FL/242/2003 | Canine | 28.43 | 29.25 |
| H4 | Turkey/MN/1066/1980 | Avian | No Ct | 17.49 |
| | Clinical sample* | Avian | No Ct | 20.87 |
| H5 | AChicken/Thailand/CUK2/2004 | Avian | No Ct | 20.13 |
| | A/Pheasant/NJ/1335/1998 | Avian | No Ct | 16.64 |
| H6 | Clinical sample* | Avian | No Ct | 19.52 |
| H10 | Clinical sample* | Avian | No Ct | 25.64 |
| | Clinical sample* | Avian | No Ct | 19.59 |
| H11 | Clinical sample* | Avian | No Ct | 15.72 |
| | Clinical sample* | Avian | No Ct | 24.55 |

| | | | | |
|---|---|---|---|---|
| * Note that subtypes of clinical samples were confirmed by nucleotide sequencing. | | | | |

| **Table 16:** Comparative sensitivity tests for influenza A virus detection between real-time RT-PCR and Directigen Flu A | | | | |
|---|---|---|---|---|
| **Virus dilutions** | **Directigen Flu A** | | **Real-time RT-PCR of M (Ct)** | |
| | **A/canine/242/0 3** | **A/Wyoming/3/03** | **A/canine/242/03** | **A/Wyoming/3/2003** |
| 10⁻¹ | ++ | ++++ | 22.42 | 19.48 |
| 10⁻² | + | +++ | 25.85 | 22.66 |
| 10⁻³ | - | - | 29.27 | 25.76 |
| 10⁻⁴ | Not done | Not done | 32.66 | 28.66 |
| 10⁻⁵ | Not done | Not done | 35.48 | 33.14 |
| 10⁻⁶ | Not done | Not done | 37.51 | 35.06 |
| 10⁻⁷ | Not done | Not done | 39.09 | 36.44 |
| 10⁻⁸ | Not done | Not done | No Ct | 38.93 |

| **Table 17.** | |
|---|---|
| Class of Amino Acid | Examples of Amino Acids |
| Nonpolar | Ala, Val, Leu, Ile, Pro, Met, Phe, Trp |
| Uncharged Polar | Gly, Ser, Thr, Cys, Tyr, Asn, Gln |
| Acidic | Asp, Glu |
| Basic | Lys, Arg, His |

| **Table 18.** | | | |
|---|---|---|---|
| **Letter Symbol** | **Amino Acid** | **Letter Symbol** | **Amino Acid** |
| A | Alanine | M | Methionine |
| B | Asparagine or aspartic acid | N | Asparagine |
| C | Cysteine | P | Proline |
| D | Aspartic Acid | Q | Glutamine |
| E | Glutamic Acid | R | Arginine |
| F | Phenylalanine | S | Serine |
| G | Glycine | T | Threonine |
| H | Histidine | V | Valine |
| I | Isoleucine | W | Tryptophan |
| K | Lysine | Y | Tyrosine |
| L | Leucine | Z | Glutamine or glutamic acid |

| **Table 19.** Amino acid differences between PB2 proteins of H3N8 equine and canine influenza viruses | | | |
|---|---|---|---|
| Position | Equine Consensus * | Canine/FL/03 | Canine/FL/04 |
| 5 | K | K | E |
| 12 | S | L | L |
| 37 | G | G | E |
| 175 | R | R | I |
| 374 | L | I | I |
| 375 | R | R | K |
| 447 | Q | Q | H |

| **Table 20.** Amino acid differences between PB1 proteins of H3N8 equine and canine influenza viruses | | | |
|---|---|---|---|
| Position | Equine Consensus * | Canine/FL/03 | Canine/FL/04 |
| 114 | V | I | I |
| 154 | D | G | G |
| 221 | A | T | T |
| 317 | M | I | I |
| 459 | I | I | V |
| 682 | I | I | V |

| **Table 21.** Amino acid differences between PA proteins of H3N8 equine and canine influenza viruses | | | |
|---|---|---|---|
| Position | Equine Consensus * | Canine/FL/03 | Canine/FL/04 |
| 27 | D | N | N |
| 62 | I | V | V |
| 213 | R | K | K |
| 337 | A | T | T |
| 343 | A | E | E |
| 345 | L | I | I |
| 353 | K | R | R |
| 400 | T | T | A |
| 450 | V | I | I |
| 460 | M | M | I |
| 673 | R | R | K |
| 675 | N | D | D |

| | | | |
|---|---|---|---|
| *Based on available genes of viruses isolated between 1963 and 1998. | | | |

| **Table 22.** Amino acid differences between NP proteins of H3N8 equine and canine influenza viruses | | | |
|---|---|---|---|
| Position | Equine Consensus * | Canine/FL/03 | Canine/FL/04 |
| 16 | G | D | D |
| 157 | A | T | T |
| 214 | R | R | K |
| 285 | V | V | I |
| 286 | A | T | T |
| 359 | A | T | T |
| 375 | D | D | N |
| 384 | R | K | K |
| 452 | R | K | K |

| **Table 23.** Amino acid differences between NA proteins of H3N8 equine and canine influenza viruses | | | |
|---|---|---|---|
| Position | Equine Consensus * | Canine/FL/03 | Canine/FL/04 |
| 9 | A/T | T | A |
| 12 | S | F | F |
| 20 | L | I | I |
| 40 | G | R | R |
| 42 | G | D | D |
| 46 | N | K | K |
| 52 | E | E | K |
| 61 | R | K | K |
| 69 | N | S | S |
| 72 | E | K | K |
| 201 | V | I | I |
| 261 | I | V | V |
| 301 | I | I | V |
| 396 | N | D | D |
| 397 | L | P | P |

| **Table 24.** Amino acid differences between M1 proteins of H3N8 equine and canine influenza viruses | | | |
|---|---|---|---|
| Position | Equine Consensus * | Canine/FL/03 | Canine/FL/04 |
| M1 161 | S | S | A |
| M1 208 | K/Q | R | R |

| | | | |
|---|---|---|---|
| *Based on available genes of viruses isolated between 1963 and 1998. | | | |

| **Table 25.** Amino acid differences between NS1 proteins of H3N8 equine and canine influenza viruses | | | |
|---|---|---|---|
| Position | Equine Consensus * | Canine/FL/03 | Canine/FL/04 |
| 44 | K | R | R |
| 59 | R | H | H |
| 71 | E | K | K |
| 86 | A | T | T |
| 88 | R | R | L |
| 140 | R | G | G |
| 216 | P | S | S |

| | | | |
|---|---|---|---|
| * Based on available genes of viruses isolated between 1963 and 1998. | | | |

### REFERENCES

U.S. Patent No. 5,106,739
U.S. Patent No. 5,034,322
U.S. Patent No. 6,455,760
U.S. Patent No. 6,696,623
U.S. Patent No. 4,683,202
U.S. Patent No. 4,683,195
U.S. Patent No. 4,800,159
U.S. Patent No. 4,965,188
U.S. Patent No. 5,994,056
U.S. Patent No. 6,814,934
Published U.S. Application No. 20040078841
Published U.S. Application No. 20040067506
Published U.S. Application No. 20040019934
Published U.S. Application No. 20030177536
Published U.S. Application No. 20030084486
Published U.S. Application No. 20040123349
Greyhound Daily News, 1/28/99. National Greyhound Association (NGA), Abilene, Kansas. http://www.NGAgreyhounds.com.
Personal communication, Dr. William Duggar, veterinarian at Palm Beach Kennel Club, West Palm Beach, Florida.
Altschul, S. F. et al. (1990) "Basic Local Alignment Search Tool" J. Mol. Biol. 215:402-410.
Altschul, S. F. et al. (1997) "Gapped BLAST and PSI-BLAST: A New Generation of Protein Database Search Programs" Nucl. Acids Res. 25:3389-3402.
An, G. (1987) "Binary Ti vectors for plant transformation and promoter analysis" Methods Enzymol. 153:292-305.
Beltz, G. A., Jacobs, K. A., Eickbush, T. H., Cherbas, P. T., Kafatos, F. C. (1983) "Isolation of multigene families and determination of homologies by filter hybridization methods" Methods of Enzymology, R. Wu, L. Grossman and K. Moldave [eds.] Academic Press, New York 100:266-285.
Burleson, F. et al. (1992) Virology: A Laboratory Manual (Academic Press).
Byars, N.E., A.C. Allison (1987) "Adjuvant formulation for use in vaccines to elicit both cell-mediated and humoral immunity" Vaccine 5:223-228.
Crawford, P.C. et al. (2005) "Transmission of equine influenza virus to dogs" Science 310:482-485.
Chang, C.P. et al. (1976) "Influenza virus isolations from dogs during a human epidemic in Taiwan" Int J Zoonoses 3:61-64.
Dacso, C.C. et al. (1984) "Sporadic occurrence of zoonotic swine influenza virus infections" J Clin Microbiol 20:833-835.
de Boer, H. A., Comstock, L. J., Vasser, M. (1983) "The tac promoter: a functional hybrid derived from the trp and lac promoters" Proc. Natl. Acad. Sci. USA 80(1):21-25.
Felsenstein, J. (1989) Cladistics 5:164.
Fields et al. (1946) Fields Virology, 3rd ed., Lippincott-Raven publishers.
Ford, R.B., Vaden, S.L. (1998) "Canine infectious tracheobronchitis" In Infectious Diseases of the Dog and Cat, 2nd edition, C.E. Greene, editor, W.B. Saunders Co., Philadelphia, PA, pp. 33-38.
Fouchier et al., (2000) Journal of Clinical Microbiology 38 (11):4096-4101.
Good, X. et al. (1994) "Reduced ethylene synthesis by transgenic tomatoes expressing S-adenosylmethionine hydrolase" Plant Molec. Biol. 26:781-790.
Guan, Y. et al. (2004) "H5N1 influenza: a protean pandemic threat" Proc Natl Acad Sci U S A 101:8156-8161.
Guo, Y. et al. (1992) "Characterization of a new avian-like influenza A virus from horses in China" Virology 188:245-255.
Houser, R.E. et al. (1980) "Evidence of prior infection with influenza A/Texas/77 (H3N2) virus in dogs with clinical parainfluenza" Can J Comp Med 44:396-402.
Karasin, A.I. et al. (2000) "Isolation and characterization of H4N6 avian influenza viruses from pigs with pneumonia in Canada" J Virol 74:9322-9327.
Karlin S. and Altschul, S. F. (1990) "Methods for Assessing the Statistical Significance of Molecular Sequence Features by Using General Scoring Schemes" Proc. Natl. Acad. Sci. USA 87:2264-2268.
Karlin S. and Altschul, S. F. (1993) "Applications and Statistics for Multiple High-Scoring Segments in Molecular Sequences" Proc. Natl. Acad. Sci. USA 90:5873-5877.
Kawaoka, Y. et al., (1989) "Avian-to-human transmission of the PB1 gene of influenza A viruses in the 1957 and 1968 pandemics" J Virol 63:4603-4608.
Keawcharoen, J. et al. (2004) "Avian influenza H5N1 in tigers and leopards" Emerg Infect Dis 10:2189-2191.
Kendal, A. P. et al. (1982) In Concepts and Procedures for Laboratory-based Influenza Surveillance. A. P. Kendal, M. S. Pereira, J. J. Skehel, Eds. (U.S. Department of Health and Human Services, Centers for Disease Control and Prevention and Pan-American Health Organization, Atlanta, GA, United States) pp. B17-B35.
Kilbourne, E.D. et al. (1975) "Demonstration of antibodies to both hemagglutinin and neuraminidase antigens of H3N2 influenza A virus in domestic dogs" Intervirology 6:315-318.
Kimura, K. et al. (1998) "Fatal case of swine influenza virus in an immunocompetent host" Mayo Clin Proc 73:243-245.
Klimov, A. I. et al. (1992a) "Sequence changes in the live attenuated, cold-adapted variants of influenza A/Leningrad/134/57 (H2N2) virus" Virology 186:795-797.
Klimov A. et al. (1992b) "Subtype H7 influenza viruses: comparative antigenic and molecular analysis of the HA-, M-, and NS-genes" Arch Virol. 122:143-161.
Kovacova, A. et al. (2002) "Sequence similarities and evolutionary relationships of influenza virus A hemagglutinins" Virus Genes 24:57-63.
Kuiken, T. et al. (2004) "Avian H5N1 influenza in cats" Science 306:241.
Kumar, S. et al. (2004) "MEGA3: Integrated software for Molecular Evolutionary Genetics Analysis and sequence alignment" Brief Bioinform 5:150-163.
Lee, L.G. et al. (1993) "Allelic discrimination by nick-translation PCR with fluorogenic probes" Nucleic Acids Res. 21(16):3761-3766.
Lewin, B. (1985) Genes II, John Wiley & Sons, Inc., p. 96.
Lipatov, A.S. et al. (2004) "Influenza: emergence and control" J Virol 78:8951-8959.
Livak, K. J. et al. (1995) "Oligonucleotides with fluorescent dyes at opposite ends provide a quenched probe system useful for detecting PCR product and nucleic acid hybridization" PCR Methods Appl. 4(6):357-362.
Maniatis, T., E.F. Fritsch, J. Sambrook (1982) "Nuclease Bal31" Molecular Cloning: A Laboratory Manual, Cold Spring Harbor Laboratory, Cold Spring Harbor, NY.
Matrosovich, M. et al. (2000) "Early alterations of the receptor-binding properties of H1, H2, and H3 avian influenza virus hemagglutinins after their introduction into mammals" J Virol 74:8502-8512.
Maertzdorf et al., (2004) Clin Microbiol. 42(3):981-986.
Merrifield, R.B. (1963) "Solid Phase Peptide Synthesis. I. The Synthesis of a Tetrapeptide" J. Amer. Chem. Soc. 85:2149-2154.
Nikitin, A. et al. (1972) "Epidemiological studies of A-Hong Kong-68 virus infection in dogs" Bull World Health Organ 47:471-479.
Nobusawa, E. et al. (1991) "Comparison of complete amino acid sequences and receptor-binding properties among 13 serotypes of hemagglutinins of influenza A viruses" Virology 182:475-485.
Patriarca, P.A. et al. (1984) "Lack of significant person-to person spread of swine influenza-like virus following fatal infection in an immunocompromised child" Am J Epidemiol 119:152-158.
Payungporn S. et al. (2006a) "Detection of canine influenza A virus (H3N8) RNA by real-time RT-PCR" (in preparation for Journal of Clinical Microbiology).
Payungporn S, et al. (2006b) "Isolation and characterization of influenza A subtype H3N8 viruses from dogs with respiratory disease in a shelter and veterinary clinic in Florida" (in preparation for Emerging Infectious Diseases).
Peiris, M. et al. (1999) "Human infection with influenza H9N2" Lancet 354:916-917.
Peiris, J.S. et al. (2004) "Re-emergence of fatal human influenza A subtype H5N1 disease" Lancet 363:617-619.
Posnett, D. N. et al. (1988) "A Novel Method for Producing Anti-peptide Antibodies" J. Biol. Chem. 263(4):1719-1725.
Putnam, Bob (February 10, 1999) "Two illnesses seen in death of dogs" St. Petersburg Times*.*
Reid, A.H. et al. (2004) "Evidence of an absence: the genetic origins of the 1918 pandemic influenza virus" Nat Rev Microbiol 2:909-914.
Rowe, T. et al. (1999) "Detection of antibody to avian influenza A (H5N1) virus in human serum by using a combination of serologic assays" J Clin Microbiol 37: 937-943.
Saiki, R. (1985) "Enzymatic amplification of beta-globin genomic sequences and restriction site analysis for diagnosis of sickle cell anemia" Science 230:1350-1354.
Sambrook, J. et al. (1989) "Plasmid Vectors" In: Molecular Cloning: A Laboratory Manual, 2d Edition, pp. 1.82-1.104. Cold Spring Harbor Laboratory Press, Cold Spring Harbor, New York.
Subbarao, K. et al. (1998) "Characterization of an avian influenza A (H5N1) virus isolated from a child with a fatal respiratory illness" Science 279:393-396.
Suzuki, Y. et al. (2000) "Sialic acid species as a determinant of the host range of influenza A viruses" J Virol 74:11825-11831.
Tam, J. P. (1988) "Synthetic Peptide Vaccine Design: Synthesis and Properties of a High-Density Multiple Antigenic Peptide System" PNAS USA 85(15):5409-5413.
Top, Jr., F.H. et al. (1977) "Swine influenza A at Fort Dix, New Jersey (January-February 1976). IV. Summary and speculation" J Infect Dis 136 Suppl:S376-S380.
Vines, A. et al. (1998) "The role of influenza A virus hemagglutinin residues 226 and 228 in receptor specificity and host range restriction" J Virol 72:7626-7631.
Wagner, R. et al. (2002) "N-Glycans attached to the stem domain of haemagglutinin efficiently regulate influenza A virus replication" J Gen Virol 83:601-609.
Webby, R. et al. (2004) "Molecular constraints to interspecies transmission of viral pathogens" Nat Med 10:S77-S81.
Webster, R.G. (1998) "Influenza: an emerging disease" Emerg Infect Dis. 4:436-441.
Webster, R.G. et al. (1992) "Evolution and ecology of influenza A viruses" Microbiol Rev. 56 :152-179.
Weis, W. et al. (1988) "Structure of the influenza virus haemagglutinin complexed with its receptor, sialic acid" Nature 333:426-431.
Womble, D.D. (2000) "GCG: The Wisconsin Package of sequence analysis programs" Methods Mol Biol 132:3-22.
Xu, D., McElroy, D., Thornburg, R. W., Wu, R. et al. (1993) "Systemic induction of a potato pin2 promoter by wounding, methyl jasmonate, and abscisic acid in transgenic rice plants" Plant Molecular Biology 22:573-588.
Yang, T. T. et al. (1996) "Optimized Codon Usage and Chromophore Mutations Provide Enhanced Sensitivity with the Green Fluorescent Protein" Nucleic Acid Research 24(22):4592-4593.
Yoon K-Y. et al. (2005) "Influenza virus infection in racing greyhounds" Emerg Infect Dis. 11:1974-1975.

## Claims

1. An isolated canine influenza A virus, the influenza virus comprising a hemagglutinin (HA) polypeptide or a polynucleotide which encodes an HA polypeptide, wherein the HA polypeptide comprises the amino acid sequence shown in SEQ ID NO: 78, or a mature sequence thereof wherein the N-terminus amino acid signal sequence of a full-length sequence has been removed.

2. The influenza virus according to claim 1, wherein the influenza virus has an HA serotype of H3.

3. The influenza virus according to claim 1, wherein the influenza virus has the serotype H3N8.

4. The influenza virus according to claim 1, wherein the influenza virus comprises a polynucleotide having the nucleotide sequence shown in SEQ ID NO: 77.

5. The influenza virus according to claim 1, further comprising a polypeptide or a polynucleotide which encodes a polypeptide, wherein the polypeptide comprises the amino acid sequence shown in any of SEQ ID NOs: 64, 66, 68, 70, 72, 74, or 76.

6. The influenza virus according to claim 1, wherein the influenza virus is provided in a physiologically acceptable carrier or buffer.

7. The influenza virus according to any preceding claim, wherein the influenza virus is attenuated or inactivated.

8. A polynucleotide expression construct comprising regulatory elements and a polynucleotide which comprises a canine influenza A virus polynucleotide selected from: (i) a polynucleotide which encodes the HA polypeptide comprising the amino acid sequence shown in SEQ ID NO: 78; (ii) a polynucleotide which encodes a mature sequence of the HA polypeptide comprising the amino acid sequence shown in SEQ ID NO: 78 wherein the N-terminus amino acid signal sequence of a full-length sequence has been removed; and (iii) a polynucleotide having the nucleotide sequence shown in SEQ ID NO: 77.

9. An isolated antibody that binds specifically to a canine influenza A virus polypeptide selected from: (i) an HA polypeptide comprising the amino acid sequence shown in SEQ ID NO: 78 and (ii) a mature sequence of the HA polypeptide comprising the amino acid sequence shown in SEQ ID NO: 78 wherein the N-terminus amino acid signal sequence of a full-length sequence has been removed.

10. An isolated, in vitro cell comprising an influenza virus of any of claims 1 to 7 or a polynucleotide which comprises a canine influenza A virus polynucleotide selected from: (i) a polynucleotide which encodes the HA polypeptide comprising the amino acid sequence shown in SEQ ID NO: 78; (ii) a polynucleotide which encodes a mature sequence of the HA polypeptide comprising the amino acid sequence shown in SEQ ID NO: 78 wherein the N-terminus amino acid signal sequence of a full-length sequence has been removed; and (iii) a polynucleotide having the nucleotide sequence shown in SEQ ID NO: 77.

11. A reassortant virus comprising a canine influenza A virus polynucleotide selected from: (i) a polynucleotide which encodes the HA polypeptide comprising the amino acid sequence shown in SEQ ID NO: 78; (ii) a polynucleotide which encodes a mature sequence of the HA polypeptide comprising the amino acid sequence shown in SEQ ID NO: 78 wherein the N-terminus amino acid signal sequence of a full-length sequence has been removed; and (iii) a polynucleotide having the nucleotide sequence shown in SEQ ID NO: 77.

12. A recombinant viral vector or recombinant polynucleotide vector comprising an isolated polynucleotide which comprises a canine influenza A virus polynucleotide selected from: (i) a polynucleotide which encodes the HA polypeptide comprising the amino acid sequence shown in SEQ ID NO: 78; (ii) a polynucleotide which encodes a mature sequence of the HA polypeptide comprising the amino acid sequence shown in SEQ ID NO: 78 wherein the N-terminus amino acid signal sequence of a full-length sequence has been removed; and (iii) a polynucleotide having the nucleotide sequence shown in SEQ ID NO: 77.

13. The vector according to claim 12, wherein the viral vector is from adenovirus, avipox, herpesvirus, vaccinia virus, canarypox, entomopox, swinepox, or West Nile virus.

14. The recombinant viral vector according to claim 12, wherein the viral vector is a canarypox viral vector.

15. The polynucleotide vector according to claim 12, wherein the polynucleotide vector is a viral vector.

16. A composition comprising an immunogen of an influenza virus of any of claims 1 to 7, optionally further comprising an adjuvant, wherein the immunogen is capable of inducing an immune response against an influenza virus that is capable of infecting a canid animal and wherein the immunogen comprises a canine influenza virus as defined in any of claims 1 to 7, or a canine influenza virus polynucleotide which comprises a canine influenza A virus polynucleotide selected from: (i) a polynucleotide which encodes the HA polypeptide comprising the amino acid sequence shown in SEQ ID NO: 78; (ii) a polynucleotide which encodes a mature sequence of the HA polypeptide comprising the amino acid sequence shown in SEQ ID NO: 78 wherein the N-terminus amino acid signal sequence of a full-length sequence has been removed; and (iii) a polynucleotide having the nucleotide sequence shown in SEQ ID NO: 77, or a polynucleotide expression construct as defined in claim 8, or a polypeptide comprising a canine influenza A virus polypeptide selected from: (i) an HA polypeptide comprising the amino acid sequence shown in SEQ ID NO: 78; and (ii) a mature sequence of the HA polypeptide comprising the amino acid sequence shown in SEQ ID NO: 78 wherein the N-terminus amino acid signal sequence of a full-length sequence has been removed, or a reassortant virus as defined in claim 11, or a vector as defined in any of claims 12 to 15, wherein the composition is provided in a physiologically acceptable carrier or buffer.

17. A canine influenza vaccine for use in inducing an immune response in a canid animal against a canine influenza virus, wherein the vaccine comprises an influenza virus of any of claims 1 to 7, or a polynucleotide expression construct of claim 8, or an isolated polypeptide comprising a canine influenza A virus polypeptide selected from: (i) an HA polypeptide comprising the amino acid sequence shown in SEQ ID NO: 78; and (ii) a mature sequence of the HA polypeptide comprising the amino acid sequence shown in SEQ ID NO: 78 wherein the N-terminus amino acid signal sequence of a full-length sequence has been removed, or a reassortant virus of claim 11, or a vector of any of claims 12 to 15, or a composition of claim 16.

18. The vaccine for use according to claim 17, wherein the vaccine comprises a pharmaceutically acceptable carrier or diluent.

19. The vaccine for use according to claim 17, wherein the canid animal is a domesticated dog.

20. The vaccine for use according to claim 17, wherein the vaccine further comprises an adjuvant.

21. The vaccine for use according to claim 17, wherein the vaccine is formulated to be parenterally administrable.

22. The vaccine for use according to claim 21, wherein the vaccine is formulated to be subcutaneously, intraperitoneally, or intramuscularly administrable.

23. The vaccine for use according to claim 17, wherein the vaccine is formulated to be nasally or orally administrable.

## Patentansprüche

1. Isoliertes Hundegrippe-A-Virus, wobei das Grippevirus ein Hämagglutinin-(HA)-Polypeptid oder ein Polynukleotid umfasst, das ein HA-Polypeptid kodiert, wobei das HA-Polypeptid die Aminosäuresequenz gemäß SEQ ID Nr. 78 oder eine reife Sequenz davon umfasst, wobei die N-terminale Aminosäuresignalsequenz einer Volllängensequenz entfernt wurde.

2. Grippevirus nach Anspruch 1, wobei das Grippevirus einen HA-Serotyp H3 hat.

3. Grippevirus nach Anspruch 1, wobei das Grippevirus den Serotyp H3N8 hat.

4. Grippevirus nach Anspruch 1, wobei das Grippevirus ein Polynukleotid mit der Nukleotidsequenz gemäß SEQ ID Nr. 77 umfasst.

5. Grippevirus nach Anspruch 1, das ferner ein Polypeptid oder Polynukleotid, das ein Polypeptid kodiert, umfasst, wobei das Polypeptid die Aminosäuresequenz gemäß SEQ ID Nr. 64, 66, 68, 70, 72, 74 oder 76 umfasst.

6. Grippevirus nach Anspruch 1, wobei das Grippevirus in einem physiologisch akzeptablen Träger oder Puffer bereitgestellt wird.

7. Grippevirus nach einem vorherigen Anspruch, wobei das Grippevirus attenuiert oder inaktiviert ist.

8. Polynukleotidexpressionskonstrukt, das regulatorische Elemente und ein Polynukleotid umfasst, das ein Hundegrippe-A-Virus-Polynukleotid umfasst, ausgewählt aus: (i) einem Polynukleotid, das das HA-Polypeptid kodiert, das die Aminosäuresequenz gemäß SEQ ID Nr. 78 umfasst; (ii) einem Polynukleotid, das eine reife Sequenz des HA-Polypeptids kodiert, das die Aminosäuresequenz gemäß SEQ ID Nr. 78 umfasst, wobei die N-terminale Aminosäuresignalsequenz einer Volllängensequenz entfernt wurde; und (iii) einem Polynukleotid mit der Nukleotidsequenz gemäß SEQ ID Nr. 77.

9. Isolierter Antikörper, der sich spezifisch an ein Hundegrippe-A-Virus-Polypeptid bindet, ausgewählt aus: (i) einem HA-Polypeptid, das die Aminosäuresequenz gemäß SEQ ID Nr. 78 umfasst, und (ii) einer reifen Sequenz des HA-Polypeptids, das die Aminosäuresequenz gemäß SEQ ID Nr. 78 umfasst, wobei die N-terminale Aminosäuresignalsequenz einer Volllängensequenz entfernt wurde.

10. Isolierte In-vitro-Zelle, die ein Grippevirus nach einem der Ansprüche 1 bis 7 oder ein Polynukleotid umfasst, das ein Hundegrippe-A-Virus-Polynukleotid umfasst, ausgewählt aus: (i) einem Polynukleotid, das das HA-Polypeptid kodiert, das die Aminosäuresequenz gemäß SEQ ID Nr. 78 umfasst; (ii) einem Polynukleotid, das eine reife Sequenz des HA-Polypeptids kodiert, das die Aminosäuresequenz gemäß SEQ ID Nr. 78 umfasst, wobei die N-terminale Aminosäuresignalsequenz einer Volllängensequenz entfernt wurde; und (iii) einem Polynukleotid mit der Nukleotidsequenz gemäß SEQ ID Nr. 77.

11. Reassortierendes Virus, das ein Hundegrippe-A-Virus-Polynukleotid umfasst, ausgewählt aus: (i) einem Polynukleotid, das das HA-Polypeptid kodiert, das die Aminosäuresequenz gemäß SEQ ID Nr. 78 umfasst; (ii) einem Polynukleotid, das eine reife Sequenz des HA-Polypeptids kodiert, das die Aminosäuresequenz gemäß SEQ ID Nr. 78 umfasst, wobei die N-terminale Aminosäuresignalsequenz einer Volllängensequenz entfernt wurde; und (iii) einem Polynukleotid mit der Nukleotidsequenz gemäß SEQ ID Nr. 77.

12. Rekombinanter viraler Vektor oder rekombinanter Polynukleotidvektor, der ein isoliertes Polynukleotid umfasst, das ein Hundegrippe-A-Virus-Polynukleotid umfasst, ausgewählt aus: (i) einem Polynukleotid, das das HA-Polypeptid kodiert, das die Aminosäuresequenz gemäß SEQ ID Nr. 78 umfasst; (ii) einem Polynukleotid, das eine reife Sequenz des HA-Polypeptids kodiert, das die Aminosäuresequenz gemäß SEQ ID Nr. 78 umfasst, wobei die N-terminale Aminosäuresignalsequenz einer Volllängensequenz entfernt wurde; und (iii) einem Polynukleotid mit der Nukleotidsequenz gemäß SEQ ID Nr. 77.

13. Vektor nach Anspruch 12, wobei der virale Vektor von Adenovirus, Vogelpocken, Herpesvirus, Kuhpockenvirus, Kanarienpocken, Entomopocken, Schweinepocken oder West-Nil-Virus stammt.

14. Rekombinanter viraler Vektor nach Anspruch 12, wobei der virale Vektor ein viraler Kanarienpocken-Vektor ist.

15. Polynukleotidvektor nach Anspruch 12, wobei der Polynukleotidvektor ein viraler Vektor ist.

16. Zusammensetzung, die ein Immunogen eines Grippevirus nach einem der Ansprüche 1 bis 7 umfasst und optional ferner einen Hilfsstoff umfasst, wobei das Immunogen eine Immunantwort gegen ein Grippevirus induzieren kann, das einen Caniden infizieren kann, und wobei das Immunogen Folgendes umfasst: ein Hundegrippevirus gemäß Definition in einem der Ansprüche 1 bis 7 oder ein Hundegrippevirus-Polynukleotid, das ein Hundegrippe-A-Virus-Polynukleotid umfasst, ausgewählt aus: (i) einem Polynukleotid, das das HA-Polypeptid kodiert, das die Aminosäuresequenz gemäß SEQ ID Nr. 78 umfasst; (ii) einem Polynukleotid, das eine reife Sequenz des HA-Polypeptids kodiert, das die Aminosäuresequenz gemäß SEQ ID Nr. 78 umfasst, wobei die N-terminale Aminosäuresignalsequenz einer Volllängensequenz entfernt wurde; und (iii) einem Polynukleotid mit der Nukleotidsequenz gemäß SEQ ID Nr. 77, oder ein Polynukleotidexpressionskonstrukt gemäß Definition in Anspruch 8 oder ein Polypeptid, das ein Hundegrippe-A-Virus-Polypeptid umfasst, ausgewählt aus: (i) einem HA-Polypeptid, das die Aminosäuresequenz gemäß SEQ ID Nr. 78 umfasst; und (ii) einer reifen Sequenz des HA-Polypeptids, das die Aminosäuresequenz gemäß SEQ ID Nr. 78 umfasst, wobei die N-terminale Aminosäuresignalsequenz einer Volllängensequenz entfernt wurde, oder ein reassortierendes Virus gemäß Definition in Anspruch 11 oder einen Vektor gemäß Definition in einem der Ansprüche 12 bis 15, wobei die Zusammensetzung in einem physiologisch akzeptablen Träger oder Puffer bereitgestellt wird.

17. Hundegrippeimpfstoff zur Verwendung beim Induzieren einer Immunantwort in einem Caniden gegen ein Hundegrippevirus, wobei der Impfstoff Folgendes umfasst: ein Grippevirus nach einem der Ansprüche 1 bis 7 oder ein Polynukleotidexpressionskonstrukt nach Anspruch 8 oder ein isoliertes Polypeptid, das ein Hundegrippe-A-Virus-Polypeptid umfasst, ausgewählt aus: (i) einem HA-Polypeptid, das die Aminosäuresequenz gemäß SEQ ID Nr. 78 umfasst; und (ii) einer reifen Sequenz des HA-Polypeptids, das die Aminosäuresequenz gemäß SEQ ID Nr. 78 umfasst, wobei die N-terminale Aminosäuresignalsequenz einer Volllängensequenz entfernt wurde, oder ein reassortierendes Virus nach Anspruch 11 oder einen Vektor nach einem der Ansprüche 12 bis 15 oder eine Zusammensetzung nach Anspruch 16.

18. Impfstoff zur Verwendung nach Anspruch 17, wobei der Impfstoff ein(en) pharmazeutisch akzeptablen/s Träger oder Verdünnungsmittel umfasst.

19. Impfstoff zur Verwendung nach Anspruch 17, wobei der Canid ein domestizierter Hund ist.

20. Impfstoff zur Verwendung nach Anspruch 17, wobei der Impfstoff ferner einen Hilfsstoff umfasst.

21. Impfstoff zur Verwendung nach Anspruch 17, wobei der Impfstoff so formuliert ist, dass er parenteral verabreicht werden kann.

22. Impfstoff zur Verwendung nach Anspruch 21, wobei der Impfstoff so formuliert ist, dass er subkutan, intraperitoneal oder intramuskulär verabreicht werden kann.

23. Impfstoff zur Verwendung nach Anspruch 17, wobei der Impfstoff so formuliert ist, dass er nasal oder oral verabreicht werden kann.

## Revendications

1. Virus de la grippe A canine isolé, le virus de la grippe comprenant un polypeptide d'hémagglutinine (HA) ou un polynucléotide codant un polypeptide HA, le polypeptide HA comprenant la séquence d'acides aminés présentée dans la SEQ ID N° 78, ou une séquence mature de celui-ci, la séquence signal d'acides aminés N-terminale d'une séquence complète ayant été retirée.

2. Virus de la grippe selon la revendication 1, dans lequel le virus de la grippe a un sérotype HA de H3.

3. Virus de la grippe selon la revendication 1, dans lequel le virus de la grippe a le sérotype H3N8.

4. Virus de la grippe selon la revendication 1, dans lequel le virus de la grippe comprend un polynucléotide ayant la séquence nucléotidique présentée dans la SEQ ID N° 77.

5. Virus de la grippe selon la revendication 1, comprenant en outre un polypeptide ou un polynucléotide qui code un polypeptide, le polypeptide comprenant la séquence d'acides aminés présentée dans l'une quelconque des SEQ ID N° : 64, 66, 68, 70, 72, 74 ou 76.

6. Virus de la grippe selon la revendication 1, dans lequel le virus de la grippe est fourni dans un véhicule ou tampon physiologiquement acceptable.

7. Virus de la grippe selon l'une quelconque des revendications précédentes, dans lequel le virus de la grippe est atténué ou inactivé.

8. Construction d'expression de polynucléotide comprenant des éléments régulateurs et un polynucléotide qui comprend un polynucléotide de virus de la grippe A canine, choisi parmi : (i) un polynucléotide qui code le polypeptide HA comprenant la séquence d'acides aminés présentée dans la SEQ ID N° 78 ; (ii) un polynucléotide qui code une séquence mature du polypeptide HA comprenant la séquence d'acides aminés présentée dans la SEQ ID N° 78, la séquence signal d'acides aminés N-terminale d'une séquence complète ayant été retirée ; et (iii) un polynucléotide ayant la séquence nucléotidique présentée dans la SEQ ID N° 77.

9. Anticorps isolé qui se lie spécifiquement à un polypeptide du virus de la grippe A canine, choisi parmi : (i) un polypeptide HA comprenant la séquence d'acides aminés présentée dans la SEQ ID N° 78 et (ii) une séquence mature du polypeptide HA comprenant la séquence d'acides aminés présentée dans la SEQ ID N° 78, la séquence signal d'acides aminés N-terminale d'une séquence complète ayant été retirée.

10. Cellule in vitro isolée comprenant un virus de la grippe de l'une quelconque des revendications 1 à 7 ou polynucléotide qui comprend un polynucléotide de virus de la grippe A canine choisi parmi : (i) un polynucléotide qui code le polypeptide HA comprenant la séquence d'acides aminés présentée dans la SEQ ID N° 78 ; (ii) un polynucléotide qui code une séquence mature du polypeptide HA comprenant la séquence d'acides aminés présentée dans la SEQ ID N° 78, la séquence signal d'acides aminés N-terminale d'une séquence complète ayant été retirée ; et (iii) un polynucléotide ayant la séquence nucléotidique présentée dans la SEQ ID N° 77.

11. Virus réassorti comprenant un polynucléotide de virus de la grippe A canine, choisi parmi : (i) un polynucléotide qui code le polypeptide HA comprenant la séquence d'acides aminés présentée dans la SEQ ID N° 78 ; (ii) un polynucléotide qui code une séquence mature du polypeptide HA comprenant la séquence d'acides aminés présentée dans la SEQ ID N° 78, la séquence signal d'acides aminés N-terminale d'une séquence complète ayant été retirée ; et (iii) un polynucléotide ayant la séquence nucléotidique présentée dans la SEQ ID N° 77.

12. Vecteur viral recombinant ou vecteur polynucléotidique recombinant comprenant un polynucléotide isolé qui comprend un polynucléotide de virus de la grippe A canine, choisi parmi : (i) un polynucléotide qui code le polypeptide HA comprenant la séquence d'acides aminés présentée dans la SEQ ID N° 78 ; (ii) un polynucléotide qui code une séquence mature du polypeptide HA comprenant la séquence d'acides aminés présentée dans la SEQ ID N° 78, la séquence signal d'acides aminés N-terminale d'une séquence complète ayant été retirée ; et (iii) un polynucléotide ayant la séquence nucléotidique présentée dans la SEQ ID N° 77.

13. Vecteur selon la revendication 12, dans lequel le vecteur viral provient de l'adénovirus, de l'avipox, du virus de l'herpès, du virus de la vaccine, de la variole du canari, de l'entomopox, de la variole du porc ou du virus du Nil occidental.

14. Vecteur viral recombinant selon la revendication 12, dans lequel le vecteur viral est un vecteur viral de la variole du canari.

15. Vecteur polynucléotidique selon la revendication 12, dans lequel le vecteur polynucléotidique est un vecteur viral.

16. Composition comprenant un immunogène d'un virus de la grippe selon l'une quelconque des revendications 1 à 7, comprenant éventuellement en outre un adjuvant, l'immunogène étant capable d'induire une réponse immunitaire contre un virus de la grippe capable d'infecter un canidé et l'immunogène comprenant un virus de la grippe canine tel que défini dans l'une quelconque des revendications 1 à 7, ou un polynucléotide de virus de la grippe canine qui comprend un polynucléotide du virus de la grippe A canine choisi parmi : (i) un polynucléotide qui code le polypeptide HA comprenant la séquence d'acides aminés présentée dans la SEQ ID N° 78 ; (ii) un polynucléotide qui code une séquence mature du polypeptide HA comprenant la séquence d'acides aminés présentée dans la SEQ ID N° 78, la séquence signal d'acides aminés N-terminale d'une séquence complète ayant été retirée ; et (iii) un polynucléotide ayant la séquence nucléotidique présentée dans la SEQ ID N° 77, ou une construction d'expression polynucléotidique telle que définie dans la revendication 8, ou un polypeptide comprenant un polypeptide du virus de la grippe A canine choisi parmi : (i) un polypeptide HA comprenant la séquence d'acides aminés présentée dans la SEQ ID N° 78 ; et (ii) une séquence mature du polypeptide HA comprenant la séquence d'acides aminés présentée dans la SEQ ID N° 78, la séquence signal d'acides aminés N-terminale d'une séquence complète ayant été retirée, ou un virus réassorti tel que défini dans la revendication 11 ou un vecteur tel que défini dans l'une quelconque des revendications 12 à 15, la composition étant fournie dans un véhicule ou tampon physiologiquement acceptable.

17. Vaccin de la grippe canine destiné à être utilisé pour induire une réponse immunitaire chez un canidé contre un virus de la grippe canine, le vaccin comprenant un virus de la grippe selon l'une quelconque des revendications 1 à 7, ou construction d'expression polynucléotidique de la revendication 8, ou polypeptide isolé comprenant un polypeptide du virus de la grippe A canine choisi parmi : (i) un polypeptide HA comprenant la séquence d'acides aminés présentée dans la SEQ ID N° 78 ; et (ii) une séquence mature du polypeptide HA comprenant la séquence d'acides aminés présentée dans la SEQ ID N° 78, la séquence signal d'acides aminés N-terminale d'une séquence complète ayant été retirée, ou un virus réassorti de la revendication 11, ou un vecteur selon l'une quelconque des revendications 12 à 15 ou une composition selon la revendication 16.

18. Vaccin à utiliser selon la revendication 17, dans lequel le vaccin comprend un véhicule ou diluant pharmaceutiquement acceptable.

19. Vaccin destiné à être utilisé selon la revendication 17, dans lequel le canidé est un chien domestique.

20. Vaccin à utiliser selon la revendication 17, dans lequel le vaccin comprend en outre un adjuvant.

21. Vaccin destiné à être utilisé selon la revendication 17, dans lequel le vaccin est formulé pour être administré par voie parentérale.

22. Vaccin destiné à être utilisé selon la revendication 21, dans lequel le vaccin est formulé pour être administrable par voie intramusculaire, intrapéritonéale ou sous-cutanée.

23. Vaccin à utiliser selon la revendication 17, dans lequel le vaccin est formulé pour être administré par voie orale ou nasale.


SEQUENCE LISTING
<110> University of Florida Research Foundation Inc. et al.
<120> Materials and Methods for Respiratory Disease Control in Canines
<130> P/53233.EP03
<150> US 60/673,443
   <151> 2005-04-21
<150> PCT/US02/015090
   <151> 2006-04-21
<150> EP 06769869
   <151> 2006-04-21
<160> 88
<170> PatentIn version 3.3
<210> 1
   <211> 2277
   <212> DNA
   <213> Influenza virus
<220>
   <221> CDS
   <222> (1)..(2277)
<400> 1
   atg gag aga ata gaa gaa ctg aga gat ctg atg tta caa tcc cgc acc         48
   Met Glu Arg Ile Glu Glu Leu Arg Asp Leu Met Leu Gln Ser Arg Thr
   1               5                   10                  15
   cgc gag ata cta aca aaa act act gtg gac cac atg gcc ata atc aag         96
   Arg Glu Ile Leu Thr Lys Thr Thr Val Asp His Met Ala Ile Ile Lys
               20                  25                  30
   aaa tac aca tca gaa aga caa gag aag aac cct gca ctt agg atg aaa        144
   Lys Tyr Thr Ser Glu Arg Gln Glu Lys Asn Pro Ala Leu Arg Met Lys
           35                  40                  45
   tgg atg atg gca atg aaa tac cca att aca gca gat aag agg ata atg        192
   Trp Met Met Ala Met Lys Tyr Pro Ile Thr Ala Asp Lys Arg Ile Met
       50                  55                  60
   gag atg att cct gag aga aat gaa cag ggt caa acc ctt tgg agc aaa        240
   Glu Met Ile Pro Glu Arg Asn Glu Gln Gly Gln Thr Leu Trp Ser Lys
   65                  70                  75                  80
   acg aac gat gct ggc tca gac cgc gta atg gta tca cct ctg gca gtg        288
   Thr Asn Asp Ala Gly Ser Asp Arg Val Met Val Ser Pro Leu Ala Val
                   85                  90                  95
   aca tgg tgg aat agg aat gga cca aca acg agc aca att cat tat cca        336
   Thr Trp Trp Asn Arg Asn Gly Pro Thr Thr Ser Thr Ile His Tyr Pro
               100                 105                 110
   aaa gtc tac aaa act tat ttt gaa aag gtt gaa aga ttg aaa cac gga        384
   Lys Val Tyr Lys Thr Tyr Phe Glu Lys Val Glu Arg Leu Lys His Gly
           115                 120                 125
   act ttt ggc ccc gtt cat ttt agg aat caa gtc aag ata aga cga aga        432
   Thr Phe Gly Pro Val His Phe Arg Asn Gln Val Lys Ile Arg Arg Arg
           130                 135                 140
   gtt gat gta aac cct ggt cac gcg gac ctc agt gcc aaa gaa gca caa      480
   Val Asp Val Asn Pro Gly His Ala Asp Leu Ser Ala Lys Glu Ala Gln
   145                 150                 155                 160
   gat gtg atc atg gaa gtt gtt ttc cca aat gaa gtg gga gcc ata att      528
   Asp Val Ile Met Glu Val Val Phe Pro Asn Glu Val Gly Ala Ile Ile
                   165                 170                 175
   cta aca tcg gaa tca caa cta aca ata acc aaa gag aaa aag gaa gaa      576
   Leu Thr Ser Glu Ser Gln Leu Thr Ile Thr Lys Glu Lys Lys Glu Glu
               180                 185                 190
   ctt cag gac tgc aaa att gct ccc ttg atg gta gca tac atg cta gaa      624
   Leu Gln Asp Cys Lys Ile Ala Pro Leu Met Val Ala Tyr Met Leu Glu
           195                 200                 205
   aga gag ttg gtc cga aaa aca agg ttc ctc cca gta gca ggc gga aca      672
   Arg Glu Leu Val Arg Lys Thr Arg Phe Leu Pro Val Ala Gly Gly Thr
       210                 215                 220
   agc agt gta tac att gaa gtg ttg cat ctg act cag gga aca tgc tgg      720
   Ser Ser Val Tyr Ile Glu Val Leu His Leu Thr Gln Gly Thr Cys Trp
   225                 230                 235                 240
   gag caa atg tac acc cca gga gga gaa gtt aga aac gat gat att gat      768
   Glu Gln Met Tyr Thr Pro Gly Gly Glu Val Arg Asn Asp Asp Ile Asp
                   245                 250                 255
   caa agt tta att att gca gcc cgg aac ata gtg aga aga gcg aca gta      816
   Gln Ser Leu Ile Ile Ala Ala Arg Asn Ile Val Arg Arg Ala Thr Val
               260                 265                 270
   tca gca gat cca cta gca tcc cta ctg gaa atg tgc cac agt aca cag      864
   Ser Ala Asp Pro Leu Ala Ser Leu Leu Glu Met Cys His Ser Thr Gln
           275                 280                 285
   att ggt gga ata agg atg gta gac atc ctt aag cag aat cca aca gag      912
   Ile Gly Gly Ile Arg Met Val Asp Ile Leu Lys Gln Asn Pro Thr Glu
       290                 295                 300
   gaa caa gct gtg gat ata tgc aaa gca gca atg gga ttg aga att agc      960
   Glu Gln Ala Val Asp Ile Cys Lys Ala Ala Met Gly Leu Arg Ile Ser
   305                 310                 315                 320
   tca tca ttc agc ttt ggt gga ttc acc ttc aaa aga aca agt gga tca     1008
   Ser Ser Phe Ser Phe Gly Gly Phe Thr Phe Lys Arg Thr Ser Gly Ser
                   325                 330                 335
   tca gtc aag aga gaa gaa gaa atg ctt acg ggc aac ctt caa aca ttg     1056
   Ser Val Lys Arg Glu Glu Glu Met Leu Thr Gly Asn Leu Gln Thr Leu
               340                 345                 350
   aaa ata aga gtg cat gag ggc tat gaa gaa ttc aca atg gtc gga aga     1104
   Lys Ile Arg Val His Glu Gly Tyr Glu Glu Phe Thr Met Val Gly Arg
           355                 360                 365
   aga gca aca gcc att atc aaa aag gca acc aga aga ttg att caa ttg     1152
   Arg Ala Thr Ala Ile Ile Lys Lys Ala Thr Arg Arg Leu Ile Gln Leu
       370                 375                 380
   ata gta agt ggg aga gat gaa caa tca att gct gaa gca ata att gta     1200
   Ile Val Ser Gly Arg Asp Glu Gln Ser Ile Ala Glu Ala Ile Ile Val
   385                 390                 395                 400
   gcc atg gtg ttt tcg caa gaa gat tgc atg ata aaa gca gtt cga ggc     1248
   Ala Met Val Phe Ser Gln Glu Asp Cys Met Ile Lys Ala Val Arg Gly
                   405                 410                 415
   gat ttg aac ttt gtt aat aga gca aat cag cgt ttg aac ccc atg cat      1296
   Asp Leu Asn Phe Val Asn Arg Ala Asn Gln Arg Leu Asn Pro Met His
               420                 425                 430
   caa ctc ttg agg cat ttc caa aaa gat gca aaa gtg ctt ttc cac aat      1344
   Gln Leu Leu Arg His Phe Gln Lys Asp Ala Lys Val Leu Phe His Asn
           435                 440                 445
   tgg gga att gaa ccc atc gac aat gta atg ggg atg att gga ata ttg      1392
   Trp Gly Ile Glu Pro Ile Asp Asn Val Met Gly Met Ile Gly Ile Leu
       450                 455                 460
   cct gac atg acc cca agc acc gag atg tca ttg aga gga gtg aga gtc      1440
   Pro Asp Met Thr Pro Ser Thr Glu Met Ser Leu Arg Gly Val Arg Val
   465                 470                 475                 480
   agc aaa atg gga gtg gat gag tac tcc agc act gag aga gtg gtg gtg      1488
   Ser Lys Met Gly Val Asp Glu Tyr Ser Ser Thr Glu Arg Val Val Val
                   485                 490                 495
   agc att gac cgt ttt tta aga gtt cgg gat caa agg gga aac ata cta      1536
   Ser Ile Asp Arg Phe Leu Arg Val Arg Asp Gln Arg Gly Asn Ile Leu
               500                 505                 510
   ctg tcc cct gaa gaa gtc agt gaa aca caa gga acg gaa aag ctg aca      1584
   Leu Ser Pro Glu Glu Val Ser Glu Thr Gln Gly Thr Glu Lys Leu Thr
           515                 520                 525
   ata att tat tcg tca tca atg atg tgg gag att aat ggt ccc gaa tca      1632
   Ile Ile Tyr Ser Ser Ser Met Met Trp Glu Ile Asn Gly Pro Glu Ser
       530                 535                 540
   gtg ttg gtc aat act tat caa tgg atc atc agg aac tgg gaa att gta      1680
   Val Leu Val Asn Thr Tyr Gln Trp Ile Ile Arg Asn Trp Glu Ile Val
   545                 550                 555                 560
   aaa att cag tgg tca cag gac ccc aca atg tta tac aat aag ata gaa      1728
   Lys Ile Gln Trp Ser Gln Asp Pro Thr Met Leu Tyr Asn Lys Ile Glu
                   565                 570                 575
   ttt gag cca ttc caa tcc ctg gtc cct agg gcc acc aga agc caa tac      1776
   Phe Glu Pro Phe Gln Ser Leu Val Pro Arg Ala Thr Arg Ser Gln Tyr
               580                 585                 590
   agc ggt ttc gta aga acc ctg ttt cag caa atg cga gat gta ctt gga      1824
   Ser Gly Phe Val Arg Thr Leu Phe Gln Gln Met Arg Asp Val Leu Gly
           595                 600                 605
   aca ttt gat act gct caa ata ata aaa ctc ctc cct ttt gcc gct gct      1872
   Thr Phe Asp Thr Ala Gln Ile Ile Lys Leu Leu Pro Phe Ala Ala Ala
       610                 615                 620
   cct ccg gaa cag agt agg atg cag ttc tct tct ttg act gtt aat gta      1920
   Pro Pro Glu Gln Ser Arg Met Gln Phe Ser Ser Leu Thr Val Asn Val
   625                 630                 635                 640
   aga ggt tcg gga atg agg ata ctt gta aga ggc aat tcc cca gtg ttc      1968
   Arg Gly Ser Gly Met Arg Ile Leu Val Arg Gly Asn Ser Pro Val Phe
                   645                 650                 655
   aac tac aat aaa gcc act aaa agg ctc aca gtc ctc gga aag gat gca      2016
   Asn Tyr Asn Lys Ala Thr Lys Arg Leu Thr Val Leu Gly Lys Asp Ala
               660                 665                 670
   ggt gcg ctt act gag gac cca gat gaa ggt acg gct gga gta gaa tct      2064
   Gly Ala Leu Thr Glu Asp Pro Asp Glu Gly Thr Ala Gly Val Glu Ser
           675                 680                 685
   gct gtt cta aga ggg ttt ctc att tta ggt aaa gag aac aag aga tat      2112
   Ala Val Leu Arg Gly Phe Leu Ile Leu Gly Lys Glu Asn Lys Arg Tyr
       690                 695                 700
   ggc cca gca cta agc atc aat gaa cta agc aaa ctt gca aaa ggg gag      2160
   Gly Pro Ala Leu Ser Ile Asn Glu Leu Ser Lys Leu Ala Lys Gly Glu
   705                 710                 715                 720
   aaa gcc aat gta cta att ggg caa ggg gac gta gtg ttg gta atg aaa      2208
   Lys Ala Asn Val Leu Ile Gly Gln Gly Asp Val Val Leu Val Met Lys
                   725                 730                 735
   cgg aaa cgt gac tct agc ata ctt act gac agc cag aca gcg acc aaa      2256
   Arg Lys Arg Asp Ser Ser Ile Leu Thr Asp Ser Gln Thr Ala Thr Lys
               740                 745                 750
   agg att cgg atg gcc atc aat                                          2277
   Arg Ile Arg Met Ala Ile Asn
           755
<210> 2
   <211> 759
   <212> PRT
   <213> Influenza virus
<400> 2
   Met Glu Arg Ile Glu Glu Leu Arg Asp Leu Met Leu Gln Ser Arg Thr
   1               5                   10                  15
   Arg Glu Ile Leu Thr Lys Thr Thr Val Asp His Met Ala Ile Ile Lys
               20                  25                  30
   Lys Tyr Thr Ser Glu Arg Gln Glu Lys Asn Pro Ala Leu Arg Met Lys
           35                  40                  45
   Trp Met Met Ala Met Lys Tyr Pro Ile Thr Ala Asp Lys Arg Ile Met
       50                  55                  60
   Glu Met Ile Pro Glu Arg Asn Glu Gln Gly Gln Thr Leu Trp Ser Lys
   65                  70                  75                  80
   Thr Asn Asp Ala Gly Ser Asp Arg Val Met Val Ser Pro Leu Ala Val
                   85                  90                  95
   Thr Trp Trp Asn Arg Asn Gly Pro Thr Thr Ser Thr Ile His Tyr Pro
               100                 105                 110
   Lys Val Tyr Lys Thr Tyr Phe Glu Lys Val Glu Arg Leu Lys His Gly
           115                 120                 125
   Thr Phe Gly Pro Val His Phe Arg Asn Gln Val Lys Ile Arg Arg Arg
       130                 135                 140
   Val Asp Val Asn Pro Gly His Ala Asp Leu Ser Ala Lys Glu Ala Gln
   145                 150                 155                 160
   Asp Val Ile Met Glu Val Val Phe Pro Asn Glu Val Gly Ala Ile Ile
   165 170 175
   Leu Thr Ser Glu Ser Gln Leu Thr Ile Thr Lys Glu Lys Lys Glu Glu
   180 185 190
   Leu Gln Asp Cys Lys Ile Ala Pro Leu Met Val Ala Tyr Met Leu Glu
   195 200 205
   Arg Glu Leu Val Arg Lys Thr Arg Phe Leu Pro Val Ala Gly Gly Thr
   210 215 220
   Ser Ser Val Tyr Ile Glu Val Leu His Leu Thr Gln Gly Thr Cys Trp
   225 230 235 240
   Glu Gln Met Tyr Thr Pro Gly Gly Glu Val Arg Asn Asp Asp Ile Asp
   245 250 255
   Gln Ser Leu Ile Ile Ala Ala Arg Asn Ile Val Arg Arg Ala Thr Val
   260 265 270
   Ser Ala Asp Pro Leu Ala Ser Leu Leu Glu Met Cys His Ser Thr Gln
   275 280 285
   Ile Gly Gly Ile Arg Met Val Asp Ile Leu Lys Gln Asn Pro Thr Glu
   290 295 300
   Glu Gln Ala Val Asp Ile Cys Lys Ala Ala Met Gly Leu Arg Ile Ser
   305 310 315 320
   Ser Ser Phe Ser Phe Gly Gly Phe Thr Phe Lys Arg Thr Ser Gly Ser
   325 330 335
   Ser Val Lys Arg Glu Glu Glu Met Leu Thr Gly Asn Leu Gln Thr Leu
   340 345 350
   Lys Ile Arg Val His Glu Gly Tyr Glu Glu Phe Thr Met Val Gly Arg
   355 360 365
   Arg Ala Thr Ala Ile Ile Lys Lys Ala Thr Arg Arg Leu Ile Gln Leu
   370 375 380
   Ile Val Ser Gly Arg Asp Glu Gln Ser Ile Ala Glu Ala Ile Ile Val
   385 390 395 400
   Ala Met Val Phe Ser Gln Glu Asp Cys Met Ile Lys Ala Val Arg Gly
   405 410 415
   Asp Leu Asn Phe Val Asn Arg Ala Asn Gln Arg Leu Asn Pro Met His
   420 425 430
   Gln Leu Leu Arg His Phe Gln Lys Asp Ala Lys Val Leu Phe His Asn
   435 440 445
   Trp Gly Ile Glu Pro Ile Asp Asn Val Met Gly Met Ile Gly Ile Leu
       450                 455                 460
   Pro Asp Met Thr Pro Ser Thr Glu Met Ser Leu Arg Gly Val Arg Val
   465                 470                 475                 480
   Ser Lys Met Gly Val Asp Glu Tyr Ser Ser Thr Glu Arg Val Val Val
                   485                 490                 495
   Ser Ile Asp Arg Phe Leu Arg Val Arg Asp Gln Arg Gly Asn Ile Leu
               500                 505                 510
   Leu Ser Pro Glu Glu Val Ser Glu Thr Gln Gly Thr Glu Lys Leu Thr
           515                 520                 525
   Ile Ile Tyr Ser Ser Ser Met Met Trp Glu Ile Asn Gly Pro Glu Ser
       530                 535                 540
   Val Leu Val Asn Thr Tyr Gln Trp Ile Ile Arg Asn Trp Glu Ile Val
   545                 550                 555                 560
   Lys Ile Gln Trp Ser Gln Asp Pro Thr Met Leu Tyr Asn Lys Ile Glu
                   565                 570                 575
   Phe Glu Pro Phe Gln Ser Leu Val Pro Arg Ala Thr Arg Ser Gln Tyr
               580                 585                 590
   Ser Gly Phe Val Arg Thr Leu Phe Gln Gln Met Arg Asp Val Leu Gly
           595                 600                 605
   Thr Phe Asp Thr Ala Gln Ile Ile Lys Leu Leu Pro Phe Ala Ala Ala
       610                 615                 620
   Pro Pro Glu Gln Ser Arg Met Gln Phe Ser Ser Leu Thr Val Asn Val
   625                 630                 635                 640
   Arg Gly Ser Gly Met Arg Ile Leu Val Arg Gly Asn Ser Pro Val Phe
                   645                 650                 655
   Asn Tyr Asn Lys Ala Thr Lys Arg Leu Thr Val Leu Gly Lys Asp Ala
               660                 665                 670
   Gly Ala Leu Thr Glu Asp Pro Asp Glu Gly Thr Ala Gly Val Glu Ser
           675                 680                 685
   Ala Val Leu Arg Gly Phe Leu Ile Leu Gly Lys Glu Asn Lys Arg Tyr
       690                 695                 700
   Gly Pro Ala Leu Ser Ile Asn Glu Leu Ser Lys Leu Ala Lys Gly Glu
   705                 710                 715                 720
   Lys Ala Asn Val Leu Ile Gly Gln Gly Asp Val Val Leu Val Met Lys
                   725                 730                 735
   Arg Lys Arg Asp Ser Ser Ile Leu Thr Asp Ser Gln Thr Ala Thr Lys
               740                 745                 750
   Arg Ile Arg Met Ala Ile Asn
           755
<210> 3
   <211> 2274
   <212> DNA
   <213> Influenza virus
<220>
   <221> CDS
   <222> (1)..(2274)
<400> 3
   atg gat gtc aat ccg act cta ctc ttc tta aag gtg cca gcg cag aat      48
   Met Asp Val Asn Pro Thr Leu Leu Phe Leu Lys Val Pro Ala Gln Asn
   1               5                   10                  15
   gct ata agc aca aca ttc cct tat act gga gat cct ccc tac agt cat      96
   Ala Ile Ser Thr Thr Phe Pro Tyr Thr Gly Asp Pro Pro Tyr Ser His
               20                  25                  30
   gga aca ggg aca gga tac acc atg gat act gtc aac aga aca cac caa     144
   Gly Thr Gly Thr Gly Tyr Thr Met Asp Thr Val Asn Arg Thr His Gln
           35                  40                  45
   tat tca gaa aaa ggg aaa tgg aca aca aac act gag att gga gca cca     192
   Tyr Ser Glu Lys Gly Lys Trp Thr Thr Asn Thr Glu Ile Gly Ala Pro
       50                  55                  60
   caa ctt aat cca atc gat gga cca ctt cct gaa gac aat gaa cca agc     240
   Gln Leu Asn Pro Ile Asp Gly Pro Leu Pro Glu Asp Asn Glu Pro Ser
   65                  70                  75                  80
   ggg tac gcc caa aca gat tgt gta ttg gaa gca atg gct ttc ctt gaa     288
   Gly Tyr Ala Gln Thr Asp Cys Val Leu Glu Ala Met Ala Phe Leu Glu
                   85                  90                  95
   gaa tcc cat cca gga atc ttt gaa aat tcg tgt ctt gaa acg atg gag     336
   Glu Ser His Pro Gly Ile Phe Glu Asn Ser Cys Leu Glu Thr Met Glu
               100                 105                 110
   gtg att cag cag aca aga gtg gac aaa cta aca caa ggc cga caa act     384
   Val Ile Gln Gln Thr Arg Val Asp Lys Leu Thr Gln Gly Arg Gln Thr
           115                 120                 125
   tat gat tgg acc ttg aat agg aat caa cct gcc gca aca gca ctt gct     432
   Tyr Asp Trp Thr Leu Asn Arg Asn Gln Pro Ala Ala Thr Ala Leu Ala
       130                 135                 140
   aat acg att gaa gta ttc aga tca aat ggt ctg act tcc aat gaa tcg     480
   Asn Thr Ile Glu Val Phe Arg Ser Asn Gly Leu Thr Ser Asn Glu Ser
   145                 150                 155                 160
   ggg aga ttg atg gac ttc ctc aaa gat gtc atg gag tcc atg aac aag     528
   Gly Arg Leu Met Asp Phe Leu Lys Asp Val Met Glu Ser Met Asn Lys
                   165                 170                 175
   gaa gaa atg gaa ata aca aca cac ttc caa cgg aag aga aga gta aga     576
   Glu Glu Met Glu Ile Thr Thr His Phe Gln Arg Lys Arg Arg Val Arg
               180                 185                 190
   gac aac atg aca aag aga atg gta aca cag aga acc ata ggg aag aaa      624
   Asp Asn Met Thr Lys Arg Met Val Thr Gln Arg Thr Ile Gly Lys Lys
           195                 200                 205
   aaa caa cga tta aac aga aag agc tat cta atc aga aca tta acc cta      672
   Lys Gln Arg Leu Asn Arg Lys Ser Tyr Leu Ile Arg Thr Leu Thr Leu
       210                 215                 220
   aac aca atg acc aag gac gct gag aga ggg aaa ttg aaa cga cga gca      720
   Asn Thr Met Thr Lys Asp Ala Glu Arg Gly Lys Leu Lys Arg Arg Ala
   225                 230                 235                     240
   atc gct acc cca ggg atg cag ata aga ggg ttt gta tat ttt gtt gaa      768
   Ile Ala Thr Pro Gly Met Gln Ile Arg Gly Phe Val Tyr Phe Val Glu
                   245                 250                 255
   aca cta gct cga aga ata tgt gaa aag ctt gaa caa tca gga ttg cca      816
   Thr Leu Ala Arg Arg Ile Cys Glu Lys Leu Glu Gln Ser Gly Leu Pro
               260                 265                 270
   gtt ggc ggt aat gag aaa aag gcc aaa ctg gct aat gtc gtc aga aaa      864
   Val Gly Gly Asn Glu Lys Lys Ala Lys Leu Ala Asn Val Val Arg Lys
           275                 280                 285
   atg atg act aat tcc caa gac act gaa ctc tcc ttc acc atc act ggg      912
   Met Met Thr Asn Ser Gln Asp Thr Glu Leu Ser Phe Thr Ile Thr Gly
       290                 295                 300
   gac aat acc aaa tgg aat gaa aat cag aac cca cgc ata ttc ctg gca      960
   Asp Asn Thr Lys Trp Asn Glu Asn Gln Asn Pro Arg Ile Phe Leu Ala
   305                 310                 315                     320
   atg atc aca tac ata act aga aac cag cca gaa tgg ttc aga aat gtt     1008
   Met Ile Thr Tyr Ile Thr Arg Asn Gln Pro Glu Trp Phe Arg Asn Val
                   325                 330                 335
   cta agc att gca ccg att atg ttc tca aat aaa atg gca aga ctg ggg     1056
   Leu Ser Ile Ala Pro Ile Met Phe Ser Asn Lys Met Ala Arg Leu Gly
               340                 345                 350
   aaa gga tat atg ttt gaa agc aaa agt atg aaa ttg aga act caa ata     1104
   Lys Gly Tyr Met Phe Glu Ser Lys Ser Met Lys Leu Arg Thr Gln Ile
           355                 360                 365
   cca gca gaa atg cta gca agc att gac cta aaa tat ttc aat gat tca     1152
   Pro Ala Glu Met Leu Ala Ser Ile Asp Leu Lys Tyr Phe Asn Asp Ser
       370                 375                 380
   aca aaa aag aaa att gag aag ata cga cca ctt ctg gtt gac ggg act     1200
   Thr Lys Lys Lys Ile Glu Lys Ile Arg Pro Leu Leu Val Asp Gly Thr
   385                 390                 395                     400
   gct tca ctg agt cct ggc atg atg atg gga atg ttc aac atg ttg agc     1248
   Ala Ser Leu Ser Pro Gly Met Met Met Gly Met Phe Asn Met Leu Ser
                   405                 410                 415
   act gtg ctg ggt gta tcc ata tta aac ctg ggc cag agg aaa tac aca     1296
   Thr Val Leu Gly Val Ser Ile Leu Asn Leu Gly Gln Arg Lys Tyr Thr
               420                 425                 430
   aag acc aca tac tgg tgg gat ggt ctg caa tca tcc gat gac ttt gct     1344
   Lys Thr Thr Tyr Trp Trp Asp Gly Leu Gln Ser Ser Asp Asp Phe Ala
           435                 440                 445
   ttg ata gtg aat gcg cct aat cat gaa gga gta caa gct gga gta gac     1392
   Leu Ile Val Asn Ala Pro Asn His Glu Gly Val Gln Ala Gly Val Asp
       450                 455                 460
   aga ttc tat aga act tgc aaa ctg gtc ggg atc aac atg agc aaa aag      1440
   Arg Phe Tyr Arg Thr Cys Lys Leu Val Gly Ile Asn Met Ser Lys Lys
   465                 470                 475                 480
   aag tcc tac ata aat aga act gga aca ttc gaa ttc aca agc ttt ttc      1488
   Lys Ser Tyr Ile Asn Arg Thr Gly Thr Phe Glu Phe Thr Ser Phe Phe
                   485                 490                 495
   tac cgg tat ggt ttt gta gcc aat ttc agc atg gaa cta ccc agt ttt      1536
   Tyr Arg Tyr Gly Phe Val Ala Asn Phe Ser Met Glu Leu Pro Ser Phe
               500                 505                 510
   ggg gtt tcc gga ata aat gaa tct gca gac atg agc att gga gtg aca      1584
   Gly Val Ser Gly Ile Asn Glu Ser Ala Asp Met Ser Ile Gly Val Thr
           515                 520                 525
   gtc atc aaa aac aac atg ata aat aat gat ctc ggt cct gcc acg gca      1632
   Val Ile Lys Asn Asn Met Ile Asn Asn Asp Leu Gly Pro Ala Thr Ala
       530                 535                 540
   caa atg gca ctc caa ctc ttc att aag gat tat cgg tac aca tac cgg      1680
   Gln Met Ala Leu Gln Leu Phe Ile Lys Asp Tyr Arg Tyr Thr Tyr Arg
   545                 550                 555                 560
   tgc cat aga ggt gat acc cag ata caa acc aga aga tct ttt gag ttg      1728
   Cys His Arg Gly Asp Thr Gln Ile Gln Thr Arg Arg Ser Phe Glu Leu
                   565                 570                 575
   aag aaa ctg tgg gaa cag act cga tca aag act ggt cta ctg gta tca      1776
   Lys Lys Leu Trp Glu Gln Thr Arg Ser Lys Thr Gly Leu Leu Val Ser
               580                 585                 590
   gat ggg ggt cca aac cta tat aac atc aga aac cta cac atc ccg gaa      1824
   Asp Gly Gly Pro Asn Leu Tyr Asn Ile Arg Asn Leu His Ile Pro Glu
           595                 600                 605
   gtc tgt tta aaa tgg gag cta atg gat gaa gat tat aag ggg agg cta      1872
   Val Cys Leu Lys Trp Glu Leu Met Asp Glu Asp Tyr Lys Gly Arg Leu
       610                 615                 620
   tgc aat cca ttg aat cct ttc gtt agt cac aaa gaa att gaa tca gtc      1920
   Cys Asn Pro Leu Asn Pro Phe Val Ser His Lys Glu Ile Glu Ser Val
   625                 630                 635                 640
   aac agt gca gta gta atg cct gcg cat ggc cct gcc aaa agc atg gag      1968
   Asn Ser Ala Val Val Met Pro Ala His Gly Pro Ala Lys Ser Met Glu
                   645                 650                 655
   tat gat gct gtt gca aca aca cat tct tgg atc ccc aag agg aac cgg      2016
   Tyr Asp Ala Val Ala Thr Thr His Ser Trp Ile Pro Lys Arg Asn Arg
               660                 665                 670
   tcc ata ttg aac aca agc caa agg gga gta ctc gaa gat gag cag atg      2064
   Ser Ile Leu Asn Thr Ser Gln Arg Gly Val Leu Glu Asp Glu Gln Met
           675                 680                 685
   tat cag aaa tgc tgc aac ctg ttt gaa aaa ttc ttc ccc agc agc tca      2112
   Tyr Gln Lys Cys Cys Asn Leu Phe Glu Lys Phe Phe Pro Ser Ser Ser
       690                 695                 700
   tac aga aga cca gtc gga att tct agt atg gtt gag gcc atg gtg tcc      2160
   Tyr Arg Arg Pro Val Gly Ile Ser Ser Met Val Glu Ala Met Val Ser
   705                 710                 715                 720
   agg gcc cgc att gat gca cga att gac ttc gaa tct gga cgg ata aag      2208
   Arg Ala Arg Ile Asp Ala Arg Ile Asp Phe Glu Ser Gly Arg Ile Lys
                   725                 730                 735
   aag gat gag ttc gct gag atc atg aag atc tgt tcc acc att gaa gag      2256
   Lys Asp Glu Phe Ala Glu Ile Met Lys Ile Cys Ser Thr Ile Glu Glu
               740                 745                 750
   ctc aga cgg caa aaa tag                                              2274
   Leu Arg Arg Gln Lys
           755
<210> 4
   <211> 757
   <212> PRT
   <213> Influenza virus
<400> 4
   Met Asp Val Asn Pro Thr Leu Leu Phe Leu Lys Val Pro Ala Gln Asn
   1               5                   10                  15
   Ala Ile Ser Thr Thr Phe Pro Tyr Thr Gly Asp Pro Pro Tyr Ser His
               20                  25                  30
   Gly Thr Gly Thr Gly Tyr Thr Met Asp Thr Val Asn Arg Thr His Gln
           35                  40                  45
   Tyr Ser Glu Lys Gly Lys Trp Thr Thr Asn Thr Glu Ile Gly Ala Pro
       50                  55                  60
   Gln Leu Asn Pro Ile Asp Gly Pro Leu Pro Glu Asp Asn Glu Pro Ser
   65                  70                  75                  80
   Gly Tyr Ala Gln Thr Asp Cys Val Leu Glu Ala Met Ala Phe Leu Glu
                   85                  90                  95
   Glu Ser His Pro Gly Ile Phe Glu Asn Ser Cys Leu Glu Thr Met Glu
               100                 105                 110
   Val Ile Gln Gln Thr Arg Val Asp Lys Leu Thr Gln Gly Arg Gln Thr
           115                 120                 125
   Tyr Asp Trp Thr Leu Asn Arg Asn Gln Pro Ala Ala Thr Ala Leu Ala
       130                 135                 140
   Asn Thr Ile Glu Val Phe Arg Ser Asn Gly Leu Thr Ser Asn Glu Ser
   145                 150                 155                 160
   Gly Arg Leu Met Asp Phe Leu Lys Asp Val Met Glu Ser Met Asn Lys
                   165                 170                 175
   Glu Glu Met Glu Ile Thr Thr His Phe Gln Arg Lys Arg Arg Val Arg
               180                 185                 190
   Asp Asn Met Thr Lys Arg Met Val Thr Gln Arg Thr Ile Gly Lys Lys
           195                 200                 205
   Lys Gln Arg Leu Asn Arg Lys Ser Tyr Leu Ile Arg Thr Leu Thr Leu
       210                 215                 220
   Asn Thr Met Thr Lys Asp Ala Glu Arg Gly Lys Leu Lys Arg Arg Ala
   225                 230                 235                 240
   Ile Ala Thr Pro Gly Met Gln Ile Arg Gly Phe Val Tyr Phe Val Glu
                   245                 250                 255
   Thr Leu Ala Arg Arg Ile Cys Glu Lys Leu Glu Gln Ser Gly Leu Pro
               260                 265                 270
   Val Gly Gly Asn Glu Lys Lys Ala Lys Leu Ala Asn Val Val Arg Lys
           275                 280                 285
   Met Met Thr Asn Ser Gln Asp Thr Glu Leu Ser Phe Thr Ile Thr Gly
       290                 295                 300
   Asp Asn Thr Lys Trp Asn Glu Asn Gln Asn Pro Arg Ile Phe Leu Ala
   305                 310                 315                 320
   Met Ile Thr Tyr Ile Thr Arg Asn Gln Pro Glu Trp Phe Arg Asn Val
                   325                 330                 335
   Leu Ser Ile Ala Pro Ile Met Phe Ser Asn Lys Met Ala Arg Leu Gly
               340                 345                 350
   Lys Gly Tyr Met Phe Glu Ser Lys Ser Met Lys Leu Arg Thr Gln Ile
           355                 360                 365
   Pro Ala Glu Met Leu Ala Ser Ile Asp Leu Lys Tyr Phe Asn Asp Ser
       370                 375                 380
   Thr Lys Lys Lys Ile Glu Lys Ile Arg Pro Leu Leu Val Asp Gly Thr
   385                 390                 395                 400
   Ala Ser Leu Ser Pro Gly Met Met Met Gly Met Phe Asn Met Leu Ser
                   405                 410                 415
   Thr Val Leu Gly Val Ser Ile Leu Asn Leu Gly Gln Arg Lys Tyr Thr
               420                 425                 430
   Lys Thr Thr Tyr Trp Trp Asp Gly Leu Gln Ser Ser Asp Asp Phe Ala
           435                 440                 445
   Leu Ile Val Asn Ala Pro Asn His Glu Gly Val Gln Ala Gly Val Asp
       450                 455                 460
   Arg Phe Tyr Arg Thr Cys Lys Leu Val Gly Ile Asn Met Ser Lys Lys
   465                 470                 475                 480
   Lys Ser Tyr Ile Asn Arg Thr Gly Thr Phe Glu Phe Thr Ser Phe Phe
   485 490 495
   Tyr Arg Tyr Gly Phe Val Ala Asn Phe Ser Met Glu Leu Pro Ser Phe
   500 505 510
   Gly Val Ser Gly Ile Asn Glu Ser Ala Asp Met Ser Ile Gly Val Thr
   515 520 525
   Val Ile Lys Asn Asn Met Ile Asn Asn Asp Leu Gly Pro Ala Thr Ala
   530 535 540
   Gln Met Ala Leu Gln Leu Phe Ile Lys Asp Tyr Arg Tyr Thr Tyr Arg
   545 550 555 560
   Cys His Arg Gly Asp Thr Gln Ile Gln Thr Arg Arg Ser Phe Glu Leu
   565 570 575
   Lys Lys Leu Trp Glu Gln Thr Arg Ser Lys Thr Gly Leu Leu Val Ser
   580 585 590
   Asp Gly Gly Pro Asn Leu Tyr Asn Ile Arg Asn Leu His Ile Pro Glu
   595 600 605
   Val Cys Leu Lys Trp Glu Leu Met Asp Glu Asp Tyr Lys Gly Arg Leu
   610 615 620
   Cys Asn Pro Leu Asn Pro Phe Val Ser His Lys Glu Ile Glu Ser Val
   625 630 635 640
   Asn Ser Ala Val Val Met Pro Ala His Gly Pro Ala Lys Ser Met Glu
   645 650 655
   Tyr Asp Ala Val Ala Thr Thr His Ser Trp Ile Pro Lys Arg Asn Arg
   660 665 670
   Ser Ile Leu Asn Thr Ser Gln Arg Gly Val Leu Glu Asp Glu Gln Met
   675 680 685
   Tyr Gln Lys Cys Cys Asn Leu Phe Glu Lys Phe Phe Pro Ser Ser Ser
   690 695 700
   Tyr Arg Arg Pro Val Gly Ile Ser Ser Met Val Glu Ala Met Val Ser
   705 710 715 720
   Arg Ala Arg Ile Asp Ala Arg Ile Asp Phe Glu Ser Gly Arg Ile Lys
   725 730 735
   Lys Asp Glu Phe Ala Glu Ile Met Lys Ile Cys Ser Thr Ile Glu Glu
   740 745 750
   Leu Arg Arg Gln Lys
   755
<210> 5
   <211> 2151
   <212> DNA
   <213> Influenza virus
<220>
   <221> CDS
   <222> (1)..(2151)
<400> 5
   atg gaa gac ttt gtg cga cag tgc ttc aat cca atg atc gtc gag ctt       48
   Met Glu Asp Phe Val Arg Gln Cys Phe Asn Pro Met Ile Val Glu Leu
   1               5                   10                  15
   gcg gaa aag gca atg aaa gaa tat gga gag aac ccg aaa atc gaa aca       96
   Ala Glu Lys Ala Met Lys Glu Tyr Gly Glu Asn Pro Lys Ile Glu Thr
               20                  25                  30
   aac aaa ttt gca gca ata tgc act cac ttg gaa gtc tgc ttc atg tac      144
   Asn Lys Phe Ala Ala Ile Cys Thr His Leu Glu Val Cys Phe Met Tyr
           35                  40                  45
   tcg gat ttt cac ttt att aat gaa ctg ggt gag tca gtg gtc ata gag      192
   Ser Asp Phe His Phe Ile Asn Glu Leu Gly Glu Ser Val Val Ile Glu
       50                  55                  60
   tct ggt gac cca aat gct ctt ttg aaa cac aga ttt gaa atc att gag      240
   Ser Gly Asp Pro Asn Ala Leu Leu Lys His Arg Phe Glu Ile Ile Glu
   65                  70                  75                  80
   ggg aga gat cga aca atg gca tgg aca gta gta aac agc atc tgc aac      288
   Gly Arg Asp Arg Thr Met Ala Trp Thr Val Val Asn Ser Ile Cys Asn
                   85                  90                  95
   acc aca aga gct gaa aaa cct aaa ttt ctt cca gat tta tac gac tat      336
   Thr Thr Arg Ala Glu Lys Pro Lys Phe Leu Pro Asp Leu Tyr Asp Tyr
               100                 105                 110
   aag gag aac aga ttt gtt gaa att ggt gtg aca agg aga gaa gtt cac      384
   Lys Glu Asn Arg Phe Val Glu Ile Gly Val Thr Arg Arg Glu Val His
           115                 120                 125
   ata tac tac ctg gag aag gcc aac aaa ata aag tct gag aaa aca cat      432
   Ile Tyr Tyr Leu Glu Lys Ala Asn Lys Ile Lys Ser Glu Lys Thr His
       130                 135                 140
   atc cac att ttc tca ttt aca gga gag gaa atg gct aca aaa gcg gac      480
   Ile His Ile Phe Ser Phe Thr Gly Glu Glu Met Ala Thr Lys Ala Asp
   145                 150                 155                 160
   tat act ctt gat gaa gag agt aga gcc agg atc aag acc aga cta ttc      528
   Tyr Thr Leu Asp Glu Glu Ser Arg Ala Arg Ile Lys Thr Arg Leu Phe
                   165                 170                 175
   acc ata aga caa gaa atg gcc agt aga ggc ctc tgg gat tcc ttt cgt      576
   Thr Ile Arg Gln Glu Met Ala Ser Arg Gly Leu Trp Asp Ser Phe Arg
               180                 185                 190
   cag tcc gag aga ggc gaa gag aca att gaa gaa aga ttt gaa ata aca      624
   Gln Ser Glu Arg Gly Glu Glu Thr Ile Glu Glu Arg Phe Glu Ile Thr
           195                 200                 205
   ggg acg atg cgc aag ctt gcc aat tac agt ctc cca ccg aac ttc tcc      672
   Gly Thr Met Arg Lys Leu Ala Asn Tyr Ser Leu Pro Pro Asn Phe Ser
       210                 215                 220
   agc ctt gaa aat ttt aga gtc tat gtg gat gga ttc gaa ccg aac ggc      720
   Ser Leu Glu Asn Phe Arg Val Tyr Val Asp Gly Phe Glu Pro Asn Gly
   225                 230                 235                 240
   tgc att gag agt aag ctt tct caa atg tcc aaa gaa gta aat gcc aga      768
   Cys Ile Glu Ser Lys Leu Ser Gln Met Ser Lys Glu Val Asn Ala Arg
                   245                 250                 255
   atc gaa cca ttt tca aag aca aca ccc cga cca ctc aaa atg cca ggt      816
   Ile Glu Pro Phe Ser Lys Thr Thr Pro Arg Pro Leu Lys Met Pro Gly
               260                 265                 270
   ggt cca ccc tgc cat cag cga tct aaa ttc ttg cta atg gat gct ctg      864
   Gly Pro Pro Cys His Gln Arg Ser Lys Phe Leu Leu Met Asp Ala Leu
           275                 280                 285
   aaa ctg agc att gag gac cca agt cac gag gga gag gga ata cca cta      912
   Lys Leu Ser Ile Glu Asp Pro Ser His Glu Gly Glu Gly Ile Pro Leu
       290                 295                 300
   tat gat gca atc aaa tgc atg aaa act ttc ttt gga tgg aaa gag ccc      960
   Tyr Asp Ala Ile Lys Cys Met Lys Thr Phe Phe Gly Trp Lys Glu Pro
   305                 310                 315                 320
   agt att gtt aaa cca cat gaa aag ggt ata aac ccg aac tat ctc caa     1008
   Ser Ile Val Lys Pro His Glu Lys Gly Ile Asn Pro Asn Tyr Leu Gln
                   325                 330                 335
   act tgg aag caa gta tta gaa gaa ata caa gac ctt gag aac gaa gaa     1056
   Thr Trp Lys Gln Val Leu Glu Glu Ile Gln Asp Leu Glu Asn Glu Glu
               340                 345                 350
   agg acc ccc aag acc aag aat atg aaa aaa aca agc caa ttg aaa tgg     1104
   Arg Thr Pro Lys Thr Lys Asn Met Lys Lys Thr Ser Gln Leu Lys Trp
           355                 360                 365
   gca cta ggt gaa aat atg gca cca gag aaa gtg gat ttt gag gat tgt     1152
   Ala Leu Gly Glu Asn Met Ala Pro Glu Lys Val Asp Phe Glu Asp Cys
       370                 375                 380
   aaa gac atc aat gat tta aaa caa tat gac agt gat gag cca gaa gca     1200
   Lys Asp Ile Asn Asp Leu Lys Gln Tyr Asp Ser Asp Glu Pro Glu Ala
   385                 390                 395                 400
   agg tct ctt gca agt tgg att caa agt gag ttc aac aaa gct tgt gag     1248
   Arg Ser Leu Ala Ser Trp Ile Gln Ser Glu Phe Asn Lys Ala Cys Glu
                   405                 410                 415
   ctg aca gat tca agc tgg ata gag ctc gat gaa att ggg gag gat gtc     1296
   Leu Thr Asp Ser Ser Trp Ile Glu Leu Asp Glu Ile Gly Glu Asp Val
               420                 425                 430
   gcc cca ata gaa tac att gcg agc atg agg aga aat tat ttt act gct     1344
   Ala Pro Ile Glu Tyr Ile Ala Ser Met Arg Arg Asn Tyr Phe Thr Ala
           435                 440                 445
   gag att tcc cat tgt aga gca aca gaa tat ata ata aaa gga gtg tac     1392
   Glu Ile Ser His Cys Arg Ala Thr Glu Tyr Ile Ile Lys Gly Val Tyr
       450                 455                 460
   atc aac act gct cta ctc aat gca tcc tgt gct gcg atg gat gaa ttt     1440
   Ile Asn Thr Ala Leu Leu Asn Ala Ser Cys Ala Ala Met Asp Glu Phe
   465                 470                 475                 480
   caa tta att ccg atg ata agt aaa tgc agg acc aaa gaa ggg aga agg     1488
   Gln Leu Ile Pro Met Ile Ser Lys Cys Arg Thr Lys Glu Gly Arg Arg
                   485                 490                 495
   aaa aca aat tta tat gga ttc ata ata aag gga agg tcc cat tta aga     1536
   Lys Thr Asn Leu Tyr Gly Phe Ile Ile Lys Gly Arg Ser His Leu Arg
               500                 505                 510
   aat gat act gac gtg gtg aac ttt gta agt atg gaa ttt tct ctc act       1584
   Asn Asp Thr Asp Val Val Asn Phe Val Ser Met Glu Phe Ser Leu Thr
           515                 520                 525
   gat cca aga ttt gag cca cac aaa tgg gaa aaa tac tgc gtt cta gaa       1632
   Asp Pro Arg Phe Glu Pro His Lys Trp Glu Lys Tyr Cys Val Leu Glu
       530                 535                 540
   att gga gac atg ctt cta aga act gct gta ggt caa gtg tca aga ccc       1680
   Ile Gly Asp Met Leu Leu Arg Thr Ala Val Gly Gln Val Ser Arg Pro
   545                 550                 555                 560
   atg ttt ttg tat gta agg aca aat gga acc tct aaa att aaa atg aaa       1728
   Met Phe Leu Tyr Val Arg Thr Asn Gly Thr Ser Lys Ile Lys Met Lys
                   565                 570                 575
   tgg gga atg gaa atg agg cgc tgc ctc ctt cag tct ctg caa cag att       1776
   Trp Gly Met Glu Met Arg Arg Cys Leu Leu Gln Ser Leu Gln Gln Ile
               580                 585                 590
   gaa agc atg atc gaa gct gag tcc tca gtc aaa gaa aag gac atg acc       1824
   Glu Ser Met Ile Glu Ala Glu Ser Ser Val Lys Glu Lys Asp Met Thr
           595                 600                 605
   aaa gaa ttt ttt gag aac aaa tca gag aca tgg cct ata gga gag tcc       1872
   Lys Glu Phe Phe Glu Asn Lys Ser Glu Thr Trp Pro Ile Gly Glu Ser
       610                 615                 620
   ccc aaa gga gtg gaa gag ggc tca atc ggg aag gtt tgc agg acc tta       1920
   Pro Lys Gly Val Glu Glu Gly Ser Ile Gly Lys Val Cys Arg Thr Leu
   625                 630                 635                 640
   tta gca aaa tct gtg ttt aac agt tta tat gca tct cca caa ctg gaa       1968
   Leu Ala Lys Ser Val Phe Asn Ser Leu Tyr Ala Ser Pro Gln Leu Glu
                   645                 650                 655
   ggg ttt tca gct gaa tct agg aaa tta ctt ctc att gtt cag gct ctt       2016
   Gly Phe Ser Ala Glu Ser Arg Lys Leu Leu Leu Ile Val Gln Ala Leu
               660                 665                 670
   aag gat gac ctg gaa cct gga acc ttt gat att ggg ggg tta tat gaa       2064
   Lys Asp Asp Leu Glu Pro Gly Thr Phe Asp Ile Gly Gly Leu Tyr Glu
           675                 680                 685
   tca att gag gag tgc ctg att aat gat ccc tgg gtt ttg ctt aat gca       2112
   Ser Ile Glu Glu Cys Leu Ile Asn Asp Pro Trp Val Leu Leu Asn Ala
       690                 695                 700
   tct tgg ttc aac tcc ttc ctt aca cat gca ctg aag tag                   2151
   Ser Trp Phe Asn Ser Phe Leu Thr His Ala Leu Lys
   705                 710                 715
<210> 6
   <211> 716
   <212> PRT
   <213> Influenza virus
<400> 6
   Met Glu Asp Phe Val Arg Gln Cys Phe Asn Pro Met Ile Val Glu Leu
   1               5                   10                  15
   Ala Glu Lys Ala Met Lys Glu Tyr Gly Glu Asn Pro Lys Ile Glu Thr
               20                  25                  30
   Asn Lys Phe Ala Ala Ile Cys Thr His Leu Glu Val Cys Phe Met Tyr
           35                  40                  45
   Ser Asp Phe His Phe Ile Asn Glu Leu Gly Glu Ser Val Val Ile Glu
       50                  55                  60
   Ser Gly Asp Pro Asn Ala Leu Leu Lys His Arg Phe Glu Ile Ile Glu
   65                  70                  75                  80
   Gly Arg Asp Arg Thr Met Ala Trp Thr Val Val Asn Ser Ile Cys Asn
                   85                  90                  95
   Thr Thr Arg Ala Glu Lys Pro Lys Phe Leu Pro Asp Leu Tyr Asp Tyr
               100                 105                 110
   Lys Glu Asn Arg Phe Val Glu Ile Gly Val Thr Arg Arg Glu Val His
           115                 120                 125
   Ile Tyr Tyr Leu Glu Lys Ala Asn Lys Ile Lys Ser Glu Lys Thr His
       130                 135                 140
   Ile His Ile Phe Ser Phe Thr Gly Glu Glu Met Ala Thr Lys Ala Asp
   145                 150                 155                 160
   Tyr Thr Leu Asp Glu Glu Ser Arg Ala Arg Ile Lys Thr Arg Leu Phe
                   165                 170                 175
   Thr Ile Arg Gln Glu Met Ala Ser Arg Gly Leu Trp Asp Ser Phe Arg
               180                 185                 190
   Gln Ser Glu Arg Gly Glu Glu Thr Ile Glu Glu Arg Phe Glu Ile Thr
           195                 200                 205
   Gly Thr Met Arg Lys Leu Ala Asn Tyr Ser Leu Pro Pro Asn Phe Ser
       210                 215                 220
   Ser Leu Glu Asn Phe Arg Val Tyr Val Asp Gly Phe Glu Pro Asn Gly
   225                 230                 235                 240
   Cys Ile Glu Ser Lys Leu Ser Gln Met Ser Lys Glu Val Asn Ala Arg
                   245                 250                 255
   Ile Glu Pro Phe Ser Lys Thr Thr Pro Arg Pro Leu Lys Met Pro Gly
               260                 265                 270
   Gly Pro Pro Cys His Gln Arg Ser Lys Phe Leu Leu Met Asp Ala Leu
           275                 280                 285
   Lys Leu Ser Ile Glu Asp Pro Ser His Glu Gly Glu Gly Ile Pro Leu
       290                 295                 300
   Tyr Asp Ala Ile Lys Cys Met Lys Thr Phe Phe Gly Trp Lys Glu Pro
   305                 310                 315                 320
   Ser Ile Val Lys Pro His Glu Lys Gly Ile Asn Pro Asn Tyr Leu Gln
                   325                 330                 335
   Thr Trp Lys Gln Val Leu Glu Glu Ile Gln Asp Leu Glu Asn Glu Glu
               340                 345                 350
   Arg Thr Pro Lys Thr Lys Asn Met Lys Lys Thr Ser Gln Leu Lys Trp
           355                 360                 365
   Ala Leu Gly Glu Asn Met Ala Pro Glu Lys Val Asp Phe Glu Asp Cys
       370                 375                 380
   Lys Asp Ile Asn Asp Leu Lys Gln Tyr Asp Ser Asp Glu Pro Glu Ala
   385                 390                 395                 400
   Arg Ser Leu Ala Ser Trp Ile Gln Ser Glu Phe Asn Lys Ala Cys Glu
                   405                 410                 415
   Leu Thr Asp Ser Ser Trp Ile Glu Leu Asp Glu Ile Gly Glu Asp Val
               420                 425                 430
   Ala Pro Ile Glu Tyr Ile Ala Ser Met Arg Arg Asn Tyr Phe Thr Ala
           435                 440                 445
   Glu Ile Ser His Cys Arg Ala Thr Glu Tyr Ile Ile Lys Gly Val Tyr
       450                 455                 460
   Ile Asn Thr Ala Leu Leu Asn Ala Ser Cys Ala Ala Met Asp Glu Phe
   465                 470                 475                 480
   Gln Leu Ile Pro Met Ile Ser Lys Cys Arg Thr Lys Glu Gly Arg Arg
                   485                 490                 495
   Lys Thr Asn Leu Tyr Gly Phe Ile Ile Lys Gly Arg Ser His Leu Arg
               500                 505                 510
   Asn Asp Thr Asp Val Val Asn Phe Val Ser Met Glu Phe Ser Leu Thr
           515                 520                 525
   Asp Pro Arg Phe Glu Pro His Lys Trp Glu Lys Tyr Cys Val Leu Glu
       530                 535                 540
   Ile Gly Asp Met Leu Leu Arg Thr Ala Val Gly Gln Val Ser Arg Pro
   545                 550                 555                 560
   Met Phe Leu Tyr Val Arg Thr Asn Gly Thr Ser Lys Ile Lys Met Lys
                   565                 570                 575
   Trp Gly Met Glu Met Arg Arg Cys Leu Leu Gln Ser Leu Gln Gln Ile
               580                 585                 590
   Glu Ser Met Ile Glu Ala Glu Ser Ser Val Lys Glu Lys Asp Met Thr
           595                 600                 605
   Lys Glu Phe Phe Glu Asn Lys Ser Glu Thr Trp Pro Ile Gly Glu Ser
       610                 615                 620
   Pro Lys Gly Val Glu Glu Gly Ser Ile Gly Lys Val Cys Arg Thr Leu
   625                 630                 635                 640
   Leu Ala Lys Ser Val Phe Asn Ser Leu Tyr Ala Ser Pro Gln Leu Glu
                   645                 650                 655
   Gly Phe Ser Ala Glu Ser Arg Lys Leu Leu Leu Ile Val Gln Ala Leu
               660                 665                 670
   Lys Asp Asp Leu Glu Pro Gly Thr Phe Asp Ile Gly Gly Leu Tyr Glu
           675                 680                 685
   Ser Ile Glu Glu Cys Leu Ile Asn Asp Pro Trp Val Leu Leu Asn Ala
       690                 695                 700
   Ser Trp Phe Asn Ser Phe Leu Thr His Ala Leu Lys
   705                 710                 715
<210> 7
   <211> 838
   <212> DNA
   <213> Influenza virus
<220>
   <221> CDS
   <222> (1)..(657)
<400> 7
   atg gat tcc aac act gtg tca agc ttt cag gta gac tgt ttt ctt tgg        48
   Met Asp Ser Asn Thr Val Ser Ser Phe Gln Val Asp Cys Phe Leu Trp
   1               5                   10                  15
   cat gtc cgc aaa cga ttc gca gac caa gaa ctg ggt gat gcc cca ttc        96
   His Val Arg Lys Arg Phe Ala Asp Gln Glu Leu Gly Asp Ala Pro Phe
               20                  25                  30
   ctt gac cgg ctt cgc cga gac cag aag tcc cta agg gga aga ggt agc       144
   Leu Asp Arg Leu Arg Arg Asp Gln Lys Ser Leu Arg Gly Arg Gly Ser
           35                  40                  45
   act ctt ggt ctg gac atc gaa aca gcc act cat gca gga aag cag ata       192
   Thr Leu Gly Leu Asp Ile Glu Thr Ala Thr His Ala Gly Lys Gln Ile
       50                  55                  60
   gtg gag cag att ctg gaa aag gaa tca gat gag gca ctt aaa atg acc       240
   Val Glu Gln Ile Leu Glu Lys Glu Ser Asp Glu Ala Leu Lys Met Thr
   65                  70                  75                  80
   att gcc tct gtt cct act tca ctc tac tta act gac atg act ctt gat       288
   Ile Ala Ser Val Pro Thr Ser Leu Tyr Leu Thr Asp Met Thr Leu Asp
                   85                  90                  95
   gag atg tca aga gac tgg ttc atg ctc atg ccc aag caa aaa gta aca       336
   Glu Met Ser Arg Asp Trp Phe Met Leu Met Pro Lys Gln Lys Val Thr
               100                 105                 110
   ggc tcc cta tgt ata aga atg gac cag gca atc atg gat aag aac atc       384
   Gly Ser Leu Cys Ile Arg Met Asp Gln Ala Ile Met Asp Lys Asn Ile
           115                 120                 125
   ata ctt aaa gca aac ttt agt gtg att ttc gaa ggg ctg gaa aca cta       432
   Ile Leu Lys Ala Asn Phe Ser Val Ile Phe Glu Gly Leu Glu Thr Leu
       130                 135                 140
   ata cta ctt aga gcc ttc acc gaa gaa gga gca gtc gtt ggc gaa att       480
   Ile Leu Leu Arg Ala Phe Thr Glu Glu Gly Ala Val Val Gly Glu Ile
   145                 150                 155                 160
   tca cca tta cct tct ctt cca gga cat act aat gag gat gtc aaa aat       528
   Ser Pro Leu Pro Ser Leu Pro Gly His Thr Asn Glu Asp Val Lys Asn
                   165                 170                 175
   gca att ggg gtc ctc atc gga gga ctt aaa tgg aat gat aat acg gtt       576
   Ala Ile Gly Val Leu Ile Gly Gly Leu Lys Trp Asn Asp Asn Thr Val
               180                 185                 190
   aga atc tct gaa act cta cag aga ttc gct tgg aga agc agt cat gag       624
   Arg Ile Ser Glu Thr Leu Gln Arg Phe Ala Trp Arg Ser Ser His Glu
           195                 200                 205
   aat ggg aga cct tca ttc cct tca aag cag aaa tgaaaaatgg agagaacaat     677
   Asn Gly Arg Pro Ser Phe Pro Ser Lys Gln Lys
       210                 215
   taagccagaa atttgaagaa ataagatggt tgattgaaga agtgcgacat agattgaaaa     737
   atacagaaaa tagttttgaa caaataacat ttatgcaagc cttacaacta ttgcttgaag     797
   tagaacaaga gataagaact ttctcgtttc agcttattta a                         838
<210> 8
   <211> 219
   <212> PRT
   <213> Influenza virus
<400> 8
   Met Asp Ser Asn Thr Val Ser Ser Phe Gln Val Asp Cys Phe Leu Trp
   1               5                   10                  15
   His Val Arg Lys Arg Phe Ala Asp Gln Glu Leu Gly Asp Ala Pro Phe
               20                  25                  30
   Leu Asp Arg Leu Arg Arg Asp Gln Lys Ser Leu Arg Gly Arg Gly Ser
           35                  40                  45
   Thr Leu Gly Leu Asp Ile Glu Thr Ala Thr His Ala Gly Lys Gln Ile
       50                  55                  60
   Val Glu Gln Ile Leu Glu Lys Glu Ser Asp Glu Ala Leu Lys Met Thr
   65                  70                  75                  80
   Ile Ala Ser Val Pro Thr Ser Leu Tyr Leu Thr Asp Met Thr Leu Asp
                   85                  90                  95
   Glu Met Ser Arg Asp Trp Phe Met Leu Met Pro Lys Gln Lys Val Thr
               100                 105                 110
   Gly Ser Leu Cys Ile Arg Met Asp Gln Ala Ile Met Asp Lys Asn Ile
           115                 120                 125
   Ile Leu Lys Ala Asn Phe Ser Val Ile Phe Glu Gly Leu Glu Thr Leu
       130                 135                 140
   Ile Leu Leu Arg Ala Phe Thr Glu Glu Gly Ala Val Val Gly Glu Ile
   145                 150                 155                 160
   Ser Pro Leu Pro Ser Leu Pro Gly His Thr Asn Glu Asp Val Lys Asn
                   165                 170                 175
   Ala Ile Gly Val Leu Ile Gly Gly Leu Lys Trp Asn Asp Asn Thr Val
               180                 185                 190
   Arg Ile Ser Glu Thr Leu Gln Arg Phe Ala Trp Arg Ser Ser His Glu
           195                 200                 205
   Asn Gly Arg Pro Ser Phe Pro Ser Lys Gln Lys
       210                 215
<210> 9
   <211> 1497
   <212> DNA
   <213> Influenza virus
<220>
   <221> CDS
   <222> (1)..(1497)
<400> 9
   atg gcg tct caa ggc acc aaa cga tcc tat gaa cag atg gaa act gat      48
   Met Ala Ser Gln Gly Thr Lys Arg Ser Tyr Glu Gln Met Glu Thr Asp
   1               5                   10                  15
   ggg gaa cgc cag aat gca act gaa atc aga gca tct gtc gga agg atg      96
   Gly Glu Arg Gln Asn Ala Thr Glu Ile Arg Ala Ser Val Gly Arg Met
               20                  25                  30
   gtg gga gga atc ggc cga ttt tat gtt cag atg tgt act gag ctt aaa     144
   Val Gly Gly Ile Gly Arg Phe Tyr Val Gln Met Cys Thr Glu Leu Lys
           35                  40                  45
   cta aac gac cat gaa ggg cgg ctg att cag aac agc ata aca ata gaa     192
   Leu Asn Asp His Glu Gly Arg Leu Ile Gln Asn Ser Ile Thr Ile Glu
       50                  55                  60
   agg atg gta ctt tcg gca ttc gac gaa aga aga aac aag tat ctc gag     240
   Arg Met Val Leu Ser Ala Phe Asp Glu Arg Arg Asn Lys Tyr Leu Glu
   65                  70                  75                  80
   gag cat ccc agt gct ggg aaa gac cct aag aaa acg gga ggc ccg ata     288
   Glu His Pro Ser Ala Gly Lys Asp Pro Lys Lys Thr Gly Gly Pro Ile
                   85                  90                  95
   tac aga agg aaa gat ggg aaa tgg atg agg gaa ctc atc ctc cat gat        336
   Tyr Arg Arg Lys Asp Gly Lys Trp Met Arg Glu Leu Ile Leu His Asp
               100                 105                 110
   aaa gaa gaa atc atg aga atc tgg cgt cag gcc aac aat ggt gaa gac        384
   Lys Glu Glu Ile Met Arg Ile Trp Arg Gln Ala Asn Asn Gly Glu Asp
           115                 120                 125
   gct act gct ggt ctt act cat atg atg atc tgg cac tcc aat ctc aat        432
   Ala Thr Ala Gly Leu Thr His Met Met Ile Trp His Ser Asn Leu Asn
       130                 135                 140
   gac acc aca tac caa aga aca agg gct ctt gtt cgg act ggg atg gat        480
   Asp Thr Thr Tyr Gln Arg Thr Arg Ala Leu Val Arg Thr Gly Met Asp
   145                 150                 155                 160
   ccc aga atg tgc tct ctg atg caa ggc tca acc ctc cca cgg aga tct        528
   Pro Arg Met Cys Ser Leu Met Gln Gly Ser Thr Leu Pro Arg Arg Ser
                   165                 170                 175
   gga gcc gct ggt gct gca gta aaa ggt gtt gga aca atg gta atg gaa        576
   Gly Ala Ala Gly Ala Ala Val Lys Gly Val Gly Thr Met Val Met Glu
               180                 185                 190
   ctc atc aga atg atc aaa cgc gga ata aat gat cgg aat ttc tgg aga        624
   Leu Ile Arg Met Ile Lys Arg Gly Ile Asn Asp Arg Asn Phe Trp Arg
           195                 200                 205
   ggt gaa aat ggt cga aaa acc aga att gct tat gaa aga atg tgc aat        672
   Gly Glu Asn Gly Arg Lys Thr Arg Ile Ala Tyr Glu Arg Met Cys Asn
       210                 215                 220
   atc ctc aaa ggg aaa ttt cag aca gca gca caa cgg gct atg atg gac        720
   Ile Leu Lys Gly Lys Phe Gln Thr Ala Ala Gln Arg Ala Met Met Asp
   225                 230                 235                 240
   cag gtg agg gaa ggc cgc aat cct gga aac gct gag att gag gat ctc        768
   Gln Val Arg Glu Gly Arg Asn Pro Gly Asn Ala Glu Ile Glu Asp Leu
                   245                 250                 255
   att ttc ttg gca cga tca gca ctt att ttg aga gga tca gta gcc cat        816
   Ile Phe Leu Ala Arg Ser Ala Leu Ile Leu Arg Gly Ser Val Ala His
               260                 265                 270
   aaa tca tgc cta cct gcc tgt gtt tat ggc ctt gca ata acc agt ggg        864
   Lys Ser Cys Leu Pro Ala Cys Val Tyr Gly Leu Ala Ile Thr Ser Gly
           275                 280                 285
   tat gac ttt gag aag gaa gga tac tct ctg gtt gga att gat cct ttc        912
   Tyr Asp Phe Glu Lys Glu Gly Tyr Ser Leu Val Gly Ile Asp Pro Phe
       290                 295                 300
   aaa cta ctc cag aac agt caa att ttc agt cta atc aga cca aaa gaa        960
   Lys Leu Leu Gln Asn Ser Gln Ile Phe Ser Leu Ile Arg Pro Lys Glu
   305                 310                 315                 320
   aac cca gca cac aag agc cag ttg gtg tgg atg gca tgc cat tct gca       1008
   Asn Pro Ala His Lys Ser Gln Leu Val Trp Met Ala Cys His Ser Ala
                   325                 330                 335
   gca ttt gag gac ctg aga gtt tta aat ttc att aga gga acc aaa gta       1056
   Ala Phe Glu Asp Leu Arg Val Leu Asn Phe Ile Arg Gly Thr Lys Val
               340                 345                 350
   atc cca aga gga cag tta aca acc aga gga gtt caa att gct tca aat       1104
   Ile Pro Arg Gly Gln Leu Thr Thr Arg Gly Val Gln Ile Ala Ser Asn
           355                 360                 365
   gaa aac atg gag aca ata aat tct agc aca ctt gaa ctg aga agc aaa      1152
   Glu Asn Met Glu Thr Ile Asn Ser Ser Thr Leu Glu Leu Arg Ser Lys
       370                 375                 380
   tat tgg gca ata agg acc aga agc gga gga aac acc agt caa cag aga      1200
   Tyr Trp Ala Ile Arg Thr Arg Ser Gly Gly Asn Thr Ser Gln Gln Arg
   385                 390                 395                 400
   gca tct gca gga cag ata agt gtg caa cct act ttc tca gta cag aga      1248
   Ala Ser Ala Gly Gln Ile Ser Val Gln Pro Thr Phe Ser Val Gln Arg
                   405                 410                 415
   aat cta ccc ttt gag aga gcg acc att atg gct gca ttc act ggt aac      1296
   Asn Leu Pro Phe Glu Arg Ala Thr Ile Met Ala Ala Phe Thr Gly Asn
               420                 425                 430
   act gaa ggg agg act tcc gac atg aga acg gaa atc ata agg atg atg      1344
   Thr Glu Gly Arg Thr Ser Asp Met Arg Thr Glu Ile Ile Arg Met Met
           435                 440                 445
   gaa aat gcc aaa tca gaa gat gtg tct ttc cag ggg cgg gga gtc ttc      1392
   Glu Asn Ala Lys Ser Glu Asp Val Ser Phe Gln Gly Arg Gly Val Phe
       450                 455                 460
   gag ctc tcg gac gaa aag gca acg aac ccg atc gtg cct tcc ttt gac      1440
   Glu Leu Ser Asp Glu Lys Ala Thr Asn Pro Ile Val Pro Ser Phe Asp
   465                 470                 475                 480
   atg agc aat gaa ggg tct tat ttc ttc gga gac aat gct gag gag ttt      1488
   Met Ser Asn Glu Gly Ser Tyr Phe Phe Gly Asp Asn Ala Glu Glu Phe
                   485                 490                 495
   gac agt taa                                                          1497
   Asp Ser
<210> 10
   <211> 498
   <212> PRT
   <213> Influenza virus
<400> 10
   Met Ala Ser Gln Gly Thr Lys Arg Ser Tyr Glu Gln Met Glu Thr Asp
   1               5                   10                  15
   Gly Glu Arg Gln Asn Ala Thr Glu Ile Arg Ala Ser Val Gly Arg Met
               20                  25                  30
   Val Gly Gly Ile Gly Arg Phe Tyr Val Gln Met Cys Thr Glu Leu Lys
           35                  40                  45
   Leu Asn Asp His Glu Gly Arg Leu Ile Gln Asn Ser Ile Thr Ile Glu
       50                  55                  60
   Arg Met Val Leu Ser Ala Phe Asp Glu Arg Arg Asn Lys Tyr Leu Glu
   65                  70                  75                  80
   Glu His Pro Ser Ala Gly Lys Asp Pro Lys Lys Thr Gly Gly Pro Ile
                   85                  90                  95
   Tyr Arg Arg Lys Asp Gly Lys Trp Met Arg Glu Leu Ile Leu His Asp
   100 105 110
   Lys Glu Glu Ile Met Arg Ile Trp Arg Gln Ala Asn Asn Gly Glu Asp
   115 120 125
   Ala Thr Ala Gly Leu Thr His Met Met Ile Trp His Ser Asn Leu Asn
   130 135 140
   Asp Thr Thr Tyr Gln Arg Thr Arg Ala Leu Val Arg Thr Gly Met Asp
   145 150 155 160
   Pro Arg Met Cys Ser Leu Met Gln Gly Ser Thr Leu Pro Arg Arg Ser
   165 170 175
   Gly Ala Ala Gly Ala Ala Val Lys Gly Val Gly Thr Met Val Met Glu
   180 185 190
   Leu Ile Arg Met Ile Lys Arg Gly Ile Asn Asp Arg Asn Phe Trp Arg
   195 200 205
   Gly Glu Asn Gly Arg Lys Thr Arg Ile Ala Tyr Glu Arg Met Cys Asn
   210 215 220
   Ile Leu Lys Gly Lys Phe Gln Thr Ala Ala Gln Arg Ala Met Met Asp
   225 230 235 240
   Gln Val Arg Glu Gly Arg Asn Pro Gly Asn Ala Glu Ile Glu Asp Leu
   245 250 255
   Ile Phe Leu Ala Arg Ser Ala Leu Ile Leu Arg Gly Ser Val Ala His
   260 265 270
   Lys Ser Cys Leu Pro Ala Cys Val Tyr Gly Leu Ala Ile Thr Ser Gly
   275 280 285
   Tyr Asp Phe Glu Lys Glu Gly Tyr Ser Leu Val Gly Ile Asp Pro Phe
   290 295 300
   Lys Leu Leu Gln Asn Ser Gln Ile Phe Ser Leu Ile Arg Pro Lys Glu
   305 310 315 320
   Asn Pro Ala His Lys Ser Gln Leu Val Trp Met Ala Cys His Ser Ala
   325 330 335
   Ala Phe Glu Asp Leu Arg Val Leu Asn Phe Ile Arg Gly Thr Lys Val
   340 345 350
   Ile Pro Arg Gly Gln Leu Thr Thr Arg Gly Val Gln Ile Ala Ser Asn
   355 360 365
   Glu Asn Met Glu Thr Ile Asn Ser Ser Thr Leu Glu Leu Arg Ser Lys
   370 375 380
   Tyr Trp Ala Ile Arg Thr Arg Ser Gly Gly Asn Thr Ser Gln Gln Arg
   385                 390                 395                 400
   Ala Ser Ala Gly Gln Ile Ser Val Gln Pro Thr Phe Ser Val Gln Arg
                   405                 410                 415
   Asn Leu Pro Phe Glu Arg Ala Thr Ile Met Ala Ala Phe Thr Gly Asn
               420                 425                 430
   Thr Glu Gly Arg Thr Ser Asp Met Arg Thr Glu Ile Ile Arg Met Met
           435                 440                 445
   Glu Asn Ala Lys Ser Glu Asp Val Ser Phe Gln Gly Arg Gly Val Phe
       450                 455                 460
   Glu Leu Ser Asp Glu Lys Ala Thr Asn Pro Ile Val Pro Ser Phe Asp
   465                 470                 475                 480
   Met Ser Asn Glu Gly Ser Tyr Phe Phe Gly Asp Asn Ala Glu Glu Phe
                   485                 490                 495
   Asp Ser
<210> 11
   <211> 1413
   <212> DNA
   <213> Influenza virus
<220>
   <221> CDS
   <222> (1)..(1413)
<400> 11
   atg aat cca aat caa aag ata ata gca att gga ttt gca tca ttg ggg        48
   Met Asn Pro Asn Gln Lys Ile Ile Ala Ile Gly Phe Ala Ser Leu Gly
   1               5                   10                  15
   ata tta atc att aat gtc att ctc cat gta gtc agc att ata gta aca        96
   Ile Leu Ile Ile Asn Val Ile Leu His Val Val Ser Ile Ile Val Thr
               20                  25                  30
   gta ctg gtc ctc aat aac aat aga aca gat ctg aac tgc aaa ggg acg       144
   Val Leu Val Leu Asn Asn Asn Arg Thr Asp Leu Asn Cys Lys Gly Thr
           35                  40                  45
   atc ata aga aag tac aat gaa aca gta aga gta gaa aaa att act caa       192
   Ile Ile Arg Lys Tyr Asn Glu Thr Val Arg Val Glu Lys Ile Thr Gln
       50                  55                  60
   tgg tat aat acc agt aca att aag tac ata gag aga cct tca aat gaa       240
   Trp Tyr Asn Thr Ser Thr Ile Lys Tyr Ile Glu Arg Pro Ser Asn Glu
   65                  70                  75                  80
   tac tac atg aac aac act gaa cca ctt tgt gag gcc caa ggc ttt gca       288
   Tyr Tyr Met Asn Asn Thr Glu Pro Leu Cys Glu Ala Gln Gly Phe Ala
                   85                  90                  95
   cca ttt tcc aaa gat aat gga ata cga att ggg tcg aga ggc cat gtt      336
   Pro Phe Ser Lys Asp Asn Gly Ile Arg Ile Gly Ser Arg Gly His Val
               100                 105                 110
   ttt gtg ata aga gaa cct ttt gta tca tgt tcg ccc tca gaa tgt aga      384
   Phe Val Ile Arg Glu Pro Phe Val Ser Cys Ser Pro Ser Glu Cys Arg
           115                 120                 125
   acc ttt ttc ctc aca cag ggc tca tta ctc aat gac aaa cat tct aac      432
   Thr Phe Phe Leu Thr Gln Gly Ser Leu Leu Asn Asp Lys His Ser Asn
       130                 135                 140
   ggc aca gta aag gac cga agt ccg tat agg act ttg atg agt gtc aaa      480
   Gly Thr Val Lys Asp Arg Ser Pro Tyr Arg Thr Leu Met Ser Val Lys
   145                 150                 155                 160
   ata ggg caa tca cct aat gta tat caa gct aga ttt gaa tcg gta gca      528
   Ile Gly Gln Ser Pro Asn Val Tyr Gln Ala Arg Phe Glu Ser Val Ala
                   165                 170                 175
   tgg tca gca aca gca tgc cat gat gga aaa aaa tgg atg aca gtt gga      576
   Trp Ser Ala Thr Ala Cys His Asp Gly Lys Lys Trp Met Thr Val Gly
               180                 185                 190
   gtc aca ggg ccc gac aat caa gca att gca gta gtg aac tat gga ggt      624
   Val Thr Gly Pro Asp Asn Gln Ala Ile Ala Val Val Asn Tyr Gly Gly
           195                 200                 205
   gtt ccg gtt gat att att aat tca tgg gca ggg gat att tta aga acc      672
   Val Pro Val Asp Ile Ile Asn Ser Trp Ala Gly Asp Ile Leu Arg Thr
       210                 215                 220
   caa gaa tca tca tgc acc tgc att aaa gga gac tgt tat tgg gta atg      720
   Gln Glu Ser Ser Cys Thr Cys Ile Lys Gly Asp Cys Tyr Trp Val Met
   225                 230                 235                 240
   act gat gga ccg gca aat agg caa gct aaa tat agg ata ttc aaa gca      768
   Thr Asp Gly Pro Ala Asn Arg Gln Ala Lys Tyr Arg Ile Phe Lys Ala
                   245                 250                 255
   aaa gat gga aga gta att gga cag act gat ata agt ttc aat ggg gga      816
   Lys Asp Gly Arg Val Ile Gly Gln Thr Asp Ile Ser Phe Asn Gly Gly
               260                 265                 270
   cac ata gag gag tgt tct tgt tac ccc aat gaa ggg aag gtg gaa tgc      864
   His Ile Glu Glu Cys Ser Cys Tyr Pro Asn Glu Gly Lys Val Glu Cys
           275                 280                 285
   ata tgc agg gac aat tgg act gga aca aat aga cca gtt ctg gta ata      912
   Ile Cys Arg Asp Asn Trp Thr Gly Thr Asn Arg Pro Val Leu Val Ile
       290                 295                 300
   tct tct gat cta tcg tac aca gtt gga tat ttg tgt gct ggc att ccc      960
   Ser Ser Asp Leu Ser Tyr Thr Val Gly Tyr Leu Cys Ala Gly Ile Pro
   305                 310                 315                 320
   act gac act cct agg gga gag gat agt caa ttc aca ggc tca tgt aca     1008
   Thr Asp Thr Pro Arg Gly Glu Asp Ser Gln Phe Thr Gly Ser Cys Thr
                   325                 330                 335
   agt cct ttg gga aat aaa gga tac ggt gta aaa ggt ttc ggg ttt cga     1056
   Ser Pro Leu Gly Asn Lys Gly Tyr Gly Val Lys Gly Phe Gly Phe Arg
               340                 345                 350
   caa gga act gac gta tgg gcc gga agg aca att agt agg act tca aga     1104
   Gln Gly Thr Asp Val Trp Ala Gly Arg Thr Ile Ser Arg Thr Ser Arg
           355                 360                 365
   tca gga ttc gaa ata ata aaa atc agg aat ggt tgg aca cag aac agt     1152
   Ser Gly Phe Glu Ile Ile Lys Ile Arg Asn Gly Trp Thr Gln Asn Ser
       370                 375                 380
   aaa gac caa atc agg agg caa gtg att atc gat gac cca aat tgg tca      1200
   Lys Asp Gln Ile Arg Arg Gln Val Ile Ile Asp Asp Pro Asn Trp Ser
   385                 390                 395                 400
   gga tat agc ggt tct ttc aca ttg ccg gtt gaa cta aca aaa aag gga      1248
   Gly Tyr Ser Gly Ser Phe Thr Leu Pro Val Glu Leu Thr Lys Lys Gly
                   405                 410                 415
   tgt ttg gtc ccc tgt ttc tgg gtt gaa atg att aga ggt aaa cct gaa      1296
   Cys Leu Val Pro Cys Phe Trp Val Glu Met Ile Arg Gly Lys Pro Glu
               420                 425                 430
   gaa aca aca ata tgg acc tct agt agc tcc att gtg atg tgt gga gta      1344
   Glu Thr Thr Ile Trp Thr Ser Ser Ser Ser Ile Val Met Cys Gly Val
           435                 440                 445
   gat cat aaa att gcc agt tgg tca tgg cac gat gga gct att ctt ccc      1392
   Asp His Lys Ile Ala Ser Trp Ser Trp His Asp Gly Ala Ile Leu Pro
       450                 455                 460
   ttt gac atc gat aag atg taa                                          1413
   Phe Asp Ile Asp Lys Met
   465                 470
<210> 12
   <211> 470
   <212> PRT
   <213> Influenza virus
<400> 12
   Met Asn Pro Asn Gln Lys Ile Ile Ala Ile Gly Phe Ala Ser Leu Gly
   1               5                   10                  15
   Ile Leu Ile Ile Asn Val Ile Leu His Val Val Ser Ile Ile Val Thr
               20                  25                  30
   Val Leu Val Leu Asn Asn Asn Arg Thr Asp Leu Asn Cys Lys Gly Thr
           35                  40                  45
   Ile Ile Arg Lys Tyr Asn Glu Thr Val Arg Val Glu Lys Ile Thr Gln
       50                  55                  60
   Trp Tyr Asn Thr Ser Thr Ile Lys Tyr Ile Glu Arg Pro Ser Asn Glu
   65                  70                  75                  80
   Tyr Tyr Met Asn Asn Thr Glu Pro Leu Cys Glu Ala Gln Gly Phe Ala
                   85                  90                  95
   Pro Phe Ser Lys Asp Asn Gly Ile Arg Ile Gly Ser Arg Gly His Val
               100                 105                 110
   Phe Val Ile Arg Glu Pro Phe Val Ser Cys Ser Pro Ser Glu Cys Arg
           115                 120                 125
   Thr Phe Phe Leu Thr Gln Gly Ser Leu Leu Asn Asp Lys His Ser Asn
       130                 135                 140
   Gly Thr Val Lys Asp Arg Ser Pro Tyr Arg Thr Leu Met Ser Val Lys
   145 150 155 160
   Ile Gly Gln Ser Pro Asn Val Tyr Gln Ala Arg Phe Glu Ser Val Ala
   165 170 175
   Trp Ser Ala Thr Ala Cys His Asp Gly Lys Lys Trp Met Thr Val Gly
   180 185 190
   Val Thr Gly Pro Asp Asn Gln Ala Ile Ala Val Val Asn Tyr Gly Gly
   195 200 205
   Val Pro Val Asp Ile Ile Asn Ser Trp Ala Gly Asp Ile Leu Arg Thr
   210 215 220
   Gln Glu Ser Ser Cys Thr Cys Ile Lys Gly Asp Cys Tyr Trp Val Met
   225 230 235 240
   Thr Asp Gly Pro Ala Asn Arg Gln Ala Lys Tyr Arg Ile Phe Lys Ala
   245 250 255
   Lys Asp Gly Arg Val Ile Gly Gln Thr Asp Ile Ser Phe Asn Gly Gly
   260 265 270
   His Ile Glu Glu Cys Ser Cys Tyr Pro Asn Glu Gly Lys Val Glu Cys
   275 280 285
   Ile Cys Arg Asp Asn Trp Thr Gly Thr Asn Arg Pro Val Leu Val Ile
   290 295 300
   Ser Ser Asp Leu Ser Tyr Thr Val Gly Tyr Leu Cys Ala Gly Ile Pro
   305 310 315 320
   Thr Asp Thr Pro Arg Gly Glu Asp Ser Gln Phe Thr Gly Ser Cys Thr
   325 330 335
   Ser Pro Leu Gly Asn Lys Gly Tyr Gly Val Lys Gly Phe Gly Phe Arg
   340 345 350
   Gln Gly Thr Asp Val Trp Ala Gly Arg Thr Ile Ser Arg Thr Ser Arg
   355 360 365
   Ser Gly Phe Glu Ile Ile Lys Ile Arg Asn Gly Trp Thr Gln Asn Ser
   370 375 380
   Lys Asp Gln Ile Arg Arg Gln Val Ile Ile Asp Asp Pro Asn Trp Ser
   385 390 395 400
   Gly Tyr Ser Gly Ser Phe Thr Leu Pro Val Glu Leu Thr Lys Lys Gly
   405 410 415
   Cys Leu Val Pro Cys Phe Trp Val Glu Met Ile Arg Gly Lys Pro Glu
               420                 425                 430
   Glu Thr Thr Ile Trp Thr Ser Ser Ser Ser Ile Val Met Cys Gly Val
           435                 440                 445
   Asp His Lys Ile Ala Ser Trp Ser Trp His Asp Gly Ala Ile Leu Pro
       450                 455                 460
   Phe Asp Ile Asp Lys Met
   465                 470
<210> 13
   <211> 982
   <212> DNA
   <213> Influenza virus
<220>
   <221> CDS
   <222> (1)..(756)
<400> 13
   atg agt ctt cta acc gag gtc gaa acg tac gtt ctc tct atc gta cca        48
   Met Ser Leu Leu Thr Glu Val Glu Thr Tyr Val Leu Ser Ile Val Pro
   1               5                   10                  15
   tca ggc ccc ctc aaa gcc gag atc gcg cag aga ctt gaa gat gtc ttt        96
   Ser Gly Pro Leu Lys Ala Glu Ile Ala Gln Arg Leu Glu Asp Val Phe
               20                  25                  30
   gca gga aag aac acc gat ctt gag gca ctc atg gaa tgg cta aag aca       144
   Ala Gly Lys Asn Thr Asp Leu Glu Ala Leu Met Glu Trp Leu Lys Thr
           35                  40                  45
   aga cca atc ctg tca cct ctg act aaa ggg att tta gga ttt gta ttc       192
   Arg Pro Ile Leu Ser Pro Leu Thr Lys Gly Ile Leu Gly Phe Val Phe
       50                  55                  60
   acg ctc acc gtg ccc agt gag cga gga ctg cag cgt aga cgc ttt gtc       240
   Thr Leu Thr Val Pro Ser Glu Arg Gly Leu Gln Arg Arg Arg Phe Val
   65                  70                  75                  80
   caa aat gcc ctt agt gga aac gga gat cca aac aac atg gac aga gca       288
   Gln Asn Ala Leu Ser Gly Asn Gly Asp Pro Asn Asn Met Asp Arg Ala
                   85                  90                  95
   gta aaa ctg tac agg aag ctt aaa aga gaa ata aca ttc cat ggg gca       336
   Val Lys Leu Tyr Arg Lys Leu Lys Arg Glu Ile Thr Phe His Gly Ala
               100                 105                 110
   aaa gag gta gca ctc agc tat tcc act ggt gca cta gcc agc tgc atg       384
   Lys Glu Val Ala Leu Ser Tyr Ser Thr Gly Ala Leu Ala Ser Cys Met
           115                 120                 125
   gga ctc ata tac aac aga atg gga act gtt aca acc gaa gtg gca ttt       432
   Gly Leu Ile Tyr Asn Arg Met Gly Thr Val Thr Thr Glu Val Ala Phe
       130                 135                 140
   ggc ctg gta tgc gcc aca tgt gaa cag att gct gat tcc cag cat cga       480
   Gly Leu Val Cys Ala Thr Cys Glu Gln Ile Ala Asp Ser Gln His Arg
   145                 150                 155                 160
   gct cac agg cag atg gtg aca aca acc aac cca ttg atc aga cat gaa       528
   Ala His Arg Gln Met Val Thr Thr Thr Asn Pro Leu Ile Arg His Glu
                   165                 170                 175
   aac aga atg gta tta gcc agt acc acg gct aaa gcc atg gaa cag atg       576
   Asn Arg Met Val Leu Ala Ser Thr Thr Ala Lys Ala Met Glu Gln Met
               180                 185                 190
   gca gga tcg agt gag cag gca gca gag gcc atg gag gtt gct agt agg       624
   Ala Gly Ser Ser Glu Gln Ala Ala Glu Ala Met Glu Val Ala Ser Arg
           195                 200                 205
   gct agg cag atg gta cag gca atg aga acc att ggg acc cac cct agc       672
   Ala Arg Gln Met Val Gln Ala Met Arg Thr Ile Gly Thr His Pro Ser
       210                 215                 220
   tcc agt gcc ggt ttg aaa gat gat ctc ctt gaa aat tta cag gcc tac       720
   Ser Ser Ala Gly Leu Lys Asp Asp Leu Leu Glu Asn Leu Gln Ala Tyr
   225                 230                 235                 240
   cag aaa cgg atg gga gtg caa atg cag cga ttc aag tgatcctctc            766
   Gln Lys Arg Met Gly Val Gln Met Gln Arg Phe Lys
                   245                 250
   gttattgcag caagtatcat tgggatcttg cacttgatat tgtggattct tgatcgtctt     826
   ttcttcaaat tcatttatcg tcgccttaaa tacgggttga aaagagggcc ttctacggaa     886
   ggagtacctg agtctatgag ggaagaatat cggcaggaac agcagaatgc tgtggatgtt     946
   gacgatggtc attttgtcaa catagagctg gagtaa                               982
<210> 14
   <211> 252
   <212> PRT
   <213> Influenza virus
<400> 14
   Met Ser Leu Leu Thr Glu Val Glu Thr Tyr Val Leu Ser Ile Val Pro
   1               5                   10                  15
   Ser Gly Pro Leu Lys Ala Glu Ile Ala Gln Arg Leu Glu Asp Val Phe
               20                  25                  30
   Ala Gly Lys Asn Thr Asp Leu Glu Ala Leu Met Glu Trp Leu Lys Thr
           35                  40                  45
   Arg Pro Ile Leu Ser Pro Leu Thr Lys Gly Ile Leu Gly Phe Val Phe
       50                  55                  60
   Thr Leu Thr Val Pro Ser Glu Arg Gly Leu Gln Arg Arg Arg Phe Val
   65                  70                  75                  80
   Gln Asn Ala Leu Ser Gly Asn Gly Asp Pro Asn Asn Met Asp Arg Ala
                   85                  90                  95
   Val Lys Leu Tyr Arg Lys Leu Lys Arg Glu Ile Thr Phe His Gly Ala
               100                 105                 110
   Lys Glu Val Ala Leu Ser Tyr Ser Thr Gly Ala Leu Ala Ser Cys Met
           115                 120                 125
   Gly Leu Ile Tyr Asn Arg Met Gly Thr Val Thr Thr Glu Val Ala Phe
       130                 135                 140
   Gly Leu Val Cys Ala Thr Cys Glu Gln Ile Ala Asp Ser Gln His Arg
   145                 150                 155                 160
   Ala His Arg Gln Met Val Thr Thr Thr Asn Pro Leu Ile Arg His Glu
                   165                 170                 175
   Asn Arg Met Val Leu Ala Ser Thr Thr Ala Lys Ala Met Glu Gln Met
               180                 185                 190
   Ala Gly Ser Ser Glu Gln Ala Ala Glu Ala Met Glu Val Ala Ser Arg
           195                 200                 205
   Ala Arg Gln Met Val Gln Ala Met Arg Thr Ile Gly Thr His Pro Ser
       210                 215                 220
   Ser Ser Ala Gly Leu Lys Asp Asp Leu Leu Glu Asn Leu Gln Ala Tyr
   225                 230                 235                 240
   Gln Lys Arg Met Gly Val Gln Met Gln Arg Phe Lys
                   245                 250
<210> 15
   <211> 1698
   <212> DNA
   <213> Influenza virus
<220>
   <221> CDS
   <222> (1)..(1698)
<400> 15
   atg aag aca acc att att ttg ata cta cta acc cat tgg gcc tac agt        48
   Met Lys Thr Thr Ile Ile Leu Ile Leu Leu Thr His Trp Ala Tyr Ser
   1               5                   10                  15
   caa aac cca atc agt ggc aac aac aca gcc aca ctg tgt ctg gga cac        96
   Gln Asn Pro Ile Ser Gly Asn Asn Thr Ala Thr Leu Cys Leu Gly His
               20                  25                  30
   cat gca gta gca aat gga aca ttg gta aaa aca atg agt gat gat caa       144
   His Ala Val Ala Asn Gly Thr Leu Val Lys Thr Met Ser Asp Asp Gln
           35                  40                  45
   att gag gtg aca aat gct aca gaa tta gtt cag agc att tca atg ggg       192
   Ile Glu Val Thr Asn Ala Thr Glu Leu Val Gln Ser Ile Ser Met Gly
       50                  55                  60
   aaa ata tgc aac aaa tca tat aga att cta gat gga aga aat tgc aca       240
   Lys Ile Cys Asn Lys Ser Tyr Arg Ile Leu Asp Gly Arg Asn Cys Thr
   65                  70                  75                  80
   tta ata gat gca atg cta gga gac ccc cac tgt gac gcc ttt cag tat       288
   Leu Ile Asp Ala Met Leu Gly Asp Pro His Cys Asp Ala Phe Gln Tyr
                   85                  90                  95
   gag agt tgg gac ctc ttc ata gaa aga agc aac gct ttc agc aat tgc      336
   Glu Ser Trp Asp Leu Phe Ile Glu Arg Ser Asn Ala Phe Ser Asn Cys
               100                 105                 110
   tac cca tat gac atc cct gac tat gca tcg ctc cga tcc att gta gca      384
   Tyr Pro Tyr Asp Ile Pro Asp Tyr Ala Ser Leu Arg Ser Ile Val Ala
           115                 120                 125
   tcc tca gga aca ttg gaa ttc aca gca gag gga ttc aca tgg aca ggt      432
   Ser Ser Gly Thr Leu Glu Phe Thr Ala Glu Gly Phe Thr Trp Thr Gly
       130                 135                 140
   gtc act caa aac gga aga agt gga gcc tgc aaa agg gga tca gcc gat      480
   Val Thr Gln Asn Gly Arg Ser Gly Ala Cys Lys Arg Gly Ser Ala Asp
   145                 150                 155                 160
   agt ttc ttt agc cga ctg aat tgg cta aca aaa tct gga agc tct tac      528
   Ser Phe Phe Ser Arg Leu Asn Trp Leu Thr Lys Ser Gly Ser Ser Tyr
                   165                 170                 175
   ccc aca ttg aat gtg aca atg cct aac aat aaa aat ttc gac aag cta      576
   Pro Thr Leu Asn Val Thr Met Pro Asn Asn Lys Asn Phe Asp Lys Leu
               180                 185                 190
   tac atc tgg ggg att cat cac ccg agc tca aat caa gag cag aca aaa      624
   Tyr Ile Trp Gly Ile His His Pro Ser Ser Asn Gln Glu Gln Thr Lys
           195                 200                 205
   ttg tac atc caa gaa tca gga cga gta aca gtc tca aca aaa aga agt      672
   Leu Tyr Ile Gln Glu Ser Gly Arg Val Thr Val Ser Thr Lys Arg Ser
       210                 215                 220
   caa caa aca ata atc cct aac atc gga tct aga ccg ttg gtc aga ggt      720
   Gln Gln Thr Ile Ile Pro Asn Ile Gly Ser Arg Pro Leu Val Arg Gly
   225                 230                 235                 240
   caa tca ggc agg ata agc ata tac tgg acc att gta aaa cct gga gat      768
   Gln Ser Gly Arg Ile Ser Ile Tyr Trp Thr Ile Val Lys Pro Gly Asp
                   245                 250                 255
   atc cta atg ata aac agt aat ggc aac tta gtt gca ccg cgg gga tat      816
   Ile Leu Met Ile Asn Ser Asn Gly Asn Leu Val Ala Pro Arg Gly Tyr
               260                 265                 270
   ttt aaa ttg aaa aca ggg aaa agc tct gta atg aga tca gat gca ccc      864
   Phe Lys Leu Lys Thr Gly Lys Ser Ser Val Met Arg Ser Asp Ala Pro
           275                 280                 285
   ata gac att tgt gtg tct gaa tgt att aca cca aat gga agc atc tcc      912
   Ile Asp Ile Cys Val Ser Glu Cys Ile Thr Pro Asn Gly Ser Ile Ser
       290                 295                 300
   aac gac aag cca ttc caa aat gtg aac aaa gtt aca tat gga aaa tgc      960
   Asn Asp Lys Pro Phe Gln Asn Val Asn Lys Val Thr Tyr Gly Lys Cys
   305                 310                 315                 320
   cct aag tat atc agg caa aac act tta aag ctg gcc act ggg atg agg     1008
   Pro Lys Tyr Ile Arg Gln Asn Thr Leu Lys Leu Ala Thr Gly Met Arg
                   325                 330                 335
   aat gta cca gaa aag caa acc aga gga atc ttt gga gca ata gcg gga     1056
   Asn Val Pro Glu Lys Gln Thr Arg Gly Ile Phe Gly Ala Ile Ala Gly
               340                 345                 350
   ttc atc gaa aac ggc tgg gaa gga atg gtt gat ggg tgg tat ggg ttc     1104
   Phe Ile Glu Asn Gly Trp Glu Gly Met Val Asp Gly Trp Tyr Gly Phe
           355                 360                 365
   cga tat caa aac tct gaa gga aca ggg caa gct gca gat cta aag agc     1152
   Arg Tyr Gln Asn Ser Glu Gly Thr Gly Gln Ala Ala Asp Leu Lys Ser
       370                 375                 380
   act caa gca gcc atc gac cag att aat gga aag tta aac aga gtg att      1200
   Thr Gln Ala Ala Ile Asp Gln Ile Asn Gly Lys Leu Asn Arg Val Ile
   385                 390                 395                 400
   gaa aga acc aat gag aaa ttc cat caa ata gag aag gaa ttc tca gaa      1248
   Glu Arg Thr Asn Glu Lys Phe His Gln Ile Glu Lys Glu Phe Ser Glu
                   405                 410                 415
   gta gaa gga aga att cag gac ctg gag aaa tat gta gaa gac acc aaa      1296
   Val Glu Gly Arg Ile Gln Asp Leu Glu Lys Tyr Val Glu Asp Thr Lys
               420                 425                 430
   ata gac cta tgg tcc tac aat gca gaa ttg ctg gtg gct cta gaa aat      1344
   Ile Asp Leu Trp Ser Tyr Asn Ala Glu Leu Leu Val Ala Leu Glu Asn
           435                 440                 445
   caa cat aca att gac tta aca gat gca gaa atg aat aaa tta ttt gag      1392
   Gln His Thr Ile Asp Leu Thr Asp Ala Glu Met Asn Lys Leu Phe Glu
       450                 455                 460
   aag act aga cgc cag tta aga gaa aac gca gaa gac atg gga ggt gga      1440
   Lys Thr Arg Arg Gln Leu Arg Glu Asn Ala Glu Asp Met Gly Gly Gly
   465                 470                 475                 480
   tgt ttc aag att tac cac aaa tgt gat aat gca tgc att gga tca ata      1488
   Cys Phe Lys Ile Tyr His Lys Cys Asp Asn Ala Cys Ile Gly Ser Ile
                   485                 490                 495
   aga act ggg aca tat gac cat tac ata tac aga gat gaa gca tta aac      1536
   Arg Thr Gly Thr Tyr Asp His Tyr Ile Tyr Arg Asp Glu Ala Leu Asn
               500                 505                 510
   aac cga ttt cag atc aaa ggt gta gag ttg aaa tca ggc tac aaa gat      1584
   Asn Arg Phe Gln Ile Lys Gly Val Glu Leu Lys Ser Gly Tyr Lys Asp
           515                 520                 525
   tgg ata ttg tgg att tca ttc gcc ata tca tgc ttc tta att tgc gtt      1632
   Trp Ile Leu Trp Ile Ser Phe Ala Ile Ser Cys Phe Leu Ile Cys Val
       530                 535                 540
   gtt cta ttg ggt ttc att atg tgg gct tgc caa aga ggc aac atc aga      1680
   Val Leu Leu Gly Phe Ile Met Trp Ala Cys Gln Arg Gly Asn Ile Arg
   545                 550                 555                 560
   tgc aac att tgc att tga                                              1698
   Cys Asn Ile Cys Ile
                   565
<210> 16
   <211> 565
   <212> PRT
   <213> Influenza virus
<400> 16
   Met Lys Thr Thr Ile Ile Leu Ile Leu Leu Thr His Trp Ala Tyr Ser
   1               5                   10                  15
   Gln Asn Pro Ile Ser Gly Asn Asn Thr Ala Thr Leu Cys Leu Gly His
               20                  25                  30
   His Ala Val Ala Asn Gly Thr Leu Val Lys Thr Met Ser Asp Asp Gln
           35                  40                  45
   Ile Glu Val Thr Asn Ala Thr Glu Leu Val Gln Ser Ile Ser Met Gly
       50                  55                  60
   Lys Ile Cys Asn Lys Ser Tyr Arg Ile Leu Asp Gly Arg Asn Cys Thr
   65                  70                  75                  80
   Leu Ile Asp Ala Met Leu Gly Asp Pro His Cys Asp Ala Phe Gln Tyr
                   85                  90                  95
   Glu Ser Trp Asp Leu Phe Ile Glu Arg Ser Asn Ala Phe Ser Asn Cys
               100                 105                 110
   Tyr Pro Tyr Asp Ile Pro Asp Tyr Ala Ser Leu Arg Ser Ile Val Ala
           115                 120                 125
   Ser Ser Gly Thr Leu Glu Phe Thr Ala Glu Gly Phe Thr Trp Thr Gly
       130                 135                 140
   Val Thr Gln Asn Gly Arg Ser Gly Ala Cys Lys Arg Gly Ser Ala Asp
   145                 150                 155                 160
   Ser Phe Phe Ser Arg Leu Asn Trp Leu Thr Lys Ser Gly Ser Ser Tyr
                   165                 170                 175
   Pro Thr Leu Asn Val Thr Met Pro Asn Asn Lys Asn Phe Asp Lys Leu
               180                 185                 190
   Tyr Ile Trp Gly Ile His His Pro Ser Ser Asn Gln Glu Gln Thr Lys
           195                 200                 205
   Leu Tyr Ile Gln Glu Ser Gly Arg Val Thr Val Ser Thr Lys Arg Ser
       210                 215                 220
   Gln Gln Thr Ile Ile Pro Asn Ile Gly Ser Arg Pro Leu Val Arg Gly
   225                 230                 235                 240
   Gln Ser Gly Arg Ile Ser Ile Tyr Trp Thr Ile Val Lys Pro Gly Asp
                   245                 250                 255
   Ile Leu Met Ile Asn Ser Asn Gly Asn Leu Val Ala Pro Arg Gly Tyr
               260                 265                 270
   Phe Lys Leu Lys Thr Gly Lys Ser Ser Val Met Arg Ser Asp Ala Pro
           275                 280                 285
   Ile Asp Ile Cys Val Ser Glu Cys Ile Thr Pro Asn Gly Ser Ile Ser
       290                 295                 300
   Asn Asp Lys Pro Phe Gln Asn Val Asn Lys Val Thr Tyr Gly Lys Cys
   305                 310                 315                 320
   Pro Lys Tyr Ile Arg Gln Asn Thr Leu Lys Leu Ala Thr Gly Met Arg
                   325                 330                 335
   Asn Val Pro Glu Lys Gln Thr Arg Gly Ile Phe Gly Ala Ile Ala Gly
               340                 345                 350
   Phe Ile Glu Asn Gly Trp Glu Gly Met Val Asp Gly Trp Tyr Gly Phe
           355                 360                 365
   Arg Tyr Gln Asn Ser Glu Gly Thr Gly Gln Ala Ala Asp Leu Lys Ser
       370                 375                 380
   Thr Gln Ala Ala Ile Asp Gln Ile Asn Gly Lys Leu Asn Arg Val Ile
   385                 390                 395                 400
   Glu Arg Thr Asn Glu Lys Phe His Gln Ile Glu Lys Glu Phe Ser Glu
                   405                 410                 415
   Val Glu Gly Arg Ile Gln Asp Leu Glu Lys Tyr Val Glu Asp Thr Lys
               420                 425                 430
   Ile Asp Leu Trp Ser Tyr Asn Ala Glu Leu Leu Val Ala Leu Glu Asn
           435                 440                 445
   Gln His Thr Ile Asp Leu Thr Asp Ala Glu Met Asn Lys Leu Phe Glu
       450                 455                 460
   Lys Thr Arg Arg Gln Leu Arg Glu Asn Ala Glu Asp Met Gly Gly Gly
   465                 470                 475                 480
   Cys Phe Lys Ile Tyr His Lys Cys Asp Asn Ala Cys Ile Gly Ser Ile
                   485                 490                 495
   Arg Thr Gly Thr Tyr Asp His Tyr Ile Tyr Arg Asp Glu Ala Leu Asn
               500                 505                 510
   Asn Arg Phe Gln Ile Lys Gly Val Glu Leu Lys Ser Gly Tyr Lys Asp
           515                 520                 525
   Trp Ile Leu Trp Ile Ser Phe Ala Ile Ser Cys Phe Leu Ile Cys Val
       530                 535                 540
   Val Leu Leu Gly Phe Ile Met Trp Ala Cys Gln Arg Gly Asn Ile Arg
   545                 550                 555                 560
   Cys Asn Ile Cys Ile
                   565
<210> 17
   <211> 2277
   <212> DNA
   <213> Influenza virus
<220>
   <221> CDS
   <222> (1)..(2277)
<400> 17
   atg gag aga ata aaa gaa ctg aga gat ctg atg tta caa tcc cgc acc        48
   Met Glu Arg Ile Lys Glu Leu Arg Asp Leu Met Leu Gln Ser Arg Thr
   1               5                   10                  15
   cgc gag ata cta aca aaa act act gtg gac cac atg gcc ata atc aag        96
   Arg Glu Ile Leu Thr Lys Thr Thr Val Asp His Met Ala Ile Ile Lys
               20                  25                  30
   aaa tac aca tca gga aga caa gag aag aac cct gca ctt agg atg aaa       144
   Lys Tyr Thr Ser Gly Arg Gln Glu Lys Asn Pro Ala Leu Arg Met Lys
           35                  40                  45
   tgg atg atg gca atg aaa tac cca att aca gca gat aag agg ata atg       192
   Trp Met Met Ala Met Lys Tyr Pro Ile Thr Ala Asp Lys Arg Ile Met
       50                  55                  60
   gag atg att cct gag aga aat gaa cag gga caa acc ctt tgg agc aaa       240
   Glu Met Ile Pro Glu Arg Asn Glu Gln Gly Gln Thr Leu Trp Ser Lys
   65                  70                  75                  80
   acg aac gat gct ggc tca gac cgc gta atg gta tca cct ctg gca gtg       288
   Thr Asn Asp Ala Gly Ser Asp Arg Val Met Val Ser Pro Leu Ala Val
                   85                  90                  95
   aca tgg tgg aat agg aat gga cca aca acg agc aca att cat tat cca       336
   Thr Trp Trp Asn Arg Asn Gly Pro Thr Thr Ser Thr Ile His Tyr Pro
               100                 105                 110
   aaa gtc tac aaa act tat ttt gaa aag gtt gaa aga ttg aaa cac gga       384
   Lys Val Tyr Lys Thr Tyr Phe Glu Lys Val Glu Arg Leu Lys His Gly
           115                 120                 125
   acc ttt ggc ccc gtt cat ttt agg aat caa gtc aag ata aga cga aga       432
   Thr Phe Gly Pro Val His Phe Arg Asn Gln Val Lys Ile Arg Arg Arg
       130                 135                 140
   gtt gat gta aac cct ggt cac gcg gac ctc agt gcc aaa gaa gca caa       480
   Val Asp Val Asn Pro Gly His Ala Asp Leu Ser Ala Lys Glu Ala Gln
   145                 150                 155                 160
   gat gtg atc atg gaa gtt gtt ttc cca aat gaa gtg gga gcc aga att       528
   Asp Val Ile Met Glu Val Val Phe Pro Asn Glu Val Gly Ala Arg Ile
                   165                 170                 175
   cta aca tcg gaa tca caa cta aca ata acc aaa gag aaa aag gaa gaa       576
   Leu Thr Ser Glu Ser Gln Leu Thr Ile Thr Lys Glu Lys Lys Glu Glu
               180                 185                 190
   ctt cag gac tgc aaa att gct ccc ttg atg gta gca tac atg cta gaa       624
   Leu Gln Asp Cys Lys Ile Ala Pro Leu Met Val Ala Tyr Met Leu Glu
           195                 200                 205
   aga gag ttg gtc cga aaa aca agg ttc ctc cca gta gca ggc gga aca       672
   Arg Glu Leu Val Arg Lys Thr Arg Phe Leu Pro Val Ala Gly Gly Thr
       210                 215                 220
   agc agt gta tac att gaa gtg ttg cat ctg act cag gga aca tgc tgg       720
   Ser Ser Val Tyr Ile Glu Val Leu His Leu Thr Gln Gly Thr Cys Trp
   225                 230                 235                 240
   gag caa atg tac acc cca gga gga gaa gtt aga aac gat gat att gat       768
   Glu Gln Met Tyr Thr Pro Gly Gly Glu Val Arg Asn Asp Asp Ile Asp
                       245                 250                 255
   caa agt tta att att gca gcc cgg aac ata gtg aga aga gcg aca gta     816
   Gln Ser Leu Ile Ile Ala Ala Arg Asn Ile Val Arg Arg Ala Thr Val
               260                 265                 270
   tca gca gat cca cta gca tcc cta ctg gaa atg tgc cac agt aca cag     864
   Ser Ala Asp Pro Leu Ala Ser Leu Leu Glu Met Cys His Ser Thr Gln
           275                 280                 285
   att ggt gga ata agg atg gta gac atc ctt aag cag aat cca aca gag     912
   Ile Gly Gly Ile Arg Met Val Asp Ile Leu Lys Gln Asn Pro Thr Glu
       290                 295                 300
   gaa caa gct gtg gat ata tgc aaa gca gca atg gga ttg aga att agc     960
   Glu Gln Ala Val Asp Ile Cys Lys Ala Ala Met Gly Leu Arg Ile Ser
   305                 310                 315                 320
   tca tca ttc agc ttt ggt gga ttc acc ttc aaa aga aca agt gga tca    1008
   Ser Ser Phe Ser Phe Gly Gly Phe Thr Phe Lys Arg Thr Ser Gly Ser
                   325                 330                 335
   tca gtc aag aga gaa gaa gaa atg ctt acg ggc aac ctt caa aca ttg    1056
   Ser Val Lys Arg Glu Glu Glu Met Leu Thr Gly Asn Leu Gln Thr Leu
               340                 345                 350
   aaa ata aga gtg cat gag ggc tat gaa gaa ttc aca atg gtc gga aga    1104
   Lys Ile Arg Val His Glu Gly Tyr Glu Glu Phe Thr Met Val Gly Arg
           355                 360                 365
   aga gca aca gcc att atc aga aag gca acc aga aga ttg att caa ttg    1152
   Arg Ala Thr Ala Ile Ile Arg Lys Ala Thr Arg Arg Leu Ile Gln Leu
       370                 375                 380
   ata gta agt ggg aga gat gaa caa tca att gct gaa gca ata att gta    1200
   Ile Val Ser Gly Arg Asp Glu Gln Ser Ile Ala Glu Ala Ile Ile Val
   385                 390                 395                 400
   gcc atg gtg ttt tcg caa gaa gat tgc atg ata aaa gca gtt cga ggc    1248
   Ala Met Val Phe Ser Gln Glu Asp Cys Met Ile Lys Ala Val Arg Gly
                   405                 410                 415
   gat ttg aac ttt gtt aat aga gca aat cag cgt ttg aac ccc atg cat    1296
   Asp Leu Asn Phe Val Asn Arg Ala Asn Gln Arg Leu Asn Pro Met His
               420                 425                 430
   caa ctc ttg agg cat ttc caa aaa gat gca aaa gtg ctt ttc caa aat    1344
   Gln Leu Leu Arg His Phe Gln Lys Asp Ala Lys Val Leu Phe Gln Asn
           435                 440                 445
   tgg gga att gaa ccc atc gac aat gta atg ggg atg att gga ata ttg    1392
   Trp Gly Ile Glu Pro Ile Asp Asn Val Met Gly Met Ile Gly Ile Leu
       450                 455                 460
   cct gac atg acc cca agc acc gag atg tca ttg aga gga gtg aga gtc    1440
   Pro Asp Met Thr Pro Ser Thr Glu Met Ser Leu Arg Gly Val Arg Val
   465                 470                 475                 480
   agc aaa atg gga gtg gat gag tac tcc agc act gag aga gtg gtg gtg    1488
   Ser Lys Met Gly Val Asp Glu Tyr Ser Ser Thr Glu Arg Val Val Val
                   485                 490                 495
   agc att gac cgt ttt tta aga gtt cgg gat caa agg gga aac ata cta    1536
   Ser Ile Asp Arg Phe Leu Arg Val Arg Asp Gln Arg Gly Asn Ile Leu
               500                 505                 510
   ctg tcc cct gaa gaa gtc agt gaa aca caa gga acg gaa aag ctg aca    1584
   Leu Ser Pro Glu Glu Val Ser Glu Thr Gln Gly Thr Glu Lys Leu Thr
           515                 520                 525
   ata att tat tcg tca tca atg atg tgg gag att aat ggt ccc gaa tca      1632
   Ile Ile Tyr Ser Ser Ser Met Met Trp Glu Ile Asn Gly Pro Glu Ser
       530                 535                 540
   gtg ttg gtc aat act tat caa tgg atc atc agg aac tgg gaa att gta      1680
   Val Leu Val Asn Thr Tyr Gln Trp Ile Ile Arg Asn Trp Glu Ile Val
   545                 550                 555                 560
   aaa att cag tgg tca cag gac ccc aca atg tta tac aat aag ata gaa      1728
   Lys Ile Gln Trp Ser Gln Asp Pro Thr Met Leu Tyr Asn Lys Ile Glu
                   565                 570                 575
   ttt gag cca ttc caa tcc ctg gtc cct agg gcc acc aga agc caa tac      1776
   Phe Glu Pro Phe Gln Ser Leu Val Pro Arg Ala Thr Arg Ser Gln Tyr
               580                 585                 590
   agc ggt ttc gta aga acc ctg ttt cag caa atg cga gat gta ctt gga      1824
   Ser Gly Phe Val Arg Thr Leu Phe Gln Gln Met Arg Asp Val Leu Gly
           595                 600                 605
   aca ttt gat act gct caa ata ata aaa ctc ctc cct ttt gcc gct gct      1872
   Thr Phe Asp Thr Ala Gln Ile Ile Lys Leu Leu Pro Phe Ala Ala Ala
       610                 615                 620
   cct ccg gaa cag agt agg atg cag ttc tct tct ttg act gtt aat gta      1920
   Pro Pro Glu Gln Ser Arg Met Gln Phe Ser Ser Leu Thr Val Asn Val
   625                 630                 635                 640
   aga ggt tcg gga atg agg ata ctt gta aga ggc aat tcc cca gtg ttc      1968
   Arg Gly Ser Gly Met Arg Ile Leu Val Arg Gly Asn Ser Pro Val Phe
                   645                 650                 655
   aac tac aat aaa gcc act aaa agg ctc aca gtc ctc gga aag gat gca      2016
   Asn Tyr Asn Lys Ala Thr Lys Arg Leu Thr Val Leu Gly Lys Asp Ala
               660                 665                 670
   ggt gcg ctt act gag gac cca gat gaa ggt acg gct gga gta gaa tct      2064
   Gly Ala Leu Thr Glu Asp Pro Asp Glu Gly Thr Ala Gly Val Glu Ser
           675                 680                 685
   gct gtt cta aga ggg ttt ctc att tta ggt aaa gaa aac aag aga tat      2112
   Ala Val Leu Arg Gly Phe Leu Ile Leu Gly Lys Glu Asn Lys Arg Tyr
       690                 695                 700
   ggc cca gca cta agc atc aat gaa cta agc aaa ctt gca aaa ggg gag      2160
   Gly Pro Ala Leu Ser Ile Asn Glu Leu Ser Lys Leu Ala Lys Gly Glu
   705                 710                 715                 720
   aaa gcc aat gta cta att ggg caa ggg gac rta gtg ttg gta atg aaa      2208
   Lys Ala Asn Val Leu Ile Gly Gln Gly Asp Xaa Val Leu Val Met Lys
                   725                 730                 735
   cgg aaa cgt gac tct agc ata ctt act gac agc cag aca gcg acc aaa      2256
   Arg Lys Arg Asp Ser Ser Ile Leu Thr Asp Ser Gln Thr Ala Thr Lys
               740                 745                 750
   agg att cgg atg gcc atc aat                                          2277
   Arg Ile Arg Met Ala Ile Asn
           755
<210> 18
   <211> 759
   <212> PRT
   <213> Influenza virus
<220>
   <221> misc_feature
   <222> (731)..(731)
   <223> The 'Xaa' at location 731 stands for Val, or Ile.
<400> 18
   Met Glu Arg Ile Lys Glu Leu Arg Asp Leu Met Leu Gln Ser Arg Thr
   1               5                   10                  15
   Arg Glu Ile Leu Thr Lys Thr Thr Val Asp His Met Ala Ile Ile Lys
               20                  25                  30
   Lys Tyr Thr Ser Gly Arg Gln Glu Lys Asn Pro Ala Leu Arg Met Lys
           35                  40                  45
   Trp Met Met Ala Met Lys Tyr Pro Ile Thr Ala Asp Lys Arg Ile Met
       50                  55                  60
   Glu Met Ile Pro Glu Arg Asn Glu Gln Gly Gln Thr Leu Trp Ser Lys
   65                  70                  75                  80
   Thr Asn Asp Ala Gly Ser Asp Arg Val Met Val Ser Pro Leu Ala Val
                   85                  90                  95
   Thr Trp Trp Asn Arg Asn Gly Pro Thr Thr Ser Thr Ile His Tyr Pro
               100                 105                 110
   Lys Val Tyr Lys Thr Tyr Phe Glu Lys Val Glu Arg Leu Lys His Gly
           115                 120                 125
   Thr Phe Gly Pro Val His Phe Arg Asn Gln Val Lys Ile Arg Arg Arg
       130                 135                 140
   Val Asp Val Asn Pro Gly His Ala Asp Leu Ser Ala Lys Glu Ala Gln
   145                 150                 155                 160
   Asp Val Ile Met Glu Val Val Phe Pro Asn Glu Val Gly Ala Arg Ile
                   165                 170                 175
   Leu Thr Ser Glu Ser Gln Leu Thr Ile Thr Lys Glu Lys Lys Glu Glu
               180                 185                 190
   Leu Gln Asp Cys Lys Ile Ala Pro Leu Met Val Ala Tyr Met Leu Glu
           195                 200                 205
   Arg Glu Leu Val Arg Lys Thr Arg Phe Leu Pro Val Ala Gly Gly Thr
       210                 215                 220
   Ser Ser Val Tyr Ile Glu Val Leu His Leu Thr Gln Gly Thr Cys Trp
   225                 230                 235                 240
   Glu Gln Met Tyr Thr Pro Gly Gly Glu Val Arg Asn Asp Asp Ile Asp
                   245                 250                 255
   Gln Ser Leu Ile Ile Ala Ala Arg Asn Ile Val Arg Arg Ala Thr Val
               260                 265                 270
   Ser Ala Asp Pro Leu Ala Ser Leu Leu Glu Met Cys His Ser Thr Gln
           275                 280                 285
   Ile Gly Gly Ile Arg Met Val Asp Ile Leu Lys Gln Asn Pro Thr Glu
       290                 295                 300
   Glu Gln Ala Val Asp Ile Cys Lys Ala Ala Met Gly Leu Arg Ile Ser
   305                 310                 315                 320
   Ser Ser Phe Ser Phe Gly Gly Phe Thr Phe Lys Arg Thr Ser Gly Ser
                   325                 330                 335
   Ser Val Lys Arg Glu Glu Glu Met Leu Thr Gly Asn Leu Gln Thr Leu
               340                 345                 350
   Lys Ile Arg Val His Glu Gly Tyr Glu Glu Phe Thr Met Val Gly Arg
           355                 360                 365
   Arg Ala Thr Ala Ile Ile Arg Lys Ala Thr Arg Arg Leu Ile Gln Leu
       370                 375                 380
   Ile Val Ser Gly Arg Asp Glu Gln Ser Ile Ala Glu Ala Ile Ile Val
   385                 390                 395                 400
   Ala Met Val Phe Ser Gln Glu Asp Cys Met Ile Lys Ala Val Arg Gly
                   405                 410                 415
   Asp Leu Asn Phe Val Asn Arg Ala Asn Gln Arg Leu Asn Pro Met His
               420                 425                 430
   Gln Leu Leu Arg His Phe Gln Lys Asp Ala Lys Val Leu Phe Gln Asn
           435                 440                 445
   Trp Gly Ile Glu Pro Ile Asp Asn Val Met Gly Met Ile Gly Ile Leu
       450                 455                 460
   Pro Asp Met Thr Pro Ser Thr Glu Met Ser Leu Arg Gly Val Arg Val
   465                 470                 475                 480
   Ser Lys Met Gly Val Asp Glu Tyr Ser Ser Thr Glu Arg Val Val Val
                   485                 490                 495
   Ser Ile Asp Arg Phe Leu Arg Val Arg Asp Gln Arg Gly Asn Ile Leu
               500                 505                 510
   Leu Ser Pro Glu Glu Val Ser Glu Thr Gln Gly Thr Glu Lys Leu Thr
           515                 520                 525
   Ile Ile Tyr Ser Ser Ser Met Met Trp Glu Ile Asn Gly Pro Glu Ser
       530                 535                 540
   Val Leu Val Asn Thr Tyr Gln Trp Ile Ile Arg Asn Trp Glu Ile Val
   545                 550                 555                 560
   Lys Ile Gln Trp Ser Gln Asp Pro Thr Met Leu Tyr Asn Lys Ile Glu
                   565                 570                 575
   Phe Glu Pro Phe Gln Ser Leu Val Pro Arg Ala Thr Arg Ser Gln Tyr
               580                 585                 590
   Ser Gly Phe Val Arg Thr Leu Phe Gln Gln Met Arg Asp Val Leu Gly
           595                 600                 605
   Thr Phe Asp Thr Ala Gln Ile Ile Lys Leu Leu Pro Phe Ala Ala Ala
       610                 615                 620
   Pro Pro Glu Gln Ser Arg Met Gln Phe Ser Ser Leu Thr Val Asn Val
   625                 630                 635                 640
   Arg Gly Ser Gly Met Arg Ile Leu Val Arg Gly Asn Ser Pro Val Phe
                   645                 650                 655
   Asn Tyr Asn Lys Ala Thr Lys Arg Leu Thr Val Leu Gly Lys Asp Ala
               660                 665                 670
   Gly Ala Leu Thr Glu Asp Pro Asp Glu Gly Thr Ala Gly Val Glu Ser
           675                 680                 685
   Ala Val Leu Arg Gly Phe Leu Ile Leu Gly Lys Glu Asn Lys Arg Tyr
       690                 695                 700
   Gly Pro Ala Leu Ser Ile Asn Glu Leu Ser Lys Leu Ala Lys Gly Glu
   705                 710                 715                 720
   Lys Ala Asn Val Leu Ile Gly Gln Gly Asp Xaa Val Leu Val Met Lys
                   725                 730                 735
   Arg Lys Arg Asp Ser Ser Ile Leu Thr Asp Ser Gln Thr Ala Thr Lys
               740                 745                 750
   Arg Ile Arg Met Ala Ile Asn
           755
<210> 19
   <211> 2274
   <212> DNA
   <213> Influenza virus
<220>
   <221> CDS
   <222> (1) .. (2274)
<210> 20
   <211> 757
   <212> PRT
   <213> Influenza virus
<400> 20
   Met Asp Val Asn Pro Thr Leu Leu Phe Leu Lys Val Pro Ala Gln Asn
   1               5                   10                  15
   Ala Ile Ser Thr Thr Phe Pro Tyr Thr Gly Asp Pro Pro Tyr Ser His
               20                  25                  30
   Gly Thr Gly Thr Gly Tyr Thr Met Asp Thr Val Asn Arg Thr His Gln
           35                  40                  45
   Tyr Ser Glu Lys Gly Lys Trp Thr Thr Asn Thr Glu Ile Gly Ala Pro
       50                  55                  60
   Gln Leu Asn Pro Ile Asp Gly Pro Leu Pro Glu Asp Asn Glu Pro Ser
   65                  70                  75                  80
   Gly Tyr Ala Gln Thr Asp Cys Val Leu Glu Ala Met Ala Phe Leu Glu
                   85                  90                  95
   Glu Ser His Pro Gly Ile Phe Glu Asn Ser Cys Leu Glu Thr Met Glu
               100                 105                 110
   Val Ile Gln Gln Thr Arg Val Asp Lys Leu Thr Gln Gly Arg Gln Thr
           115                 120                 125
   Tyr Asp Trp Thr Leu Asn Arg Asn Gln Pro Ala Ala Thr Ala Leu Ala
       130                 135                 140
   Asn Thr Ile Glu Val Phe Arg Ser Asn Gly Leu Thr Ser Asn Glu Ser
   145                 150                 155                 160
   Gly Arg Leu Met Asp Phe Leu Lys Asp Val Met Glu Ser Met Asn Lys
                   165                 170                 175
   Glu Glu Met Glu Ile Thr Thr His Phe Gln Arg Lys Arg Arg Val Arg
               180                 185                 190
   Asp Asn Met Thr Lys Arg Met Val Thr Gln Arg Thr Ile Gly Lys Lys
           195                 200                 205
   Lys Gln Arg Leu Asn Arg Lys Ser Tyr Leu Ile Arg Thr Leu Thr Leu
       210                 215                 220
   Asn Thr Met Thr Lys Asp Ala Glu Arg Gly Lys Leu Lys Arg Arg Ala
   225                 230                 235                 240
   Ile Ala Thr Pro Gly Met Gln Ile Arg Gly Phe Val Tyr Phe Val Glu
                   245                 250                 255
   Thr Leu Ala Arg Arg Ile Cys Glu Lys Leu Glu Gln Ser Gly Leu Pro
               260                 265                 270
   Val Gly Gly Asn Glu Lys Lys Ala Lys Leu Ala Asn Val Val Arg Lys
           275                 280                 285
   Met Met Thr Asn Ser Gln Asp Thr Glu Leu Ser Phe Thr Ile Thr Gly
       290                 295                 300
   Asp Asn Thr Lys Trp Asn Glu Asn Gln Asn Pro Arg Ile Phe Leu Ala
   305 310 315 320
   Met Ile Thr Tyr Ile Thr Arg Asn Gln Pro Glu Trp Phe Arg Asn Val
   325 330 335
   Leu Ser Ile Ala Pro Ile Met Phe Ser Asn Lys Met Ala Arg Leu Gly
   340 345 350
   Lys Gly Tyr Met Phe Glu Ser Lys Ser Met Lys Leu Arg Thr Gln Ile
   355 360 365
   Pro Ala Glu Met Leu Ala Ser Ile Asp Leu Lys Tyr Phe Asn Asp Ser
   370 375 380
   Thr Lys Lys Lys Ile Glu Lys Ile Arg Pro Leu Leu Val Asp Gly Thr
   385 390 395 400
   Ala Ser Leu Ser Pro Gly Met Met Met Gly Met Phe Asn Met Leu Ser
   405 410 415
   Thr Val Leu Gly Val Ser Ile Leu Asn Leu Gly Gln Arg Lys Tyr Thr
   420 425 430
   Lys Thr Thr Tyr Trp Trp Asp Gly Leu Gln Ser Ser Asp Asp Phe Ala
   435 440 445
   Leu Ile Val Asn Ala Pro Asn His Glu Gly Ile Gln Ala Gly Val Asp
   450 455 460
   Arg Phe Tyr Arg Thr Cys Lys Leu Val Gly Ile Asn Met Ser Lys Lys
   465 470 475 480
   Lys Ser Tyr Ile Asn Arg Thr Gly Thr Phe Glu Phe Thr Ser Phe Phe
   485 490 495
   Tyr Arg Tyr Gly Phe Val Ala Asn Phe Ser Met Glu Leu Pro Ser Phe
   500 505 510
   Gly Val Ser Gly Ile Asn Glu Ser Ala Asp Met Ser Ile Gly Val Thr
   515 520 525
   Val Ile Lys Asn Asn Met Ile Asn Asn Asp Leu Gly Pro Ala Thr Ala
   530 535 540
   Gln Met Ala Leu Gln Leu Phe Ile Lys Asp Tyr Arg Tyr Thr Tyr Arg
   545 550 555 560
   Cys His Arg Gly Asp Thr Gln Ile Gln Thr Arg Arg Ser Phe Glu Leu
   565 570 575
   Lys Lys Leu Trp Glu Gln Thr Arg Ser Lys Thr Gly Leu Leu Val Ser
               580                 585                 590
   Asp Gly Gly Pro Asn Leu Tyr Asn Ile Arg Asn Leu His Ile Pro Glu
           595                 600                 605
   Val Cys Leu Lys Trp Glu Leu Met Asp Glu Asp Tyr Lys Gly Arg Leu
       610                 615                 620
   Cys Asn Pro Leu Asn Pro Phe Val Ser His Lys Glu Ile Glu Ser Val
   625                 630                 635                 640
   Asn Ser Ala Val Val Met Pro Ala His Gly Pro Ala Lys Ser Met Glu
                   645                 650                 655
   Tyr Asp Ala Val Ala Thr Thr His Ser Trp Ile Pro Lys Arg Asn Arg
               660                 665                 670
   Ser Ile Leu Asn Thr Ser Gln Arg Gly Ile Leu Glu Asp Glu Gln Met
           675                 680                 685
   Tyr Gln Lys Cys Cys Asn Leu Phe Glu Lys Phe Phe Pro Ser Ser Ser
       690                 695                 700
   Tyr Arg Arg Pro Val Gly Ile Ser Ser Met Val Glu Ala Met Val Ser
   705                 710                 715                 720
   Arg Ala Arg Ile Asp Ala Arg Ile Asp Phe Glu Ser Gly Arg Ile Lys
                   725                 730                 735
   Lys Asp Glu Phe Ala Glu Ile Met Lys Ile Cys Ser Thr Ile Glu Glu
               740                 745                 750
   Leu Arg Arg Gln Lys
           755
<210> 21
   <211> 2151
   <212> DNA
   <213> Influenza virus
<220>
   <221> CDS
   <222> (1) .. (2151)
<400> 21
   atg gaa gac ttt gtg cga cag tgc ttc aat cca atg atc gtc gag ctt      48
   Met Glu Asp Phe Val Arg Gln Cys Phe Asn Pro Met Ile Val Glu Leu
   1               5                   10                  15
   gcg gaa aag gca atg aaa gaa tat gga gag aac ccg aaa atc gaa aca      96
   Ala Glu Lys Ala Met Lys Glu Tyr Gly Glu Asn Pro Lys Ile Glu Thr
               20                  25                  30
   aac aaa ttt gca gca ata tgc act cac ttg gaa gtc tgc ttc atg tac     144
   Asn Lys Phe Ala Ala Ile Cys Thr His Leu Glu Val Cys Phe Met Tyr
               35                  40                  45
   tcg gat ttt cac ttt att aat gaa ctg ggt gag tca gtg gtc ata gag        192
   Ser Asp Phe His Phe Ile Asn Glu Leu Gly Glu Ser Val Val Ile Glu
       50                  55                  60
   tct ggt gac cca aat gct ctt ttg aaa cac aga ttt gaa atc att gag        240
   Ser Gly Asp Pro Asn Ala Leu Leu Lys His Arg Phe Glu Ile Ile Glu
   65                  70                  75                  80
   ggg aga gat cga aca atg gca tgg aca gta gta aac agc atc tgc aac        288
   Gly Arg Asp Arg Thr Met Ala Trp Thr Val Val Asn Ser Ile Cys Asn
                   85                  90                  95
   acc aca aga gct gaa aaa cct aaa ttt ctt cca gat tta tac gac tat        336
   Thr Thr Arg Ala Glu Lys Pro Lys Phe Leu Pro Asp Leu Tyr Asp Tyr
               100                 105                 110
   aag gag aac aga ttt gtt gaa att ggt gtg aca agg aga gaa gtt cac        384
   Lys Glu Asn Arg Phe Val Glu Ile Gly Val Thr Arg Arg Glu Val His
           115                 120                 125
   ata tac tac ctg gag aag gcc aac aaa ata aag tct gag aaa aca cat        432
   Ile Tyr Tyr Leu Glu Lys Ala Asn Lys Ile Lys Ser Glu Lys Thr His
       130                 135                 140
   atc cac att ttc tca ttt aca gga gag gaa atg gct aca aaa gcg gac        480
   Ile His Ile Phe Ser Phe Thr Gly Glu Glu Met Ala Thr Lys Ala Asp
   145                 150                 155                 160
   tat act ctt gat gaa gag agt aga gcc agg atc aag acc aga cta ttc        528
   Tyr Thr Leu Asp Glu Glu Ser Arg Ala Arg Ile Lys Thr Arg Leu Phe
                   165                 170                 175
   act ata aga caa gaa atg gcc agt aga ggc ctc tgg gat tcc ttt cgt        576
   Thr Ile Arg Gln Glu Met Ala Ser Arg Gly Leu Trp Asp Ser Phe Arg
               180                 185                 190
   cag tcc gag aga ggc gaa gag aca att gaa gaa aga ttt gaa atc aca        624
   Gln Ser Glu Arg Gly Glu Glu Thr Ile Glu Glu Arg Phe Glu Ile Thr
           195                 200                 205
   ggg acg atg cgc aag ctt gcc aat tac agt ctc cca ccg aac ttc tcc        672
   Gly Thr Met Arg Lys Leu Ala Asn Tyr Ser Leu Pro Pro Asn Phe Ser
       210                 215                 220
   agc ctt gaa aat ttt aga gtc tat gtg gat gga ttc gaa ccg aac ggc        720
   Ser Leu Glu Asn Phe Arg Val Tyr Val Asp Gly Phe Glu Pro Asn Gly
   225                 230                 235                 240
   tgc att gag agt aag ctt tct caa atg tcc aaa gaa gta aat gcc aga        768
   Cys Ile Glu Ser Lys Leu Ser Gln Met Ser Lys Glu Val Asn Ala Arg
                   245                 250                 255
   atc gaa cca ttt tca aag aca aca ccc cga cca ctc aaa atg cca ggt        816
   Ile Glu Pro Phe Ser Lys Thr Thr Pro Arg Pro Leu Lys Met Pro Gly
               260                 265                 270
   ggt cca ccc tgc cat cag cga tct aaa ttc ttg cta atg gat gct ctg        864
   Gly Pro Pro Cys His Gln Arg Ser Lys Phe Leu Leu Met Asp Ala Leu
           275                 280                 285
   aaa ctg agc att gag gac cca agt cac gag gga gag gga ata cca cta        912
   Lys Leu Ser Ile Glu Asp Pro Ser His Glu Gly Glu Gly Ile Pro Leu
       290                 295                 300
   tat gat gca atc aaa tgc atg aaa act ttc ttt gga tgg aaa gag ccc        960
   Tyr Asp Ala Ile Lys Cys Met Lys Thr Phe Phe Gly Trp Lys Glu Pro
   305                 310                 315                 320
   agt att gtt aaa cca cat gaa aag ggt ata aac ccg aac tat ctc caa      1008
   Ser Ile Val Lys Pro His Glu Lys Gly Ile Asn Pro Asn Tyr Leu Gln
                   325                 330                 335
   act tgg aag caa gta tta gaa gaa ata caa gac ctt gag aac gaa gaa      1056
   Thr Trp Lys Gln Val Leu Glu Glu Ile Gln Asp Leu Glu Asn Glu Glu
               340                 345                 350
   agg acc ccc aag acc aag aat atg aaa aaa aca agc caa ttg aaa tgg      1104
   Arg Thr Pro Lys Thr Lys Asn Met Lys Lys Thr Ser Gln Leu Lys Trp
           355                 360                 365
   gca cta ggt gaa aat atg gca cca gag aaa gtg gat ttt gag gat tgt      1152
   Ala Leu Gly Glu Asn Met Ala Pro Glu Lys Val Asp Phe Glu Asp Cys
       370                 375                 380
   aaa gac atc aat gat tta aaa caa tat gac agt gat gag cca gaa aca      1200
   Lys Asp Ile Asn Asp Leu Lys Gln Tyr Asp Ser Asp Glu Pro Glu Thr
   385                 390                 395                 400
   agg tct ctt gca agt tgg att caa agt gag ttc aac aaa gct tgt gag      1248
   Arg Ser Leu Ala Ser Trp Ile Gln Ser Glu Phe Asn Lys Ala Cys Glu
                   405                 410                 415
   ctg aca gat tca agc tgg ata gag ctc gat gaa att ggg gag gat gtc      1296
   Leu Thr Asp Ser Ser Trp Ile Glu Leu Asp Glu Ile Gly Glu Asp Val
               420                 425                 430
   gcc cca ata gaa tac att gcg agc atg agg aga aat tat ttt act gct      1344
   Ala Pro Ile Glu Tyr Ile Ala Ser Met Arg Arg Asn Tyr Phe Thr Ala
           435                 440                 445
   gag att tcc cat tgt aga gca aca gaa tat ata atg aaa gga gtg tac      1392
   Glu Ile Ser His Cys Arg Ala Thr Glu Tyr Ile Met Lys Gly Val Tyr
       450                 455                 460
   atc aac act gct cta ctc aat gca tcc tgt gct gcg atg gat gaa ttt      1440
   Ile Asn Thr Ala Leu Leu Asn Ala Ser Cys Ala Ala Met Asp Glu Phe
   465                 470                 475                 480
   caa tta att ccg atg ata agt aaa tgc agg acc aaa gaa ggg aga agg      1488
   Gln Leu Ile Pro Met Ile Ser Lys Cys Arg Thr Lys Glu Gly Arg Arg
                   485                 490                 495
   aaa aca aat tta tat gga ttc ata ata aag gga agg tcc cat tta aga      1536
   Lys Thr Asn Leu Tyr Gly Phe Ile Ile Lys Gly Arg Ser His Leu Arg
               500                 505                 510
   aat gat act gac gtg gtg aac ttt gta agt atg gaa ttt tct ctc act      1584
   Asn Asp Thr Asp Val Val Asn Phe Val Ser Met Glu Phe Ser Leu Thr
           515                 520                 525
   gat cca aga ttt gag cca cac aaa tgg gaa aaa tac tgc gtt cta gaa      1632
   Asp Pro Arg Phe Glu Pro His Lys Trp Glu Lys Tyr Cys Val Leu Glu
       530                 535                 540
   att gga gac atg ctt cta aga act gct gta ggt caa gtg tca aga ccc      1680
   Ile Gly Asp Met Leu Leu Arg Thr Ala Val Gly Gln Val Ser Arg Pro
   545                 550                 555                 560
   atg ttt ttg tat gta agg aca aat gga acc tct aaa att aaa atg aaa      1728
   Met Phe Leu Tyr Val Arg Thr Asn Gly Thr Ser Lys Ile Lys Met Lys
                   565                 570                 575
   tgg gga atg gaa atg agg cgc tgc ctc ctt cag tct ctg caa cag att      1776
   Trp Gly Met Glu Met Arg Arg Cys Leu Leu Gln Ser Leu Gln Gln Ile
               580                 585                 590
   gaa agc atg atc gaa gct gag tcc tca gtc aaa gaa aag gac atg acc      1824
   Glu Ser Met Ile Glu Ala Glu Ser Ser Val Lys Glu Lys Asp Met Thr
           595                 600                 605
   aaa gaa ttt ttt gag aac aaa tca gag aca tgg cct ata gga gag tcc      1872
   Lys Glu Phe Phe Glu Asn Lys Ser Glu Thr Trp Pro Ile Gly Glu Ser
       610                 615                 620
   ccc aaa gga gtg gaa gag ggc tca atc ggg aag gtt tgc agg acc tta      1920
   Pro Lys Gly Val Glu Glu Gly Ser Ile Gly Lys Val Cys Arg Thr Leu
   625                 630                 635                 640
   tta gca aaa tct gtg ttt aac agt tta tat gca tct cca caa ctg gaa      1968
   Leu Ala Lys Ser Val Phe Asn Ser Leu Tyr Ala Ser Pro Gln Leu Glu
                   645                 650                 655
   ggg ttt tca gct gaa tct agg aaa tta ctt ctc att gtt cag gct ctt      2016
   Gly Phe Ser Ala Glu Ser Arg Lys Leu Leu Leu Ile Val Gln Ala Leu
               660                 665                 670
   agg gat gac ctg gaa cct gga acc ttt gat att ggg ggg tta tat gaa      2064
   Arg Asp Asp Leu Glu Pro Gly Thr Phe Asp Ile Gly Gly Leu Tyr Glu
           675                 680                 685
   tca att gag gag tgc ctg att aat gat ccc tgg gtt ttg ctt aat gca      2112
   Ser Ile Glu Glu Cys Leu Ile Asn Asp Pro Trp Val Leu Leu Asn Ala
       690                 695                 700
   tct tgg ttc aac tcc ttc ctt aca cat gca ctg aag tag                  2151
   Ser Trp Phe Asn Ser Phe Leu Thr His Ala Leu Lys
   705                 710                 715
<210> 22
   <211> 716
   <212> PRT
   <213> Influenza virus
<400> 22
   Met Glu Asp Phe Val Arg Gln Cys Phe Asn Pro Met Ile Val Glu Leu
   1               5                   10                  15
   Ala Glu Lys Ala Met Lys Glu Tyr Gly Glu Asn Pro Lys Ile Glu Thr
               20                  25                  30
   Asn Lys Phe Ala Ala Ile Cys Thr His Leu Glu Val Cys Phe Met Tyr
           35                  40                  45
   Ser Asp Phe His Phe Ile Asn Glu Leu Gly Glu Ser Val Val Ile Glu
       50                  55                  60
   Ser Gly Asp Pro Asn Ala Leu Leu Lys His Arg Phe Glu Ile Ile Glu
   65                  70                  75                  80
   Gly Arg Asp Arg Thr Met Ala Trp Thr Val Val Asn Ser Ile Cys Asn
                   85                  90                  95
   Thr Thr Arg Ala Glu Lys Pro Lys Phe Leu Pro Asp Leu Tyr Asp Tyr
               100                 105                 110
   Lys Glu Asn Arg Phe Val Glu Ile Gly Val Thr Arg Arg Glu Val His
           115                     120                     125
   Ile Tyr Tyr Leu Glu Lys Ala Asn Lys Ile Lys Ser Glu Lys Thr His
       130                     135                     140
   Ile His Ile Phe Ser Phe Thr Gly Glu Glu Met Ala Thr Lys Ala Asp
   145                     150                     155                 160
   Tyr Thr Leu Asp Glu Glu Ser Arg Ala Arg Ile Lys Thr Arg Leu Phe
                   165                     170                     175
   Thr Ile Arg Gln Glu Met Ala Ser Arg Gly Leu Trp Asp Ser Phe Arg
               180                     185                     190
   Gln Ser Glu Arg Gly Glu Glu Thr Ile Glu Glu Arg Phe Glu Ile Thr
           195                     200                     205
   Gly Thr Met Arg Lys Leu Ala Asn Tyr Ser Leu Pro Pro Asn Phe Ser
       210                     215                     220
   Ser Leu Glu Asn Phe Arg Val Tyr Val Asp Gly Phe Glu Pro Asn Gly
   225                     230                     235                 240
   Cys Ile Glu Ser Lys Leu Ser Gln Met Ser Lys Glu Val Asn Ala Arg
                   245                     250                     255
   Ile Glu Pro Phe Ser Lys Thr Thr Pro Arg Pro Leu Lys Met Pro Gly
               260                     265                     270
   Gly Pro Pro Cys His Gln Arg Ser Lys Phe Leu Leu Met Asp Ala Leu
           275                     280                     285
   Lys Leu Ser Ile Glu Asp Pro Ser His Glu Gly Glu Gly Ile Pro Leu
       290                     295                     300
   Tyr Asp Ala Ile Lys Cys Met Lys Thr Phe Phe Gly Trp Lys Glu Pro
   305                     310                     315                 320
   Ser Ile Val Lys Pro His Glu Lys Gly Ile Asn Pro Asn Tyr Leu Gln
                   325                     330                     335
   Thr Trp Lys Gln Val Leu Glu Glu Ile Gln Asp Leu Glu Asn Glu Glu
               340                     345                     350
   Arg Thr Pro Lys Thr Lys Asn Met Lys Lys Thr Ser Gln Leu Lys Trp
           355                     360                     365
   Ala Leu Gly Glu Asn Met Ala Pro Glu Lys Val Asp Phe Glu Asp Cys
       370                     375                     380
   Lys Asp Ile Asn Asp Leu Lys Gln Tyr Asp Ser Asp Glu Pro Glu Thr
   385                     390                     395                 400
   Arg Ser Leu Ala Ser Trp Ile Gln Ser Glu Phe Asn Lys Ala Cys Glu
                   405                 410                 415
   Leu Thr Asp Ser Ser Trp Ile Glu Leu Asp Glu Ile Gly Glu Asp Val
               420                 425                 430
   Ala Pro Ile Glu Tyr Ile Ala Ser Met Arg Arg Asn Tyr Phe Thr Ala
           435                 440                 445
   Glu Ile Ser His Cys Arg Ala Thr Glu Tyr Ile Met Lys Gly Val Tyr
       450                 455                 460
   Ile Asn Thr Ala Leu Leu Asn Ala Ser Cys Ala Ala Met Asp Glu Phe
   465                 470                 475                 480
   Gln Leu Ile Pro Met Ile Ser Lys Cys Arg Thr Lys Glu Gly Arg Arg
                   485                 490                 495
   Lys Thr Asn Leu Tyr Gly Phe Ile Ile Lys Gly Arg Ser His Leu Arg
               500                 505                 510
   Asn Asp Thr Asp Val Val Asn Phe Val Ser Met Glu Phe Ser Leu Thr
           515                 520                 525
   Asp Pro Arg Phe Glu Pro His Lys Trp Glu Lys Tyr Cys Val Leu Glu
       530                 535                 540
   Ile Gly Asp Met Leu Leu Arg Thr Ala Val Gly Gln Val Ser Arg Pro
   545                 550                 555                 560
   Met Phe Leu Tyr Val Arg Thr Asn Gly Thr Ser Lys Ile Lys Met Lys
                   565                 570                 575
   Trp Gly Met Glu Met Arg Arg Cys Leu Leu Gln Ser Leu Gln Gln Ile
               580                 585                 590
   Glu Ser Met Ile Glu Ala Glu Ser Ser Val Lys Glu Lys Asp Met Thr
           595                 600                 605
   Lys Glu Phe Phe Glu Asn Lys Ser Glu Thr Trp Pro Ile Gly Glu Ser
       610                 615                 620
   Pro Lys Gly Val Glu Glu Gly Ser Ile Gly Lys Val Cys Arg Thr Leu
   625                 630                 635                 640
   Leu Ala Lys Ser Val Phe Asn Ser Leu Tyr Ala Ser Pro Gln Leu Glu
                   645                 650                 655
   Gly Phe Ser Ala Glu Ser Arg Lys Leu Leu Leu Ile Val Gln Ala Leu
               660                 665                 670
   Arg Asp Asp Leu Glu Pro Gly Thr Phe Asp Ile Gly Gly Leu Tyr Glu
   675 680 685
   Ser Ile Glu Glu Cys Leu Ile Asn Asp Pro Trp Val Leu Leu Asn Ala
   690 695 700
   Ser Trp Phe Asn Ser Phe Leu Thr His Ala Leu Lys
   705 710 715
<210> 23
   <211> 838
   <212> DNA
   <213> Influenza virus
<220>
   <221> CDS
   <222> (1) .. (657)
<400> 23
   atg gat tcc aac act gtg tca agc ttt cag gta gac tgt ttt ctt tgg        48
   Met Asp Ser Asn Thr Val Ser Ser Phe Gln Val Asp Cys Phe Leu Trp
   1               5                   10                  15
   cat gtc cgc aaa cga ttc gca gac caa gaa ctg ggt gat gcc cca ttc        96
   His Val Arg Lys Arg Phe Ala Asp Gln Glu Leu Gly Asp Ala Pro Phe
               20                  25                  30
   ctt gac cgg ctt cgc cga gac cag aag tcc cta agg gga aga ggt agc       144
   Leu Asp Arg Leu Arg Arg Asp Gln Lys Ser Leu Arg Gly Arg Gly Ser
           35                  40                  45
   act ctt ggt ctg gac atc gaa aca gcc act cat gca gga aag cag ata       192
   Thr Leu Gly Leu Asp Ile Glu Thr Ala Thr His Ala Gly Lys Gln Ile
       50                  55                  60
   gtg gag cag att ctg gaa aag gaa tca gat gag gca ctt aaa atg acc       240
   Val Glu Gln Ile Leu Glu Lys Glu Ser Asp Glu Ala Leu Lys Met Thr
   65                  70                  75                  80
   att gcc tct gtt cct act tca cgc tac tta act gac atg act ctt gat       288
   Ile Ala Ser Val Pro Thr Ser Arg Tyr Leu Thr Asp Met Thr Leu Asp
                   85                  90                  95
   gag atg tca aga gac tgg ttc atg ctc atg ccc aag caa aaa gta aca       336
   Glu Met Ser Arg Asp Trp Phe Met Leu Met Pro Lys Gln Lys Val Thr
               100                 105                 110
   ggc tcc cta tgt ata aga atg gac cag gca atc atg gat aag aac atc       384
   Gly Ser Leu Cys Ile Arg Met Asp Gln Ala Ile Met Asp Lys Asn Ile
           115                 120                 125
   ata ctt aaa gca aac ttt agt gtg att ttc gaa ggg ctg gaa aca cta       432
   Ile Leu Lys Ala Asn Phe Ser Val Ile Phe Glu Gly Leu Glu Thr Leu
       130                 135                 140
   ata cta ctt aga gcc ttc acc gaa gaa gga gca gtc gtt ggc gaa att       480
   Ile Leu Leu Arg Ala Phe Thr Glu Glu Gly Ala Val Val Gly Glu Ile
   145                 150                 155                 160
   tca cca tta cct tct ctt cca gga cat act aat gag gat gtc aaa aat       528
   Ser Pro Leu Pro Ser Leu Pro Gly His Thr Asn Glu Asp Val Lys Asn
                   165                 170                 175
   gca att ggg gtc ctc atc gga gga ctt aaa tgg aat gat aat acg gtt       576
   Ala Ile Gly Val Leu Ile Gly Gly Leu Lys Trp Asn Asp Asn Thr Val
               180                 185                 190
   aga atc tct gaa act cta cag aga ttc gct tgg aga agc agt cat gag       624
   Arg Ile Ser Glu Thr Leu Gln Arg Phe Ala Trp Arg Ser Ser His Glu
           195                 200                 205
   aat ggg aga cct tca ttc cct tca aag cag aaa tgaaaaatgg agagaacaat     677
   Asn Gly Arg Pro Ser Phe Pro Ser Lys Gln Lys
       210                 215
   taagccagaa atttgaagaa ataagatggt tgattgaaga agtgcgacat agattgaaaa     737
   atacagaaaa tagttttgaa caaataacat ttatgcaagc cttacaacta ttgcttgaag     797
   tagaacaaga gataagaact ttctcgtttc agcttattta a                         838
<210> 24
   <211> 219
   <212> PRT
   <213> Influenza virus
<400> 24
   Met Asp Ser Asn Thr Val Ser Ser Phe Gln Val Asp Cys Phe Leu Trp
   1               5                   10                  15
   His Val Arg Lys Arg Phe Ala Asp Gln Glu Leu Gly Asp Ala Pro Phe
               20                  25                  30
   Leu Asp Arg Leu Arg Arg Asp Gln Lys Ser Leu Arg Gly Arg Gly Ser
           35                  40                  45
   Thr Leu Gly Leu Asp Ile Glu Thr Ala Thr His Ala Gly Lys Gln Ile
       50                  55                  60
   Val Glu Gln Ile Leu Glu Lys Glu Ser Asp Glu Ala Leu Lys Met Thr
   65                  70                  75                  80
   Ile Ala Ser Val Pro Thr Ser Arg Tyr Leu Thr Asp Met Thr Leu Asp
                   85                  90                  95
   Glu Met Ser Arg Asp Trp Phe Met Leu Met Pro Lys Gln Lys Val Thr
               100                 105                 110
   Gly Ser Leu Cys Ile Arg Met Asp Gln Ala Ile Met Asp Lys Asn Ile
           115                 120                 125
   Ile Leu Lys Ala Asn Phe Ser Val Ile Phe Glu Gly Leu Glu Thr Leu
       130                 135                 140
   Ile Leu Leu Arg Ala Phe Thr Glu Glu Gly Ala Val Val Gly Glu Ile
   145                 150                 155                 160
   Ser Pro Leu Pro Ser Leu Pro Gly His Thr Asn Glu Asp Val Lys Asn
                   165                 170                 175
   Ala Ile Gly Val Leu Ile Gly Gly Leu Lys Trp Asn Asp Asn Thr Val
               180                 185                 190
   Arg Ile Ser Glu Thr Leu Gln Arg Phe Ala Trp Arg Ser Ser His Glu
           195                 200                 205
   Asn Gly Arg Pro Ser Phe Pro Ser Lys Gln Lys
       210                 215
<210> 25
   <211> 1497
   <212> DNA
   <213> Influenza virus
<220>
   <221> CDS
   <222> (1) .. (1497)
<400> 25
   atg gcg tct caa ggc acc aaa cga tcc tat gaa cag atg gaa act gat      48
   Met Ala Ser Gln Gly Thr Lys Arg Ser Tyr Glu Gln Met Glu Thr Asp
   1               5                   10                  15
   ggg gaa cgc cag aat gca act gaa atc aga gca tct gtc gga agg atg      96
   Gly Glu Arg Gln Asn Ala Thr Glu Ile Arg Ala Ser Val Gly Arg Met
               20                  25                  30
   gtg gga gga atc ggc cgg ttt tat gtt cag atg tgt act gag ctt aaa     144
   Val Gly Gly Ile Gly Arg Phe Tyr Val Gln Met Cys Thr Glu Leu Lys
           35                  40                  45
   cta aac gac cat gaa ggg cgg ctg att cag aac agc ata aca ata gaa     192
   Leu Asn Asp His Glu Gly Arg Leu Ile Gln Asn Ser Ile Thr Ile Glu
       50                  55                  60
   agg atg gta ctt tcg gca ttc gac gaa aga aga aac aag tat ctc gag     240
   Arg Met Val Leu Ser Ala Phe Asp Glu Arg Arg Asn Lys Tyr Leu Glu
   65                  70                  75                  80
   gag cat ccc agt gct ggg aaa gac cct aag aaa acg gga ggc ccg ata     288
   Glu His Pro Ser Ala Gly Lys Asp Pro Lys Lys Thr Gly Gly Pro Ile
                   85                  90                  95
   tac aga agg aaa gat ggg aaa tgg atg agg gaa ctc atc ctc cat gat     336
   Tyr Arg Arg Lys Asp Gly Lys Trp Met Arg Glu Leu Ile Leu His Asp
               100                 105                 110
   aaa gaa gaa atc atg aga atc tgg cgt cag gcc aac aat ggt gaa gac     384
   Lys Glu Glu Ile Met Arg Ile Trp Arg Gln Ala Asn Asn Gly Glu Asp
           115                 120                 125
   gct act gct ggt ctt act cat atg atg atc tgg cac tcc aat ctc aat     432
   Ala Thr Ala Gly Leu Thr His Met Met Ile Trp His Ser Asn Leu Asn
       130                 135                 140
   gac acc aca tac caa aga aca agg gct ctt gtt cgg act ggg atg gat     480
   Asp Thr Thr Tyr Gln Arg Thr Arg Ala Leu Val Arg Thr Gly Met Asp
   145                 150                 155                 160
   ccc aga atg tgc tct ctg atg caa ggc tca acc ctc cca cgg aga tct     528
   Pro Arg Met Cys Ser Leu Met Gln Gly Ser Thr Leu Pro Arg Arg Ser
                   165                 170                 175
   gga gcc gct ggt gct gca gta aaa ggt gtt gga aca atg gta atg gaa     576
   Gly Ala Ala Gly Ala Ala Val Lys Gly Val Gly Thr Met Val Met Glu
               180                 185                 190
   ctc atc aga atg atc aaa cgc gga ata aat gat cgg aat ttc tgg aga      624
   Leu Ile Arg Met Ile Lys Arg Gly Ile Asn Asp Arg Asn Phe Trp Arg
           195                 200                 205
   ggt gaa aat ggt cga aga acc aga att gct tat gaa aga atg tgc aat      672
   Gly Glu Asn Gly Arg Arg Thr Arg Ile Ala Tyr Glu Arg Met Cys Asn
       210                 215                 220
   atc ctc aaa ggg aaa ttt cag aca gca gca caa cgg gct atg atg gac      720
   Ile Leu Lys Gly Lys Phe Gln Thr Ala Ala Gln Arg Ala Met Met Asp
   225                 230                 235                 240
   cag gtg agg gaa ggc cgc aat cct gga aac gct gag att gag gat ctc      768
   Gln Val Arg Glu Gly Arg Asn Pro Gly Asn Ala Glu Ile Glu Asp Leu
                   245                 250                 255
   att ttc ttg gca cga tca gca ctt att ttg aga gga tca gta gcc cat      816
   Ile Phe Leu Ala Arg Ser Ala Leu Ile Leu Arg Gly Ser Val Ala His
               260                 265                 270
   aaa tca tgc cta cct gcc tgt gtt tat ggc ctt gca gta acc agt ggg      864
   Lys Ser Cys Leu Pro Ala Cys Val Tyr Gly Leu Ala Val Thr Ser Gly
           275                 280                 285
   tat gac ttt gag aag gaa gga tac tct ctg gtt gga att gat cct ttc      912
   Tyr Asp Phe Glu Lys Glu Gly Tyr Ser Leu Val Gly Ile Asp Pro Phe
       290                 295                 300
   aaa cta ctc cag aac agt caa att ttc agt cta atc aga cca aaa gaa      960
   Lys Leu Leu Gln Asn Ser Gln Ile Phe Ser Leu Ile Arg Pro Lys Glu
   305                 310                 315                 320
   aac cca gca cac aag agc cag ttg gtg tgg atg gca tgc cat tct gca     1008
   Asn Pro Ala His Lys Ser Gln Leu Val Trp Met Ala Cys His Ser Ala
                   325                 330                 335
   gca ttt gag gac ctg aga gtt tta aat ttc att aga gga acc aaa gta     1056
   Ala Phe Glu Asp Leu Arg Val Leu Asn Phe Ile Arg Gly Thr Lys Val
               340                 345                 350
   atc cca aga gga cag tta aca acc aga gga gtt caa att gct tca aat     1104
   Ile Pro Arg Gly Gln Leu Thr Thr Arg Gly Val Gln Ile Ala Ser Asn
           355                 360                 365
   gaa aac atg gag aca ata gat tct agc aca ctt gaa ctg aga agc aaa     1152
   Glu Asn Met Glu Thr Ile Asp Ser Ser Thr Leu Glu Leu Arg Ser Lys
       370                 375                 380
   tat tgg gca ata agg acc aga agc gga gga aac acc agt caa cag aga     1200
   Tyr Trp Ala Ile Arg Thr Arg Ser Gly Gly Asn Thr Ser Gln Gln Arg
   385                 390                 395                 400
   gca tct gca gga cag ata agt gtg caa cct act ttc tca gta cag aga     1248
   Ala Ser Ala Gly Gln Ile Ser Val Gln Pro Thr Phe Ser Val Gln Arg
                   405                 410                 415
   aat ctt ccc ttt gag aga gca acc att atg gct gca ttc act ggt aac     1296
   Asn Leu Pro Phe Glu Arg Ala Thr Ile Met Ala Ala Phe Thr Gly Asn
               420                 425                 430
   act gaa ggg agg act tcc gac atg aga acg gaa atc ata agg atg atg     1344
   Thr Glu Gly Arg Thr Ser Asp Met Arg Thr Glu Ile Ile Arg Met Met
           435                 440                 445
   gaa aat gcc aaa tca gaa gat gtg tct ttc cag ggg cgg gga gtc ttc     1392
   Glu Asn Ala Lys Ser Glu Asp Val Ser Phe Gln Gly Arg Gly Val Phe
       450                 455                 460
   gag ctc tcg gac gaa aag gca acg aac ccg atc gtg cct tcc ttt gac      1440
   Glu Leu Ser Asp Glu Lys Ala Thr Asn Pro Ile Val Pro Ser Phe Asp
   465                 470                 475                 480
   atg agc aat gaa ggg tct tat ttc ttc gga gac aat gct gag gag ttt      1488
   Met Ser Asn Glu Gly Ser Tyr Phe Phe Gly Asp Asn Ala Glu Glu Phe
                   485                 490                 495
   gac agt taa                                                          1497
   Asp Ser
<210> 26
   <211> 498
   <212> PRT
   <213> Influenza virus
<400> 26
   Met Ala Ser Gln Gly Thr Lys Arg Ser Tyr Glu Gln Met Glu Thr Asp
   1               5                   10                  15
   Gly Glu Arg Gln Asn Ala Thr Glu Ile Arg Ala Ser Val Gly Arg Met
               20                  25                  30
   Val Gly Gly Ile Gly Arg Phe Tyr Val Gln Met Cys Thr Glu Leu Lys
           35                  40                  45
   Leu Asn Asp His Glu Gly Arg Leu Ile Gln Asn Ser Ile Thr Ile Glu
       50                  55                  60
   Arg Met Val Leu Ser Ala Phe Asp Glu Arg Arg Asn Lys Tyr Leu Glu
   65                  70                  75                  80
   Glu His Pro Ser Ala Gly Lys Asp Pro Lys Lys Thr Gly Gly Pro Ile
                   85                  90                  95
   Tyr Arg Arg Lys Asp Gly Lys Trp Met Arg Glu Leu Ile Leu His Asp
               100                 105                 110
   Lys Glu Glu Ile Met Arg Ile Trp Arg Gln Ala Asn Asn Gly Glu Asp
           115                 120                 125
   Ala Thr Ala Gly Leu Thr His Met Met Ile Trp His Ser Asn Leu Asn
       130                 135                 140
   Asp Thr Thr Tyr Gln Arg Thr Arg Ala Leu Val Arg Thr Gly Met Asp
   145                 150                 155                 160
   Pro Arg Met Cys Ser Leu Met Gln Gly Ser Thr Leu Pro Arg Arg Ser
                   165                 170                 175
   Gly Ala Ala Gly Ala Ala Val Lys Gly Val Gly Thr Met Val Met Glu
               180                 185                 190
   Leu Ile Arg Met Ile Lys Arg Gly Ile Asn Asp Arg Asn Phe Trp Arg
           195                 200                 205
   Gly Glu Asn Gly Arg Arg Thr Arg Ile Ala Tyr Glu Arg Met Cys Asn
       210                 215                 220
   Ile Leu Lys Gly Lys Phe Gln Thr Ala Ala Gln Arg Ala Met Met Asp
   225                 230                 235                 240
   Gln Val Arg Glu Gly Arg Asn Pro Gly Asn Ala Glu Ile Glu Asp Leu
                   245                 250                 255
   Ile Phe Leu Ala Arg Ser Ala Leu Ile Leu Arg Gly Ser Val Ala His
               260                 265                 270
   Lys Ser Cys Leu Pro Ala Cys Val Tyr Gly Leu Ala Val Thr Ser Gly
           275                 280                 285
   Tyr Asp Phe Glu Lys Glu Gly Tyr Ser Leu Val Gly Ile Asp Pro Phe
       290                 295                 300
   Lys Leu Leu Gln Asn Ser Gln Ile Phe Ser Leu Ile Arg Pro Lys Glu
   305                 310                 315                 320
   Asn Pro Ala His Lys Ser Gln Leu Val Trp Met Ala Cys His Ser Ala
                   325                 330                 335
   Ala Phe Glu Asp Leu Arg Val Leu Asn Phe Ile Arg Gly Thr Lys Val
               340                 345                 350
   Ile Pro Arg Gly Gln Leu Thr Thr Arg Gly Val Gln Ile Ala Ser Asn
           355                 360                 365
   Glu Asn Met Glu Thr Ile Asp Ser Ser Thr Leu Glu Leu Arg Ser Lys
       370                 375                 380
   Tyr Trp Ala Ile Arg Thr Arg Ser Gly Gly Asn Thr Ser Gln Gln Arg
   385                 390                 395                 400
   Ala Ser Ala Gly Gln Ile Ser Val Gln Pro Thr Phe Ser Val Gln Arg
                   405                 410                 415
   Asn Leu Pro Phe Glu Arg Ala Thr Ile Met Ala Ala Phe Thr Gly Asn
               420                 425                 430
   Thr Glu Gly Arg Thr Ser Asp Met Arg Thr Glu Ile Ile Arg Met Met
           435                 440                 445
   Glu Asn Ala Lys Ser Glu Asp Val Ser Phe Gln Gly Arg Gly Val Phe
       450                 455                 460
   Glu Leu Ser Asp Glu Lys Ala Thr Asn Pro Ile Val Pro Ser Phe Asp
   465 470 475 480
   Met Ser Asn Glu Gly Ser Tyr Phe Phe Gly Asp Asn Ala Glu Glu Phe
   485 490 495
   Asp Ser
<210> 27
   <211> 1410
   <212> DNA
   <213> Influenza virus
<220>
   <221> CDS
   <222> (1) .. (1410)
<400> 27
   atg aat cca aat caa aag ata ata aca att gga ttt gca tca ttg ggg      48
   Met Asn Pro Asn Gln Lys Ile Ile Thr Ile Gly Phe Ala Ser Leu Gly
   1               5                   10                  15
   ata tta atc att aat gtc att ctc cat gta gtc agc att ata gta aca      96
   Ile Leu Ile Ile Asn Val Ile Leu His Val Val Ser Ile Ile Val Thr
               20                  25                  30
   gta ctg gtc ctc aat aac aat aga aca gat ctg aac tgc aaa ggg acg     144
   Val Leu Val Leu Asn Asn Asn Arg Thr Asp Leu Asn Cys Lys Gly Thr
           35                  40                  45
   atc ata aga gag tac aat gaa aca gta aga gta gaa aaa att act caa     192
   Ile Ile Arg Glu Tyr Asn Glu Thr Val Arg Val Glu Lys Ile Thr Gln
       50                  55                  60
   tgg tat aat acc agt aca att aag tac ata gag aga cct tca aat gaa     240
   Trp Tyr Asn Thr Ser Thr Ile Lys Tyr Ile Glu Arg Pro Ser Asn Glu
   65                  70                  75                  80
   tac tac atg aac aac act gaa cca ctt tgt gag gcc caa ggc ttt gca     288
   Tyr Tyr Met Asn Asn Thr Glu Pro Leu Cys Glu Ala Gln Gly Phe Ala
                   85                  90                  95
   cca ttt tcc aaa gat aat gga ata cga att ggg tcg aga ggc cat gtt     336
   Pro Phe Ser Lys Asp Asn Gly Ile Arg Ile Gly Ser Arg Gly His Val
               100                 105                 110
   ttt gtg ata aga gaa cct ttt gta tca tgt tcg ccc tca gaa tgt aga     384
   Phe Val Ile Arg Glu Pro Phe Val Ser Cys Ser Pro Ser Glu Cys Arg
           115                 120                 125
   acc ttt ttc ctc aca cag ggc tca tta ctc aat gac aaa cat tct aac     432
   Thr Phe Phe Leu Thr Gln Gly Ser Leu Leu Asn Asp Lys His Ser Asn
       130                 135                 140
   ggc aca gta aag gac cga agt ccg tat agg act ttg atg agt gtc aaa     480
   Gly Thr Val Lys Asp Arg Ser Pro Tyr Arg Thr Leu Met Ser Val Lys
   145                 150                 155                 160
   ata ggg caa tca cct aat gta tat caa gct agg ttt gaa tcg gtg gca     528
   Ile Gly Gln Ser Pro Asn Val Tyr Gln Ala Arg Phe Glu Ser Val Ala
                   165                 170                 175
   tgg tca gca aca gca tgc cat gat gga aaa aaa tgg atg aca gtt gga     576
   Trp Ser Ala Thr Ala Cys His Asp Gly Lys Lys Trp Met Thr Val Gly
               180                 185                 190
   gtc aca ggg ccc gac aat caa gca att gca gta gtg aac tat gga ggt      624
   Val Thr Gly Pro Asp Asn Gln Ala Ile Ala Val Val Asn Tyr Gly Gly
           195                 200                 205
   gtt ccg gtt gat att att aat tca tgg gca ggg gat att tta aga acc      672
   Val Pro Val Asp Ile Ile Asn Ser Trp Ala Gly Asp Ile Leu Arg Thr
       210                 215                 220
   caa gaa tca tca tgc acc tgc att aaa gga gac tgt tat tgg gta atg      720
   Gln Glu Ser Ser Cys Thr Cys Ile Lys Gly Asp Cys Tyr Trp Val Met
   225                 230                 235                 240
   act gat gga ccg gca aat agg caa gct aaa tat agg ata ttc aaa gca      768
   Thr Asp Gly Pro Ala Asn Arg Gln Ala Lys Tyr Arg Ile Phe Lys Ala
                   245                 250                 255
   aaa gat gga aga gta att gga cag act gat ata agt ttc aat ggg gga      816
   Lys Asp Gly Arg Val Ile Gly Gln Thr Asp Ile Ser Phe Asn Gly Gly
               260                 265                 270
   cac ata gag gag tgt tct tgt tac ccc aat gaa ggg aag gtg gaa tgc      864
   His Ile Glu Glu Cys Ser Cys Tyr Pro Asn Glu Gly Lys Val Glu Cys
           275                 280                 285
   ata tgc agg gac aat tgg act gga aca aat aga cca att ctg gta ata      912
   Ile Cys Arg Asp Asn Trp Thr Gly Thr Asn Arg Pro Ile Leu Val Ile
       290                 295                 300
   tct tct gat cta tcg tac aca gtt gga tat ttg tgt gct ggc att ccc      960
   Ser Ser Asp Leu Ser Tyr Thr Val Gly Tyr Leu Cys Ala Gly Ile Pro
   305                 310                 315                 320
   act gac act cct agg gga gag gat agt caa ttc aca ggc tca tgt aca     1008
   Thr Asp Thr Pro Arg Gly Glu Asp Ser Gln Phe Thr Gly Ser Cys Thr
                   325                 330                 335
   agt cct ttg gga aat aaa gga tac ggt gta aaa ggt ttc ggg ttt cga     1056
   Ser Pro Leu Gly Asn Lys Gly Tyr Gly Val Lys Gly Phe Gly Phe Arg
               340                 345                 350
   caa gga act gac gta tgg gcc gga agg aca att agt agg act tca aga     1104
   Gln Gly Thr Asp Val Trp Ala Gly Arg Thr Ile Ser Arg Thr Ser Arg
           355                 360                 365
   tca gga ttc gaa ata ata aaa atc agg aat ggt tgg aca cag aac agt     1152
   Ser Gly Phe Glu Ile Ile Lys Ile Arg Asn Gly Trp Thr Gln Asn Ser
       370                 375                 380
   aaa gac caa atc agg agg caa gtg att atc gat gac cca aat tgg tca     1200
   Lys Asp Gln Ile Arg Arg Gln Val Ile Ile Asp Asp Pro Asn Trp Ser
   385                 390                 395                 400
   gga tat agc ggt tct ttc aca ttg ccg gtt gaa cta aca aaa aag gga     1248
   Gly Tyr Ser Gly Ser Phe Thr Leu Pro Val Glu Leu Thr Lys Lys Gly
                   405                 410                 415
   tgt ttg gtc ccc tgt ttc tgg gtt gaa atg att aga ggt aaa cct gaa     1296
   Cys Leu Val Pro Cys Phe Trp Val Glu Met Ile Arg Gly Lys Pro Glu
               420                 425                 430
   gaa aca aca ata tgg acc tct agc agc tcc att gtg atg tgt gga gta     1344
   Glu Thr Thr Ile Trp Thr Ser Ser Ser Ser Ile Val Met Cys Gly Val
           435                 440                 445
   gat cat aaa att gcc agt tgg tca tgg cac gat gga gct att ctt ccc     1392
   Asp His Lys Ile Ala Ser Trp Ser Trp His Asp Gly Ala Ile Leu Pro
       450                 455                 460
   ttt gac atc gat aag atg                                         1410
   Phe Asp Ile Asp Lys Met
   465                 470
<210> 28
   <211> 470
   <212> PRT
   <213> Influenza virus
<400> 28
   Met Asn Pro Asn Gln Lys Ile Ile Thr Ile Gly Phe Ala Ser Leu Gly
   1               5                   10                  15
   Ile Leu Ile Ile Asn Val Ile Leu His Val Val Ser Ile Ile Val Thr
               20                  25                  30
   Val Leu Val Leu Asn Asn Asn Arg Thr Asp Leu Asn Cys Lys Gly Thr
           35                  40                  45
   Ile Ile Arg Glu Tyr Asn Glu Thr Val Arg Val Glu Lys Ile Thr Gln
       50                  55                  60
   Trp Tyr Asn Thr Ser Thr Ile Lys Tyr Ile Glu Arg Pro Ser Asn Glu
   65                  70                  75                  80
   Tyr Tyr Met Asn Asn Thr Glu Pro Leu Cys Glu Ala Gln Gly Phe Ala
                   85                  90                  95
   Pro Phe Ser Lys Asp Asn Gly Ile Arg Ile Gly Ser Arg Gly His Val
               100                 105                 110
   Phe Val Ile Arg Glu Pro Phe Val Ser Cys Ser Pro Ser Glu Cys Arg
           115                 120                 125
   Thr Phe Phe Leu Thr Gln Gly Ser Leu Leu Asn Asp Lys His Ser Asn
       130                 135                 140
   Gly Thr Val Lys Asp Arg Ser Pro Tyr Arg Thr Leu Met Ser Val Lys
   145                 150                 155                 160
   Ile Gly Gln Ser Pro Asn Val Tyr Gln Ala Arg Phe Glu Ser Val Ala
                   165                 170                 175
   Trp Ser Ala Thr Ala Cys His Asp Gly Lys Lys Trp Met Thr Val Gly
               180                 185                 190
   Val Thr Gly Pro Asp Asn Gln Ala Ile Ala Val Val Asn Tyr Gly Gly
           195                 200                 205
   Val Pro Val Asp Ile Ile Asn Ser Trp Ala Gly Asp Ile Leu Arg Thr
       210                 215                 220
   Gln Glu Ser Ser Cys Thr Cys Ile Lys Gly Asp Cys Tyr Trp Val Met
   225                 230                 235                 240
   Thr Asp Gly Pro Ala Asn Arg Gln Ala Lys Tyr Arg Ile Phe Lys Ala
                   245                 250                 255
   Lys Asp Gly Arg Val Ile Gly Gln Thr Asp Ile Ser Phe Asn Gly Gly
               260                 265                 270
   His Ile Glu Glu Cys Ser Cys Tyr Pro Asn Glu Gly Lys Val Glu Cys
           275                 280                 285
   Ile Cys Arg Asp Asn Trp Thr Gly Thr Asn Arg Pro Ile Leu Val Ile
       290                 295                 300
   Ser Ser Asp Leu Ser Tyr Thr Val Gly Tyr Leu Cys Ala Gly Ile Pro
   305                 310                 315                 320
   Thr Asp Thr Pro Arg Gly Glu Asp Ser Gln Phe Thr Gly Ser Cys Thr
                   325                 330                 335
   Ser Pro Leu Gly Asn Lys Gly Tyr Gly Val Lys Gly Phe Gly Phe Arg
               340                 345                 350
   Gln Gly Thr Asp Val Trp Ala Gly Arg Thr Ile Ser Arg Thr Ser Arg
           355                 360                 365
   Ser Gly Phe Glu Ile Ile Lys Ile Arg Asn Gly Trp Thr Gln Asn Ser
       370                 375                 380
   Lys Asp Gln Ile Arg Arg Gln Val Ile Ile Asp Asp Pro Asn Trp Ser
   385                 390                 395                 400
   Gly Tyr Ser Gly Ser Phe Thr Leu Pro Val Glu Leu Thr Lys Lys Gly
                   405                 410                 415
   Cys Leu Val Pro Cys Phe Trp Val Glu Met Ile Arg Gly Lys Pro Glu
               420                 425                 430
   Glu Thr Thr Ile Trp Thr Ser Ser Ser Ser Ile Val Met Cys Gly Val
           435                 440                 445
   Asp His Lys Ile Ala Ser Trp Ser Trp His Asp Gly Ala Ile Leu Pro
       450                 455                 460
   Phe Asp Ile Asp Lys Met
   465                 470
<210> 29
   <211> 982
   <212> DNA
   <213> Influenza virus
<220>
   <221> CDS
   <222> (1) .. (756)
<400> 29
   atg agt ctt cta acc gag gtc gaa acg tac gtt ctc tct atc gta cca 48
   Met Ser Leu Leu Thr Glu Val Glu Thr Tyr Val Leu Ser Ile Val Pro
   1 5 10 15
   tca ggc ccc ctc aaa gcc gag atc gcg cag aga ctt gaa gat gtc ttt 96
   Ser Gly Pro Leu Lys Ala Glu Ile Ala Gln Arg Leu Glu Asp Val Phe
   20 25 30
   gca ggg aag aac acc gat ctt gag gca ctc atg gaa tgg cta aag aca 144
   Ala Gly Lys Asn Thr Asp Leu Glu Ala Leu Met Glu Trp Leu Lys Thr
   35 40 45
   aga cca atc ctg tca cct ctg act aaa ggg att tta gga ttt gta ttc 192
   Arg Pro Ile Leu Ser Pro Leu Thr Lys Gly Ile Leu Gly Phe Val Phe
   50 55 60
   acg ctc acc gtg ccc agt gag cga gga ctg cag cgt aga cgc ttt gtc 240
   Thr Leu Thr Val Pro Ser Glu Arg Gly Leu Gln Arg Arg Arg Phe Val
   65 70 75 80
   caa aat gcc ctt agt gga aac gga gat cca aac aac atg gac aga gca 288
   Gln Asn Ala Leu Ser Gly Asn Gly Asp Pro Asn Asn Met Asp Arg Ala
   85 90 95
   gta aaa ctg tac agg aag ctt aaa aga gaa ata aca ttc cat ggg gca 336
   Val Lys Leu Tyr Arg Lys Leu Lys Arg Glu Ile Thr Phe His Gly Ala
   100 105 110
   aaa gag gtg gca ctc agc tat tcc act ggt gca cta gcc agc tgc atg 384
   Lys Glu Val Ala Leu Ser Tyr Ser Thr Gly Ala Leu Ala Ser Cys Met
   115 120 125
   gga ctc ata tac aac aga atg gga act gtt aca acc gaa gtg gca ttt 432
   Gly Leu Ile Tyr Asn Arg Met Gly Thr Val Thr Thr Glu Val Ala Phe
   130 135 140
   ggc ctg gta tgc gcc aca tgt gaa cag att gct gat tcc cag cat cga 480
   Gly Leu Val Cys Ala Thr Cys Glu Gln Ile Ala Asp Ser Gln His Arg
   145 150 155 160
   tct cac agg cag atg gtg aca aca acc aac cca tta atc aga cat gaa 528
   Ser His Arg Gln Met Val Thr Thr Thr Asn Pro Leu Ile Arg His Glu
   165 170 175
   aac aga atg gta tta gcc agt acc acg gct aaa gcc atg gaa cag atg 576
   Asn Arg Met Val Leu Ala Ser Thr Thr Ala Lys Ala Met Glu Gln Met
   180 185 190
   gca gga tcg agt gag cag gca gca gag gcc atg gag gtt gct agt agg 624
   Ala Gly Ser Ser Glu Gln Ala Ala Glu Ala Met Glu Val Ala Ser Arg
   195 200 205
   gct agg cag atg gta cag gca atg aga acc att ggg acc cac cct agc 672
   Ala Arg Gln Met Val Gln Ala Met Arg Thr Ile Gly Thr His Pro Ser
   210 215 220
   tcc agt gcc ggt ttg aaa gat gat ctc ctt gaa aat tta cag gcc tac 720
   Ser Ser Ala Gly Leu Lys Asp Asp Leu Leu Glu Asn Leu Gln Ala Tyr
   225 230 235 240
   cag aaa cgg atg gga gtg caa atg cag cga ttc aag tgatcctctc 766
   Gln Lys Arg Met Gly Val Gln Met Gln Arg Phe Lys
   245 250
   gttattgcag caagtatcat tgggatcttg cacttgatat tgtggattct tgatcgtctt 826
   ttcttcaaat tcatttatcg tcgccttaaa tacgggttga aaagagggcc ttctacggaa 886
   ggagtacctg agtctatgag ggaagaatat cggcaggaac agcagaatgc tgtggatgtt 946
   gacgatggtc attttgtcaa catagagctg gagtaa 982
<210> 30
   <211> 252
   <212> PRT
   <213> Influenza virus
<400> 30
   Met Ser Leu Leu Thr Glu Val Glu Thr Tyr Val Leu Ser Ile Val Pro
   1               5                   10                  15
   Ser Gly Pro Leu Lys Ala Glu Ile Ala Gln Arg Leu Glu Asp Val Phe
               20                  25                  30
   Ala Gly Lys Asn Thr Asp Leu Glu Ala Leu Met Glu Trp Leu Lys Thr
           35                  40                  45
   Arg Pro Ile Leu Ser Pro Leu Thr Lys Gly Ile Leu Gly Phe Val Phe
       50                  55                  60
   Thr Leu Thr Val Pro Ser Glu Arg Gly Leu Gln Arg Arg Arg Phe Val
   65                  70                  75                  80
   Gln Asn Ala Leu Ser Gly Asn Gly Asp Pro Asn Asn Met Asp Arg Ala
                   85                  90                  95
   Val Lys Leu Tyr Arg Lys Leu Lys Arg Glu Ile Thr Phe His Gly Ala
               100                 105                 110
   Lys Glu Val Ala Leu Ser Tyr Ser Thr Gly Ala Leu Ala Ser Cys Met
           115                 120                 125
   Gly Leu Ile Tyr Asn Arg Met Gly Thr Val Thr Thr Glu Val Ala Phe
       130                 135                 140
   Gly Leu Val Cys Ala Thr Cys Glu Gln Ile Ala Asp Ser Gln His Arg
   145                 150                 155                 160
   Ser His Arg Gln Met Val Thr Thr Thr Asn Pro Leu Ile Arg His Glu
                   165                 170                 175
   Asn Arg Met Val Leu Ala Ser Thr Thr Ala Lys Ala Met Glu Gln Met
               180                 185                 190
   Ala Gly Ser Ser Glu Gln Ala Ala Glu Ala Met Glu Val Ala Ser Arg
           195                 200                 205
   Ala Arg Gln Met Val Gln Ala Met Arg Thr Ile Gly Thr His Pro Ser
       210                 215                 220
   Ser Ser Ala Gly Leu Lys Asp Asp Leu Leu Glu Asn Leu Gln Ala Tyr
   225                 230                 235                 240
   Gln Lys Arg Met Gly Val Gln Met Gln Arg Phe Lys
                   245                 250
<210> 31
   <211> 1698
   <212> DNA
   <213> Influenza virus
<220>
   <221> CDS
   <222> (1) .. (1698)
<400> 31
   atg aag aca acc att att ttg ata cta ctg acc cat tgg gcc tac agt        48
   Met Lys Thr Thr Ile Ile Leu Ile Leu Leu Thr His Trp Ala Tyr Ser
   1               5                   10                  15
   caa aac cca atc agt gac aac aac aca gcc aca ctg tgt ctg gga cac        96
   Gln Asn Pro Ile Ser Asp Asn Asn Thr Ala Thr Leu Cys Leu Gly His
               20                  25                  30
   cat gca gta gca aat gga aca ttg gta aaa aca ata agt gat gat caa       144
   His Ala Val Ala Asn Gly Thr Leu Val Lys Thr Ile Ser Asp Asp Gln
           35                  40                  45
   att gag gtg aca aat gct aca gaa tta gtt cag agc att tca atg ggg       192
   Ile Glu Val Thr Asn Ala Thr Glu Leu Val Gln Ser Ile Ser Met Gly
       50                  55                  60
   aaa ata tgc aac aaa tca tat aga att cta gat gga aga aat tgc aca       240
   Lys Ile Cys Asn Lys Ser Tyr Arg Ile Leu Asp Gly Arg Asn Cys Thr
   65                  70                  75                  80
   tta ata gat gca atg cta gga gac ccc cac tgt gac gcc ttt cag tat       288
   Leu Ile Asp Ala Met Leu Gly Asp Pro His Cys Asp Ala Phe Gln Tyr
                   85                  90                  95
   gag agt tgg gac ctc ttt ata gaa aga agc agc gct ttc agc aat tgc       336
   Glu Ser Trp Asp Leu Phe Ile Glu Arg Ser Ser Ala Phe Ser Asn Cys
               100                 105                 110
   tac cca tat gac atc cct gac tat gca tcg ctc cga tcc att gta gca       384
   Tyr Pro Tyr Asp Ile Pro Asp Tyr Ala Ser Leu Arg Ser Ile Val Ala
           115                 120                 125
   tcc tca gga aca ttg gaa ttc aca gca gag gga ttc aca tgg aca ggt       432
   Ser Ser Gly Thr Leu Glu Phe Thr Ala Glu Gly Phe Thr Trp Thr Gly
       130                 135                 140
   gtc act caa aac gga aga agt gga gcc tgc aaa agg gga tca gcc gat       480
   Val Thr Gln Asn Gly Arg Ser Gly Ala Cys Lys Arg Gly Ser Ala Asp
   145                 150                 155                 160
   agt ttc ttt agc cga ctg aat tgg cta aca aaa tct gga agc tct tac       528
   Ser Phe Phe Ser Arg Leu Asn Trp Leu Thr Lys Ser Gly Ser Ser Tyr
                   165                 170                 175
   ccc aca ttg aat gtg aca atg cct aac aat aaa aat ttc gac aag cta       576
   Pro Thr Leu Asn Val Thr Met Pro Asn Asn Lys Asn Phe Asp Lys Leu
               180                 185                 190
   tac atc tgg ggg att cat cac ccg agc tca aat caa gag cag aca aaa       624
   Tyr Ile Trp Gly Ile His His Pro Ser Ser Asn Gln Glu Gln Thr Lys
           195                 200                 205
   ttg tac atc caa gaa tca gga cga gta aca gtc tca aca aaa aga agt       672
   Leu Tyr Ile Gln Glu Ser Gly Arg Val Thr Val Ser Thr Lys Arg Ser
       210                 215                 220
   caa caa aca ata atc cct aac atc gga tct aga ccg ttg gtc aga ggt       720
   Gln Gln Thr Ile Ile Pro Asn Ile Gly Ser Arg Pro Leu Val Arg Gly
   225                 230                 235                 240
   caa tca ggc agg ata agc ata tac tgg acc att gta aaa cct gga gat       768
   Gln Ser Gly Arg Ile Ser Ile Tyr Trp Thr Ile Val Lys Pro Gly Asp
                   245                 250                 255
   atc cta atg ata aac agt aat ggc aac tta gtt gca ccg cgg gga tat       816
   Ile Leu Met Ile Asn Ser Asn Gly Asn Leu Val Ala Pro Arg Gly Tyr
               260                 265                 270
   ttt aaa ttg aaa aca ggg aaa agc tct gta atg aga tca gat gta ccc       864
   Phe Lys Leu Lys Thr Gly Lys Ser Ser Val Met Arg Ser Asp Val Pro
           275                 280                 285
   ata gac att tgt gtg tct gaa tgt att aca cca aat gga agc atc tcc       912
   Ile Asp Ile Cys Val Ser Glu Cys Ile Thr Pro Asn Gly Ser Ile Ser
       290                 295                 300
   aac gac aag cca ttc caa aat gtg aac aaa gtt aca tat gga aaa tgc       960
   Asn Asp Lys Pro Phe Gln Asn Val Asn Lys Val Thr Tyr Gly Lys Cys
   305                 310                 315                 320
   ccc aag tat atc agg caa aac act tta aag ctg gcc act ggg atg agg      1008
   Pro Lys Tyr Ile Arg Gln Asn Thr Leu Lys Leu Ala Thr Gly Met Arg
                   325                 330                 335
   aat gta cca gaa aag caa acc aga gga atc ttt gga gca ata gcg gga      1056
   Asn Val Pro Glu Lys Gln Thr Arg Gly Ile Phe Gly Ala Ile Ala Gly
               340                 345                 350
   ttc atc gaa aac ggc tgg gaa gga atg gtt gat ggg tgg tat ggg ttc      1104
   Phe Ile Glu Asn Gly Trp Glu Gly Met Val Asp Gly Trp Tyr Gly Phe
           355                 360                 365
   cga tat caa aac tct gaa gga aca ggg caa gct gca gat cta aag agc      1152
   Arg Tyr Gln Asn Ser Glu Gly Thr Gly Gln Ala Ala Asp Leu Lys Ser
       370                 375                 380
   act caa gca gcc atc gac cag att aat gga aag tta aac aga gtg att      1200
   Thr Gln Ala Ala Ile Asp Gln Ile Asn Gly Lys Leu Asn Arg Val Ile
   385                 390                 395                 400
   gaa aga acc aat gag aaa ttc cat caa ata gag aag gaa ttc tca gaa      1248
   Glu Arg Thr Asn Glu Lys Phe His Gln Ile Glu Lys Glu Phe Ser Glu
                   405                 410                 415
   gta gaa gga aga att cag gac ttg gag aaa tat gta gaa gac acc aaa      1296
   Val Glu Gly Arg Ile Gln Asp Leu Glu Lys Tyr Val Glu Asp Thr Lys
               420                 425                 430
   ata gac cta tgg tcc tac aat gca gaa ttg ctg gtg gct cta gaa aat      1344
   Ile Asp Leu Trp Ser Tyr Asn Ala Glu Leu Leu Val Ala Leu Glu Asn
           435                 440                 445
   caa cat aca att gac tta aca gat gca gaa atg aat aaa tta ttt gag      1392
   Gln His Thr Ile Asp Leu Thr Asp Ala Glu Met Asn Lys Leu Phe Glu
       450                 455                 460
   aag act aga cgc cag tta aga gaa aac gca gaa gac atg gga ggt gga      1440
   Lys Thr Arg Arg Gln Leu Arg Glu Asn Ala Glu Asp Met Gly Gly Gly
   465                 470                 475                 480
   tgt ttc aag att tac cac aaa tgt gat aat gca tgc att gga tca ata      1488
   Cys Phe Lys Ile Tyr His Lys Cys Asp Asn Ala Cys Ile Gly Ser Ile
                   485                 490                 495
   aga act ggg aca tat gac cat tac ata tac aga gat gaa gca tta aac      1536
   Arg Thr Gly Thr Tyr Asp His Tyr Ile Tyr Arg Asp Glu Ala Leu Asn
               500                 505                 510
   aac cga ttt cag atc aaa ggt gta gag ttg aaa tca ggc tac aaa gat      1584
   Asn Arg Phe Gln Ile Lys Gly Val Glu Leu Lys Ser Gly Tyr Lys Asp
           515                 520                 525
   tgg ata ctg tgg att tca ttc gcc ata tca tgc ttc tta att tgc gtt      1632
   Trp Ile Leu Trp Ile Ser Phe Ala Ile Ser Cys Phe Leu Ile Cys Val
       530                 535                 540
   gtt cta ttg ggt ttc att atg tgg gct tgc caa aaa ggc aac atc aga      1680
   Val Leu Leu Gly Phe Ile Met Trp Ala Cys Gln Lys Gly Asn Ile Arg
   545                 550                 555                 560
   tgc aac att tgc att tga                                              1698
   Cys Asn Ile Cys Ile
                   565
<210> 32
   <211> 565
   <212> PRT
   <213> Influenza virus
<400> 32
   Met Lys Thr Thr Ile Ile Leu Ile Leu Leu Thr His Trp Ala Tyr Ser
   1               5                   10                  15
   Gln Asn Pro Ile Ser Asp Asn Asn Thr Ala Thr Leu Cys Leu Gly His
               20                  25                  30
   His Ala Val Ala Asn Gly Thr Leu Val Lys Thr Ile Ser Asp Asp Gln
           35                  40                  45
   Ile Glu Val Thr Asn Ala Thr Glu Leu Val Gln Ser Ile Ser Met Gly
       50                  55                  60
   Lys Ile Cys Asn Lys Ser Tyr Arg Ile Leu Asp Gly Arg Asn Cys Thr
   65                  70                  75                  80
   Leu Ile Asp Ala Met Leu Gly Asp Pro His Cys Asp Ala Phe Gln Tyr
                   85                  90                  95
   Glu Ser Trp Asp Leu Phe Ile Glu Arg Ser Ser Ala Phe Ser Asn Cys
               100                 105                 110
   Tyr Pro Tyr Asp Ile Pro Asp Tyr Ala Ser Leu Arg Ser Ile Val Ala
           115                 120                 125
   Ser Ser Gly Thr Leu Glu Phe Thr Ala Glu Gly Phe Thr Trp Thr Gly
       130                 135                 140
   Val Thr Gln Asn Gly Arg Ser Gly Ala Cys Lys Arg Gly Ser Ala Asp
   145                 150                 155                 160
   Ser Phe Phe Ser Arg Leu Asn Trp Leu Thr Lys Ser Gly Ser Ser Tyr
                   165                 170                 175
   Pro Thr Leu Asn Val Thr Met Pro Asn Asn Lys Asn Phe Asp Lys Leu
               180                 185                 190
   Tyr Ile Trp Gly Ile His His Pro Ser Ser Asn Gln Glu Gln Thr Lys
           195                 200                 205
   Leu Tyr Ile Gln Glu Ser Gly Arg Val Thr Val Ser Thr Lys Arg Ser
       210                 215                 220
   Gln Gln Thr Ile Ile Pro Asn Ile Gly Ser Arg Pro Leu Val Arg Gly
   225                 230                 235                 240
   Gln Ser Gly Arg Ile Ser Ile Tyr Trp Thr Ile Val Lys Pro Gly Asp
                   245                 250                 255
   Ile Leu Met Ile Asn Ser Asn Gly Asn Leu Val Ala Pro Arg Gly Tyr
               260                 265                 270
   Phe Lys Leu Lys Thr Gly Lys Ser Ser Val Met Arg Ser Asp Val Pro
           275                 280                 285
   Ile Asp Ile Cys Val Ser Glu Cys Ile Thr Pro Asn Gly Ser Ile Ser
       290                 295                 300
   Asn Asp Lys Pro Phe Gln Asn Val Asn Lys Val Thr Tyr Gly Lys Cys
   305                 310                 315                 320
   Pro Lys Tyr Ile Arg Gln Asn Thr Leu Lys Leu Ala Thr Gly Met Arg
                   325                 330                 335
   Asn Val Pro Glu Lys Gln Thr Arg Gly Ile Phe Gly Ala Ile Ala Gly
               340                 345                 350
   Phe Ile Glu Asn Gly Trp Glu Gly Met Val Asp Gly Trp Tyr Gly Phe
           355                 360                 365
   Arg Tyr Gln Asn Ser Glu Gly Thr Gly Gln Ala Ala Asp Leu Lys Ser
       370                 375                 380
   Thr Gln Ala Ala Ile Asp Gln Ile Asn Gly Lys Leu Asn Arg Val Ile
   385                 390                 395                 400
   Glu Arg Thr Asn Glu Lys Phe His Gln Ile Glu Lys Glu Phe Ser Glu
                   405                 410                 415
   Val Glu Gly Arg Ile Gln Asp Leu Glu Lys Tyr Val Glu Asp Thr Lys
   420 425 430
   Ile Asp Leu Trp Ser Tyr Asn Ala Glu Leu Leu Val Ala Leu Glu Asn
   435 440 445
   Gln His Thr Ile Asp Leu Thr Asp Ala Glu Met Asn Lys Leu Phe Glu
   450 455 460
   Lys Thr Arg Arg Gln Leu Arg Glu Asn Ala Glu Asp Met Gly Gly Gly
   465 470 475 480
   Cys Phe Lys Ile Tyr His Lys Cys Asp Asn Ala Cys Ile Gly Ser Ile
   485 490 495
   Arg Thr Gly Thr Tyr Asp His Tyr Ile Tyr Arg Asp Glu Ala Leu Asn
   500 505 510
   Asn Arg Phe Gln Ile Lys Gly Val Glu Leu Lys Ser Gly Tyr Lys Asp
   515 520 525
   Trp Ile Leu Trp Ile Ser Phe Ala Ile Ser Cys Phe Leu Ile Cys Val
   530 535 540
   Val Leu Leu Gly Phe Ile Met Trp Ala Cys Gln Lys Gly Asn Ile Arg
   545 550 555 560
   Cys Asn Ile Cys Ile
   565
<210> 33
   <211> 549
   <212> PRT
   <213> Influenza virus
<400> 33
   Gln Asn Pro Ile Ser Gly Asn Asn Thr Ala Thr Leu Cys Leu Gly His
   1 5 10 15
   His Ala Val Ala Asn Gly Thr Leu Val Lys Thr Met Ser Asp Asp Gln
   20 25 30
   Ile Glu Val Thr Asn Ala Thr Glu Leu Val Gln Ser Ile Ser Met Gly
   35 40 45
   Lys Ile Cys Asn Lys Ser Tyr Arg Ile Leu Asp Gly Arg Asn Cys Thr
   50 55 60
   Leu Ile Asp Ala Met Leu Gly Asp Pro His Cys Asp Ala Phe Gln Tyr
   65 70 75 80
   Glu Ser Trp Asp Leu Phe Ile Glu Arg Ser Asn Ala Phe Ser Asn Cys
                   85                  90                  95
   Tyr Pro Tyr Asp Ile Pro Asp Tyr Ala Ser Leu Arg Ser Ile Val Ala
               100                 105                 110
   Ser Ser Gly Thr Leu Glu Phe Thr Ala Glu Gly Phe Thr Trp Thr Gly
           115                 120                 125
   Val Thr Gln Asn Gly Arg Ser Gly Ala Cys Lys Arg Gly Ser Ala Asp
       130                 135                 140
   Ser Phe Phe Ser Arg Leu Asn Trp Leu Thr Lys Ser Gly Ser Ser Tyr
   145                 150                 155                 160
   Pro Thr Leu Asn Val Thr Met Pro Asn Asn Lys Asn Phe Asp Lys Leu
                   165                 170                 175
   Tyr Ile Trp Gly Ile His His Pro Ser Ser Asn Gln Glu Gln Thr Lys
               180                 185                 190
   Leu Tyr Ile Gln Glu Ser Gly Arg Val Thr Val Ser Thr Lys Arg Ser
           195                 200                 205
   Gln Gln Thr Ile Ile Pro Asn Ile Gly Ser Arg Pro Leu Val Arg Gly
       210                 215                 220
   Gln Ser Gly Arg Ile Ser Ile Tyr Trp Thr Ile Val Lys Pro Gly Asp
   225                 230                 235                 240
   Ile Leu Met Ile Asn Ser Asn Gly Asn Leu Val Ala Pro Arg Gly Tyr
                   245                 250                 255
   Phe Lys Leu Lys Thr Gly Lys Ser Ser Val Met Arg Ser Asp Ala Pro
               260                 265                 270
   Ile Asp Ile Cys Val Ser Glu Cys Ile Thr Pro Asn Gly Ser Ile Ser
           275                 280                 285
   Asn Asp Lys Pro Phe Gln Asn Val Asn Lys Val Thr Tyr Gly Lys Cys
       290                 295                 300
   Pro Lys Tyr Ile Arg Gln Asn Thr Leu Lys Leu Ala Thr Gly Met Arg
   305                 310                 315                 320
   Asn Val Pro Glu Lys Gln Thr Arg Gly Ile Phe Gly Ala Ile Ala Gly
                   325                 330                 335
   Phe Ile Glu Asn Gly Trp Glu Gly Met Val Asp Gly Trp Tyr Gly Phe
               340                 345                 350
   Arg Tyr Gln Asn Ser Glu Gly Thr Gly Gln Ala Ala Asp Leu Lys Ser
   355 360 365
   Thr Gln Ala Ala Ile Asp Gln Ile Asn Gly Lys Leu Asn Arg Val Ile
   370 375 380
   Glu Arg Thr Asn Glu Lys Phe His Gln Ile Glu Lys Glu Phe Ser Glu
   385 390 395 400
   Val Glu Gly Arg Ile Gln Asp Leu Glu Lys Tyr Val Glu Asp Thr Lys
   405 410 415
   Ile Asp Leu Trp Ser Tyr Asn Ala Glu Leu Leu Val Ala Leu Glu Asn
   420 425 430
   Gln His Thr Ile Asp Leu Thr Asp Ala Glu Met Asn Lys Leu Phe Glu
   435 440 445
   Lys Thr Arg Arg Gln Leu Arg Glu Asn Ala Glu Asp Met Gly Gly Gly
   450 455 460
   Cys Phe Lys Ile Tyr His Lys Cys Asp Asn Ala Cys Ile Gly Ser Ile
   465 470 475 480
   Arg Thr Gly Thr Tyr Asp His Tyr Ile Tyr Arg Asp Glu Ala Leu Asn
   485 490 495
   Asn Arg Phe Gln Ile Lys Gly Val Glu Leu Lys Ser Gly Tyr Lys Asp
   500 505 510
   Trp Ile Leu Trp Ile Ser Phe Ala Ile Ser Cys Phe Leu Ile Cys Val
   515 520 525
   Val Leu Leu Gly Phe Ile Met Trp Ala Cys Gln Arg Gly Asn Ile Arg
   530 535 540
   Cys Asn Ile Cys Ile
   545
<210> 34
   <211> 549
   <212> PRT
   <213> Influenza virus
<400> 34
   Gln Asn Pro Ile Ser Asp Asn Asn Thr Ala Thr Leu Cys Leu Gly His
   1               5                   10                  15
   His Ala Val Ala Asn Gly Thr Leu Val Lys Thr Ile Ser Asp Asp Gln
               20                  25                  30
   Ile Glu Val Thr Asn Ala Thr Glu Leu Val Gln Ser Ile Ser Met Gly
           35                  40                  45
   Lys Ile Cys Asn Lys Ser Tyr Arg Ile Leu Asp Gly Arg Asn Cys Thr
       50                  55                  60
   Leu Ile Asp Ala Met Leu Gly Asp Pro His Cys Asp Ala Phe Gln Tyr
   65                  70                  75                  80
   Glu Ser Trp Asp Leu Phe Ile Glu Arg Ser Ser Ala Phe Ser Asn Cys
                   85                  90                  95
   Tyr Pro Tyr Asp Ile Pro Asp Tyr Ala Ser Leu Arg Ser Ile Val Ala
               100                 105                 110
   Ser Ser Gly Thr Leu Glu Phe Thr Ala Glu Gly Phe Thr Trp Thr Gly
           115                 120                 125
   Val Thr Gln Asn Gly Arg Ser Gly Ala Cys Lys Arg Gly Ser Ala Asp
       130                 135                 140
   Ser Phe Phe Ser Arg Leu Asn Trp Leu Thr Lys Ser Gly Ser Ser Tyr
   145                 150                 155                 160
   Pro Thr Leu Asn Val Thr Met Pro Asn Asn Lys Asn Phe Asp Lys Leu
                   165                 170                 175
   Tyr Ile Trp Gly Ile His His Pro Ser Ser Asn Gln Glu Gln Thr Lys
               180                 185                 190
   Leu Tyr Ile Gln Glu Ser Gly Arg Val Thr Val Ser Thr Lys Arg Ser
           195                 200                 205
   Gln Gln Thr Ile Ile Pro Asn Ile Gly Ser Arg Pro Leu Val Arg Gly
       210                 215                 220
   Gln Ser Gly Arg Ile Ser Ile Tyr Trp Thr Ile Val Lys Pro Gly Asp
   225                 230                 235                 240
   Ile Leu Met Ile Asn Ser Asn Gly Asn Leu Val Ala Pro Arg Gly Tyr
                   245                 250                 255
   Phe Lys Leu Lys Thr Gly Lys Ser Ser Val Met Arg Ser Asp Val Pro
               260                 265                 270
   Ile Asp Ile Cys Val Ser Glu Cys Ile Thr Pro Asn Gly Ser Ile Ser
           275                 280                 285
   Asn Asp Lys Pro Phe Gln Asn Val Asn Lys Val Thr Tyr Gly Lys Cys
       290                 295                 300
   Pro Lys Tyr Ile Arg Gln Asn Thr Leu Lys Leu Ala Thr Gly Met Arg
   305                 310                 315                 320
   Asn Val Pro Glu Lys Gln Thr Arg Gly Ile Phe Gly Ala Ile Ala Gly
                   325                 330                 335
   Phe Ile Glu Asn Gly Trp Glu Gly Met Val Asp Gly Trp Tyr Gly Phe
               340                 345                 350
   Arg Tyr Gln Asn Ser Glu Gly Thr Gly Gln Ala Ala Asp Leu Lys Ser
           355                 360                 365
   Thr Gln Ala Ala Ile Asp Gln Ile Asn Gly Lys Leu Asn Arg Val Ile
       370                 375                 380
   Glu Arg Thr Asn Glu Lys Phe His Gln Ile Glu Lys Glu Phe Ser Glu
   385                 390                 395                 400
   Val Glu Gly Arg Ile Gln Asp Leu Glu Lys Tyr Val Glu Asp Thr Lys
                   405                 410                 415
   Ile Asp Leu Trp Ser Tyr Asn Ala Glu Leu Leu Val Ala Leu Glu Asn
               420                 425                 430
   Gln His Thr Ile Asp Leu Thr Asp Ala Glu Met Asn Lys Leu Phe Glu
           435                 440                 445
   Lys Thr Arg Arg Gln Leu Arg Glu Asn Ala Glu Asp Met Gly Gly Gly
       450                 455                 460
   Cys Phe Lys Ile Tyr His Lys Cys Asp Asn Ala Cys Ile Gly Ser Ile
   465                 470                 475                 480
   Arg Thr Gly Thr Tyr Asp His Tyr Ile Tyr Arg Asp Glu Ala Leu Asn
                   485                 490                 495
   Asn Arg Phe Gln Ile Lys Gly Val Glu Leu Lys Ser Gly Tyr Lys Asp
               500                 505                 510
   Trp Ile Leu Trp Ile Ser Phe Ala Ile Ser Cys Phe Leu Ile Cys Val
           515                 520                 525
   Val Leu Leu Gly Phe Ile Met Trp Ala Cys Gln Lys Gly Asn Ile Arg
       530                 535                 540
   Cys Asn Ile Cys Ile
   545
<210> 35
   <211> 9
   <212> DNA
   <213> Influenza virus
<400> 35
   gagagttgg 9
<210> 36
   <211> 9
   <212> DNA
   <213> Influenza virus
<400> 36
   ccgttggtc 9
<210> 37
   <211> 9
   <212> DNA
   <213> Influenza virus
<400> 37
   caaaccaga 9
<210> 38
   <211> 9
   <212> DNA
   <213> Influenza virus
<400> 38
   agaactggg 9
<210> 39
   <211> 15
   <212> DNA
   <213> Influenza virus
<400> 39
   tatgagagtt gggac 15
<210> 40
   <211> 15
   <212> DNA
   <213> Influenza virus
<400> 40
   agaccgttgg tcaga 15
<210> 41
   <211> 15
   <212> DNA
   <213> Influenza virus
<400> 41
   aagcaaacca gagga 15
<210> 42
   <211> 15
   <212> DNA
   <213> Influenza virus
<400> 42
   ataagaactg ggaca 15
<210> 43
   <211> 9
   <212> DNA
   <213> Influenza virus
<400> 43
   acaatgagt 9
<210> 44
   <211> 15
   <212> DNA
   <213> Influenza virus
<400> 44
   aaaacaatga gtgat 15
<210> 45
   <211> 9
   <212> DNA
   <213> Influenza virus
<400> 45
   gatgtaccc 9
<210> 46
   <211> 15
   <212> DNA
   <213> Influenza virus
<400> 46
   tcagatgtac ccata 15
<210> 47
   <211> 2280
   <212> DNA
   <213> Influenza virus
<220>
   <221> CDS
   <222> (1)..(2280)
<400> 47
   atg gag aga ata aaa gaa ctg aga gat ctg atg tta caa tcc cgc acc       48
   Met Glu Arg Ile Lys Glu Leu Arg Asp Leu Met Leu Gln Ser Arg Thr
   1               5                   10                  15
   cgc gag ata cta aca aaa act act gtg gac cac atg gcc ata atc aag       96
   Arg Glu Ile Leu Thr Lys Thr Thr Val Asp His Met Ala Ile Ile Lys
               20                  25                  30
   aaa tac aca tca gga aga caa gag aag aac cct gca ctt agg atg aaa      144
   Lys Tyr Thr Ser Gly Arg Gln Glu Lys Asn Pro Ala Leu Arg Met Lys
           35                  40                  45
   tgg atg atg gca atg aaa tac cca att aca gca gat aag agg ata atg      192
   Trp Met Met Ala Met Lys Tyr Pro Ile Thr Ala Asp Lys Arg Ile Met
       50                  55                  60
   gag atg att cct gag aga aat gaa cag gga caa acc ctt tgg agc aaa      240
   Glu Met Ile Pro Glu Arg Asn Glu Gln Gly Gln Thr Leu Trp Ser Lys
   65                  70                  75                  80
   acg aac gat gct ggc tca gac cgc gta atg gta tca cct ctg gca gtg      288
   Thr Asn Asp Ala Gly Ser Asp Arg Val Met Val Ser Pro Leu Ala Val
                   85                  90                  95
   aca tgg tgg aat agg aat gga cca aca acg aac aca att cat tat cca      336
   Thr Trp Trp Asn Arg Asn Gly Pro Thr Thr Asn Thr Ile His Tyr Pro
               100                 105                 110
   aaa gtc tac aaa act tat ttt gaa aag gtt gaa aga ttg aaa cac gga      384
   Lys Val Tyr Lys Thr Tyr Phe Glu Lys Val Glu Arg Leu Lys His Gly
           115                 120                 125
   acc ttt ggc ccc gtt cat ttt agg aat caa gtc aag ata aga cga aga      432
   Thr Phe Gly Pro Val His Phe Arg Asn Gln Val Lys Ile Arg Arg Arg
       130                 135                 140
   gtt gat gta aac cct ggt cac gcg gac ctc agt gct aaa gaa gca caa      480
   Val Asp Val Asn Pro Gly His Ala Asp Leu Ser Ala Lys Glu Ala Gln
   145                 150                 155                 160
   gat gtg atc atg gaa gtt gtt ttc cca aat gaa gtg gga gcc aga att      528
   Asp Val Ile Met Glu Val Val Phe Pro Asn Glu Val Gly Ala Arg Ile
                   165                 170                 175
   cta aca tca gaa tca caa cta aca ata acc aaa gag aaa aag gaa gaa      576
   Leu Thr Ser Glu Ser Gln Leu Thr Ile Thr Lys Glu Lys Lys Glu Glu
               180                 185                 190
   ctt cag gac tgc aaa att gct ccc ttg atg gta gca tac atg cta gaa      624
   Leu Gln Asp Cys Lys Ile Ala Pro Leu Met Val Ala Tyr Met Leu Glu
           195                 200                 205
   aga gag ttg gtc cga aaa aca agg ttc ctc cca gta gta ggc gga aca      672
   Arg Glu Leu Val Arg Lys Thr Arg Phe Leu Pro Val Val Gly Gly Thr
       210                 215                 220
   agc agt gta tac att gaa gtg ttg cat ctg act cag gga aca tgc tgg      720
   Ser Ser Val Tyr Ile Glu Val Leu His Leu Thr Gln Gly Thr Cys Trp
   225                 230                 235                 240
   gag caa atg tac acc cca gga gga aaa gtt aga aac gat gat att gat      768
   Glu Gln Met Tyr Thr Pro Gly Gly Lys Val Arg Asn Asp Asp Ile Asp
                   245                 250                 255
   caa agt tta att att gca gcc cgg aac ata gtg aga aga gca aca gta      816
   Gln Ser Leu Ile Ile Ala Ala Arg Asn Ile Val Arg Arg Ala Thr Val
               260                 265                 270
   tca gca gat cca cta gca tcc cta ctg gaa atg tgc cac agt aca cag      864
   Ser Ala Asp Pro Leu Ala Ser Leu Leu Glu Met Cys His Ser Thr Gln
           275                 280                 285
   att ggt gga aca agg atg gta gac atc ctt aag cag aac cca aca gag      912
   Ile Gly Gly Thr Arg Met Val Asp Ile Leu Lys Gln Asn Pro Thr Glu
       290                 295                 300
   gaa caa gct gtg gat ata tgc aaa gca gca atg gga ttg aga att agc      960
   Glu Gln Ala Val Asp Ile Cys Lys Ala Ala Met Gly Leu Arg Ile Ser
   305                 310                 315                 320
   tca tca ttc agc ttt ggt gga ttc acc ttc aaa agg aca agt gga tca     1008
   Ser Ser Phe Ser Phe Gly Gly Phe Thr Phe Lys Arg Thr Ser Gly Ser
                   325                 330                 335
   tca gtc aag aga gaa gaa gaa atg ctt acg ggc aac ctt caa aca ttg     1056
   Ser Val Lys Arg Glu Glu Glu Met Leu Thr Gly Asn Leu Gln Thr Leu
               340                 345                 350
   aaa ata aga gtg cat gag ggc tat gaa gaa ttc aca atg gtc gga aga     1104
   Lys Ile Arg Val His Glu Gly Tyr Glu Glu Phe Thr Met Val Gly Arg
           355                 360                 365
   aga gca aca gcc att atc aga aag gca acc aga aga ttg att caa ttg     1152
   Arg Ala Thr Ala Ile Ile Arg Lys Ala Thr Arg Arg Leu Ile Gln Leu
       370                 375                 380
   ata gta agt ggg aga gat gaa caa tca att gct gaa gca ata att gta     1200
   Ile Val Ser Gly Arg Asp Glu Gln Ser Ile Ala Glu Ala Ile Ile Val
   385                 390                 395                 400
   gcc atg gtg ttt tcg caa gaa gat tgc atg ata aaa gca gtt cga ggc       1248
   Ala Met Val Phe Ser Gln Glu Asp Cys Met Ile Lys Ala Val Arg Gly
                   405                 410                 415
   gat ttg aac ttt gtt aat aga gca aat cag cgt ttg aac ccc atg cat       1296
   Asp Leu Asn Phe Val Asn Arg Ala Asn Gln Arg Leu Asn Pro Met His
               420                 425                 430
   caa ctc ttg agg cat ttc caa aaa gat gca aaa gtg ctt ttc caa aat       1344
   Gln Leu Leu Arg His Phe Gln Lys Asp Ala Lys Val Leu Phe Gln Asn
           435                 440                 445
   tgg gga att gaa ccc atc gac aat gta atg ggg atg att gga ata ttg       1392
   Trp Gly Ile Glu Pro Ile Asp Asn Val Met Gly Met Ile Gly Ile Leu
       450                 455                 460
   cct gac atg acc cca agc acc gag atg tca ttg aga gga gtg aga gtc       1440
   Pro Asp Met Thr Pro Ser Thr Glu Met Ser Leu Arg Gly Val Arg Val
   465                 470                 475                 480
   agc aaa atg gga gtg gat gag tac tcc agc act gag aga gtg gtg gtg       1488
   Ser Lys Met Gly Val Asp Glu Tyr Ser Ser Thr Glu Arg Val Val Val
                   485                 490                 495
   agc att gac cgt ttt tta aga gtt cgg gat caa agg gga aac ata cta       1536
   Ser Ile Asp Arg Phe Leu Arg Val Arg Asp Gln Arg Gly Asn Ile Leu
               500                 505                 510
   ctg tcc cct gaa gaa gtc agt gaa aca caa gga acg gaa aag ctg aca       1584
   Leu Ser Pro Glu Glu Val Ser Glu Thr Gln Gly Thr Glu Lys Leu Thr
           515                 520                 525
   ata att tat tcg tca tca atg atg tgg gag att aat ggt ccc gaa tca       1632
   Ile Ile Tyr Ser Ser Ser Met Met Trp Glu Ile Asn Gly Pro Glu Ser
       530                 535                 540
   gtg ttg gtc aat act tat caa tgg atc atc aga aac tgg gaa att gta       1680
   Val Leu Val Asn Thr Tyr Gln Trp Ile Ile Arg Asn Trp Glu Ile Val
   545                 550                 555                 560
   aaa att cag tgg tca cag gac ccc aca atg tta tac aat aag ata gaa       1728
   Lys Ile Gln Trp Ser Gln Asp Pro Thr Met Leu Tyr Asn Lys Ile Glu
                   565                 570                 575
   ttt gaa cca ttc caa tcc ctg gtc cct agg gcc acc aga agc caa tac       1776
   Phe Glu Pro Phe Gln Ser Leu Val Pro Arg Ala Thr Arg Ser Gln Tyr
               580                 585                 590
   agc ggt ttc gta aga acc ctg ttt cag caa atg cga gat gta ctt gga       1824
   Ser Gly Phe Val Arg Thr Leu Phe Gln Gln Met Arg Asp Val Leu Gly
           595                 600                 605
   aca ttt gat act gct caa ata ata aaa ctc ctc cct ttt gcc gct gct       1872
   Thr Phe Asp Thr Ala Gln Ile Ile Lys Leu Leu Pro Phe Ala Ala Ala
       610                 615                 620
   cct ccg gaa cag agt agg atg cag ttc tct tct ttg act gtt aat gta       1920
   Pro Pro Glu Gln Ser Arg Met Gln Phe Ser Ser Leu Thr Val Asn Val
   625                 630                 635                 640
   aga ggt tcg gga atg agg ata ctt gta aga ggc aat tcc cca gtg ttc       1968
   Arg Gly Ser Gly Met Arg Ile Leu Val Arg Gly Asn Ser Pro Val Phe
                   645                 650                 655
   aac tac aat aaa gtc act aaa agg ctc aca gtc ctc gga aag gat gca       2016
   Asn Tyr Asn Lys Val Thr Lys Arg Leu Thr Val Leu Gly Lys Asp Ala
                   660                 665                 670
   ggt gcg ctt act gag gac cca gat gaa ggt acg gct gga gta gag tct      2064
   Gly Ala Leu Thr Glu Asp Pro Asp Glu Gly Thr Ala Gly Val Glu Ser
           675                 680                 685
   gct gtt cta aga ggg ttt ctc att tta ggt aaa gaa aac aag aga tat      2112
   Ala Val Leu Arg Gly Phe Leu Ile Leu Gly Lys Glu Asn Lys Arg Tyr
       690                 695                 700
   ggc cca gca cta agc atc aat gaa ctt agc aaa ctt gca aaa ggg gag      2160
   Gly Pro Ala Leu Ser Ile Asn Glu Leu Ser Lys Leu Ala Lys Gly Glu
   705                 710                 715                 720
   aaa gcc aat gta cta att ggg caa ggg gac gta gtg ttg gta atg aaa      2208
   Lys Ala Asn Val Leu Ile Gly Gln Gly Asp Val Val Leu Val Met Lys
                   725                 730                 735
   cgg aaa cgt gac tct agc ata ctt act gac agc cag aca gcg acc aaa      2256
   Arg Lys Arg Asp Ser Ser Ile Leu Thr Asp Ser Gln Thr Ala Thr Lys
               740                 745                 750
   agg att cgg atg gcc atc aat tag                                      2280
   Arg Ile Arg Met Ala Ile Asn
           755
<210> 48
   <211> 759
   <212> PRT
   <213> Influenza virus
<400> 48
   Met Glu Arg Ile Lys Glu Leu Arg Asp Leu Met Leu Gln Ser Arg Thr
   1               5                   10                  15
   Arg Glu Ile Leu Thr Lys Thr Thr Val Asp His Met Ala Ile Ile Lys
               20                  25                  30
   Lys Tyr Thr Ser Gly Arg Gln Glu Lys Asn Pro Ala Leu Arg Met Lys
           35                  40                  45
   Trp Met Met Ala Met Lys Tyr Pro Ile Thr Ala Asp Lys Arg Ile Met
       50                  55                  60
   Glu Met Ile Pro Glu Arg Asn Glu Gln Gly Gln Thr Leu Trp Ser Lys
   65                  70                  75                  80
   Thr Asn Asp Ala Gly Ser Asp Arg Val Met Val Ser Pro Leu Ala Val
                   85                  90                  95
   Thr Trp Trp Asn Arg Asn Gly Pro Thr Thr Asn Thr Ile His Tyr Pro
               100                 105                 110
   Lys Val Tyr Lys Thr Tyr Phe Glu Lys Val Glu Arg Leu Lys His Gly
           115                 120                 125
   Thr Phe Gly Pro Val His Phe Arg Asn Gln Val Lys Ile Arg Arg Arg
       130                 135                 140
   Val Asp Val Asn Pro Gly His Ala Asp Leu Ser Ala Lys Glu Ala Gln
   145                 150                 155                 160
   Asp Val Ile Met Glu Val Val Phe Pro Asn Glu Val Gly Ala Arg Ile
                   165                 170                 175
   Leu Thr Ser Glu Ser Gln Leu Thr Ile Thr Lys Glu Lys Lys Glu Glu
               180                 185                 190
   Leu Gln Asp Cys Lys Ile Ala Pro Leu Met Val Ala Tyr Met Leu Glu
           195                 200                 205
   Arg Glu Leu Val Arg Lys Thr Arg Phe Leu Pro Val Val Gly Gly Thr
       210                 215                 220
   Ser Ser Val Tyr Ile Glu Val Leu His Leu Thr Gln Gly Thr Cys Trp
   225                 230                 235                 240
   Glu Gln Met Tyr Thr Pro Gly Gly Lys Val Arg Asn Asp Asp Ile Asp
                   245                 250                 255
   Gln Ser Leu Ile Ile Ala Ala Arg Asn Ile Val Arg Arg Ala Thr Val
               260                 265                 270
   Ser Ala Asp Pro Leu Ala Ser Leu Leu Glu Met Cys His Ser Thr Gln
           275                 280                 285
   Ile Gly Gly Thr Arg Met Val Asp Ile Leu Lys Gln Asn Pro Thr Glu
       290                 295                 300
   Glu Gln Ala Val Asp Ile Cys Lys Ala Ala Met Gly Leu Arg Ile Ser
   305                 310                 315                 320
   Ser Ser Phe Ser Phe Gly Gly Phe Thr Phe Lys Arg Thr Ser Gly Ser
                   325                 330                 335
   Ser Val Lys Arg Glu Glu Glu Met Leu Thr Gly Asn Leu Gln Thr Leu
               340                 345                 350
   Lys Ile Arg Val His Glu Gly Tyr Glu Glu Phe Thr Met Val Gly Arg
           355                 360                 365
   Arg Ala Thr Ala Ile Ile Arg Lys Ala Thr Arg Arg Leu Ile Gln Leu
       370                 375                 380
   Ile Val Ser Gly Arg Asp Glu Gln Ser Ile Ala Glu Ala Ile Ile Val
   385                 390                 395                 400
   Ala Met Val Phe Ser Gln Glu Asp Cys Met Ile Lys Ala Val Arg Gly
                   405                 410                 415
   Asp Leu Asn Phe Val Asn Arg Ala Asn Gln Arg Leu Asn Pro Met His
               420                 425                 430
   Gln Leu Leu Arg His Phe Gln Lys Asp Ala Lys Val Leu Phe Gln Asn
           435                 440                 445
   Trp Gly Ile Glu Pro Ile Asp Asn Val Met Gly Met Ile Gly Ile Leu
       450                 455                 460
   Pro Asp Met Thr Pro Ser Thr Glu Met Ser Leu Arg Gly Val Arg Val
   465                 470                 475                 480
   Ser Lys Met Gly Val Asp Glu Tyr Ser Ser Thr Glu Arg Val Val Val
                   485                 490                 495
   Ser Ile Asp Arg Phe Leu Arg Val Arg Asp Gln Arg Gly Asn Ile Leu
               500                 505                 510
   Leu Ser Pro Glu Glu Val Ser Glu Thr Gln Gly Thr Glu Lys Leu Thr
           515                 520                 525
   Ile Ile Tyr Ser Ser Ser Met Met Trp Glu Ile Asn Gly Pro Glu Ser
       530                 535                 540
   Val Leu Val Asn Thr Tyr Gln Trp Ile Ile Arg Asn Trp Glu Ile Val
   545                 550                 555                 560
   Lys Ile Gln Trp Ser Gln Asp Pro Thr Met Leu Tyr Asn Lys Ile Glu
                   565                 570                 575
   Phe Glu Pro Phe Gln Ser Leu Val Pro Arg Ala Thr Arg Ser Gln Tyr
               580                 585                 590
   Ser Gly Phe Val Arg Thr Leu Phe Gln Gln Met Arg Asp Val Leu Gly
           595                 600                 605
   Thr Phe Asp Thr Ala Gln Ile Ile Lys Leu Leu Pro Phe Ala Ala Ala
       610                 615                 620
   Pro Pro Glu Gln Ser Arg Met Gln Phe Ser Ser Leu Thr Val Asn Val
   625                 630                 635                 640
   Arg Gly Ser Gly Met Arg Ile Leu Val Arg Gly Asn Ser Pro Val Phe
                   645                 650                 655
   Asn Tyr Asn Lys Val Thr Lys Arg Leu Thr Val Leu Gly Lys Asp Ala
               660                 665                 670
   Gly Ala Leu Thr Glu Asp Pro Asp Glu Gly Thr Ala Gly Val Glu Ser
           675                 680                 685
   Ala Val Leu Arg Gly Phe Leu Ile Leu Gly Lys Glu Asn Lys Arg Tyr
   690 695 700
   Gly Pro Ala Leu Ser Ile Asn Glu Leu Ser Lys Leu Ala Lys Gly Glu
   705 710 715 720
   Lys Ala Asn Val Leu Ile Gly Gln Gly Asp Val Val Leu Val Met Lys
   725 730 735
   Arg Lys Arg Asp Ser Ser Ile Leu Thr Asp Ser Gln Thr Ala Thr Lys
   740 745 750
   Arg Ile Arg Met Ala Ile Asn
   755
<210> 49
   <211> 2274
   <212> DNA
   <213> Influenza virus
<220>
   <221> CDS
   <222> (1)..(2274)
<400> 49
   atg gat gtc aat ccg act cta ctt ttc tta aag gtg cca gcg caa aat      48
   Met Asp Val Asn Pro Thr Leu Leu Phe Leu Lys Val Pro Ala Gln Asn
   1               5                   10                  15
   gct ata agc aca aca ttc cct tat act gga gat cct ccc tac agt cat      96
   Ala Ile Ser Thr Thr Phe Pro Tyr Thr Gly Asp Pro Pro Tyr Ser His
               20                  25                  30
   gga aca ggg aca gga tac acc atg gat act gtc aac aga aca cac caa     144
   Gly Thr Gly Thr Gly Tyr Thr Met Asp Thr Val Asn Arg Thr His Gln
           35                  40                  45
   tat tca gaa aaa ggg aaa tgg aca aca aac act gag att gga gca cca     192
   Tyr Ser Glu Lys Gly Lys Trp Thr Thr Asn Thr Glu Ile Gly Ala Pro
       50                  55                  60
   caa ctt aat cca atc gat gga cca ctt cct gaa gac aat gaa cca agt     240
   Gln Leu Asn Pro Ile Asp Gly Pro Leu Pro Glu Asp Asn Glu Pro Ser
   65                  70                  75                  80
   ggg tac gcc caa aca gat tgt gta ttg gaa gca atg gct ttc ctt gaa     288
   Gly Tyr Ala Gln Thr Asp Cys Val Leu Glu Ala Met Ala Phe Leu Glu
                   85                  90                  95
   gaa tcc cat ccc gga atc ttt gaa aat tcg tgt ctt gaa acg atg gag     336
   Glu Ser His Pro Gly Ile Phe Glu Asn Ser Cys Leu Glu Thr Met Glu
               100                 105                 110
   gtg att cag cag aca aga gtg gac aaa cta aca caa ggc cga caa act     384
   Val Ile Gln Gln Thr Arg Val Asp Lys Leu Thr Gln Gly Arg Gln Thr
           115                 120                 125
   tat gat tgg acc ttg aat agg aat caa cct gcc gca aca gca ctt gct     432
   Tyr Asp Trp Thr Leu Asn Arg Asn Gln Pro Ala Ala Thr Ala Leu Ala
       130                 135                 140
   aat acg att gaa gta ttc aga tca aat ggt ctg acc tcc aat gaa tcg     480
   Asn Thr Ile Glu Val Phe Arg Ser Asn Gly Leu Thr Ser Asn Glu Ser
   145                 150                 155                 160
   ggg aga ttg atg gac ttc ctc aaa gat gtc atg gag tcc atg aac aag      528
   Gly Arg Leu Met Asp Phe Leu Lys Asp Val Met Glu Ser Met Asn Lys
                   165                 170                 175
   gag gaa atg gaa ata aca aca cac ttc caa cgg aag aga aga gta aga      576
   Glu Glu Met Glu Ile Thr Thr His Phe Gln Arg Lys Arg Arg Val Arg
               180                 185                 190
   gac aac atg aca aag aga atg ata aca cag aga acc ata gga aag aaa      624
   Asp Asn Met Thr Lys Arg Met Ile Thr Gln Arg Thr Ile Gly Lys Lys
           195                 200                 205
   aaa caa cga tta agc aga aag agc tat cta atc aga aca tta acc cta      672
   Lys Gln Arg Leu Ser Arg Lys Ser Tyr Leu Ile Arg Thr Leu Thr Leu
       210                 215                 220
   aac aca atg acc aag gac gct gag aga ggg aaa ttg aaa cga cga gca      720
   Asn Thr Met Thr Lys Asp Ala Glu Arg Gly Lys Leu Lys Arg Arg Ala
   225                 230                 235                 240
   atc gct acc cca ggg atg cag ata aga gga ttt gta tat ttt gtt gaa      768
   Ile Ala Thr Pro Gly Met Gln Ile Arg Gly Phe Val Tyr Phe Val Glu
                   245                 250                 255
   aca cta gct cga aga ata tgt gaa aag ctt gaa caa tca gga ttg cca      816
   Thr Leu Ala Arg Arg Ile Cys Glu Lys Leu Glu Gln Ser Gly Leu Pro
               260                 265                 270
   gtt ggc ggt aat gag aaa aag gcc aaa ctg gct aat gtc gtc aga aaa      864
   Val Gly Gly Asn Glu Lys Lys Ala Lys Leu Ala Asn Val Val Arg Lys
           275                 280                 285
   atg atg act aat tcc caa gac act gaa ctc tcc ttc acc atc act ggg      912
   Met Met Thr Asn Ser Gln Asp Thr Glu Leu Ser Phe Thr Ile Thr Gly
       290                 295                 300
   gac aat acc aaa tgg aat gaa aat cag aac cca cgc ata ttc ctg gca      960
   Asp Asn Thr Lys Trp Asn Glu Asn Gln Asn Pro Arg Ile Phe Leu Ala
   305                 310                 315                 320
   atg atc aca tac ata act aga gat cag cca gaa tgg ttc aga aat gtt     1008
   Met Ile Thr Tyr Ile Thr Arg Asp Gln Pro Glu Trp Phe Arg Asn Val
                   325                 330                 335
   cta agc att gca ccg att atg ttc tca aat aaa atg gca aga ctg ggg     1056
   Leu Ser Ile Ala Pro Ile Met Phe Ser Asn Lys Met Ala Arg Leu Gly
               340                 345                 350
   aaa gga tat atg ttt gaa agc aaa agt atg aaa ttg aga act caa ata     1104
   Lys Gly Tyr Met Phe Glu Ser Lys Ser Met Lys Leu Arg Thr Gln Ile
           355                 360                 365
   cca gca gaa atg cta gca agc att gac cta aaa tat ttc aat gat tca     1152
   Pro Ala Glu Met Leu Ala Ser Ile Asp Leu Lys Tyr Phe Asn Asp Ser
       370                 375                 380
   aca aaa aag aaa att gaa aag ata cga cca ctc ctg gtt gac ggg act     1200
   Thr Lys Lys Lys Ile Glu Lys Ile Arg Pro Leu Leu Val Asp Gly Thr
   385                 390                 395                 400
   gct tca ctg agt cct ggc atg atg atg gga atg ttc aac atg ttg agc     1248
   Ala Ser Leu Ser Pro Gly Met Met Met Gly Met Phe Asn Met Leu Ser
                   405                 410                 415
   act gtg ctg ggt gta tcc ata tta aac ctg ggc cag agg aaa tat aca     1296
   Thr Val Leu Gly Val Ser Ile Leu Asn Leu Gly Gln Arg Lys Tyr Thr
               420                 425                 430
   aag acc aca tac tgg tgg gat ggt ctg caa tca tcc gat gac ttt gct      1344
   Lys Thr Thr Tyr Trp Trp Asp Gly Leu Gln Ser Ser Asp Asp Phe Ala
           435                 440                 445
   ttg ata gtg aat gcg cct aat cat gaa gga ata caa gct gga gta gac      1392
   Leu Ile Val Asn Ala Pro Asn His Glu Gly Ile Gln Ala Gly Val Asp
       450                 455                 460
   aga ttc tat aga act tgc aaa ctg gtc ggg atc aac atg agc aaa aag      1440
   Arg Phe Tyr Arg Thr Cys Lys Leu Val Gly Ile Asn Met Ser Lys Lys
   465                 470                 475                 480
   aag tcc tac ata aat aga act gga aca ttc gaa ttc aca agc ttt ttc      1488
   Lys Ser Tyr Ile Asn Arg Thr Gly Thr Phe Glu Phe Thr Ser Phe Phe
                   485                 490                 495
   tac cgg tat ggt ttt gta gcc aat ttc agc atg gaa cta ccc agt ttt      1536
   Tyr Arg Tyr Gly Phe Val Ala Asn Phe Ser Met Glu Leu Pro Ser Phe
               500                 505                 510
   ggg gtt tcc gga ata aat gaa tct gca gac atg agc att gga gtg aca      1584
   Gly Val Ser Gly Ile Asn Glu Ser Ala Asp Met Ser Ile Gly Val Thr
           515                 520                 525
   gtc atc aaa aac aac atg ata aat aat gat ctc ggt cct gcc acg gca      1632
   Val Ile Lys Asn Asn Met Ile Asn Asn Asp Leu Gly Pro Ala Thr Ala
       530                 535                 540
   caa atg gca ctc caa ctc ttc att aag gat tat cgg tac aca tac cgg      1680
   Gln Met Ala Leu Gln Leu Phe Ile Lys Asp Tyr Arg Tyr Thr Tyr Arg
   545                 550                 555                 560
   tgc cat aga ggt gat acc cag ata caa acc aga aga tct ttt gag ttg      1728
   Cys His Arg Gly Asp Thr Gln Ile Gln Thr Arg Arg Ser Phe Glu Leu
                   565                 570                 575
   aag aaa ctg tgg gaa cag act cga tca aag act ggt cta ctg gta tca      1776
   Lys Lys Leu Trp Glu Gln Thr Arg Ser Lys Thr Gly Leu Leu Val Ser
               580                 585                 590
   gat ggg ggt cca aac cta tat aac atc aga aac cta cac atc ccg gaa      1824
   Asp Gly Gly Pro Asn Leu Tyr Asn Ile Arg Asn Leu His Ile Pro Glu
           595                 600                 605
   gtc tgt tta aaa tgg gag cta atg gat gaa gat tat aag ggg agg cta      1872
   Val Cys Leu Lys Trp Glu Leu Met Asp Glu Asp Tyr Lys Gly Arg Leu
       610                 615                 620
   tgc aat cca ttg aat cct ttc gtt agt cac aaa gaa att gaa tca gtc      1920
   Cys Asn Pro Leu Asn Pro Phe Val Ser His Lys Glu Ile Glu Ser Val
   625                 630                 635                 640
   aac agt gca gta gta atg cct gct cat ggc cct gcc aaa agc atg gag      1968
   Asn Ser Ala Val Val Met Pro Ala His Gly Pro Ala Lys Ser Met Glu
                   645                 650                 655
   tat gat gct gtt gca aca aca cat tct tgg atc ccc aag agg aac cgg      2016
   Tyr Asp Ala Val Ala Thr Thr His Ser Trp Ile Pro Lys Arg Asn Arg
               660                 665                 670
   tcc ata ttg aac aca agc caa agg gga ata cta gaa gat gag cag atg      2064
   Ser Ile Leu Asn Thr Ser Gln Arg Gly Ile Leu Glu Asp Glu Gln Met
           675                 680                 685
   tat cag aaa tgc tgc aac ctg ttt gaa aaa ttc ttc ccc agc agc tca      2112
   Tyr Gln Lys Cys Cys Asn Leu Phe Glu Lys Phe Phe Pro Ser Ser Ser
       690                 695                 700
   tac aga aga cca gtc gga att tct agt atg gtt gag gcc atg gta tcc      2160
   Tyr Arg Arg Pro Val Gly Ile Ser Ser Met Val Glu Ala Met Val Ser
   705                 710                 715                 720
   agg gcc cgc att gat gca cga att gac ttc gaa tct gga cgg ata aag         2208
   Arg Ala Arg Ile Asp Ala Arg Ile Asp Phe Glu Ser Gly Arg Ile Lys
                   725                 730                 735
   aag gat gag ttc gct gag atc atg aag atc tgt tcc acc att gaa gag         2256
   Lys Asp Glu Phe Ala Glu Ile Met Lys Ile Cys Ser Thr Ile Glu Glu
               740                 745                 750
   ctc aga cgg caa aaa tag                                                 2274
   Leu Arg Arg Gln Lys
           755
<210> 50
   <211> 757
   <212> PRT
   <213> Influenza virus
<400> 50
   Met Asp Val Asn Pro Thr Leu Leu Phe Leu Lys Val Pro Ala Gln Asn
   1               5                   10                  15
   Ala Ile Ser Thr Thr Phe Pro Tyr Thr Gly Asp Pro Pro Tyr Ser His
               20                  25                  30
   Gly Thr Gly Thr Gly Tyr Thr Met Asp Thr Val Asn Arg Thr His Gln
           35                  40                  45
   Tyr Ser Glu Lys Gly Lys Trp Thr Thr Asn Thr Glu Ile Gly Ala Pro
       50                  55                  60
   Gln Leu Asn Pro Ile Asp Gly Pro Leu Pro Glu Asp Asn Glu Pro Ser
   65                  70                  75                  80
   Gly Tyr Ala Gln Thr Asp Cys Val Leu Glu Ala Met Ala Phe Leu Glu
                   85                  90                  95
   Glu Ser His Pro Gly Ile Phe Glu Asn Ser Cys Leu Glu Thr Met Glu
               100                 105                 110
   Val Ile Gln Gln Thr Arg Val Asp Lys Leu Thr Gln Gly Arg Gln Thr
           115                 120                 125
   Tyr Asp Trp Thr Leu Asn Arg Asn Gln Pro Ala Ala Thr Ala Leu Ala
       130                 135                 140
   Asn Thr Ile Glu Val Phe Arg Ser Asn Gly Leu Thr Ser Asn Glu Ser
   145                 150                 155                 160
   Gly Arg Leu Met Asp Phe Leu Lys Asp Val Met Glu Ser Met Asn Lys
                   165                 170                 175
   Glu Glu Met Glu Ile Thr Thr His Phe Gln Arg Lys Arg Arg Val Arg
               180                 185                 190
   Asp Asn Met Thr Lys Arg Met Ile Thr Gln Arg Thr Ile Gly Lys Lys
           195                 200                 205
   Lys Gln Arg Leu Ser Arg Lys Ser Tyr Leu Ile Arg Thr Leu Thr Leu
       210                 215                 220
   Asn Thr Met Thr Lys Asp Ala Glu Arg Gly Lys Leu Lys Arg Arg Ala
   225                 230                 235                 240
   Ile Ala Thr Pro Gly Met Gln Ile Arg Gly Phe Val Tyr Phe Val Glu
                   245                 250                 255
   Thr Leu Ala Arg Arg Ile Cys Glu Lys Leu Glu Gln Ser Gly Leu Pro
               260                 265                 270
   Val Gly Gly Asn Glu Lys Lys Ala Lys Leu Ala Asn Val Val Arg Lys
           275                 280                 285
   Met Met Thr Asn Ser Gln Asp Thr Glu Leu Ser Phe Thr Ile Thr Gly
       290                 295                 300
   Asp Asn Thr Lys Trp Asn Glu Asn Gln Asn Pro Arg Ile Phe Leu Ala
   305                 310                 315                 320
   Met Ile Thr Tyr Ile Thr Arg Asp Gln Pro Glu Trp Phe Arg Asn Val
                   325                 330                 335
   Leu Ser Ile Ala Pro Ile Met Phe Ser Asn Lys Met Ala Arg Leu Gly
               340                 345                 350
   Lys Gly Tyr Met Phe Glu Ser Lys Ser Met Lys Leu Arg Thr Gln Ile
           355                 360                 365
   Pro Ala Glu Met Leu Ala Ser Ile Asp Leu Lys Tyr Phe Asn Asp Ser
       370                 375                 380
   Thr Lys Lys Lys Ile Glu Lys Ile Arg Pro Leu Leu Val Asp Gly Thr
   385                 390                 395                 400
   Ala Ser Leu Ser Pro Gly Met Met Met Gly Met Phe Asn Met Leu Ser
                   405                 410                 415
   Thr Val Leu Gly Val Ser Ile Leu Asn Leu Gly Gln Arg Lys Tyr Thr
               420                 425                 430
   Lys Thr Thr Tyr Trp Trp Asp Gly Leu Gln Ser Ser Asp Asp Phe Ala
           435                 440                 445
   Leu Ile Val Asn Ala Pro Asn His Glu Gly Ile Gln Ala Gly Val Asp
       450                 455                 460
   Arg Phe Tyr Arg Thr Cys Lys Leu Val Gly Ile Asn Met Ser Lys Lys
   465                 470                 475                 480
   Lys Ser Tyr Ile Asn Arg Thr Gly Thr Phe Glu Phe Thr Ser Phe Phe
                   485                 490                 495
   Tyr Arg Tyr Gly Phe Val Ala Asn Phe Ser Met Glu Leu Pro Ser Phe
               500                 505                 510
   Gly Val Ser Gly Ile Asn Glu Ser Ala Asp Met Ser Ile Gly Val Thr
           515                 520                 525
   Val Ile Lys Asn Asn Met Ile Asn Asn Asp Leu Gly Pro Ala Thr Ala
       530                 535                 540
   Gln Met Ala Leu Gln Leu Phe Ile Lys Asp Tyr Arg Tyr Thr Tyr Arg
   545                 550                 555                 560
   Cys His Arg Gly Asp Thr Gln Ile Gln Thr Arg Arg Ser Phe Glu Leu
                   565                 570                 575
   Lys Lys Leu Trp Glu Gln Thr Arg Ser Lys Thr Gly Leu Leu Val Ser
               580                 585                 590
   Asp Gly Gly Pro Asn Leu Tyr Asn Ile Arg Asn Leu His Ile Pro Glu
           595                 600                 605
   Val Cys Leu Lys Trp Glu Leu Met Asp Glu Asp Tyr Lys Gly Arg Leu
       610                 615                 620
   Cys Asn Pro Leu Asn Pro Phe Val Ser His Lys Glu Ile Glu Ser Val
   625                 630                 635                 640
   Asn Ser Ala Val Val Met Pro Ala His Gly Pro Ala Lys Ser Met Glu
                   645                 650                 655
   Tyr Asp Ala Val Ala Thr Thr His Ser Trp Ile Pro Lys Arg Asn Arg
               660                 665                 670
   Ser Ile Leu Asn Thr Ser Gln Arg Gly Ile Leu Glu Asp Glu Gln Met
           675                 680                 685
   Tyr Gln Lys Cys Cys Asn Leu Phe Glu Lys Phe Phe Pro Ser Ser Ser
       690                 695                 700
   Tyr Arg Arg Pro Val Gly Ile Ser Ser Met Val Glu Ala Met Val Ser
   705                 710                 715                 720
   Arg Ala Arg Ile Asp Ala Arg Ile Asp Phe Glu Ser Gly Arg Ile Lys
                   725                 730                 735
   Lys Asp Glu Phe Ala Glu Ile Met Lys Ile Cys Ser Thr Ile Glu Glu
               740                 745                 750
   Leu Arg Arg Gln Lys
           755
<210> 51
   <211> 2151
   <212> DNA
   <213> Influenza virus
<220>
   <221> CDS
   <222> (1)..(2151)
<400> 51
   atg gaa gac ttt gtg cga cag tgc ttc aat cca atg atc gtc gag ctt        48
   Met Glu Asp Phe Val Arg Gln Cys Phe Asn Pro Met Ile Val Glu Leu
   1               5                   10                  15
   gcg gaa aag gca atg aaa gaa tat gga gag aac ccg aaa atc gaa aca        96
   Ala Glu Lys Ala Met Lys Glu Tyr Gly Glu Asn Pro Lys Ile Glu Thr
               20                  25                  30
   aac aaa ttt gca gca ata tgc act cac ttg gaa gtc tgc ttc atg tac       144
   Asn Lys Phe Ala Ala Ile Cys Thr His Leu Glu Val Cys Phe Met Tyr
           35                  40                  45
   tcg gat ttc cac ttt ata aat gaa ctg ggt gag tca gtg gtc ata gag       192
   Ser Asp Phe His Phe Ile Asn Glu Leu Gly Glu Ser Val Val Ile Glu
       50                  55                  60
   tct ggt gac cca aat gct ctt ttg aaa cac aga ttt gaa atc att gag       240
   Ser Gly Asp Pro Asn Ala Leu Leu Lys His Arg Phe Glu Ile Ile Glu
   65                  70                  75                  80
   ggg aga gat cga aca atg gca tgg aca gta gta aac agc atc tgc aac       288
   Gly Arg Asp Arg Thr Met Ala Trp Thr Val Val Asn Ser Ile Cys Asn
                   85                  90                  95
   acc aca aga gct gaa aaa cct aaa ttt ctt cca gat tta tac gac tat       336
   Thr Thr Arg Ala Glu Lys Pro Lys Phe Leu Pro Asp Leu Tyr Asp Tyr
               100                 105                 110
   aaa gag aac aga ttt gtt gaa att ggt gtg aca agg aga gaa gtt cac       384
   Lys Glu Asn Arg Phe Val Glu Ile Gly Val Thr Arg Arg Glu Val His
           115                 120                 125
   ata tac tac ctg gag aag gcc aac aaa ata aag tct gag aaa aca cat       432
   Ile Tyr Tyr Leu Glu Lys Ala Asn Lys Ile Lys Ser Glu Lys Thr His
       130                 135                 140
   atc cac att ttc tca ttt aca gga gaa gaa atg gct aca aaa gcg gac       480
   Ile His Ile Phe Ser Phe Thr Gly Glu Glu Met Ala Thr Lys Ala Asp
   145                 150                 155                 160
   tat act ctt gat gaa gag agt aga gcc agg atc aag acc aga cta ttc       528
   Tyr Thr Leu Asp Glu Glu Ser Arg Ala Arg Ile Lys Thr Arg Leu Phe
                   165                 170                 175
   act ata aga caa gaa atg gcc agt aga ggc ctc tgg gat tcc ttt cgt       576
   Thr Ile Arg Gln Glu Met Ala Ser Arg Gly Leu Trp Asp Ser Phe Arg
               180                 185                 190
   cag tcc gag aga ggc gaa gag aca att gaa gaa aga ttt gaa atc aca       624
   Gln Ser Glu Arg Gly Glu Glu Thr Ile Glu Glu Arg Phe Glu Ile Thr
           195                 200                 205
   ggg acg atg cgc aag ctt gcc aat tac agt ctc cca ccg aac ttc tcc      672
   Gly Thr Met Arg Lys Leu Ala Asn Tyr Ser Leu Pro Pro Asn Phe Ser
       210                 215                 220
   agc ctt gaa aat ttt aga gtc tat ata gat gga ttc gaa ccg aac ggc      720
   Ser Leu Glu Asn Phe Arg Val Tyr Ile Asp Gly Phe Glu Pro Asn Gly
   225                 230                 235                 240
   tgc att gag agt aag ctt tct caa atg tcc aaa gaa gta aat gcc aaa      768
   Cys Ile Glu Ser Lys Leu Ser Gln Met Ser Lys Glu Val Asn Ala Lys
                   245                 250                 255
   ata gaa cca ttt tca aag aca aca ccc cga cca ctc aaa atg cca ggt      816
   Ile Glu Pro Phe Ser Lys Thr Thr Pro Arg Pro Leu Lys Met Pro Gly
               260                 265                 270
   ggt cca ccc tgc cat cag cga tcc aaa ttc ttg cta atg gat gct ctg      864
   Gly Pro Pro Cys His Gln Arg Ser Lys Phe Leu Leu Met Asp Ala Leu
           275                 280                 285
   aaa ctg agc att gag gac cca agt cac gag gga gag ggg ata cca cta      912
   Lys Leu Ser Ile Glu Asp Pro Ser His Glu Gly Glu Gly Ile Pro Leu
       290                 295                 300
   tat gat gca atc aaa tgc atg aaa act ttc ttt gga tgg aaa gag ccc      960
   Tyr Asp Ala Ile Lys Cys Met Lys Thr Phe Phe Gly Trp Lys Glu Pro
   305                 310                 315                 320
   agt att gtt aaa cca cat aaa aag ggt ata aac ccg aac tat ctc caa     1008
   Ser Ile Val Lys Pro His Lys Lys Gly Ile Asn Pro Asn Tyr Leu Gln
                   325                 330                 335
   act tgg aag caa gta tta gaa gaa ata caa gac ctt gag aac gaa gaa     1056
   Thr Trp Lys Gln Val Leu Glu Glu Ile Gln Asp Leu Glu Asn Glu Glu
               340                 345                 350
   agg acc ccc aag acc aag aat atg aaa aaa aca agc caa ttg aaa tgg     1104
   Arg Thr Pro Lys Thr Lys Asn Met Lys Lys Thr Ser Gln Leu Lys Trp
           355                 360                 365
   gca cta ggt gaa aat atg gca cca gag aaa gtg gat ttt gag gat tgt     1152
   Ala Leu Gly Glu Asn Met Ala Pro Glu Lys Val Asp Phe Glu Asp Cys
       370                 375                 380
   aaa gac atc aat gat tta aaa caa tat gac agt gat gag cca gaa gca     1200
   Lys Asp Ile Asn Asp Leu Lys Gln Tyr Asp Ser Asp Glu Pro Glu Ala
   385                 390                 395                 400
   agg tct ctt gca agt tgg att caa agt gag ttc aac aag gct tgt gag     1248
   Arg Ser Leu Ala Ser Trp Ile Gln Ser Glu Phe Asn Lys Ala Cys Glu
                   405                 410                 415
   ctg aca gat tca agc tgg ata gag ctc gat gaa att ggg gag gat gtc     1296
   Leu Thr Asp Ser Ser Trp Ile Glu Leu Asp Glu Ile Gly Glu Asp Val
               420                 425                 430
   gcc cca ata gaa tac att gcg agc atg agg aga aat tat ttt act gct     1344
   Ala Pro Ile Glu Tyr Ile Ala Ser Met Arg Arg Asn Tyr Phe Thr Ala
           435                 440                 445
   gag att tcc cat tgt aga gca aca gaa tat ata atg aaa gga gtg tac     1392
   Glu Ile Ser His Cys Arg Ala Thr Glu Tyr Ile Met Lys Gly Val Tyr
       450                 455                 460
   atc aac act gct cta ctc aat gca tcc tgt gct gcg atg gat gaa ttt     1440
   Ile Asn Thr Ala Leu Leu Asn Ala Ser Cys Ala Ala Met Asp Glu Phe
   465                 470                 475                 480
   caa tta att ccg atg ata agt aaa tgc agg acc aaa gaa ggg aga agg      1488
   Gln Leu Ile Pro Met Ile Ser Lys Cys Arg Thr Lys Glu Gly Arg Arg
                   485                 490                 495
   aaa aca aat tta tat gga ttc ata ata aag gga agg tcc cat tta aga      1536
   Lys Thr Asn Leu Tyr Gly Phe Ile Ile Lys Gly Arg Ser His Leu Arg
               500                 505                 510
   aat gat act gac gtg gtg aac ttt gta agt atg gaa ttt tct ctc act      1584
   Asn Asp Thr Asp Val Val Asn Phe Val Ser Met Glu Phe Ser Leu Thr
           515                 520                 525
   gat cca aga ttt gag cca cac aaa tgg gaa aaa tac tgc gtt cta gaa      1632
   Asp Pro Arg Phe Glu Pro His Lys Trp Glu Lys Tyr Cys Val Leu Glu
       530                 535                 540
   att gga gac atg ctt tta aga act gct gta ggt caa gtg tca aga ccc      1680
   Ile Gly Asp Met Leu Leu Arg Thr Ala Val Gly Gln Val Ser Arg Pro
   545                 550                 555                 560
   atg ttt ttg tat gta agg aca aat gga acc tct aaa att aaa atg aaa      1728
   Met Phe Leu Tyr Val Arg Thr Asn Gly Thr Ser Lys Ile Lys Met Lys
                   565                 570                 575
   tgg gga atg gaa atg agg cgc tgc ctc ctt cag tct ctg caa cag att      1776
   Trp Gly Met Glu Met Arg Arg Cys Leu Leu Gln Ser Leu Gln Gln Ile
               580                 585                 590
   gaa agc atg atc gaa gct gag tcc tca gtc aaa gaa aag gac atg acc      1824
   Glu Ser Met Ile Glu Ala Glu Ser Ser Val Lys Glu Lys Asp Met Thr
           595                 600                 605
   aaa gaa ttt ttt gag aac aaa tca gag aca tgg cct ata gga gag tcc      1872
   Lys Glu Phe Phe Glu Asn Lys Ser Glu Thr Trp Pro Ile Gly Glu Ser
       610                 615                 620
   ccc aaa gga gtg gaa gag ggc tca atc ggg aag gtt tgc agg acc tta      1920
   Pro Lys Gly Val Glu Glu Gly Ser Ile Gly Lys Val Cys Arg Thr Leu
   625                 630                 635                 640
   tta gca aaa tct gtg ttt aac agt tta tat gca tct cca caa ctg gaa      1968
   Leu Ala Lys Ser Val Phe Asn Ser Leu Tyr Ala Ser Pro Gln Leu Glu
                   645                 650                 655
   gga ttt tca gct gaa tct agg aaa tta ctt ctc att gtt cag gct ctt      2016
   Gly Phe Ser Ala Glu Ser Arg Lys Leu Leu Leu Ile Val Gln Ala Leu
               660                 665                 670
   aga gat gac ctg gaa cct gga acc ttt gat att ggg ggg tta tat gaa      2064
   Arg Asp Asp Leu Glu Pro Gly Thr Phe Asp Ile Gly Gly Leu Tyr Glu
           675                 680                 685
   tca att gag gag tgc ctg att aat gat ccc tgg gtt ttg ctt aat gca      2112
   Ser Ile Glu Glu Cys Leu Ile Asn Asp Pro Trp Val Leu Leu Asn Ala
       690                 695                 700
   tct tgg ttc aac tcc ttc ctc aca cat gca ctg aag tag                  2151
   Ser Trp Phe Asn Ser Phe Leu Thr His Ala Leu Lys
   705                 710                 715
<210> 52
   <211> 716
   <212> PRT
   <213> Influenza virus
<400> 52
   Met Glu Asp Phe Val Arg Gln Cys Phe Asn Pro Met Ile Val Glu Leu
   1               5                   10                  15
   Ala Glu Lys Ala Met Lys Glu Tyr Gly Glu Asn Pro Lys Ile Glu Thr
               20                  25                  30
   Asn Lys Phe Ala Ala Ile Cys Thr His Leu Glu Val Cys Phe Met Tyr
           35                  40                  45
   Ser Asp Phe His Phe Ile Asn Glu Leu Gly Glu Ser Val Val Ile Glu
       50                  55                  60
   Ser Gly Asp Pro Asn Ala Leu Leu Lys His Arg Phe Glu Ile Ile Glu
   65                  70                  75                  80
   Gly Arg Asp Arg Thr Met Ala Trp Thr Val Val Asn Ser Ile Cys Asn
                   85                  90                  95
   Thr Thr Arg Ala Glu Lys Pro Lys Phe Leu Pro Asp Leu Tyr Asp Tyr
               100                 105                 110
   Lys Glu Asn Arg Phe Val Glu Ile Gly Val Thr Arg Arg Glu Val His
           115                 120                 125
   Ile Tyr Tyr Leu Glu Lys Ala Asn Lys Ile Lys Ser Glu Lys Thr His
       130                 135                 140
   Ile His Ile Phe Ser Phe Thr Gly Glu Glu Met Ala Thr Lys Ala Asp
   145                 150                 155                 160
   Tyr Thr Leu Asp Glu Glu Ser Arg Ala Arg Ile Lys Thr Arg Leu Phe
                   165                 170                 175
   Thr Ile Arg Gln Glu Met Ala Ser Arg Gly Leu Trp Asp Ser Phe Arg
               180                 185                 190
   Gln Ser Glu Arg Gly Glu Glu Thr Ile Glu Glu Arg Phe Glu Ile Thr
           195                 200                 205
   Gly Thr Met Arg Lys Leu Ala Asn Tyr Ser Leu Pro Pro Asn Phe Ser
       210                 215                 220
   Ser Leu Glu Asn Phe Arg Val Tyr Ile Asp Gly Phe Glu Pro Asn Gly
   225                 230                 235                 240
   Cys Ile Glu Ser Lys Leu Ser Gln Met Ser Lys Glu Val Asn Ala Lys
                   245                 250                 255
   Ile Glu Pro Phe Ser Lys Thr Thr Pro Arg Pro Leu Lys Met Pro Gly
               260                 265                 270
   Gly Pro Pro Cys His Gln Arg Ser Lys Phe Leu Leu Met Asp Ala Leu
           275                 280                 285
   Lys Leu Ser Ile Glu Asp Pro Ser His Glu Gly Glu Gly Ile Pro Leu
       290                 295                 300
   Tyr Asp Ala Ile Lys Cys Met Lys Thr Phe Phe Gly Trp Lys Glu Pro
   305                 310                 315                 320
   Ser Ile Val Lys Pro His Lys Lys Gly Ile Asn Pro Asn Tyr Leu Gln
                   325                 330                 335
   Thr Trp Lys Gln Val Leu Glu Glu Ile Gln Asp Leu Glu Asn Glu Glu
               340                 345                 350
   Arg Thr Pro Lys Thr Lys Asn Met Lys Lys Thr Ser Gln Leu Lys Trp
           355                 360                 365
   Ala Leu Gly Glu Asn Met Ala Pro Glu Lys Val Asp Phe Glu Asp Cys
       370                 375                 380
   Lys Asp Ile Asn Asp Leu Lys Gln Tyr Asp Ser Asp Glu Pro Glu Ala
   385                 390                 395                 400
   Arg Ser Leu Ala Ser Trp Ile Gln Ser Glu Phe Asn Lys Ala Cys Glu
                   405                 410                 415
   Leu Thr Asp Ser Ser Trp Ile Glu Leu Asp Glu Ile Gly Glu Asp Val
               420                 425                 430
   Ala Pro Ile Glu Tyr Ile Ala Ser Met Arg Arg Asn Tyr Phe Thr Ala
           435                 440                 445
   Glu Ile Ser His Cys Arg Ala Thr Glu Tyr Ile Met Lys Gly Val Tyr
       450                 455                 460
   Ile Asn Thr Ala Leu Leu Asn Ala Ser Cys Ala Ala Met Asp Glu Phe
   465                 470                 475                 480
   Gln Leu Ile Pro Met Ile Ser Lys Cys Arg Thr Lys Glu Gly Arg Arg
                   485                 490                 495
   Lys Thr Asn Leu Tyr Gly Phe Ile Ile Lys Gly Arg Ser His Leu Arg
               500                 505                 510
   Asn Asp Thr Asp Val Val Asn Phe Val Ser Met Glu Phe Ser Leu Thr
           515                 520                 525
   Asp Pro Arg Phe Glu Pro His Lys Trp Glu Lys Tyr Cys Val Leu Glu
       530                 535                 540
   Ile Gly Asp Met Leu Leu Arg Thr Ala Val Gly Gln Val Ser Arg Pro
   545                 550                 555                 560
   Met Phe Leu Tyr Val Arg Thr Asn Gly Thr Ser Lys Ile Lys Met Lys
                   565                 570                 575
   Trp Gly Met Glu Met Arg Arg Cys Leu Leu Gln Ser Leu Gln Gln Ile
               580                 585                 590
   Glu Ser Met Ile Glu Ala Glu Ser Ser Val Lys Glu Lys Asp Met Thr
           595                 600                 605
   Lys Glu Phe Phe Glu Asn Lys Ser Glu Thr Trp Pro Ile Gly Glu Ser
       610                 615                 620
   Pro Lys Gly Val Glu Glu Gly Ser Ile Gly Lys Val Cys Arg Thr Leu
   625                 630                 635                 640
   Leu Ala Lys Ser Val Phe Asn Ser Leu Tyr Ala Ser Pro Gln Leu Glu
                   645                 650                 655
   Gly Phe Ser Ala Glu Ser Arg Lys Leu Leu Leu Ile Val Gln Ala Leu
               660                 665                 670
   Arg Asp Asp Leu Glu Pro Gly Thr Phe Asp Ile Gly Gly Leu Tyr Glu
           675                 680                 685
   Ser Ile Glu Glu Cys Leu Ile Asn Asp Pro Trp Val Leu Leu Asn Ala
       690                 695                 700
   Ser Trp Phe Asn Ser Phe Leu Thr His Ala Leu Lys
   705                 710                 715
<210> 53
   <211> 844
   <212> DNA
   <213> Influenza virus
<220>
   <221> CDS
   <222> (1)..(690)
<400> 53
   atg gat tcc aac act gtg tca agc ttt cag gta gac tgt ttt ctt tgg      48
   Met Asp Ser Asn Thr Val Ser Ser Phe Gln Val Asp Cys Phe Leu Trp
   1               5                   10                  15
   cat gtc cgt aaa cga ttc gca gac caa gaa ctg ggt gat gcc cca ttc      96
   His Val Arg Lys Arg Phe Ala Asp Gln Glu Leu Gly Asp Ala Pro Phe
               20                  25                  30
   ctt gac cgg ctt cgc cga gac cag aag tcc cta agg gga aga ggt agc     144
   Leu Asp Arg Leu Arg Arg Asp Gln Lys Ser Leu Arg Gly Arg Gly Ser
           35                  40                  45
   act ctt ggt ctg gac atc gaa aca gcc act cat gca gga aag cag ata     192
   Thr Leu Gly Leu Asp Ile Glu Thr Ala Thr His Ala Gly Lys Gln Ile
       50                  55                  60
   gtg gag cag att ctg gaa aag gaa tca gat gag gca ctt aaa atg acc        240
   Val Glu Gln Ile Leu Glu Lys Glu Ser Asp Glu Ala Leu Lys Met Thr
   65                  70                  75                  80
   att gcc tct gtt cct gct tca cgc tac tta act gac atg act ctt gat        288
   Ile Ala Ser Val Pro Ala Ser Arg Tyr Leu Thr Asp Met Thr Leu Asp
                   85                  90                  95
   gag atg tca aga gac tgg ttc atg ctc atg ccc aag caa aaa gta aca        336
   Glu Met Ser Arg Asp Trp Phe Met Leu Met Pro Lys Gln Lys Val Thr
               100                 105                 110
   ggc tcc cta tgt ata aga atg gac cag gca atc atg gat aag aac atc        384
   Gly Ser Leu Cys Ile Arg Met Asp Gln Ala Ile Met Asp Lys Asn Ile
           115                 120                 125
   ata ctt aaa gca aac ttt agt gtg att ttc gaa agg ctg gaa aca cta        432
   Ile Leu Lys Ala Asn Phe Ser Val Ile Phe Glu Arg Leu Glu Thr Leu
       130                 135                 140
   ata cta ctt aga gcc ttc acc gaa gaa gga gca gtc gtt ggc gaa att        480
   Ile Leu Leu Arg Ala Phe Thr Glu Glu Gly Ala Val Val Gly Glu Ile
   145                 150                 155                 160
   tca cca tta cct tct ctt cca gga cat act aat gag gat gtc aaa aat        528
   Ser Pro Leu Pro Ser Leu Pro Gly His Thr Asn Glu Asp Val Lys Asn
                   165                 170                 175
   gca att ggg gtc ctc atc gga gga ctt aaa tgg aat gat aat acg gtt        576
   Ala Ile Gly Val Leu Ile Gly Gly Leu Lys Trp Asn Asp Asn Thr Val
               180                 185                 190
   aga atc tct gaa act cta cag aga ttc gct tgg aga agc agt cat gaa        624
   Arg Ile Ser Glu Thr Leu Gln Arg Phe Ala Trp Arg Ser Ser His Glu
           195                 200                 205
   aat ggg aga cct tca ttc cct tca aaa cag aaa cga aaa atg gag aga        672
   Asn Gly Arg Pro Ser Phe Pro Ser Lys Gln Lys Arg Lys Met Glu Arg
       210                 215                 220
   aca att aag cca gaa att tgaagaaata agatggttga ttgaagaagt               720
   Thr Ile Lys Pro Glu Ile
   225                 230
   gcgacataga ttgaaaaata cagaaaatag ttttgaacaa ataacattta tgcaagcctt      780
   acaactattg cttgaagtag aacaagagat aagaactttc tcgtttcagc ttatttaatg      840
   ataa                                                                   844
<210> 54
   <211> 230
   <212> PRT
   <213> Influenza virus
<400> 54
   Met Asp Ser Asn Thr Val Ser Ser Phe Gln Val Asp Cys Phe Leu Trp
   1               5                   10                  15
   His Val Arg Lys Arg Phe Ala Asp Gln Glu Leu Gly Asp Ala Pro Phe
               20                  25                  30
   Leu Asp Arg Leu Arg Arg Asp Gln Lys Ser Leu Arg Gly Arg Gly Ser
           35                  40                  45
   Thr Leu Gly Leu Asp Ile Glu Thr Ala Thr His Ala Gly Lys Gln Ile
       50                  55                  60
   Val Glu Gln Ile Leu Glu Lys Glu Ser Asp Glu Ala Leu Lys Met Thr
   65                  70                  75                  80
   Ile Ala Ser Val Pro Ala Ser Arg Tyr Leu Thr Asp Met Thr Leu Asp
                   85                  90                  95
   Glu Met Ser Arg Asp Trp Phe Met Leu Met Pro Lys Gln Lys Val Thr
               100                 105                 110
   Gly Ser Leu Cys Ile Arg Met Asp Gln Ala Ile Met Asp Lys Asn Ile
           115                 120                 125
   Ile Leu Lys Ala Asn Phe Ser Val Ile Phe Glu Arg Leu Glu Thr Leu
       130                 135                 140
   Ile Leu Leu Arg Ala Phe Thr Glu Glu Gly Ala Val Val Gly Glu Ile
   145                 150                 155                 160
   Ser Pro Leu Pro Ser Leu Pro Gly His Thr Asn Glu Asp Val Lys Asn
                   165                 170                 175
   Ala Ile Gly Val Leu Ile Gly Gly Leu Lys Trp Asn Asp Asn Thr Val
               180                 185                 190
   Arg Ile Ser Glu Thr Leu Gln Arg Phe Ala Trp Arg Ser Ser His Glu
           195                 200                 205
   Asn Gly Arg Pro Ser Phe Pro Ser Lys Gln Lys Arg Lys Met Glu Arg
       210                 215                 220
   Thr Ile Lys Pro Glu Ile
   225                 230
<210> 55
   <211> 1497
   <212> DNA
   <213> Influenza virus
<220>
   <221> CDS
   <222> (1)..(1497)
<400> 55
   atg gcg tct caa ggc acc aaa cga tcc tat gaa cag atg gaa act gat        48
   Met Ala Ser Gln Gly Thr Lys Arg Ser Tyr Glu Gln Met Glu Thr Asp
   1               5                   10                  15
   ggg gaa cgc cag aat gca act gaa atc aga gca tct gtc gga agg atg        96
   Gly Glu Arg Gln Asn Ala Thr Glu Ile Arg Ala Ser Val Gly Arg Met
               20                  25                  30
   gtg gga gga atc gga cgg ttt tat gtc cag atg tgt act gag ctt aaa       144
   Val Gly Gly Ile Gly Arg Phe Tyr Val Gln Met Cys Thr Glu Leu Lys
           35                  40                  45
   cta aac gac cat gaa ggg cgg ctg att cag aac agc ata aca ata gaa      192
   Leu Asn Asp His Glu Gly Arg Leu Ile Gln Asn Ser Ile Thr Ile Glu
       50                  55                  60
   agg atg gtg ctt tcg gca ttc gac gaa aga aga aac aag tat ctc gag      240
   Arg Met Val Leu Ser Ala Phe Asp Glu Arg Arg Asn Lys Tyr Leu Glu
   65                  70                  75                  80
   gag cat ccc agt gct ggg aaa gac cct aag aaa acg gga ggc ccg ata      288
   Glu His Pro Ser Ala Gly Lys Asp Pro Lys Lys Thr Gly Gly Pro Ile
                   85                  90                  95
   tac aga aga aaa gat ggg aaa tgg atg agg gaa ctc atc ctc cat gat      336
   Tyr Arg Arg Lys Asp Gly Lys Trp Met Arg Glu Leu Ile Leu His Asp
               100                 105                 110
   aaa gaa gaa atc atg aga atc tgg cgt cag gcc aac aat ggt gaa gac      384
   Lys Glu Glu Ile Met Arg Ile Trp Arg Gln Ala Asn Asn Gly Glu Asp
           115                 120                 125
   gct act gct ggt ctt act cat atg atg atc tgg cac tcc aat ctc aat      432
   Ala Thr Ala Gly Leu Thr His Met Met Ile Trp His Ser Asn Leu Asn
       130                 135                 140
   gac acc aca tac caa aga aca agg gct ctt gtt cgg act ggg atg gat      480
   Asp Thr Thr Tyr Gln Arg Thr Arg Ala Leu Val Arg Thr Gly Met Asp
   145                 150                 155                 160
   ccc aga atg tgc tct ctg atg caa ggc tca acc ctc cca cgg aga tct      528
   Pro Arg Met Cys Ser Leu Met Gln Gly Ser Thr Leu Pro Arg Arg Ser
                   165                 170                 175
   gga gcc gct ggt gct gca gta aaa ggc gtt gga aca atg gta atg gaa      576
   Gly Ala Ala Gly Ala Ala Val Lys Gly Val Gly Thr Met Val Met Glu
               180                 185                 190
   ctc atc aga atg atc aag cgc gga ata aat gat cgg aat ttc tgg aga      624
   Leu Ile Arg Met Ile Lys Arg Gly Ile Asn Asp Arg Asn Phe Trp Arg
           195                 200                 205
   ggt gaa aat ggt cga aga acc aga att gct tat gaa aga atg tgc aat      672
   Gly Glu Asn Gly Arg Arg Thr Arg Ile Ala Tyr Glu Arg Met Cys Asn
       210                 215                 220
   atc ctc aaa ggg aaa ttt cag aca gca gca caa cgg gct atg atg gac      720
   Ile Leu Lys Gly Lys Phe Gln Thr Ala Ala Gln Arg Ala Met Met Asp
   225                 230                 235                 240
   cag gtg agg gaa ggc cgc aat cct gga aac gct gag att gag gat ctc      768
   Gln Val Arg Glu Gly Arg Asn Pro Gly Asn Ala Glu Ile Glu Asp Leu
                   245                 250                 255
   att ttc ttg gca cga tca gca ctt att ttg aga gga tca gta gcc cat      816
   Ile Phe Leu Ala Arg Ser Ala Leu Ile Leu Arg Gly Ser Val Ala His
               260                 265                 270
   aaa tca tgc cta cct gcc tgt gtt tat ggc ctt gca gta acc agt ggg      864
   Lys Ser Cys Leu Pro Ala Cys Val Tyr Gly Leu Ala Val Thr Ser Gly
           275                 280                 285
   tat gac ttt gag aag gaa gga tac tct ctg gtt gga att gat cct ttc      912
   Tyr Asp Phe Glu Lys Glu Gly Tyr Ser Leu Val Gly Ile Asp Pro Phe
       290                 295                 300
   aaa cta ctc cag aac agt caa att ttc agt cta atc aga cca aaa gaa      960
   Lys Leu Leu Gln Asn Ser Gln Ile Phe Ser Leu Ile Arg Pro Lys Glu
   305                 310                 315                 320
   aac cca gca cac aaa agc cag ttg gtg tgg atg gca tgc cat tct gca      1008
   Asn Pro Ala His Lys Ser Gln Leu Val Trp Met Ala Cys His Ser Ala
                   325                 330                 335
   gca ttt gag gat ctg aga gtt tta aat ttc att aga gga acc aaa gta      1056
   Ala Phe Glu Asp Leu Arg Val Leu Asn Phe Ile Arg Gly Thr Lys Val
               340                 345                 350
   atc cca aga gga cag tta aca acc aga gga gtt caa att gct tca aat      1104
   Ile Pro Arg Gly Gln Leu Thr Thr Arg Gly Val Gln Ile Ala Ser Asn
           355                 360                 365
   gaa aac atg gag aca ata aat tct agc aca ctt gaa ctg aga agc aaa      1152
   Glu Asn Met Glu Thr Ile Asn Ser Ser Thr Leu Glu Leu Arg Ser Lys
       370                 375                 380
   tat tgg gca ata agg acc aga agc gga gga aac acc agt caa cag aga      1200
   Tyr Trp Ala Ile Arg Thr Arg Ser Gly Gly Asn Thr Ser Gln Gln Arg
   385                 390                 395                 400
   gca tct gca gga cag ata agt gtg caa cct act ttc tca gta cag aga      1248
   Ala Ser Ala Gly Gln Ile Ser Val Gln Pro Thr Phe Ser Val Gln Arg
                   405                 410                 415
   aat ctt ccc ttt gag aga gca acc att atg gct gca ttc act ggt aac      1296
   Asn Leu Pro Phe Glu Arg Ala Thr Ile Met Ala Ala Phe Thr Gly Asn
               420                 425                 430
   act gaa gga agg act tcc gac atg aga acg gaa atc ata agg atg atg      1344
   Thr Glu Gly Arg Thr Ser Asp Met Arg Thr Glu Ile Ile Arg Met Met
           435                 440                 445
   gaa aat gcc aaa tca gaa gat gtg tct ttc cag ggg cgg gga gtc ttc      1392
   Glu Asn Ala Lys Ser Glu Asp Val Ser Phe Gln Gly Arg Gly Val Phe
       450                 455                 460
   gag ctc tcg gac gaa aag gca acg aac ccg atc gtg cct tcc ttt gac      1440
   Glu Leu Ser Asp Glu Lys Ala Thr Asn Pro Ile Val Pro Ser Phe Asp
   465                 470                 475                 480
   atg agc aat gaa ggg tct tat ttc ttc gga gac aat gct gag gag ttt      1488
   Met Ser Asn Glu Gly Ser Tyr Phe Phe Gly Asp Asn Ala Glu Glu Phe
                   485                 490                 495
   gac agt taa                                                          1497
   Asp Ser
<210> 56
   <211> 498
   <212> PRT
   <213> Influenza virus
<400> 56
   Met Ala Ser Gln Gly Thr Lys Arg Ser Tyr Glu Gln Met Glu Thr Asp
   1               5                   10                  15
   Gly Glu Arg Gln Asn Ala Thr Glu Ile Arg Ala Ser Val Gly Arg Met
               20                  25                  30
   Val Gly Gly Ile Gly Arg Phe Tyr Val Gln Met Cys Thr Glu Leu Lys
           35                  40                  45
   Leu Asn Asp His Glu Gly Arg Leu Ile Gln Asn Ser Ile Thr Ile Glu
       50                  55                  60
   Arg Met Val Leu Ser Ala Phe Asp Glu Arg Arg Asn Lys Tyr Leu Glu
   65                  70                  75                  80
   Glu His Pro Ser Ala Gly Lys Asp Pro Lys Lys Thr Gly Gly Pro Ile
                   85                  90                  95
   Tyr Arg Arg Lys Asp Gly Lys Trp Met Arg Glu Leu Ile Leu His Asp
               100                 105                 110
   Lys Glu Glu Ile Met Arg Ile Trp Arg Gln Ala Asn Asn Gly Glu Asp
           115                 120                 125
   Ala Thr Ala Gly Leu Thr His Met Met Ile Trp His Ser Asn Leu Asn
       130                 135                 140
   Asp Thr Thr Tyr Gln Arg Thr Arg Ala Leu Val Arg Thr Gly Met Asp
   145                 150                 155                 160
   Pro Arg Met Cys Ser Leu Met Gln Gly Ser Thr Leu Pro Arg Arg Ser
                   165                 170                 175
   Gly Ala Ala Gly Ala Ala Val Lys Gly Val Gly Thr Met Val Met Glu
               180                 185                 190
   Leu Ile Arg Met Ile Lys Arg Gly Ile Asn Asp Arg Asn Phe Trp Arg
           195                 200                 205
   Gly Glu Asn Gly Arg Arg Thr Arg Ile Ala Tyr Glu Arg Met Cys Asn
       210                 215                 220
   Ile Leu Lys Gly Lys Phe Gln Thr Ala Ala Gln Arg Ala Met Met Asp
   225                 230                 235                 240
   Gln Val Arg Glu Gly Arg Asn Pro Gly Asn Ala Glu Ile Glu Asp Leu
                   245                 250                 255
   Ile Phe Leu Ala Arg Ser Ala Leu Ile Leu Arg Gly Ser Val Ala His
               260                 265                 270
   Lys Ser Cys Leu Pro Ala Cys Val Tyr Gly Leu Ala Val Thr Ser Gly
           275                 280                 285
   Tyr Asp Phe Glu Lys Glu Gly Tyr Ser Leu Val Gly Ile Asp Pro Phe
       290                 295                 300
   Lys Leu Leu Gln Asn Ser Gln Ile Phe Ser Leu Ile Arg Pro Lys Glu
   305                 310                 315                 320
   Asn Pro Ala His Lys Ser Gln Leu Val Trp Met Ala Cys His Ser Ala
                   325                 330                 335
   Ala Phe Glu Asp Leu Arg Val Leu Asn Phe Ile Arg Gly Thr Lys Val
               340                 345                 350
   Ile Pro Arg Gly Gln Leu Thr Thr Arg Gly Val Gln Ile Ala Ser Asn
           355                 360                 365
   Glu Asn Met Glu Thr Ile Asn Ser Ser Thr Leu Glu Leu Arg Ser Lys
       370                 375                 380
   Tyr Trp Ala Ile Arg Thr Arg Ser Gly Gly Asn Thr Ser Gln Gln Arg
   385                 390                 395                 400
   Ala Ser Ala Gly Gln Ile Ser Val Gln Pro Thr Phe Ser Val Gln Arg
                   405                 410                 415
   Asn Leu Pro Phe Glu Arg Ala Thr Ile Met Ala Ala Phe Thr Gly Asn
               420                 425                 430
   Thr Glu Gly Arg Thr Ser Asp Met Arg Thr Glu Ile Ile Arg Met Met
           435                 440                 445
   Glu Asn Ala Lys Ser Glu Asp Val Ser Phe Gln Gly Arg Gly Val Phe
       450                 455                 460
   Glu Leu Ser Asp Glu Lys Ala Thr Asn Pro Ile Val Pro Ser Phe Asp
   465                 470                 475                 480
   Met Ser Asn Glu Gly Ser Tyr Phe Phe Gly Asp Asn Ala Glu Glu Phe
                   485                 490                 495
   Asp Ser
<210> 57
   <211> 1413
   <212> DNA
   <213> Influenza virus
<220>
   <221> CDS
   <222> (1)..(1413)
<400> 57
   atg aat cca aat caa aag ata ata gca att gga ttt gca tca ttg ggg       48
   Met Asn Pro Asn Gln Lys Ile Ile Ala Ile Gly Phe Ala Ser Leu Gly
   1               5                   10                  15
   ata tta atc att aat gtc att ctc cat gta gtc agc att ata gta aca       96
   Ile Leu Ile Ile Asn Val Ile Leu His Val Val Ser Ile Ile Val Thr
               20                  25                  30
   gta ctg gtc ctc aat aac aat aga aca gat ctg aac tgc aaa ggg acg      144
   Val Leu Val Leu Asn Asn Asn Arg Thr Asp Leu Asn Cys Lys Gly Thr
                   35                  40                  45
   atc ata aga gaa tac aat gaa aca gta aga gta gaa aaa ctt act caa      192
   Ile Ile Arg Glu Tyr Asn Glu Thr Val Arg Val Glu Lys Leu Thr Gln
       50                  55                  60
   tgg tat aat acc agt aca att aag tac ata gag aga cct tca aat gaa      240
   Trp Tyr Asn Thr Ser Thr Ile Lys Tyr Ile Glu Arg Pro Ser Asn Glu
   65                  70                  75                  80
   tac tac atg aat aac act gaa cca ctt tgt gag gcc caa ggc ttt gca      288
   Tyr Tyr Met Asn Asn Thr Glu Pro Leu Cys Glu Ala Gln Gly Phe Ala
                   85                  90                  95
   cca ttt tcc aaa gat aat gga ata cga att ggg tcg aga ggc cat gtt      336
   Pro Phe Ser Lys Asp Asn Gly Ile Arg Ile Gly Ser Arg Gly His Val
               100                 105                 110
   ttt gtg ata aga gaa cct ttt gta tca tgt tcg ccc tca gaa tgt aga      384
   Phe Val Ile Arg Glu Pro Phe Val Ser Cys Ser Pro Ser Glu Cys Arg
           115                 120                 125
   acc ttt ttc ctc aca cag ggc tca tta ctc aat gac aaa cat tct aac      432
   Thr Phe Phe Leu Thr Gln Gly Ser Leu Leu Asn Asp Lys His Ser Asn
       130                 135                 140
   ggc aca ata aag gat cga agt ccg tat agg act ttg atg agt gtc aaa      480
   Gly Thr Ile Lys Asp Arg Ser Pro Tyr Arg Thr Leu Met Ser Val Lys
   145                 150                 155                 160
   ata ggg caa tca cct aat gta tat caa gct agg ttt gaa tcg gtg gca      528
   Ile Gly Gln Ser Pro Asn Val Tyr Gln Ala Arg Phe Glu Ser Val Ala
                   165                 170                 175
   tgg tca gca aca gca tgc cat gat gga aaa aaa tgg atg aca gtt gga      576
   Trp Ser Ala Thr Ala Cys His Asp Gly Lys Lys Trp Met Thr Val Gly
               180                 185                 190
   gtc aca ggg ccc gac aat caa gca att gca gta gtg aac tat gga ggt      624
   Val Thr Gly Pro Asp Asn Gln Ala Ile Ala Val Val Asn Tyr Gly Gly
           195                 200                 205
   gtt ccg gtt gat att att aat tca tgg gca ggg gat att tta aga acc      672
   Val Pro Val Asp Ile Ile Asn Ser Trp Ala Gly Asp Ile Leu Arg Thr
       210                 215                 220
   caa gaa tca tca tgc acc tgc att aaa gga gac tgt tat tgg gta atg      720
   Gln Glu Ser Ser Cys Thr Cys Ile Lys Gly Asp Cys Tyr Trp Val Met
   225                 230                 235                 240
   act gat gga ccg gca aat agg caa gct aaa tat agg ata ttc aaa gca      768
   Thr Asp Gly Pro Ala Asn Arg Gln Ala Lys Tyr Arg Ile Phe Lys Ala
                   245                 250                 255
   aaa gat gga aga gta att gga caa act gat ata agt ttc aat ggg gga      816
   Lys Asp Gly Arg Val Ile Gly Gln Thr Asp Ile Ser Phe Asn Gly Gly
               260                 265                 270
   cac ata gag gag tgt tct tgt tac ccc aat gaa ggg aag gtg gaa tgc      864
   His Ile Glu Glu Cys Ser Cys Tyr Pro Asn Glu Gly Lys Val Glu Cys
           275                 280                 285
   ata tgc agg gac aat tgg act gga aca aat aga cca att ctg gta ata      912
   Ile Cys Arg Asp Asn Trp Thr Gly Thr Asn Arg Pro Ile Leu Val Ile
       290                 295                 300
   tct tct gat cta tcg tac aca gtt gga tat ttg tgt gct ggc att ccc      960
   Ser Ser Asp Leu Ser Tyr Thr Val Gly Tyr Leu Cys Ala Gly Ile Pro
   305                 310                 315                 320
   act gac act cct agg gga gag gat agt caa ttc aca ggc tca tgt aca      1008
   Thr Asp Thr Pro Arg Gly Glu Asp Ser Gln Phe Thr Gly Ser Cys Thr
                   325                     330                     335
   agt cct ttg gga aat aaa gga tac ggt gta aaa ggc ttc ggg ttt cga      1056
   Ser Pro Leu Gly Asn Lys Gly Tyr Gly Val Lys Gly Phe Gly Phe Arg
               340                     345                     350
   caa gga act gac gta tgg gcc gga agg aca att agt agg act tca aga      1104
   Gln Gly Thr Asp Val Trp Ala Gly Arg Thr Ile Ser Arg Thr Ser Arg
           355                     360                     365
   tca gga ttc gaa ata ata aaa atc agg aat ggt tgg aca cag aac agt      1152
   Ser Gly Phe Glu Ile Ile Lys Ile Arg Asn Gly Trp Thr Gln Asn Ser
       370                     375                     380
   aag gac caa atc agg agg caa gtg att atc gat gac cca aat tgg tca      1200
   Lys Asp Gln Ile Arg Arg Gln Val Ile Ile Asp Asp Pro Asn Trp Ser
   385                     390                     395                     400
   gga tat agc ggt tct ttc aca ttg ccg gtt gaa ctg aca aaa aag gga      1248
   Gly Tyr Ser Gly Ser Phe Thr Leu Pro Val Glu Leu Thr Lys Lys Gly
                   405                     410                     415
   tgt ttg gtc ccc tgt ttc tgg gtt gaa atg att aga ggt aaa cct gaa      1296
   Cys Leu Val Pro Cys Phe Trp Val Glu Met Ile Arg Gly Lys Pro Glu
               420                     425                     430
   gaa aca aca ata tgg acc tct agc agc tcc att gtg atg tgt gga gta      1344
   Glu Thr Thr Ile Trp Thr Ser Ser Ser Ser Ile Val Met Cys Gly Val
           435                     440                     445
   gat cat aaa att gcc agt tgg tca tgg cac gat gga gct att ctt ccc      1392
   Asp His Lys Ile Ala Ser Trp Ser Trp His Asp Gly Ala Ile Leu Pro
       450                     455                     460
   ttt gac atc gat aag atg taa                                          1413
   Phe Asp Ile Asp Lys Met
   465                     470
<210> 58
   <211> 470
   <212> PRT
   <213> Influenza virus
<400> 58
   Met Asn Pro Asn Gln Lys Ile Ile Ala Ile Gly Phe Ala Ser Leu Gly
   1               5                   10                  15
   Ile Leu Ile Ile Asn Val Ile Leu His Val Val Ser Ile Ile Val Thr
               20                  25                  30
   Val Leu Val Leu Asn Asn Asn Arg Thr Asp Leu Asn Cys Lys Gly Thr
           35                  40                  45
   Ile Ile Arg Glu Tyr Asn Glu Thr Val Arg Val Glu Lys Leu Thr Gln
       50                  55                  60
   Trp Tyr Asn Thr Ser Thr Ile Lys Tyr Ile Glu Arg Pro Ser Asn Glu
   65                  70                  75                  80
   Tyr Tyr Met Asn Asn Thr Glu Pro Leu Cys Glu Ala Gln Gly Phe Ala
                   85                  90                  95
   Pro Phe Ser Lys Asp Asn Gly Ile Arg Ile Gly Ser Arg Gly His Val
               100                 105                 110
   Phe Val Ile Arg Glu Pro Phe Val Ser Cys Ser Pro Ser Glu Cys Arg
           115                 120                 125
   Thr Phe Phe Leu Thr Gln Gly Ser Leu Leu Asn Asp Lys His Ser Asn
       130                 135                 140
   Gly Thr Ile Lys Asp Arg Ser Pro Tyr Arg Thr Leu Met Ser Val Lys
   145                 150                 155                 160
   Ile Gly Gln Ser Pro Asn Val Tyr Gln Ala Arg Phe Glu Ser Val Ala
                   165                 170                 175
   Trp Ser Ala Thr Ala Cys His Asp Gly Lys Lys Trp Met Thr Val Gly
               180                 185                 190
   Val Thr Gly Pro Asp Asn Gln Ala Ile Ala Val Val Asn Tyr Gly Gly
           195                 200                 205
   Val Pro Val Asp Ile Ile Asn Ser Trp Ala Gly Asp Ile Leu Arg Thr
       210                 215                 220
   Gln Glu Ser Ser Cys Thr Cys Ile Lys Gly Asp Cys Tyr Trp Val Met
   225                 230                 235                 240
   Thr Asp Gly Pro Ala Asn Arg Gln Ala Lys Tyr Arg Ile Phe Lys Ala
                   245                 250                 255
   Lys Asp Gly Arg Val Ile Gly Gln Thr Asp Ile Ser Phe Asn Gly Gly
               260                 265                 270
   His Ile Glu Glu Cys Ser Cys Tyr Pro Asn Glu Gly Lys Val Glu Cys
           275                 280                 285
   Ile Cys Arg Asp Asn Trp Thr Gly Thr Asn Arg Pro Ile Leu Val Ile
       290                 295                 300
   Ser Ser Asp Leu Ser Tyr Thr Val Gly Tyr Leu Cys Ala Gly Ile Pro
   305                 310                 315                 320
   Thr Asp Thr Pro Arg Gly Glu Asp Ser Gln Phe Thr Gly Ser Cys Thr
                   325                 330                 335
   Ser Pro Leu Gly Asn Lys Gly Tyr Gly Val Lys Gly Phe Gly Phe Arg
               340                 345                 350
   Gln Gly Thr Asp Val Trp Ala Gly Arg Thr Ile Ser Arg Thr Ser Arg
   355 360 365
   Ser Gly Phe Glu Ile Ile Lys Ile Arg Asn Gly Trp Thr Gln Asn Ser
   370 375 380
   Lys Asp Gln Ile Arg Arg Gln Val Ile Ile Asp Asp Pro Asn Trp Ser
   385 390 395 400
   Gly Tyr Ser Gly Ser Phe Thr Leu Pro Val Glu Leu Thr Lys Lys Gly
   405 410 415
   Cys Leu Val Pro Cys Phe Trp Val Glu Met Ile Arg Gly Lys Pro Glu
   420 425 430
   Glu Thr Thr Ile Trp Thr Ser Ser Ser Ser Ile Val Met Cys Gly Val
   435 440 445
   Asp His Lys Ile Ala Ser Trp Ser Trp His Asp Gly Ala Ile Leu Pro
   450 455 460
   Phe Asp Ile Asp Lys Met
   465 470
<210> 59
   <211> 981
   <212> DNA
   <213> Influenza virus
<220>
   <221> CDS
   <222> (1)..(756)
<400> 59
   atg agt ctt cta acc gag gtc gaa acg tac gtt ctc tct atc gta cca      48
   Met Ser Leu Leu Thr Glu Val Glu Thr Tyr Val Leu Ser Ile Val Pro
   1               5                   10                  15
   tca ggc ccc ctc aaa gcc gag atc gcg cag aga ctt gaa gat gtc ttt      96
   Ser Gly Pro Leu Lys Ala Glu Ile Ala Gln Arg Leu Glu Asp Val Phe
               20                  25                  30
   gcg gga aag aac acc gat ctt gag gca ctc atg gaa tgg cta aag aca     144
   Ala Gly Lys Asn Thr Asp Leu Glu Ala Leu Met Glu Trp Leu Lys Thr
           35                  40                  45
   aga cca atc ctg tca cct ctg act aaa ggg att tta gga ttt gta ttc     192
   Arg Pro Ile Leu Ser Pro Leu Thr Lys Gly Ile Leu Gly Phe Val Phe
       50                  55                  60
   acg ctc acc gtg ccc agt gag cga gga ctg cag cgt aga cgc ttt gtc     240
   Thr Leu Thr Val Pro Ser Glu Arg Gly Leu Gln Arg Arg Arg Phe Val
   65                  70                  75                  80
   caa aat gcc ctt agt gga aac gga gat cca aac aac atg gac aga gca     288
   Gln Asn Ala Leu Ser Gly Asn Gly Asp Pro Asn Asn Met Asp Arg Ala
                   85                  90                  95
   gta aaa ctg tac agg aag ctt aaa aga gaa ata aca ttc cat ggg gca     336
   Val Lys Leu Tyr Arg Lys Leu Lys Arg Glu Ile Thr Phe His Gly Ala
               100                 105                 110
   aaa gag gtg gca ctc agc tat tcc act ggt gca cta gcc agc tgc atg      384
   Lys Glu Val Ala Leu Ser Tyr Ser Thr Gly Ala Leu Ala Ser Cys Met
           115                 120                 125
   gga ctc ata tac aac aga atg gga act gtt aca acc gaa gtg gca ttt      432
   Gly Leu Ile Tyr Asn Arg Met Gly Thr Val Thr Thr Glu Val Ala Phe
       130                 135                 140
   ggc ctg gta tgc gcc aca tgt gaa cag att gct gat tcc cag cat cga      480
   Gly Leu Val Cys Ala Thr Cys Glu Gln Ile Ala Asp Ser Gln His Arg
   145                 150                 155                 160
   tct cac agg cag atg gtg aca aca acc aac cca tta atc aga cat gaa      528
   Ser His Arg Gln Met Val Thr Thr Thr Asn Pro Leu Ile Arg His Glu
                   165                 170                 175
   aac aga atg gta tta gcc agt acc acg gct aaa gcc atg gaa cag atg      576
   Asn Arg Met Val Leu Ala Ser Thr Thr Ala Lys Ala Met Glu Gln Met
               180                 185                 190
   gca gga tcg agt gag cag gca gca gag gcc atg gag gtt gct agt agg      624
   Ala Gly Ser Ser Glu Gln Ala Ala Glu Ala Met Glu Val Ala Ser Arg
           195                 200                 205
   gct agg cag atg gta cag gca atg aga acc att ggg acc cac cct agc      672
   Ala Arg Gln Met Val Gln Ala Met Arg Thr Ile Gly Thr His Pro Ser
       210                 215                 220
   tcc agt gcc ggt ttg aaa gat gat ctc ctt gaa aat tta cag gcc tac      720
   Ser Ser Ala Gly Leu Lys Asp Asp Leu Leu Glu Asn Leu Gln Ala Tyr
   225                 230                 235                 240
   cag aaa cgg atg gga gtg caa atg cag cga ttc aag tgatcctctc           766
   Gln Lys Arg Met Gly Val Gln Met Gln Arg Phe Lys
                   245                 250
   gtcattgcag caagtatcat tgggatcttg cacttgatat tgtggattct tgatcgtctt    826
   ttcttcaaat tcatttatcg tcgccttaaa tacgggttga aaagagggcc ttctacggaa    886
   ggagtacctg agtctatgag ggaagaatat cggcaggaac agcagaatgc tgtggatgtt    946
   gacgatggtc attttgtcaa catagagctg gagta                               981
<210> 60
   <211> 252
   <212> PRT
   <213> Influenza virus
<400> 60
   Met Ser Leu Leu Thr Glu Val Glu Thr Tyr Val Leu Ser Ile Val Pro
   1               5                   10                  15
   Ser Gly Pro Leu Lys Ala Glu Ile Ala Gln Arg Leu Glu Asp Val Phe
               20                  25                  30
   Ala Gly Lys Asn Thr Asp Leu Glu Ala Leu Met Glu Trp Leu Lys Thr
           35                  40                  45
   Arg Pro Ile Leu Ser Pro Leu Thr Lys Gly Ile Leu Gly Phe Val Phe
       50                  55                  60
   Thr Leu Thr Val Pro Ser Glu Arg Gly Leu Gln Arg Arg Arg Phe Val
   65                  70                  75                  80
   Gln Asn Ala Leu Ser Gly Asn Gly Asp Pro Asn Asn Met Asp Arg Ala
                   85                  90                  95
   Val Lys Leu Tyr Arg Lys Leu Lys Arg Glu Ile Thr Phe His Gly Ala
               100                 105                 110
   Lys Glu Val Ala Leu Ser Tyr Ser Thr Gly Ala Leu Ala Ser Cys Met
           115                 120                 125
   Gly Leu Ile Tyr Asn Arg Met Gly Thr Val Thr Thr Glu Val Ala Phe
       130                 135                 140
   Gly Leu Val Cys Ala Thr Cys Glu Gln Ile Ala Asp Ser Gln His Arg
   145                 150                 155                 160
   Ser His Arg Gln Met Val Thr Thr Thr Asn Pro Leu Ile Arg His Glu
                   165                 170                 175
   Asn Arg Met Val Leu Ala Ser Thr Thr Ala Lys Ala Met Glu Gln Met
               180                 185                 190
   Ala Gly Ser Ser Glu Gln Ala Ala Glu Ala Met Glu Val Ala Ser Arg
           195                 200                 205
   Ala Arg Gln Met Val Gln Ala Met Arg Thr Ile Gly Thr His Pro Ser
       210                 215                 220
   Ser Ser Ala Gly Leu Lys Asp Asp Leu Leu Glu Asn Leu Gln Ala Tyr
   225                 230                 235                 240
   Gln Lys Arg Met Gly Val Gln Met Gln Arg Phe Lys
                   245                 250
<210> 61
   <211> 1698
   <212> DNA
   <213> Influenza virus
<220>
   <221> CDS
   <222> (1)..(1698)
<400> 61
   atg aag aca acc att att tta ata cta ctg acc cat tgg gcc tac agt        48
   Met Lys Thr Thr Ile Ile Leu Ile Leu Leu Thr His Trp Ala Tyr Ser
   1               5                   10                  15
   caa aac cca atc agt ggc aat aac aca gcc aca ctg tgt ctg gga cac        96
   Gln Asn Pro Ile Ser Gly Asn Asn Thr Ala Thr Leu Cys Leu Gly His
               20                  25                  30
   cat gca gta gca aat gga aca ttg gta aaa aca atg agt gat gat caa       144
   His Ala Val Ala Asn Gly Thr Leu Val Lys Thr Met Ser Asp Asp Gln
           35                  40                  45
   att gag gtg aca aat gct aca gaa tta gtt cag agc att tca atg ggg      192
   Ile Glu Val Thr Asn Ala Thr Glu Leu Val Gln Ser Ile Ser Met Gly
       50                  55                  60
   aaa ata tgc aac aaa tca tat aga att cta gat gga aga aat tgc aca      240
   Lys Ile Cys Asn Lys Ser Tyr Arg Ile Leu Asp Gly Arg Asn Cys Thr
   65                  70                  75                  80
   tta ata gat gca atg cta gga gac ccc cac tgt gac gcc ttt cag tat      288
   Leu Ile Asp Ala Met Leu Gly Asp Pro His Cys Asp Ala Phe Gln Tyr
                   85                  90                  95
   gag agt tgg gac ctc ttt ata gaa aga agc agc gct ttc agc aat tgc      336
   Glu Ser Trp Asp Leu Phe Ile Glu Arg Ser Ser Ala Phe Ser Asn Cys
               100                 105                 110
   tac cca tat gac atc cct gac tat gca tcg ctc cga tcc att gta gca      384
   Tyr Pro Tyr Asp Ile Pro Asp Tyr Ala Ser Leu Arg Ser Ile Val Ala
           115                 120                 125
   tcc tca ggg aca gtg gaa ttc aca gca gag gga ttc aca tgg aca ggt      432
   Ser Ser Gly Thr Val Glu Phe Thr Ala Glu Gly Phe Thr Trp Thr Gly
       130                 135                 140
   gta act caa aac gga aga agt gga gcc tgc aaa agg gga tca gcc gat      480
   Val Thr Gln Asn Gly Arg Ser Gly Ala Cys Lys Arg Gly Ser Ala Asp
   145                 150                 155                 160
   agt ttc ttt agc cga ctg aat tgg cta aca aaa tct gga agc tct tac      528
   Ser Phe Phe Ser Arg Leu Asn Trp Leu Thr Lys Ser Gly Ser Ser Tyr
                   165                 170                 175
   ccc aca ttg aat gtg aca atg cct aac aat aaa aat ttc gac aag cta      576
   Pro Thr Leu Asn Val Thr Met Pro Asn Asn Lys Asn Phe Asp Lys Leu
               180                 185                 190
   tac atc tgg ggg att cat cac ccg agc tca aat caa gag cag aca aaa      624
   Tyr Ile Trp Gly Ile His His Pro Ser Ser Asn Gln Glu Gln Thr Lys
           195                 200                 205
   ttg tac atc caa gaa tca gga cga gta aca gtc tca aca aaa aga agt      672
   Leu Tyr Ile Gln Glu Ser Gly Arg Val Thr Val Ser Thr Lys Arg Ser
       210                 215                 220
   caa caa aca ata atc cct aac atc gga tct aga ccg ttg gtc aga ggt      720
   Gln Gln Thr Ile Ile Pro Asn Ile Gly Ser Arg Pro Leu Val Arg Gly
   225                 230                 235                 240
   caa tca ggc agg ata agc ata tac tgg acc att gta aaa cct gga gat      768
   Gln Ser Gly Arg Ile Ser Ile Tyr Trp Thr Ile Val Lys Pro Gly Asp
                   245                 250                 255
   atc cta atg ata aac agt aat ggc aac tta gtt gca ccg cgg gga tat      816
   Ile Leu Met Ile Asn Ser Asn Gly Asn Leu Val Ala Pro Arg Gly Tyr
               260                 265                 270
   ttt aaa ttg aac aca ggg aaa agc tct gta atg aga tcc gat gta ccc      864
   Phe Lys Leu Asn Thr Gly Lys Ser Ser Val Met Arg Ser Asp Val Pro
           275                 280                 285
   ata gac att tgt gtg tct gaa tgt att aca cca aat gga agc atc tcc      912
   Ile Asp Ile Cys Val Ser Glu Cys Ile Thr Pro Asn Gly Ser Ile Ser
       290                 295                 300
   aac gac aag cca ttc caa aat gtg aac aaa gtt aca tat gga aaa tgc      960
   Asn Asp Lys Pro Phe Gln Asn Val Asn Lys Val Thr Tyr Gly Lys Cys
   305                 310                 315                 320
   ccc aag tat atc agg caa aac act tta aag ctg gcc act ggg atg agg      1008
   Pro Lys Tyr Ile Arg Gln Asn Thr Leu Lys Leu Ala Thr Gly Met Arg
                   325                 330                 335
   aat gta cca gaa aag caa acc aga gga atc ttt gga gca ata gcg gga      1056
   Asn Val Pro Glu Lys Gln Thr Arg Gly Ile Phe Gly Ala Ile Ala Gly
               340                 345                 350
   ttc atc gaa aac ggc tgg gaa gga atg gtt gat ggg tgg tat ggg ttc      1104
   Phe Ile Glu Asn Gly Trp Glu Gly Met Val Asp Gly Trp Tyr Gly Phe
           355                 360                 365
   cga tat caa aac tct gaa gga aca ggg caa gct gca gat cta aag agc      1152
   Arg Tyr Gln Asn Ser Glu Gly Thr Gly Gln Ala Ala Asp Leu Lys Ser
       370                 375                 380
   act caa gca gcc atc gac cag att aat gga aag tta aac aga gtg att      1200
   Thr Gln Ala Ala Ile Asp Gln Ile Asn Gly Lys Leu Asn Arg Val Ile
   385                 390                 395                 400
   gaa aga acc aat gag aaa ttc cat caa ata gag aag gaa ttc tca gaa      1248
   Glu Arg Thr Asn Glu Lys Phe His Gln Ile Glu Lys Glu Phe Ser Glu
                   405                 410                 415
   gta gaa gga aga att cag gac ttg gag aaa tat gta gaa gac acc aaa      1296
   Val Glu Gly Arg Ile Gln Asp Leu Glu Lys Tyr Val Glu Asp Thr Lys
               420                 425                 430
   ata gac cta tgg tcc tac aat gca gaa ttg ctg gtg gct cta gaa aat      1344
   Ile Asp Leu Trp Ser Tyr Asn Ala Glu Leu Leu Val Ala Leu Glu Asn
           435                 440                 445
   caa cat aca att gac tta aca gat gca gaa atg aat aaa tta ttt gag      1392
   Gln His Thr Ile Asp Leu Thr Asp Ala Glu Met Asn Lys Leu Phe Glu
       450                 455                 460
   aag act aga cgc cag tta aga gaa aac gca gaa gac atg gga ggt gga      1440
   Lys Thr Arg Arg Gln Leu Arg Glu Asn Ala Glu Asp Met Gly Gly Gly
   465                 470                 475                 480
   tgt ttc aag att tac cac aaa tgt gat aat gca tgc att gaa tca ata      1488
   Cys Phe Lys Ile Tyr His Lys Cys Asp Asn Ala Cys Ile Glu Ser Ile
                   485                 490                 495
   aga act ggg aca tat gac cat tac ata tac aaa gat gaa gca tta aac      1536
   Arg Thr Gly Thr Tyr Asp His Tyr Ile Tyr Lys Asp Glu Ala Leu Asn
               500                 505                 510
   aat cga ttt cag atc aaa ggt gta gag ttg aaa tca ggc tac aaa gat      1584
   Asn Arg Phe Gln Ile Lys Gly Val Glu Leu Lys Ser Gly Tyr Lys Asp
           515                 520                 525
   tgg ata ctg tgg att tca ttc gcc ata tca tgc ttc tta att tgc gtt      1632
   Trp Ile Leu Trp Ile Ser Phe Ala Ile Ser Cys Phe Leu Ile Cys Val
       530                 535                 540
   gtt cta ttg ggt ttc att atg tgg gct tgc caa aaa ggc aac atc aga      1680
   Val Leu Leu Gly Phe Ile Met Trp Ala Cys Gln Lys Gly Asn Ile Arg
   545                 550                 555                 560
   tgc aac att tgc att tga                                              1698
   Cys Asn Ile Cys Ile
                   565
<210> 62
   <211> 565
   <212> PRT
   <213> Influenza virus
<400> 62
   Met Lys Thr Thr Ile Ile Leu Ile Leu Leu Thr His Trp Ala Tyr Ser
   1               5                   10                  15
   Gln Asn Pro Ile Ser Gly Asn Asn Thr Ala Thr Leu Cys Leu Gly His
               20                  25                  30
   His Ala Val Ala Asn Gly Thr Leu Val Lys Thr Met Ser Asp Asp Gln
           35                  40                  45
   Ile Glu Val Thr Asn Ala Thr Glu Leu Val Gln Ser Ile Ser Met Gly
       50                  55                  60
   Lys Ile Cys Asn Lys Ser Tyr Arg Ile Leu Asp Gly Arg Asn Cys Thr
   65                  70                  75                  80
   Leu Ile Asp Ala Met Leu Gly Asp Pro His Cys Asp Ala Phe Gln Tyr
                   85                  90                  95
   Glu Ser Trp Asp Leu Phe Ile Glu Arg Ser Ser Ala Phe Ser Asn Cys
               100                 105                 110
   Tyr Pro Tyr Asp Ile Pro Asp Tyr Ala Ser Leu Arg Ser Ile Val Ala
           115                 120                 125
   Ser Ser Gly Thr Val Glu Phe Thr Ala Glu Gly Phe Thr Trp Thr Gly
       130                 135                 140
   Val Thr Gln Asn Gly Arg Ser Gly Ala Cys Lys Arg Gly Ser Ala Asp
   145                 150                 155                 160
   Ser Phe Phe Ser Arg Leu Asn Trp Leu Thr Lys Ser Gly Ser Ser Tyr
                   165                 170                 175
   Pro Thr Leu Asn Val Thr Met Pro Asn Asn Lys Asn Phe Asp Lys Leu
               180                 185                 190
   Tyr Ile Trp Gly Ile His His Pro Ser Ser Asn Gln Glu Gln Thr Lys
           195                 200                 205
   Leu Tyr Ile Gln Glu Ser Gly Arg Val Thr Val Ser Thr Lys Arg Ser
       210                 215                 220
   Gln Gln Thr Ile Ile Pro Asn Ile Gly Ser Arg Pro Leu Val Arg Gly
   225                 230                 235                 240
   Gln Ser Gly Arg Ile Ser Ile Tyr Trp Thr Ile Val Lys Pro Gly Asp
                   245                 250                 255
   Ile Leu Met Ile Asn Ser Asn Gly Asn Leu Val Ala Pro Arg Gly Tyr
               260                 265                 270
   Phe Lys Leu Asn Thr Gly Lys Ser Ser Val Met Arg Ser Asp Val Pro
           275                 280                 285
   Ile Asp Ile Cys Val Ser Glu Cys Ile Thr Pro Asn Gly Ser Ile Ser
       290                 295                 300
   Asn Asp Lys Pro Phe Gln Asn Val Asn Lys Val Thr Tyr Gly Lys Cys
   305                 310                 315                 320
   Pro Lys Tyr Ile Arg Gln Asn Thr Leu Lys Leu Ala Thr Gly Met Arg
                   325                 330                 335
   Asn Val Pro Glu Lys Gln Thr Arg Gly Ile Phe Gly Ala Ile Ala Gly
               340                 345                 350
   Phe Ile Glu Asn Gly Trp Glu Gly Met Val Asp Gly Trp Tyr Gly Phe
           355                 360                 365
   Arg Tyr Gln Asn Ser Glu Gly Thr Gly Gln Ala Ala Asp Leu Lys Ser
       370                 375                 380
   Thr Gln Ala Ala Ile Asp Gln Ile Asn Gly Lys Leu Asn Arg Val Ile
   385                 390                 395                 400
   Glu Arg Thr Asn Glu Lys Phe His Gln Ile Glu Lys Glu Phe Ser Glu
                   405                 410                 415
   Val Glu Gly Arg Ile Gln Asp Leu Glu Lys Tyr Val Glu Asp Thr Lys
               420                 425                 430
   Ile Asp Leu Trp Ser Tyr Asn Ala Glu Leu Leu Val Ala Leu Glu Asn
           435                 440                 445
   Gln His Thr Ile Asp Leu Thr Asp Ala Glu Met Asn Lys Leu Phe Glu
       450                 455                 460
   Lys Thr Arg Arg Gln Leu Arg Glu Asn Ala Glu Asp Met Gly Gly Gly
   465                 470                 475                 480
   Cys Phe Lys Ile Tyr His Lys Cys Asp Asn Ala Cys Ile Glu Ser Ile
                   485                 490                 495
   Arg Thr Gly Thr Tyr Asp His Tyr Ile Tyr Lys Asp Glu Ala Leu Asn
               500                 505                 510
   Asn Arg Phe Gln Ile Lys Gly Val Glu Leu Lys Ser Gly Tyr Lys Asp
           515                 520                 525
   Trp Ile Leu Trp Ile Ser Phe Ala Ile Ser Cys Phe Leu Ile Cys Val
       530                 535                 540
   Val Leu Leu Gly Phe Ile Met Trp Ala Cys Gln Lys Gly Asn Ile Arg
   545                 550                 555                 560
   Cys Asn Ile Cys Ile
                   565
<210> 63
   <211> 2280
   <212> DNA
   <213> Influenza virus
<220>
   <221> CDS
   <222> (1)..(2280)
<400> 63
   atg gag aga ata aaa gaa ctg aga gat ctg atg tta caa tcc cgc acc      48
   Met Glu Arg Ile Lys Glu Leu Arg Asp Leu Met Leu Gln Ser Arg Thr
   1               5                   10                  15
   cgc gag ata cta aca aaa act act gtg gac cac atg gcc ata atc aag      96
   Arg Glu Ile Leu Thr Lys Thr Thr Val Asp His Met Ala Ile Ile Lys
               20                  25                  30
   aaa tac aca tca gga aga caa gag aag aac cct gca ctt agg atg aaa     144
   Lys Tyr Thr Ser Gly Arg Gln Glu Lys Asn Pro Ala Leu Arg Met Lys
           35                  40                  45
   tgg atg atg gca atg aaa tac cca att aca gca gat aag agg ata atg     192
   Trp Met Met Ala Met Lys Tyr Pro Ile Thr Ala Asp Lys Arg Ile Met
       50                  55                  60
   gag atg att cct gag aga aat gaa cag gga caa acc ctt tgg agc aaa     240
   Glu Met Ile Pro Glu Arg Asn Glu Gln Gly Gln Thr Leu Trp Ser Lys
   65                  70                  75                  80
   acg aac gat gct ggc tca gac cgc gta atg gta tca cct ctg gca gtg     288
   Thr Asn Asp Ala Gly Ser Asp Arg Val Met Val Ser Pro Leu Ala Val
                   85                  90                  95
   aca tgg tgg aat agg aat gga cca aca acg aac aca att cat tat cca     336
   Thr Trp Trp Asn Arg Asn Gly Pro Thr Thr Asn Thr Ile His Tyr Pro
               100                 105                 110
   aaa gtc tac aaa act tat ttt gaa aag gtt gaa aga ttg aaa cac gga     384
   Lys Val Tyr Lys Thr Tyr Phe Glu Lys Val Glu Arg Leu Lys His Gly
           115                 120                 125
   acc ttt ggc ccc gtt cat ttt agg aat caa gtc aag ata aga cga aga     432
   Thr Phe Gly Pro Val His Phe Arg Asn Gln Val Lys Ile Arg Arg Arg
       130                 135                 140
   gtt gat gta aac cct ggt cac gcg gac ctc agt gct aaa gaa gca caa     480
   Val Asp Val Asn Pro Gly His Ala Asp Leu Ser Ala Lys Glu Ala Gln
   145                 150                 155                 160
   gat gtg atc atg gaa gtt gtt ttc cca aat gaa gtg gga gcc aga att     528
   Asp Val Ile Met Glu Val Val Phe Pro Asn Glu Val Gly Ala Arg Ile
                   165                 170                 175
   cta aca tca gaa tca caa cta aca ata acc aaa gag aaa aag gaa gaa     576
   Leu Thr Ser Glu Ser Gln Leu Thr Ile Thr Lys Glu Lys Lys Glu Glu
               180                 185                 190
   ctt cag gac tgc aaa att gct ccc ttg atg gta gca tac atg cta gaa      624
   Leu Gln Asp Cys Lys Ile Ala Pro Leu Met Val Ala Tyr Met Leu Glu
           195                 200                 205
   aga gag ttg gtc cga aaa aca agg ttc ctc cca gta gta ggc gga aca      672
   Arg Glu Leu Val Arg Lys Thr Arg Phe Leu Pro Val Val Gly Gly Thr
       210                 215                 220
   agc agt gta tac att gaa gtg ttg cat ctg act cag gga aca tgc tgg      720
   Ser Ser Val Tyr Ile Glu Val Leu His Leu Thr Gln Gly Thr Cys Trp
   225                 230                 235                 240
   gag caa atg tac acc cca gga gga aaa gtt aga aac gat gat att gat      768
   Glu Gln Met Tyr Thr Pro Gly Gly Lys Val Arg Asn Asp Asp Ile Asp
                   245                 250                 255
   caa agt tta att att gca gcc cgg aac ata gtg aga aga gca aca gta      816
   Gln Ser Leu Ile Ile Ala Ala Arg Asn Ile Val Arg Arg Ala Thr Val
               260                 265                 270
   tca gca gat cca cta gca tcc cta ctg gaa atg tgc cac agt aca cag      864
   Ser Ala Asp Pro Leu Ala Ser Leu Leu Glu Met Cys His Ser Thr Gln
           275                 280                 285
   att ggt gga aca agg atg gta gac atc ctt aag cag aac cca aca gag      912
   Ile Gly Gly Thr Arg Met Val Asp Ile Leu Lys Gln Asn Pro Thr Glu
       290                 295                 300
   gaa caa gct gtg gat ata tgc aaa gca gca atg gga ttg aga att agc      960
   Glu Gln Ala Val Asp Ile Cys Lys Ala Ala Met Gly Leu Arg Ile Ser
   305                 310                 315                 320
   tca tca ttc agc ttt ggt gga ttc acc ttc aaa agg aca agt gga tca     1008
   Ser Ser Phe Ser Phe Gly Gly Phe Thr Phe Lys Arg Thr Ser Gly Ser
                   325                 330                 335
   tca gtc aag aga gaa gaa gaa atg ctt acg ggc aac ctt caa aca ttg     1056
   Ser Val Lys Arg Glu Glu Glu Met Leu Thr Gly Asn Leu Gln Thr Leu
               340                 345                 350
   aaa ata aga gtg cat gag ggc tat gaa gaa ttc aca atg gtc gga aga     1104
   Lys Ile Arg Val His Glu Gly Tyr Glu Glu Phe Thr Met Val Gly Arg
           355                 360                 365
   aga gca aca gcc att atc aga aag gca acc aga aga ttg att caa ttg     1152
   Arg Ala Thr Ala Ile Ile Arg Lys Ala Thr Arg Arg Leu Ile Gln Leu
       370                 375                 380
   ata gta agt ggg aga gat gaa caa tca att gct gaa gca ata att gta     1200
   Ile Val Ser Gly Arg Asp Glu Gln Ser Ile Ala Glu Ala Ile Ile Val
   385                 390                 395                 400
   gcc atg gtg ttt tcg caa gaa gat tgc atg ata aaa gca gtt cga ggc     1248
   Ala Met Val Phe Ser Gln Glu Asp Cys Met Ile Lys Ala Val Arg Gly
                   405                 410                 415
   gat ttg aac ttt gtt aat aga gca aat cag cgt ttg aac ccc atg cat     1296
   Asp Leu Asn Phe Val Asn Arg Ala Asn Gln Arg Leu Asn Pro Met His
               420                 425                 430
   caa ctc ttg agg cat ttc caa aaa gat gca aaa gtg ctt ttc caa aat     1344
   Gln Leu Leu Arg His Phe Gln Lys Asp Ala Lys Val Leu Phe Gln Asn
           435                 440                 445
   tgg gga att gaa ccc atc gac aat gta atg ggg atg att gga ata ttg     1392
   Trp Gly Ile Glu Pro Ile Asp Asn Val Met Gly Met Ile Gly Ile Leu
       450                 455                 460
   cct gac atg acc cca agc acc gag atg tca ttg aga gga gtg aga gtc     1440
   Pro Asp Met Thr Pro Ser Thr Glu Met Ser Leu Arg Gly Val Arg Val
   465 470 475 480
   agc aaa atg gga gtg gat gag tac tcc agc act gag aga gtg gtg gtg 1488
   Ser Lys Met Gly Val Asp Glu Tyr Ser Ser Thr Glu Arg Val Val Val
   485 490 495
   agc att gac cgt ttt tta aga gtt cgg gat caa agg gga aac ata cta 1536
   Ser Ile Asp Arg Phe Leu Arg Val Arg Asp Gln Arg Gly Asn Ile Leu
   500 505 510
   ctg tcc cct gaa gaa gtc agt gaa aca caa gga acg gaa aag ctg aca 1584
   Leu Ser Pro Glu Glu Val Ser Glu Thr Gln Gly Thr Glu Lys Leu Thr
   515 520 525
   ata att tat tcg tca tca atg atg tgg gag att aat ggt ccc gaa tca 1632
   Ile Ile Tyr Ser Ser Ser Met Met Trp Glu Ile Asn Gly Pro Glu Ser
   530 535 540
   gtg ttg gtc aat act tat caa tgg atc atc aga aac tgg gaa att gta 1680
   Val Leu Val Asn Thr Tyr Gln Trp Ile Ile Arg Asn Trp Glu Ile Val
   545 550 555 560
   aaa att cag tgg tca cag gac ccc aca atg tta tac aat aag ata gaa 1728
   Lys Ile Gln Trp Ser Gln Asp Pro Thr Met Leu Tyr Asn Lys Ile Glu
   565 570 575
   ttt gaa cca ttc caa tcc ctg gtc cct agg gcc acc aga agc caa tac 1776
   Phe Glu Pro Phe Gln Ser Leu Val Pro Arg Ala Thr Arg Ser Gln Tyr
   580 585 590
   agc ggt ttc gta aga acc ctg ttt cag caa atg cga gat gta ctt gga 1824
   Ser Gly Phe Val Arg Thr Leu Phe Gln Gln Met Arg Asp Val Leu Gly
   595 600 605
   aca ttt gat act gct caa ata ata aaa ctc ctc cct ttt gcc gct gct 1872
   Thr Phe Asp Thr Ala Gln Ile Ile Lys Leu Leu Pro Phe Ala Ala Ala
   610 615 620
   cct ccg gaa cag agt agg atg cag ttc tct tct ttg act gtt aat gta 1920
   Pro Pro Glu Gln Ser Arg Met Gln Phe Ser Ser Leu Thr Val Asn Val
   625 630 635 640
   aga ggt tcg gga atg agg ata ctt gta aga ggc aat tcc cca gtg ttc 1968
   Arg Gly Ser Gly Met Arg Ile Leu Val Arg Gly Asn Ser Pro Val Phe
   645 650 655
   aac tac aat aaa gtc act aaa agg ctc aca gtc ctc gga aag gat gca 2016
   Asn Tyr Asn Lys Val Thr Lys Arg Leu Thr Val Leu Gly Lys Asp Ala
   660 665 670
   ggt gcg ctt act gag gac cca gat gaa ggt acg gct gga gta gag tct 2064
   Gly Ala Leu Thr Glu Asp Pro Asp Glu Gly Thr Ala Gly Val Glu Ser
   675 680 685
   gct gtt cta aga ggg ttt ctc att tta ggt aaa gaa aac aag aga tat 2112
   Ala Val Leu Arg Gly Phe Leu Ile Leu Gly Lys Glu Asn Lys Arg Tyr
   690 695 700
   ggc cca gca cta agc atc aat gaa ctt agc aaa ctt gca aaa ggg gag 2160
   Gly Pro Ala Leu Ser Ile Asn Glu Leu Ser Lys Leu Ala Lys Gly Glu
   705 710 715 720
   aaa gcc aat gta cta att ggg caa ggg gac gta gtg ttg gta atg aaa 2208
   Lys Ala Asn Val Leu Ile Gly Gln Gly Asp Val Val Leu Val Met Lys
   725 730 735
   cgg aaa cgt gac tct agc ata ctt act gac agc cag aca gcg acc aaa 2256
   Arg Lys Arg Asp Ser Ser Ile Leu Thr Asp Ser Gln Thr Ala Thr Lys
                   740                 745                 750
   agg att cgg atg gcc atc aat tag                                          2280
   Arg Ile Arg Met Ala Ile Asn
           755
<210> 64
   <211> 759
   <212> PRT
   <213> Influenza virus
<400> 64
   Met Glu Arg Ile Lys Glu Leu Arg Asp Leu Met Leu Gln Ser Arg Thr
   1               5                   10                  15
   Arg Glu Ile Leu Thr Lys Thr Thr Val Asp His Met Ala Ile Ile Lys
               20                  25                  30
   Lys Tyr Thr Ser Gly Arg Gln Glu Lys Asn Pro Ala Leu Arg Met Lys
           35                  40                  45
   Trp Met Met Ala Met Lys Tyr Pro Ile Thr Ala Asp Lys Arg Ile Met
       50                  55                  60
   Glu Met Ile Pro Glu Arg Asn Glu Gln Gly Gln Thr Leu Trp Ser Lys
   65                  70                  75                  80
   Thr Asn Asp Ala Gly Ser Asp Arg Val Met Val Ser Pro Leu Ala Val
                   85                  90                  95
   Thr Trp Trp Asn Arg Asn Gly Pro Thr Thr Asn Thr Ile His Tyr Pro
               100                 105                 110
   Lys Val Tyr Lys Thr Tyr Phe Glu Lys Val Glu Arg Leu Lys His Gly
           115                 120                 125
   Thr Phe Gly Pro Val His Phe Arg Asn Gln Val Lys Ile Arg Arg Arg
       130                 135                 140
   Val Asp Val Asn Pro Gly His Ala Asp Leu Ser Ala Lys Glu Ala Gln
   145                 150                 155                 160
   Asp Val Ile Met Glu Val Val Phe Pro Asn Glu Val Gly Ala Arg Ile
                   165                 170                 175
   Leu Thr Ser Glu Ser Gln Leu Thr Ile Thr Lys Glu Lys Lys Glu Glu
               180                 185                 190
   Leu Gln Asp Cys Lys Ile Ala Pro Leu Met Val Ala Tyr Met Leu Glu
           195                 200                 205
   Arg Glu Leu Val Arg Lys Thr Arg Phe Leu Pro Val Val Gly Gly Thr
       210                 215                 220
   Ser Ser Val Tyr Ile Glu Val Leu His Leu Thr Gln Gly Thr Cys Trp
   225                 230                 235                 240
   Glu Gln Met Tyr Thr Pro Gly Gly Lys Val Arg Asn Asp Asp Ile Asp
                   245                 250                 255
   Gln Ser Leu Ile Ile Ala Ala Arg Asn Ile Val Arg Arg Ala Thr Val
               260                 265                 270
   Ser Ala Asp Pro Leu Ala Ser Leu Leu Glu Met Cys His Ser Thr Gln
           275                 280                 285
   Ile Gly Gly Thr Arg Met Val Asp Ile Leu Lys Gln Asn Pro Thr Glu
       290                 295                 300
   Glu Gln Ala Val Asp Ile Cys Lys Ala Ala Met Gly Leu Arg Ile Ser
   305                 310                 315                 320
   Ser Ser Phe Ser Phe Gly Gly Phe Thr Phe Lys Arg Thr Ser Gly Ser
                   325                 330                 335
   Ser Val Lys Arg Glu Glu Glu Met Leu Thr Gly Asn Leu Gln Thr Leu
               340                 345                 350
   Lys Ile Arg Val His Glu Gly Tyr Glu Glu Phe Thr Met Val Gly Arg
           355                 360                 365
   Arg Ala Thr Ala Ile Ile Arg Lys Ala Thr Arg Arg Leu Ile Gln Leu
       370                 375                 380
   Ile Val Ser Gly Arg Asp Glu Gln Ser Ile Ala Glu Ala Ile Ile Val
   385                 390                 395                 400
   Ala Met Val Phe Ser Gln Glu Asp Cys Met Ile Lys Ala Val Arg Gly
                   405                 410                 415
   Asp Leu Asn Phe Val Asn Arg Ala Asn Gln Arg Leu Asn Pro Met His
               420                 425                 430
   Gln Leu Leu Arg His Phe Gln Lys Asp Ala Lys Val Leu Phe Gln Asn
           435                 440                 445
   Trp Gly Ile Glu Pro Ile Asp Asn Val Met Gly Met Ile Gly Ile Leu
       450                 455                 460
   Pro Asp Met Thr Pro Ser Thr Glu Met Ser Leu Arg Gly Val Arg Val
   465                 470                 475                 480
   Ser Lys Met Gly Val Asp Glu Tyr Ser Ser Thr Glu Arg Val Val Val
                   485                 490                 495
   Ser Ile Asp Arg Phe Leu Arg Val Arg Asp Gln Arg Gly Asn Ile Leu
               500                 505                 510
   Leu Ser Pro Glu Glu Val Ser Glu Thr Gln Gly Thr Glu Lys Leu Thr
           515                 520                 525
   Ile Ile Tyr Ser Ser Ser Met Met Trp Glu Ile Asn Gly Pro Glu Ser
       530                 535                 540
   Val Leu Val Asn Thr Tyr Gln Trp Ile Ile Arg Asn Trp Glu Ile Val
   545                 550                 555                 560
   Lys Ile Gln Trp Ser Gln Asp Pro Thr Met Leu Tyr Asn Lys Ile Glu
                   565                 570                 575
   Phe Glu Pro Phe Gln Ser Leu Val Pro Arg Ala Thr Arg Ser Gln Tyr
               580                 585                 590
   Ser Gly Phe Val Arg Thr Leu Phe Gln Gln Met Arg Asp Val Leu Gly
           595                 600                 605
   Thr Phe Asp Thr Ala Gln Ile Ile Lys Leu Leu Pro Phe Ala Ala Ala
       610                 615                 620
   Pro Pro Glu Gln Ser Arg Met Gln Phe Ser Ser Leu Thr Val Asn Val
   625                 630                 635                 640
   Arg Gly Ser Gly Met Arg Ile Leu Val Arg Gly Asn Ser Pro Val Phe
                   645                 650                 655
   Asn Tyr Asn Lys Val Thr Lys Arg Leu Thr Val Leu Gly Lys Asp Ala
               660                 665                 670
   Gly Ala Leu Thr Glu Asp Pro Asp Glu Gly Thr Ala Gly Val Glu Ser
           675                 680                 685
   Ala Val Leu Arg Gly Phe Leu Ile Leu Gly Lys Glu Asn Lys Arg Tyr
       690                 695                 700
   Gly Pro Ala Leu Ser Ile Asn Glu Leu Ser Lys Leu Ala Lys Gly Glu
   705                 710                 715                 720
   Lys Ala Asn Val Leu Ile Gly Gln Gly Asp Val Val Leu Val Met Lys
                   725                 730                 735
   Arg Lys Arg Asp Ser Ser Ile Leu Thr Asp Ser Gln Thr Ala Thr Lys
               740                 745                 750
   Arg Ile Arg Met Ala Ile Asn
           755
<210> 65
   <211> 2274
   <212> DNA
   <213> Influenza virus
<220>
   <221> CDS
   <222> (1)..(2274)
<400> 65
   atg gat gtc aat ccg act cta ctt ttc tta aag gtg cca gcg caa aat       48
   Met Asp Val Asn Pro Thr Leu Leu Phe Leu Lys Val Pro Ala Gln Asn
   1               5                   10                  15
   gct ata agc aca aca ttc cct tat act gga gat cct ccc tac agt cat       96
   Ala Ile Ser Thr Thr Phe Pro Tyr Thr Gly Asp Pro Pro Tyr Ser His
               20                  25                  30
   gga aca ggg aca gga tac acc atg gat act gtc aac aga aca cac caa      144
   Gly Thr Gly Thr Gly Tyr Thr Met Asp Thr Val Asn Arg Thr His Gln
           35                  40                  45
   tat tca gaa aaa ggg aaa tgg aca aca aac act gag att gga gca cca      192
   Tyr Ser Glu Lys Gly Lys Trp Thr Thr Asn Thr Glu Ile Gly Ala Pro
       50                  55                  60
   caa ctt aat cca atc gat gga cca ctt cct gaa gac aat gaa cca agt      240
   Gln Leu Asn Pro Ile Asp Gly Pro Leu Pro Glu Asp Asn Glu Pro Ser
   65                  70                  75                  80
   ggg tac gcc caa aca gat tgt gta ttg gaa gca atg gct ttc ctt gaa      288
   Gly Tyr Ala Gln Thr Asp Cys Val Leu Glu Ala Met Ala Phe Leu Glu
                   85                  90                  95
   gaa tcc cat ccc gga atc ttt gaa aat tcg tgt ctt gaa acg atg gag      336
   Glu Ser His Pro Gly Ile Phe Glu Asn Ser Cys Leu Glu Thr Met Glu
               100                 105                 110
   gtg att cag cag aca aga gtg gac aaa cta aca caa ggc cga caa act      384
   Val Ile Gln Gln Thr Arg Val Asp Lys Leu Thr Gln Gly Arg Gln Thr
           115                 120                 125
   tat gat tgg acc ttg aat agg aat caa cct gcc gca aca gca ctt gct      432
   Tyr Asp Trp Thr Leu Asn Arg Asn Gln Pro Ala Ala Thr Ala Leu Ala
       130                 135                 140
   aat acg att gaa gta ttc aga tca aat ggt ctg acc tcc aat gaa tcg      480
   Asn Thr Ile Glu Val Phe Arg Ser Asn Gly Leu Thr Ser Asn Glu Ser
   145                 150                 155                 160
   ggg aga ttg atg gac ttc ctc aaa gat gtc atg gag tcc atg aac aag      528
   Gly Arg Leu Met Asp Phe Leu Lys Asp Val Met Glu Ser Met Asn Lys
                   165                 170                 175
   gag gaa atg gaa ata aca aca cac ttc caa cgg aag aga aga gta aga      576
   Glu Glu Met Glu Ile Thr Thr His Phe Gln Arg Lys Arg Arg Val Arg
               180                 185                 190
   gac aac atg aca aag aga atg ata aca cag aga acc ata gga aag aaa      624
   Asp Asn Met Thr Lys Arg Met Ile Thr Gln Arg Thr Ile Gly Lys Lys
           195                 200                 205
   aaa caa cga tta agc aga aag agc tat cta atc aga aca tta acc cta      672
   Lys Gln Arg Leu Ser Arg Lys Ser Tyr Leu Ile Arg Thr Leu Thr Leu
       210                 215                 220
   aac aca atg acc aag gac gct gag aga ggg aaa ttg aaa cga cga gca      720
   Asn Thr Met Thr Lys Asp Ala Glu Arg Gly Lys Leu Lys Arg Arg Ala
   225                 230                 235                 240
   atc gct acc cca ggg atg cag ata aga gga ttt gta tat ttt gtt gaa       768
   Ile Ala Thr Pro Gly Met Gln Ile Arg Gly Phe Val Tyr Phe Val Glu
                   245                 250                 255
   aca cta gct cga aga ata tgt gaa aag ctt gaa caa tca gga ttg cca       816
   Thr Leu Ala Arg Arg Ile Cys Glu Lys Leu Glu Gln Ser Gly Leu Pro
               260                 265                 270
   gtt ggc ggt aat gag aaa aag gcc aaa ctg gct aat gtc gtc aga aaa       864
   Val Gly Gly Asn Glu Lys Lys Ala Lys Leu Ala Asn Val Val Arg Lys
           275                 280                 285
   atg atg act aat tcc caa gac act gaa ctc tcc ttc acc atc act ggg       912
   Met Met Thr Asn Ser Gln Asp Thr Glu Leu Ser Phe Thr Ile Thr Gly
       290                 295                 300
   gac aat acc aaa tgg aat gaa aat cag aac cca cgc ata ttc ctg gca       960
   Asp Asn Thr Lys Trp Asn Glu Asn Gln Asn Pro Arg Ile Phe Leu Ala
   305                 310                 315                 320
   atg atc aca tac ata act aga gat cag cca gaa tgg ttc aga aat gtt      1008
   Met Ile Thr Tyr Ile Thr Arg Asp Gln Pro Glu Trp Phe Arg Asn Val
                   325                 330                 335
   cta agc att gca ccg att atg ttc tca aat aaa atg gca aga ctg ggg      1056
   Leu Ser Ile Ala Pro Ile Met Phe Ser Asn Lys Met Ala Arg Leu Gly
               340                 345                 350
   aaa gga tat atg ttt gaa agc aaa agt atg aaa ttg aga act caa ata      1104
   Lys Gly Tyr Met Phe Glu Ser Lys Ser Met Lys Leu Arg Thr Gln Ile
           355                 360                 365
   cca gca gaa atg cta gca agc att gac cta aaa tat ttc aat gat tca      1152
   Pro Ala Glu Met Leu Ala Ser Ile Asp Leu Lys Tyr Phe Asn Asp Ser
       370                 375                 380
   aca aaa aag aaa att gaa aag ata cga cca ctc ctg gtt gac ggg act      1200
   Thr Lys Lys Lys Ile Glu Lys Ile Arg Pro Leu Leu Val Asp Gly Thr
   385                 390                 395                 400
   gct tca ctg agt cct ggc atg atg atg gga atg ttc aac atg ttg agc      1248
   Ala Ser Leu Ser Pro Gly Met Met Met Gly Met Phe Asn Met Leu Ser
                   405                 410                 415
   act gtg ctg ggt gta tcc ata tta aac ctg ggc cag agg aaa tat aca      1296
   Thr Val Leu Gly Val Ser Ile Leu Asn Leu Gly Gln Arg Lys Tyr Thr
               420                 425                 430
   aag acc aca tac tgg tgg gat ggt ctg caa tca tcc gat gac ttt gct      1344
   Lys Thr Thr Tyr Trp Trp Asp Gly Leu Gln Ser Ser Asp Asp Phe Ala
           435                 440                 445
   ttg ata gtg aat gcg cct aat cat gaa gga ata caa gct gga gta gac      1392
   Leu Ile Val Asn Ala Pro Asn His Glu Gly Ile Gln Ala Gly Val Asp
       450                 455                 460
   aga ttc tat aga act tgc aaa ctg gtc ggg atc aac atg agc aaa aag      1440
   Arg Phe Tyr Arg Thr Cys Lys Leu Val Gly Ile Asn Met Ser Lys Lys
   465                 470                 475                 480
   aag tcc tac ata aat aga act gga aca ttc gaa ttc aca agc ttt ttc      1488
   Lys Ser Tyr Ile Asn Arg Thr Gly Thr Phe Glu Phe Thr Ser Phe Phe
                   485                 490                 495
   tac cgg tat ggt ttt gta gcc aat ttc agc atg gaa cta ccc agt ttt      1536
   Tyr Arg Tyr Gly Phe Val Ala Asn Phe Ser Met Glu Leu Pro Ser Phe
               500                 505                 510
   ggg gtt tcc gga ata aat gaa tct gca gac atg agc att gga gtg aca      1584
   Gly Val Ser Gly Ile Asn Glu Ser Ala Asp Met Ser Ile Gly Val Thr
           515                 520                 525
   gtc atc aaa aac aac atg ata aat aat gat ctc ggt cct gcc acg gca      1632
   Val Ile Lys Asn Asn Met Ile Asn Asn Asp Leu Gly Pro Ala Thr Ala
       530                 535                 540
   caa atg gca ctc caa ctc ttc att aag gat tat cgg tac aca tac cgg      1680
   Gln Met Ala Leu Gln Leu Phe Ile Lys Asp Tyr Arg Tyr Thr Tyr Arg
   545                 550                 555                 560
   tgc cat aga ggt gat acc cag ata caa acc aga aga tct ttt gag ttg      1728
   Cys His Arg Gly Asp Thr Gln Ile Gln Thr Arg Arg Ser Phe Glu Leu
                   565                 570                 575
   aag aaa ctg tgg gaa cag act cga tca aag act ggt cta ctg gta tca      1776
   Lys Lys Leu Trp Glu Gln Thr Arg Ser Lys Thr Gly Leu Leu Val Ser
               580                 585                 590
   gat ggg ggt cca aac cta tat aac atc aga aac cta cac atc ccg gaa      1824
   Asp Gly Gly Pro Asn Leu Tyr Asn Ile Arg Asn Leu His Ile Pro Glu
           595                 600                 605
   gtc tgt tta aaa tgg gag cta atg gat gaa gat tat aag ggg agg cta      1872
   Val Cys Leu Lys Trp Glu Leu Met Asp Glu Asp Tyr Lys Gly Arg Leu
       610                 615                 620
   tgc aat cca ttg aat cct ttc gtt agt cac aaa gaa att gaa tca gtc      1920
   Cys Asn Pro Leu Asn Pro Phe Val Ser His Lys Glu Ile Glu Ser Val
   625                 630                 635                 640
   aac agt gca gta gta atg cct gct cat ggc cct gcc aaa agc atg gag      1968
   Asn Ser Ala Val Val Met Pro Ala His Gly Pro Ala Lys Ser Met Glu
                   645                 650                 655
   tat gat gct gtt gca aca aca cat tct tgg atc ccc aag agg aac cgg      2016
   Tyr Asp Ala Val Ala Thr Thr His Ser Trp Ile Pro Lys Arg Asn Arg
               660                 665                 670
   tcc ata ttg aac aca agc caa agg gga ata cta gaa gat gag cag atg      2064
   Ser Ile Leu Asn Thr Ser Gln Arg Gly Ile Leu Glu Asp Glu Gln Met
           675                 680                 685
   tat cag aaa tgc tgc aac ctg ttt gaa aaa ttc ttc ccc agc agc tca      2112
   Tyr Gln Lys Cys Cys Asn Leu Phe Glu Lys Phe Phe Pro Ser Ser Ser
       690                 695                 700
   tac aga aga cca gtc gga att tct agt atg gtt gag gcc atg gta tcc      2160
   Tyr Arg Arg Pro Val Gly Ile Ser Ser Met Val Glu Ala Met Val Ser
   705                 710                 715                 720
   agg gcc cgc att gat gca cga att gac ttc gaa tct gga cgg ata aag      2208
   Arg Ala Arg Ile Asp Ala Arg Ile Asp Phe Glu Ser Gly Arg Ile Lys
                   725                 730                 735
   aag gat gag ttc gct gag atc atg aag atc tgt tcc acc att gaa gag      2256
   Lys Asp Glu Phe Ala Glu Ile Met Lys Ile Cys Ser Thr Ile Glu Glu
               740                 745                 750
   ctc aga cgg caa aaa tag                                              2274
   Leu Arg Arg Gln Lys
           755
<210> 66
   <211> 757
   <212> PRT
   <213> Influenza virus
<400> 66
   Met Asp Val Asn Pro Thr Leu Leu Phe Leu Lys Val Pro Ala Gln Asn
   1               5                   10                  15
   Ala Ile Ser Thr Thr Phe Pro Tyr Thr Gly Asp Pro Pro Tyr Ser His
               20                  25                  30
   Gly Thr Gly Thr Gly Tyr Thr Met Asp Thr Val Asn Arg Thr His Gln
           35                  40                  45
   Tyr Ser Glu Lys Gly Lys Trp Thr Thr Asn Thr Glu Ile Gly Ala Pro
       50                  55                  60
   Gln Leu Asn Pro Ile Asp Gly Pro Leu Pro Glu Asp Asn Glu Pro Ser
   65                  70                  75                  80
   Gly Tyr Ala Gln Thr Asp Cys Val Leu Glu Ala Met Ala Phe Leu Glu
                   85                  90                  95
   Glu Ser His Pro Gly Ile Phe Glu Asn Ser Cys Leu Glu Thr Met Glu
               100                 105                 110
   Val Ile Gln Gln Thr Arg Val Asp Lys Leu Thr Gln Gly Arg Gln Thr
           115                 120                 125
   Tyr Asp Trp Thr Leu Asn Arg Asn Gln Pro Ala Ala Thr Ala Leu Ala
       130                 135                 140
   Asn Thr Ile Glu Val Phe Arg Ser Asn Gly Leu Thr Ser Asn Glu Ser
   145                 150                 155                 160
   Gly Arg Leu Met Asp Phe Leu Lys Asp Val Met Glu Ser Met Asn Lys
                   165                 170                 175
   Glu Glu Met Glu Ile Thr Thr His Phe Gln Arg Lys Arg Arg Val Arg
               180                 185                 190
   Asp Asn Met Thr Lys Arg Met Ile Thr Gln Arg Thr Ile Gly Lys Lys
           195                 200                 205
   Lys Gln Arg Leu Ser Arg Lys Ser Tyr Leu Ile Arg Thr Leu Thr Leu
       210                 215                 220
   Asn Thr Met Thr Lys Asp Ala Glu Arg Gly Lys Leu Lys Arg Arg Ala
   225                 230                 235                 240
   Ile Ala Thr Pro Gly Met Gln Ile Arg Gly Phe Val Tyr Phe Val Glu
                   245                 250                 255
   Thr Leu Ala Arg Arg Ile Cys Glu Lys Leu Glu Gln Ser Gly Leu Pro
               260                 265                 270
   Val Gly Gly Asn Glu Lys Lys Ala Lys Leu Ala Asn Val Val Arg Lys
           275                 280                 285
   Met Met Thr Asn Ser Gln Asp Thr Glu Leu Ser Phe Thr Ile Thr Gly
       290                 295                 300
   Asp Asn Thr Lys Trp Asn Glu Asn Gln Asn Pro Arg Ile Phe Leu Ala
   305                 310                 315                 320
   Met Ile Thr Tyr Ile Thr Arg Asp Gln Pro Glu Trp Phe Arg Asn Val
                   325                 330                 335
   Leu Ser Ile Ala Pro Ile Met Phe Ser Asn Lys Met Ala Arg Leu Gly
               340                 345                 350
   Lys Gly Tyr Met Phe Glu Ser Lys Ser Met Lys Leu Arg Thr Gln Ile
           355                 360                 365
   Pro Ala Glu Met Leu Ala Ser Ile Asp Leu Lys Tyr Phe Asn Asp Ser
       370                 375                 380
   Thr Lys Lys Lys Ile Glu Lys Ile Arg Pro Leu Leu Val Asp Gly Thr
   385                 390                 395                 400
   Ala Ser Leu Ser Pro Gly Met Met Met Gly Met Phe Asn Met Leu Ser
                   405                 410                 415
   Thr Val Leu Gly Val Ser Ile Leu Asn Leu Gly Gln Arg Lys Tyr Thr
               420                 425                 430
   Lys Thr Thr Tyr Trp Trp Asp Gly Leu Gln Ser Ser Asp Asp Phe Ala
           435                 440                 445
   Leu Ile Val Asn Ala Pro Asn His Glu Gly Ile Gln Ala Gly Val Asp
       450                 455                 460
   Arg Phe Tyr Arg Thr Cys Lys Leu Val Gly Ile Asn Met Ser Lys Lys
   465                 470                 475                 480
   Lys Ser Tyr Ile Asn Arg Thr Gly Thr Phe Glu Phe Thr Ser Phe Phe
                   485                 490                 495
   Tyr Arg Tyr Gly Phe Val Ala Asn Phe Ser Met Glu Leu Pro Ser Phe
               500                 505                 510
   Gly Val Ser Gly Ile Asn Glu Ser Ala Asp Met Ser Ile Gly Val Thr
           515                 520                 525
   Val Ile Lys Asn Asn Met Ile Asn Asn Asp Leu Gly Pro Ala Thr Ala
       530                 535                 540
   Gln Met Ala Leu Gln Leu Phe Ile Lys Asp Tyr Arg Tyr Thr Tyr Arg
   545                 550                 555                 560
   Cys His Arg Gly Asp Thr Gln Ile Gln Thr Arg Arg Ser Phe Glu Leu
                   565                 570                 575
   Lys Lys Leu Trp Glu Gln Thr Arg Ser Lys Thr Gly Leu Leu Val Ser
               580                 585                 590
   Asp Gly Gly Pro Asn Leu Tyr Asn Ile Arg Asn Leu His Ile Pro Glu
           595                 600                 605
   Val Cys Leu Lys Trp Glu Leu Met Asp Glu Asp Tyr Lys Gly Arg Leu
       610                 615                 620
   Cys Asn Pro Leu Asn Pro Phe Val Ser His Lys Glu Ile Glu Ser Val
   625                 630                 635                 640
   Asn Ser Ala Val Val Met Pro Ala His Gly Pro Ala Lys Ser Met Glu
                   645                 650                 655
   Tyr Asp Ala Val Ala Thr Thr His Ser Trp Ile Pro Lys Arg Asn Arg
               660                 665                 670
   Ser Ile Leu Asn Thr Ser Gln Arg Gly Ile Leu Glu Asp Glu Gln Met
           675                 680                 685
   Tyr Gln Lys Cys Cys Asn Leu Phe Glu Lys Phe Phe Pro Ser Ser Ser
       690                 695                 700
   Tyr Arg Arg Pro Val Gly Ile Ser Ser Met Val Glu Ala Met Val Ser
   705                 710                 715                 720
   Arg Ala Arg Ile Asp Ala Arg Ile Asp Phe Glu Ser Gly Arg Ile Lys
                   725                 730                 735
   Lys Asp Glu Phe Ala Glu Ile Met Lys Ile Cys Ser Thr Ile Glu Glu
               740                 745                 750
   Leu Arg Arg Gln Lys
           755
<210> 67
   <211> 2151
   <212> DNA
   <213> Influenza virus
<220>
   <221> CDS
   <222> (1)..(2151)
<400> 67
   atg gaa gac ttt gtg cga cag tgc ttc aat cca atg atc gtc gag ctt 48
   Met Glu Asp Phe Val Arg Gln Cys Phe Asn Pro Met Ile Val Glu Leu
   1               5                   10                  15
   gcg gaa aag gca atg aaa gaa tat gga gag aac ccg aaa atc gaa aca      96
   Ala Glu Lys Ala Met Lys Glu Tyr Gly Glu Asn Pro Lys Ile Glu Thr
               20                  25                  30
   aac aaa ttt gca gca ata tgc act cac ttg gaa gtc tgc ttc atg tac     144
   Asn Lys Phe Ala Ala Ile Cys Thr His Leu Glu Val Cys Phe Met Tyr
           35                  40                  45
   tcg gat ttc cac ttt ata aat gaa ctg ggt gag tca gtg gtc ata gag     192
   Ser Asp Phe His Phe Ile Asn Glu Leu Gly Glu Ser Val Val Ile Glu
       50                  55                  60
   tct ggt gac cca aat gct ctt ttg aaa cac aga ttt gaa atc att gag     240
   Ser Gly Asp Pro Asn Ala Leu Leu Lys His Arg Phe Glu Ile Ile Glu
   65                  70                  75                  80
   ggg aga gat cga aca atg gca tgg aca gta gta aac agc atc tgc aac     288
   Gly Arg Asp Arg Thr Met Ala Trp Thr Val Val Asn Ser Ile Cys Asn
                   85                  90                  95
   acc aca aga gct gaa aaa cct aaa ttt ctt cca gat tta tac gac tat     336
   Thr Thr Arg Ala Glu Lys Pro Lys Phe Leu Pro Asp Leu Tyr Asp Tyr
               100                 105                 110
   aaa gag aac aga ttt gtt gaa att ggt gtg aca agg aga gaa gtt cac     384
   Lys Glu Asn Arg Phe Val Glu Ile Gly Val Thr Arg Arg Glu Val His
           115                 120                 125
   ata tac tac ctg gag aag gcc aac aaa ata aag tct gag aaa aca cat     432
   Ile Tyr Tyr Leu Glu Lys Ala Asn Lys Ile Lys Ser Glu Lys Thr His
       130                 135                 140
   atc cac att ttc tca ttt aca gga gaa gaa atg gct aca aaa gcg gac     480
   Ile His Ile Phe Ser Phe Thr Gly Glu Glu Met Ala Thr Lys Ala Asp
   145                 150                 155                 160
   tat act ctt gat gaa gag agt aga gcc agg atc aag acc aga cta ttc     528
   Tyr Thr Leu Asp Glu Glu Ser Arg Ala Arg Ile Lys Thr Arg Leu Phe
                   165                 170                 175
   act ata aga caa gaa atg gcc agt aga ggc ctc tgg gat tcc ttt cgt     576
   Thr Ile Arg Gln Glu Met Ala Ser Arg Gly Leu Trp Asp Ser Phe Arg
               180                 185                 190
   cag tcc gag aga ggc gaa gag aca att gaa gaa aga ttt gaa atc aca     624
   Gln Ser Glu Arg Gly Glu Glu Thr Ile Glu Glu Arg Phe Glu Ile Thr
           195                 200                 205
   ggg acg atg cgc aag ctt gcc aat tac agt ctc cca ccg aac ttc tcc     672
   Gly Thr Met Arg Lys Leu Ala Asn Tyr Ser Leu Pro Pro Asn Phe Ser
       210                 215                 220
   agc ctt gaa aat ttt aga gtc tat ata gat gga ttc gaa ccg aac ggc     720
   Ser Leu Glu Asn Phe Arg Val Tyr Ile Asp Gly Phe Glu Pro Asn Gly
   225                 230                 235                 240
   tgc att gag agt aag ctt tct caa atg tcc aaa gaa gta aat gcc aaa     768
   Cys Ile Glu Ser Lys Leu Ser Gln Met Ser Lys Glu Val Asn Ala Lys
                   245                 250                 255
   ata gaa cca ttt tca aag aca aca ccc cga cca ctc aaa atg cca ggt     816
   Ile Glu Pro Phe Ser Lys Thr Thr Pro Arg Pro Leu Lys Met Pro Gly
               260                 265                 270
   ggt cca ccc tgc cat cag cga tcc aaa ttc ttg cta atg gat gct ctg     864
   Gly Pro Pro Cys His Gln Arg Ser Lys Phe Leu Leu Met Asp Ala Leu
                   275                     280                     285
   aaa ctg agc att gag gac cca agt cac gag gga gag ggg ata cca cta      912
   Lys Leu Ser Ile Glu Asp Pro Ser His Glu Gly Glu Gly Ile Pro Leu
       290                     295                     300
   tat gat gca atc aaa tgc atg aaa act ttc ttt gga tgg aaa gag ccc      960
   Tyr Asp Ala Ile Lys Cys Met Lys Thr Phe Phe Gly Trp Lys Glu Pro
   305                     310                     315                     320
   agt att gtt aaa cca cat aaa aag ggt ata aac ccg aac tat ctc caa     1008
   Ser Ile Val Lys Pro His Lys Lys Gly Ile Asn Pro Asn Tyr Leu Gln
                   325                     330                     335
   act tgg aag caa gta tta gaa gaa ata caa gac ctt gag aac gaa gaa     1056
   Thr Trp Lys Gln Val Leu Glu Glu Ile Gln Asp Leu Glu Asn Glu Glu
               340                     345                     350
   agg acc ccc aag acc aag aat atg aaa aaa aca agc caa ttg aaa tgg     1104
   Arg Thr Pro Lys Thr Lys Asn Met Lys Lys Thr Ser Gln Leu Lys Trp
           355                     360                     365
   gca cta ggt gaa aat atg gca cca gag aaa gtg gat ttt gag gat tgt     1152
   Ala Leu Gly Glu Asn Met Ala Pro Glu Lys Val Asp Phe Glu Asp Cys
       370                     375                     380
   aaa gac atc aat gat tta aaa caa tat gac agt gat gag cca gaa gca     1200
   Lys Asp Ile Asn Asp Leu Lys Gln Tyr Asp Ser Asp Glu Pro Glu Ala
   385                     390                     395                     400
   agg tct ctt gca agt tgg att caa agt gag ttc aac aag gct tgt gag     1248
   Arg Ser Leu Ala Ser Trp Ile Gln Ser Glu Phe Asn Lys Ala Cys Glu
                   405                     410                     415
   ctg aca gat tca agc tgg ata gag ctc gat gaa att ggg gag gat gtc     1296
   Leu Thr Asp Ser Ser Trp Ile Glu Leu Asp Glu Ile Gly Glu Asp Val
               420                     425                     430
   gcc cca ata gaa tac att gcg agc atg agg aga aat tat ttt act gct     1344
   Ala Pro Ile Glu Tyr Ile Ala Ser Met Arg Arg Asn Tyr Phe Thr Ala
           435                     440                     445
   gag att tcc cat tgt aga gca aca gaa tat ata atg aaa gga gtg tac     1392
   Glu Ile Ser His Cys Arg Ala Thr Glu Tyr Ile Met Lys Gly Val Tyr
       450                     455                     460
   atc aac act gct cta ctc aat gca tcc tgt gct gcg atg gat gaa ttt     1440
   Ile Asn Thr Ala Leu Leu Asn Ala Ser Cys Ala Ala Met Asp Glu Phe
   465                     470                     475                     480
   caa tta att ccg atg ata agt aaa tgc agg acc aaa gaa ggg aga agg     1488
   Gln Leu Ile Pro Met Ile Ser Lys Cys Arg Thr Lys Glu Gly Arg Arg
                   485                     490                     495
   aaa aca aat tta tat gga ttc ata ata aag gga agg tcc cat tta aga     1536
   Lys Thr Asn Leu Tyr Gly Phe Ile Ile Lys Gly Arg Ser His Leu Arg
               500                     505                     510
   aat gat act gac gtg gtg aac ttt gta agt atg gaa ttt tct ctc act     1584
   Asn Asp Thr Asp Val Val Asn Phe Val Ser Met Glu Phe Ser Leu Thr
           515                     520                     525
   gat cca aga ttt gag cca cac aaa tgg gaa aaa tac tgc gtt cta gaa     1632
   Asp Pro Arg Phe Glu Pro His Lys Trp Glu Lys Tyr Cys Val Leu Glu
       530                     535                     540
   att gga gac atg ctt tta aga act gct gta ggt caa gtg tca aga ccc     1680
   Ile Gly Asp Met Leu Leu Arg Thr Ala Val Gly Gln Val Ser Arg Pro
   545                     550                     555                     560
   atg ttt ttg tat gta agg aca aat gga acc tct aaa att aaa atg aaa      1728
   Met Phe Leu Tyr Val Arg Thr Asn Gly Thr Ser Lys Ile Lys Met Lys
                   565                 570                 575
   tgg gga atg gaa atg agg cgc tgc ctc ctt cag tct ctg caa cag att      1776
   Trp Gly Met Glu Met Arg Arg Cys Leu Leu Gln Ser Leu Gln Gln Ile
               580                 585                 590
   gaa agc atg atc gaa gct gag tcc tca gtc aaa gaa aag gac atg acc      1824
   Glu Ser Met Ile Glu Ala Glu Ser Ser Val Lys Glu Lys Asp Met Thr
           595                 600                 605
   aaa gaa ttt ttt gag aac aaa tca gag aca tgg cct ata gga gag tcc      1872
   Lys Glu Phe Phe Glu Asn Lys Ser Glu Thr Trp Pro Ile Gly Glu Ser
       610                 615                 620
   ccc aaa gga gtg gaa gag ggc tca atc ggg aag gtt tgc agg acc tta      1920
   Pro Lys Gly Val Glu Glu Gly Ser Ile Gly Lys Val Cys Arg Thr Leu
   625                 630                 635                 640
   tta gca aaa tct gtg ttt aac agt tta tat gca tct cca caa ctg gaa      1968
   Leu Ala Lys Ser Val Phe Asn Ser Leu Tyr Ala Ser Pro Gln Leu Glu
                   645                 650                 655
   gga ttt tca gct gaa tct agg aaa tta ctt ctc att gtt cag gct ctt      2016
   Gly Phe Ser Ala Glu Ser Arg Lys Leu Leu Leu Ile Val Gln Ala Leu
               660                 665                 670
   aga gat gac ctg gaa cct gga acc ttt gat att ggg ggg tta tat gaa      2064
   Arg Asp Asp Leu Glu Pro Gly Thr Phe Asp Ile Gly Gly Leu Tyr Glu
           675                 680                 685
   tca att gag gag tgc ctg att aat gat ccc tgg gtt ttg ctt aat gca      2112
   Ser Ile Glu Glu Cys Leu Ile Asn Asp Pro Trp Val Leu Leu Asn Ala
       690                 695                 700
   tct tgg ttc aac tcc ttc ctc aca cat gca ctg aag tag                  2151
   Ser Trp Phe Asn Ser Phe Leu Thr His Ala Leu Lys
   705                 710                 715
<210> 68
   <211> 716
   <212> PRT
   <213> Influenza virus
<400> 68
   Met Glu Asp Phe Val Arg Gln Cys Phe Asn Pro Met Ile Val Glu Leu
   1               5                   10                  15
   Ala Glu Lys Ala Met Lys Glu Tyr Gly Glu Asn Pro Lys Ile Glu Thr
               20                  25                  30
   Asn Lys Phe Ala Ala Ile Cys Thr His Leu Glu Val Cys Phe Met Tyr
           35                  40                  45
   Ser Asp Phe His Phe Ile Asn Glu Leu Gly Glu Ser Val Val Ile Glu
       50                  55                  60
   Ser Gly Asp Pro Asn Ala Leu Leu Lys His Arg Phe Glu Ile Ile Glu
   65                  70                  75                  80
   Gly Arg Asp Arg Thr Met Ala Trp Thr Val Val Asn Ser Ile Cys Asn
                   85                  90                  95
   Thr Thr Arg Ala Glu Lys Pro Lys Phe Leu Pro Asp Leu Tyr Asp Tyr
               100                 105                 110
   Lys Glu Asn Arg Phe Val Glu Ile Gly Val Thr Arg Arg Glu Val His
           115                 120                 125
   Ile Tyr Tyr Leu Glu Lys Ala Asn Lys Ile Lys Ser Glu Lys Thr His
       130                 135                 140
   Ile His Ile Phe Ser Phe Thr Gly Glu Glu Met Ala Thr Lys Ala Asp
   145                 150                 155                 160
   Tyr Thr Leu Asp Glu Glu Ser Arg Ala Arg Ile Lys Thr Arg Leu Phe
                   165                 170                 175
   Thr Ile Arg Gln Glu Met Ala Ser Arg Gly Leu Trp Asp Ser Phe Arg
               180                 185                 190
   Gln Ser Glu Arg Gly Glu Glu Thr Ile Glu Glu Arg Phe Glu Ile Thr
           195                 200                 205
   Gly Thr Met Arg Lys Leu Ala Asn Tyr Ser Leu Pro Pro Asn Phe Ser
       210                 215                 220
   Ser Leu Glu Asn Phe Arg Val Tyr Ile Asp Gly Phe Glu Pro Asn Gly
   225                 230                 235                 240
   Cys Ile Glu Ser Lys Leu Ser Gln Met Ser Lys Glu Val Asn Ala Lys
                   245                 250                 255
   Ile Glu Pro Phe Ser Lys Thr Thr Pro Arg Pro Leu Lys Met Pro Gly
               260                 265                 270
   Gly Pro Pro Cys His Gln Arg Ser Lys Phe Leu Leu Met Asp Ala Leu
           275                 280                 285
   Lys Leu Ser Ile Glu Asp Pro Ser His Glu Gly Glu Gly Ile Pro Leu
       290                 295                 300
   Tyr Asp Ala Ile Lys Cys Met Lys Thr Phe Phe Gly Trp Lys Glu Pro
   305                 310                 315                 320
   Ser Ile Val Lys Pro His Lys Lys Gly Ile Asn Pro Asn Tyr Leu Gln
                   325                 330                 335
   Thr Trp Lys Gln Val Leu Glu Glu Ile Gln Asp Leu Glu Asn Glu Glu
               340                 345                 350
   Arg Thr Pro Lys Thr Lys Asn Met Lys Lys Thr Ser Gln Leu Lys Trp
   355 360 365
   Ala Leu Gly Glu Asn Met Ala Pro Glu Lys Val Asp Phe Glu Asp Cys
   370 375 380
   Lys Asp Ile Asn Asp Leu Lys Gln Tyr Asp Ser Asp Glu Pro Glu Ala
   385 390 395 400
   Arg Ser Leu Ala Ser Trp Ile Gln Ser Glu Phe Asn Lys Ala Cys Glu
   405 410 415
   Leu Thr Asp Ser Ser Trp Ile Glu Leu Asp Glu Ile Gly Glu Asp Val
   420 425 430
   Ala Pro Ile Glu Tyr Ile Ala Ser Met Arg Arg Asn Tyr Phe Thr Ala
   435 440 445
   Glu Ile Ser His Cys Arg Ala Thr Glu Tyr Ile Met Lys Gly Val Tyr
   450 455 460
   Ile Asn Thr Ala Leu Leu Asn Ala Ser Cys Ala Ala Met Asp Glu Phe
   465 470 475 480
   Gln Leu Ile Pro Met Ile Ser Lys Cys Arg Thr Lys Glu Gly Arg Arg
   485 490 495
   Lys Thr Asn Leu Tyr Gly Phe Ile Ile Lys Gly Arg Ser His Leu Arg
   500 505 510
   Asn Asp Thr Asp Val Val Asn Phe Val Ser Met Glu Phe Ser Leu Thr
   515 520 525
   Asp Pro Arg Phe Glu Pro His Lys Trp Glu Lys Tyr Cys Val Leu Glu
   530 535 540
   Ile Gly Asp Met Leu Leu Arg Thr Ala Val Gly Gln Val Ser Arg Pro
   545 550 555 560
   Met Phe Leu Tyr Val Arg Thr Asn Gly Thr Ser Lys Ile Lys Met Lys
   565 570 575
   Trp Gly Met Glu Met Arg Arg Cys Leu Leu Gln Ser Leu Gln Gln Ile
   580 585 590
   Glu Ser Met Ile Glu Ala Glu Ser Ser Val Lys Glu Lys Asp Met Thr
   595 600 605
   Lys Glu Phe Phe Glu Asn Lys Ser Glu Thr Trp Pro Ile Gly Glu Ser
   610 615 620
   Pro Lys Gly Val Glu Glu Gly Ser Ile Gly Lys Val Cys Arg Thr Leu
   625 630 635 640
   Leu Ala Lys Ser Val Phe Asn Ser Leu Tyr Ala Ser Pro Gln Leu Glu
                   645                 650                 655
   Gly Phe Ser Ala Glu Ser Arg Lys Leu Leu Leu Ile Val Gln Ala Leu
               660                 665                 670
   Arg Asp Asp Leu Glu Pro Gly Thr Phe Asp Ile Gly Gly Leu Tyr Glu
           675                 680                 685
   Ser Ile Glu Glu Cys Leu Ile Asn Asp Pro Trp Val Leu Leu Asn Ala
       690                 695                 700
   Ser Trp Phe Asn Ser Phe Leu Thr His Ala Leu Lys
   705                 710                 715
<210> 69
   <211> 844
   <212> DNA
   <213> Influenza virus
<220>
   <221> CDS
   <222> (1)..(690)
<400> 69
   atg gat tcc aac act gtg tca agc ttt cag gta gac tgt ttt ctt tgg       48
   Met Asp Ser Asn Thr Val Ser Ser Phe Gln Val Asp Cys Phe Leu Trp
   1               5                   10                  15
   cat gtc cgt aaa cga ttc gca gac caa gaa ctg ggt gat gcc cca ttc       96
   His Val Arg Lys Arg Phe Ala Asp Gln Glu Leu Gly Asp Ala Pro Phe
               20                  25                  30
   ctt gac cgg ctt cgc cga gac cag aag tcc cta agg gga aga ggt agc      144
   Leu Asp Arg Leu Arg Arg Asp Gln Lys Ser Leu Arg Gly Arg Gly Ser
           35                  40                  45
   act ctt ggt ctg gac atc gaa aca gcc act cat gca gga aag cag ata      192
   Thr Leu Gly Leu Asp Ile Glu Thr Ala Thr His Ala Gly Lys Gln Ile
       50                  55                  60
   gtg gag cag att ctg gaa aag gaa tca gat gag gca ctt aaa atg acc      240
   Val Glu Gln Ile Leu Glu Lys Glu Ser Asp Glu Ala Leu Lys Met Thr
   65                  70                  75                  80
   att gcc tct gtt cct gct tca cgc tac tta act gac atg act ctt gat      288
   Ile Ala Ser Val Pro Ala Ser Arg Tyr Leu Thr Asp Met Thr Leu Asp
                   85                  90                  95
   gag atg tca aga gac tgg ttc atg ctc atg ccc aag caa aaa gta aca      336
   Glu Met Ser Arg Asp Trp Phe Met Leu Met Pro Lys Gln Lys Val Thr
               100                 105                 110
   ggc tcc cta tgt ata aga atg gac cag gca atc atg gat aag aac atc      384
   Gly Ser Leu Cys Ile Arg Met Asp Gln Ala Ile Met Asp Lys Asn Ile
           115                 120                 125
   ata ctt aaa gca aac ttt agt gtg att ttc gaa agg ctg gaa aca cta      432
   Ile Leu Lys Ala Asn Phe Ser Val Ile Phe Glu Arg Leu Glu Thr Leu
       130                 135                 140
   ata cta ctt aga gcc ttc acc gaa gaa gga gca gtc gtt ggc gaa att      480
   Ile Leu Leu Arg Ala Phe Thr Glu Glu Gly Ala Val Val Gly Glu Ile
   145                 150                 155                 160
   tca cca tta cct tct ctt cca gga cat act aat gag gat gtc aaa aat      528
   Ser Pro Leu Pro Ser Leu Pro Gly His Thr Asn Glu Asp Val Lys Asn
                   165                 170                 175
   gca att ggg gtc ctc atc gga gga ctt aaa tgg aat gat aat acg gtt      576
   Ala Ile Gly Val Leu Ile Gly Gly Leu Lys Trp Asn Asp Asn Thr Val
               180                 185                 190
   aga atc tct gaa act cta cag aga ttc gct tgg aga agc agt cat gaa      624
   Arg Ile Ser Glu Thr Leu Gln Arg Phe Ala Trp Arg Ser Ser His Glu
           195                 200                 205
   aat ggg aga cct tca ttc cct tca aaa cag aaa cga aaa atg gag aga      672
   Asn Gly Arg Pro Ser Phe Pro Ser Lys Gln Lys Arg Lys Met Glu Arg
       210                 215                 220
   aca att aag cca gaa att tgaagaaata agatggttga ttgaagaagt             720
   Thr Ile Lys Pro Glu Ile
   225                 230
   gcgacataga ttgaaaaata cagaaaatag ttttgaacaa ataacattta tgcaagcctt    780
   acaactattg cttgaagtag aacaagagat aagaactttc tcgtttcagc ttatttaatg    840
   ataa                                                                 844
<210> 70
   <211> 230
   <212> PRT
   <213> Influenza virus
<400> 70
   Met Asp Ser Asn Thr Val Ser Ser Phe Gln Val Asp Cys Phe Leu Trp
   1 5 10 15
   His Val Arg Lys Arg Phe Ala Asp Gln Glu Leu Gly Asp Ala Pro Phe
   20 25 30
   Leu Asp Arg Leu Arg Arg Asp Gln Lys Ser Leu Arg Gly Arg Gly Ser
   35 40 45
   Thr Leu Gly Leu Asp Ile Glu Thr Ala Thr His Ala Gly Lys Gln Ile
   50 55 60
   Val Glu Gln Ile Leu Glu Lys Glu Ser Asp Glu Ala Leu Lys Met Thr
   65 70 75 80
   Ile Ala Ser Val Pro Ala Ser Arg Tyr Leu Thr Asp Met Thr Leu Asp
   85 90 95
   Glu Met Ser Arg Asp Trp Phe Met Leu Met Pro Lys Gln Lys Val Thr
   100 105 110
   Gly Ser Leu Cys Ile Arg Met Asp Gln Ala Ile Met Asp Lys Asn Ile
   115 120 125
   Ile Leu Lys Ala Asn Phe Ser Val Ile Phe Glu Arg Leu Glu Thr Leu
   130 135 140
   Ile Leu Leu Arg Ala Phe Thr Glu Glu Gly Ala Val Val Gly Glu Ile
   145 150 155 160
   Ser Pro Leu Pro Ser Leu Pro Gly His Thr Asn Glu Asp Val Lys Asn
   165 170 175
   Ala Ile Gly Val Leu Ile Gly Gly Leu Lys Trp Asn Asp Asn Thr Val
   180 185 190
   Arg Ile Ser Glu Thr Leu Gln Arg Phe Ala Trp Arg Ser Ser His Glu
   195 200 205
   Asn Gly Arg Pro Ser Phe Pro Ser Lys Gln Lys Arg Lys Met Glu Arg
   210 215 220
   Thr Ile Lys Pro Glu Ile
   225 230
<210> 71
   <211> 1497
   <212> DNA
   <213> Influenza virus
<220>
   <221> CDS
   <222> (1)..(1497)
<400> 71
   atg gcg tct caa ggc acc aaa cga tcc tat gaa cag atg gaa act gat         48
   Met Ala Ser Gln Gly Thr Lys Arg Ser Tyr Glu Gln Met Glu Thr Asp
   1               5                   10                  15
   ggg gaa cgc cag aat gca act gaa atc aga gca tct gtc gga agg atg         96
   Gly Glu Arg Gln Asn Ala Thr Glu Ile Arg Ala Ser Val Gly Arg Met
               20                  25                  30
   gtg gga gga atc gga cgg ttt tat gtc cag atg tgt act gag ctt aaa        144
   Val Gly Gly Ile Gly Arg Phe Tyr Val Gln Met Cys Thr Glu Leu Lys
           35                  40                  45
   cta aac gac cat gaa ggg cgg ctg att cag aac agc ata aca ata gaa        192
   Leu Asn Asp His Glu Gly Arg Leu Ile Gln Asn Ser Ile Thr Ile Glu
       50                  55                  60
   agg atg gtg ctt tcg gca ttc gac gaa aga aga aac aag tat ctc gag        240
   Arg Met Val Leu Ser Ala Phe Asp Glu Arg Arg Asn Lys Tyr Leu Glu
   65                  70                  75                  80
   gag cat ccc agt gct ggg aaa gac cct aag aaa acg gga ggc ccg ata        288
   Glu His Pro Ser Ala Gly Lys Asp Pro Lys Lys Thr Gly Gly Pro Ile
                   85                  90                  95
   tac aga aga aaa gat ggg aaa tgg atg agg gaa ctc atc ctc cat gat        336
   Tyr Arg Arg Lys Asp Gly Lys Trp Met Arg Glu Leu Ile Leu His Asp
               100                 105                 110
   aaa gaa gaa atc atg aga atc tgg cgt cag gcc aac aat ggt gaa gac        384
   Lys Glu Glu Ile Met Arg Ile Trp Arg Gln Ala Asn Asn Gly Glu Asp
           115                 120                 125
   gct act gct ggt ctt act cat atg atg atc tgg cac tcc aat ctc aat      432
   Ala Thr Ala Gly Leu Thr His Met Met Ile Trp His Ser Asn Leu Asn
       130                 135                 140
   gac acc aca tac caa aga aca agg gct ctt gtt cgg act ggg atg gat      480
   Asp Thr Thr Tyr Gln Arg Thr Arg Ala Leu Val Arg Thr Gly Met Asp
   145                 150                 155                 160
   ccc aga atg tgc tct ctg atg caa ggc tca acc ctc cca cgg aga tct      528
   Pro Arg Met Cys Ser Leu Met Gln Gly Ser Thr Leu Pro Arg Arg Ser
                   165                 170                 175
   gga gcc gct ggt gct gca gta aaa ggc gtt gga aca atg gta atg gaa      576
   Gly Ala Ala Gly Ala Ala Val Lys Gly Val Gly Thr Met Val Met Glu
               180                 185                 190
   ctc atc aga atg atc aag cgc gga ata aat gat cgg aat ttc tgg aga      624
   Leu Ile Arg Met Ile Lys Arg Gly Ile Asn Asp Arg Asn Phe Trp Arg
           195                 200                 205
   ggt gaa aat ggt cga aga acc aga att gct tat gaa aga atg tgc aat      672
   Gly Glu Asn Gly Arg Arg Thr Arg Ile Ala Tyr Glu Arg Met Cys Asn
       210                 215                 220
   atc ctc aaa ggg aaa ttt cag aca gca gca caa cgg gct atg atg gac      720
   Ile Leu Lys Gly Lys Phe Gln Thr Ala Ala Gln Arg Ala Met Met Asp
   225                 230                 235                 240
   cag gtg agg gaa ggc cgc aat cct gga aac gct gag att gag gat ctc      768
   Gln Val Arg Glu Gly Arg Asn Pro Gly Asn Ala Glu Ile Glu Asp Leu
                   245                 250                 255
   att ttc ttg gca cga tca gca ctt att ttg aga gga tca gta gcc cat      816
   Ile Phe Leu Ala Arg Ser Ala Leu Ile Leu Arg Gly Ser Val Ala His
               260                 265                 270
   aaa tca tgc cta cct gcc tgt gtt tat ggc ctt gca cta acc agt ggg      864
   Lys Ser Cys Leu Pro Ala Cys Val Tyr Gly Leu Ala Leu Thr Ser Gly
           275                 280                 285
   tat gac ttt gag aag gaa gga tac tct ctg gtt gga att gat cct ttc      912
   Tyr Asp Phe Glu Lys Glu Gly Tyr Ser Leu Val Gly Ile Asp Pro Phe
       290                 295                 300
   aaa cta ctc cag aac agt caa att ttc agt cta atc aga cca aaa gaa      960
   Lys Leu Leu Gln Asn Ser Gln Ile Phe Ser Leu Ile Arg Pro Lys Glu
   305                 310                 315                 320
   aac cca gca cac aaa agc cag ttg gtg tgg atg gca tgc cat tct gca     1008
   Asn Pro Ala His Lys Ser Gln Leu Val Trp Met Ala Cys His Ser Ala
                   325                 330                 335
   gca ttt gag gat ctg aga gtt tta aat ttc att aga gga acc aaa gta     1056
   Ala Phe Glu Asp Leu Arg Val Leu Asn Phe Ile Arg Gly Thr Lys Val
               340                 345                 350
   atc cca aga gga cag tta aca acc aga gga gtt caa att gct tca aat     1104
   Ile Pro Arg Gly Gln Leu Thr Thr Arg Gly Val Gln Ile Ala Ser Asn
           355                 360                 365
   gaa aac atg gag aca ata aat tct agc aca ctt gaa ctg aga agc aaa     1152
   Glu Asn Met Glu Thr Ile Asn Ser Ser Thr Leu Glu Leu Arg Ser Lys
       370                 375                 380
   tat tgg gca ata agg acc aga agc gga gga aac acc agt caa cag aga     1200
   Tyr Trp Ala Ile Arg Thr Arg Ser Gly Gly Asn Thr Ser Gln Gln Arg
   385                 390                 395                 400
   gca tct gca gga cag ata agt gtg caa cct act ttc tca gta cag aga      1248
   Ala Ser Ala Gly Gln Ile Ser Val Gln Pro Thr Phe Ser Val Gln Arg
                   405                 410                 415
   aat ctt ccc ttt gag aga gca acc att atg gct gca ttc act ggt aac      1296
   Asn Leu Pro Phe Glu Arg Ala Thr Ile Met Ala Ala Phe Thr Gly Asn
               420                 425                 430
   act gaa gga agg act tcc gac atg aga acg gaa atc ata agg atg atg      1344
   Thr Glu Gly Arg Thr Ser Asp Met Arg Thr Glu Ile Ile Arg Met Met
           435                 440                 445
   gaa aat gcc aaa tca gaa gat gtg tct ttc cag ggg cgg gga gtc ttc      1392
   Glu Asn Ala Lys Ser Glu Asp Val Ser Phe Gln Gly Arg Gly Val Phe
       450                 455                 460
   gag ctc tcg gac gaa aag gca acg aac ccg atc gtg cct tcc ttt gac      1440
   Glu Leu Ser Asp Glu Lys Ala Thr Asn Pro Ile Val Pro Ser Phe Asp
   465                 470                 475                 480
   atg agc aat gaa ggg tct tat ttc ttc gga gac aat gct gag gag ttt      1488
   Met Ser Asn Glu Gly Ser Tyr Phe Phe Gly Asp Asn Ala Glu Glu Phe
                   485                 490                 495
   gac agt taa                                                          1497
   Asp Ser
<210> 72
   <211> 498
   <212> PRT
   <213> Influenza virus
<400> 72
   Met Ala Ser Gln Gly Thr Lys Arg Ser Tyr Glu Gln Met Glu Thr Asp
   1               5                   10                  15
   Gly Glu Arg Gln Asn Ala Thr Glu Ile Arg Ala Ser Val Gly Arg Met
               20                  25                  30
   Val Gly Gly Ile Gly Arg Phe Tyr Val Gln Met Cys Thr Glu Leu Lys
           35                  40                  45
   Leu Asn Asp His Glu Gly Arg Leu Ile Gln Asn Ser Ile Thr Ile Glu
       50                  55                  60
   Arg Met Val Leu Ser Ala Phe Asp Glu Arg Arg Asn Lys Tyr Leu Glu
   65                  70                  75                  80
   Glu His Pro Ser Ala Gly Lys Asp Pro Lys Lys Thr Gly Gly Pro Ile
                   85                  90                  95
   Tyr Arg Arg Lys Asp Gly Lys Trp Met Arg Glu Leu Ile Leu His Asp
               100                 105                 110
   Lys Glu Glu Ile Met Arg Ile Trp Arg Gln Ala Asn Asn Gly Glu Asp
           115                 120                 125
   Ala Thr Ala Gly Leu Thr His Met Met Ile Trp His Ser Asn Leu Asn
       130                 135                 140
   Asp Thr Thr Tyr Gln Arg Thr Arg Ala Leu Val Arg Thr Gly Met Asp
   145                 150                 155                 160
   Pro Arg Met Cys Ser Leu Met Gln Gly Ser Thr Leu Pro Arg Arg Ser
                   165                 170                 175
   Gly Ala Ala Gly Ala Ala Val Lys Gly Val Gly Thr Met Val Met Glu
               180                 185                 190
   Leu Ile Arg Met Ile Lys Arg Gly Ile Asn Asp Arg Asn Phe Trp Arg
           195                 200                 205
   Gly Glu Asn Gly Arg Arg Thr Arg Ile Ala Tyr Glu Arg Met Cys Asn
       210                 215                 220
   Ile Leu Lys Gly Lys Phe Gln Thr Ala Ala Gln Arg Ala Met Met Asp
   225                 230                 235                 240
   Gln Val Arg Glu Gly Arg Asn Pro Gly Asn Ala Glu Ile Glu Asp Leu
                   245                 250                 255
   Ile Phe Leu Ala Arg Ser Ala Leu Ile Leu Arg Gly Ser Val Ala His
               260                 265                 270
   Lys Ser Cys Leu Pro Ala Cys Val Tyr Gly Leu Ala Leu Thr Ser Gly
           275                 280                 285
   Tyr Asp Phe Glu Lys Glu Gly Tyr Ser Leu Val Gly Ile Asp Pro Phe
       290                 295                 300
   Lys Leu Leu Gln Asn Ser Gln Ile Phe Ser Leu Ile Arg Pro Lys Glu
   305                 310                 315                 320
   Asn Pro Ala His Lys Ser Gln Leu Val Trp Met Ala Cys His Ser Ala
                   325                 330                 335
   Ala Phe Glu Asp Leu Arg Val Leu Asn Phe Ile Arg Gly Thr Lys Val
               340                 345                 350
   Ile Pro Arg Gly Gln Leu Thr Thr Arg Gly Val Gln Ile Ala Ser Asn
           355                 360                 365
   Glu Asn Met Glu Thr Ile Asn Ser Ser Thr Leu Glu Leu Arg Ser Lys
       370                 375                 380
   Tyr Trp Ala Ile Arg Thr Arg Ser Gly Gly Asn Thr Ser Gln Gln Arg
   385                 390                 395                 400
   Ala Ser Ala Gly Gln Ile Ser Val Gln Pro Thr Phe Ser Val Gln Arg
                   405                 410                 415
   Asn Leu Pro Phe Glu Arg Ala Thr Ile Met Ala Ala Phe Thr Gly Asn
               420                 425                 430
   Thr Glu Gly Arg Thr Ser Asp Met Arg Thr Glu Ile Ile Arg Met Met
           435                 440                 445
   Glu Asn Ala Lys Ser Glu Asp Val Ser Phe Gln Gly Arg Gly Val Phe
       450                 455                 460
   Glu Leu Ser Asp Glu Lys Ala Thr Asn Pro Ile Val Pro Ser Phe Asp
   465                 470                 475                 480
   Met Ser Asn Glu Gly Ser Tyr Phe Phe Gly Asp Asn Ala Glu Glu Phe
                   485                 490                 495
   Asp Ser
<210> 73
   <211> 1413
   <212> DNA
   <213> Influenza virus
<220>
   <221> CDS
   <222> (1)..(1413)
<400> 73
   atg aat cca aat caa aag ata ata gca att gga ttt gca tca ttg ggg      48
   Met Asn Pro Asn Gln Lys Ile Ile Ala Ile Gly Phe Ala Ser Leu Gly
   1               5                   10                  15
   ata tta atc att aat gtc att ctc cat gta gtc agc att ata gta aca      96
   Ile Leu Ile Ile Asn Val Ile Leu His Val Val Ser Ile Ile Val Thr
               20                  25                  30
   gta ctg gtc ctc aat aac aat aga aca gat ctg aac tgc aaa ggg acg     144
   Val Leu Val Leu Asn Asn Asn Arg Thr Asp Leu Asn Cys Lys Gly Thr
           35                  40                  45
   atc ata aga gaa tac aat gaa aca gta aga gta gaa aaa ctt act caa     192
   Ile Ile Arg Glu Tyr Asn Glu Thr Val Arg Val Glu Lys Leu Thr Gln
       50                  55                  60
   tgg tat aat acc agt aca att aag tac ata gag aga cct tca aat gaa     240
   Trp Tyr Asn Thr Ser Thr Ile Lys Tyr Ile Glu Arg Pro Ser Asn Glu
   65                  70                  75                  80
   tac tac atg aat aac act gaa cca ctt tgt gag gcc caa ggc ttt gca     288
   Tyr Tyr Met Asn Asn Thr Glu Pro Leu Cys Glu Ala Gln Gly Phe Ala
                   85                  90                  95
   cca ttt tcc aaa gat aat gga ata cga att ggg tcg aga ggc cat gtt     336
   Pro Phe Ser Lys Asp Asn Gly Ile Arg Ile Gly Ser Arg Gly His Val
               100                 105                 110
   ttt gtg ata aga gaa cct ttt gta tca tgt tcg ccc tca gaa tgt aga     384
   Phe Val Ile Arg Glu Pro Phe Val Ser Cys Ser Pro Ser Glu Cys Arg
           115                 120                 125
   acc ttt ttc ctc aca cag ggc tca tta ctc aat gac aaa cat tct aac      432
   Thr Phe Phe Leu Thr Gln Gly Ser Leu Leu Asn Asp Lys His Ser Asn
       130                 135                 140
   ggc aca ata aag gat cga agt ccg tat agg act ttg atg agt gtc aaa      480
   Gly Thr Ile Lys Asp Arg Ser Pro Tyr Arg Thr Leu Met Ser Val Lys
   145                 150                 155                 160
   ata ggg caa tca cct aat gta tat caa gct agg ttt gaa tcg gtg gca      528
   Ile Gly Gln Ser Pro Asn Val Tyr Gln Ala Arg Phe Glu Ser Val Ala
                   165                 170                 175
   tgg tca gca aca gca tgc cat gat gga aaa aaa tgg atg aca gtt gga      576
   Trp Ser Ala Thr Ala Cys His Asp Gly Lys Lys Trp Met Thr Val Gly
               180                 185                 190
   gtc aca ggg ccc gac aat caa gca att gca gta gtg aac tat gga ggt      624
   Val Thr Gly Pro Asp Asn Gln Ala Ile Ala Val Val Asn Tyr Gly Gly
           195                 200                 205
   gtt ccg gtt gat att att aat tca tgg gca ggg gat att tta aga acc      672
   Val Pro Val Asp Ile Ile Asn Ser Trp Ala Gly Asp Ile Leu Arg Thr
       210                 215                 220
   caa gaa tca tca tgc acc tgc att aaa gga gac tgt tat tgg gta atg      720
   Gln Glu Ser Ser Cys Thr Cys Ile Lys Gly Asp Cys Tyr Trp Val Met
   225                 230                 235                 240
   act gat gga ccg gca aat agg caa gct aaa tat agg ata ttc aaa gca      768
   Thr Asp Gly Pro Ala Asn Arg Gln Ala Lys Tyr Arg Ile Phe Lys Ala
                   245                 250                 255
   aaa gat gga aga gta att gga caa act gat ata agt ttc aat ggg gga      816
   Lys Asp Gly Arg Val Ile Gly Gln Thr Asp Ile Ser Phe Asn Gly Gly
               260                 265                 270
   cac ata gag gag tgt tct tgt tac ccc aat gaa ggg aag gtg gaa tgc      864
   His Ile Glu Glu Cys Ser Cys Tyr Pro Asn Glu Gly Lys Val Glu Cys
           275                 280                 285
   ata tgc agg gac aat tgg act gga aca aat aga cca att ctg gta ata      912
   Ile Cys Arg Asp Asn Trp Thr Gly Thr Asn Arg Pro Ile Leu Val Ile
       290                 295                 300
   tct tct gat cta tcg tac aca gtt gga tat ttg tgt gct ggc att ccc      960
   Ser Ser Asp Leu Ser Tyr Thr Val Gly Tyr Leu Cys Ala Gly Ile Pro
   305                 310                 315                 320
   act gac act cct agg gga gag gat agt caa ttc aca ggc tca tgt aca     1008
   Thr Asp Thr Pro Arg Gly Glu Asp Ser Gln Phe Thr Gly Ser Cys Thr
                   325                 330                 335
   agt cct ttg gga aat aaa gga tac ggt gta aaa ggc ttc ggg ttt cga     1056
   Ser Pro Leu Gly Asn Lys Gly Tyr Gly Val Lys Gly Phe Gly Phe Arg
               340                 345                 350
   caa gga act gac gta tgg gcc gga agg aca att agt agg act tca aga     1104
   Gln Gly Thr Asp Val Trp Ala Gly Arg Thr Ile Ser Arg Thr Ser Arg
           355                 360                 365
   tca gga ttc gaa ata ata aaa atc agg aat ggt tgg aca cag aac agt     1152
   Ser Gly Phe Glu Ile Ile Lys Ile Arg Asn Gly Trp Thr Gln Asn Ser
       370                 375                 380
   aag gac caa atc agg agg caa gtg att atc gat gac cca aat tgg tca     1200
   Lys Asp Gln Ile Arg Arg Gln Val Ile Ile Asp Asp Pro Asn Trp Ser
   385                 390                 395                 400
   gga tat agc ggt tct ttc aca ttg ccg gtt gaa ctg aca aaa aag gga      1248
   Gly Tyr Ser Gly Ser Phe Thr Leu Pro Val Glu Leu Thr Lys Lys Gly
                   405                 410                 415
   tgt ttg gtc ccc tgt ttc tgg gtt gaa atg att aga ggt aaa cct gaa      1296
   Cys Leu Val Pro Cys Phe Trp Val Glu Met Ile Arg Gly Lys Pro Glu
               420                 425                 430
   gaa aca aca ata tgg acc tct agc agc tcc att gtg atg tgt gga gta      1344
   Glu Thr Thr Ile Trp Thr Ser Ser Ser Ser Ile Val Met Cys Gly Val
           435                 440                 445
   gat cat aaa att gcc agt tgg tca tgg cac gat gga gct att ctt ccc      1392
   Asp His Lys Ile Ala Ser Trp Ser Trp His Asp Gly Ala Ile Leu Pro
       450                 455                 460
   ttt gac atc gat aag atg taa                                          1413
   Phe Asp Ile Asp Lys Met
   465                 470
<210> 74
   <211> 470
   <212> PRT
   <213> Influenza virus
<400> 74
   Met Asn Pro Asn Gln Lys Ile Ile Ala Ile Gly Phe Ala Ser Leu Gly
   1               5                   10                  15
   Ile Leu Ile Ile Asn Val Ile Leu His Val Val Ser Ile Ile Val Thr
               20                  25                  30
   Val Leu Val Leu Asn Asn Asn Arg Thr Asp Leu Asn Cys Lys Gly Thr
           35                  40                  45
   Ile Ile Arg Glu Tyr Asn Glu Thr Val Arg Val Glu Lys Leu Thr Gln
       50                  55                  60
   Trp Tyr Asn Thr Ser Thr Ile Lys Tyr Ile Glu Arg Pro Ser Asn Glu
   65                  70                  75                  80
   Tyr Tyr Met Asn Asn Thr Glu Pro Leu Cys Glu Ala Gln Gly Phe Ala
                   85                  90                  95
   Pro Phe Ser Lys Asp Asn Gly Ile Arg Ile Gly Ser Arg Gly His Val
               100                 105                 110
   Phe Val Ile Arg Glu Pro Phe Val Ser Cys Ser Pro Ser Glu Cys Arg
           115                 120                 125
   Thr Phe Phe Leu Thr Gln Gly Ser Leu Leu Asn Asp Lys His Ser Asn
       130                 135                 140
   Gly Thr Ile Lys Asp Arg Ser Pro Tyr Arg Thr Leu Met Ser Val Lys
   145                 150                 155                 160
   Ile Gly Gln Ser Pro Asn Val Tyr Gln Ala Arg Phe Glu Ser Val Ala
                   165                 170                 175
   Trp Ser Ala Thr Ala Cys His Asp Gly Lys Lys Trp Met Thr Val Gly
               180                 185                 190
   Val Thr Gly Pro Asp Asn Gln Ala Ile Ala Val Val Asn Tyr Gly Gly
           195                 200                 205
   Val Pro Val Asp Ile Ile Asn Ser Trp Ala Gly Asp Ile Leu Arg Thr
       210                 215                 220
   Gln Glu Ser Ser Cys Thr Cys Ile Lys Gly Asp Cys Tyr Trp Val Met
   225                 230                 235                 240
   Thr Asp Gly Pro Ala Asn Arg Gln Ala Lys Tyr Arg Ile Phe Lys Ala
                   245                 250                 255
   Lys Asp Gly Arg Val Ile Gly Gln Thr Asp Ile Ser Phe Asn Gly Gly
               260                 265                 270
   His Ile Glu Glu Cys Ser Cys Tyr Pro Asn Glu Gly Lys Val Glu Cys
           275                 280                 285
   Ile Cys Arg Asp Asn Trp Thr Gly Thr Asn Arg Pro Ile Leu Val Ile
       290                 295                 300
   Ser Ser Asp Leu Ser Tyr Thr Val Gly Tyr Leu Cys Ala Gly Ile Pro
   305                 310                 315                 320
   Thr Asp Thr Pro Arg Gly Glu Asp Ser Gln Phe Thr Gly Ser Cys Thr
                   325                 330                 335
   Ser Pro Leu Gly Asn Lys Gly Tyr Gly Val Lys Gly Phe Gly Phe Arg
               340                 345                 350
   Gln Gly Thr Asp Val Trp Ala Gly Arg Thr Ile Ser Arg Thr Ser Arg
           355                 360                 365
   Ser Gly Phe Glu Ile Ile Lys Ile Arg Asn Gly Trp Thr Gln Asn Ser
       370                 375                 380
   Lys Asp Gln Ile Arg Arg Gln Val Ile Ile Asp Asp Pro Asn Trp Ser
   385                 390                 395                 400
   Gly Tyr Ser Gly Ser Phe Thr Leu Pro Val Glu Leu Thr Lys Lys Gly
                   405                 410                 415
   Cys Leu Val Pro Cys Phe Trp Val Glu Met Ile Arg Gly Lys Pro Glu
               420                 425                 430
   Glu Thr Thr Ile Trp Thr Ser Ser Ser Ser Ile Val Met Cys Gly Val
   435 440 445
   Asp His Lys Ile Ala Ser Trp Ser Trp His Asp Gly Ala Ile Leu Pro
   450 455 460
   Phe Asp Ile Asp Lys Met
   465 470
<210> 75
   <211> 981
   <212> DNA
   <213> Influenza virus
<220>
   <221> CDS
   <222> (1)..(756)
<400> 75
   atg agt ctt cta acc gag gtc gaa acg tac gtt ctc tct atc gta cca       48
   Met Ser Leu Leu Thr Glu Val Glu Thr Tyr Val Leu Ser Ile Val Pro
   1               5                   10                  15
   tca ggc ccc ctc aaa gcc gag atc gcg cag aga ctt gaa gat gtc ttt       96
   Ser Gly Pro Leu Lys Ala Glu Ile Ala Gln Arg Leu Glu Asp Val Phe
               20                  25                  30
   gcg gga aag aac acc gat ctt gag gca ctc atg gaa tgg cta aag aca      144
   Ala Gly Lys Asn Thr Asp Leu Glu Ala Leu Met Glu Trp Leu Lys Thr
           35                  40                  45
   aga cca atc ctg tca cct ctg act aaa ggg att tta gga ttt gta ttc      192
   Arg Pro Ile Leu Ser Pro Leu Thr Lys Gly Ile Leu Gly Phe Val Phe
       50                  55                  60
   acg ctc acc gtg ccc agt gag cga gga ctg cag cgt aga cgc ttt gtc      240
   Thr Leu Thr Val Pro Ser Glu Arg Gly Leu Gln Arg Arg Arg Phe Val
   65                  70                  75                  80
   caa aat gcc ctt agt gga aac gga gat cca aac aac atg gac aga gca      288
   Gln Asn Ala Leu Ser Gly Asn Gly Asp Pro Asn Asn Met Asp Arg Ala
                   85                  90                  95
   gta aaa ctg tac agg aag ctt aaa aga gaa ata aca ttc cat ggg gca      336
   Val Lys Leu Tyr Arg Lys Leu Lys Arg Glu Ile Thr Phe His Gly Ala
               100                 105                 110
   aaa gag gtg gca ctc agc tat tcc act ggt gca cta gcc agc tgc atg      384
   Lys Glu Val Ala Leu Ser Tyr Ser Thr Gly Ala Leu Ala Ser Cys Met
           115                 120                 125
   gga ctc ata tac aac aga atg gga act gtt aca acc gaa gtg gca ttt      432
   Gly Leu Ile Tyr Asn Arg Met Gly Thr Val Thr Thr Glu Val Ala Phe
       130                 135                 140
   ggc ctg gta tgc gcc aca tgt gaa cag att gct gat tcc cag cat cga      480
   Gly Leu Val Cys Ala Thr Cys Glu Gln Ile Ala Asp Ser Gln His Arg
   145                 150                 155                 160
   tct cac agg cag atg gtg aca aca acc aac cca tta atc aga cat gaa      528
   Ser His Arg Gln Met Val Thr Thr Thr Asn Pro Leu Ile Arg His Glu
                   165                 170                 175
   aac aga atg gta tta gcc agt acc acg gct aaa gcc atg gaa cag atg      576
   Asn Arg Met Val Leu Ala Ser Thr Thr Ala Lys Ala Met Glu Gln Met
               180                 185                 190
   gca gga tcg agt gag cag gca gca gag gcc atg gag gtt gct agt agg      624
   Ala Gly Ser Ser Glu Gln Ala Ala Glu Ala Met Glu Val Ala Ser Arg
           195                 200                 205
   gct agg cag atg gta cag gca atg aga acc att ggg acc cac cct agc      672
   Ala Arg Gln Met Val Gln Ala Met Arg Thr Ile Gly Thr His Pro Ser
       210                 215                 220
   tcc agt gcc ggt ttg aaa gat gat ctc ctt gaa aat tta cag gcc tac      720
   Ser Ser Ala Gly Leu Lys Asp Asp Leu Leu Glu Asn Leu Gln Ala Tyr
   225                 230                 235                 240
   cag aaa cgg atg gga gtg caa atg cag cga ttc aag tgatcctctc           766
   Gln Lys Arg Met Gly Val Gln Met Gln Arg Phe Lys
                   245                 250
   gtcattgcag caagtatcat tgggatcttg cacttgatat tgtggattct tgatcgtctt    826
   ttcttcaaat tcatttatcg tcgccttaaa tacgggttga aaagagggcc ttctacggaa    886
   ggagtacctg agtctatgag ggaagaatat cggcaggaac agcagaatgc tgtggatgtt    946
   gacgatggtc attttgtcaa catagagctg gagta                               981
<210> 76
   <211> 252
   <212> PRT
   <213> Influenza virus
<400> 76
   Met Ser Leu Leu Thr Glu Val Glu Thr Tyr Val Leu Ser Ile Val Pro
   1               5                   10                  15
   Ser Gly Pro Leu Lys Ala Glu Ile Ala Gln Arg Leu Glu Asp Val Phe
               20                  25                  30
   Ala Gly Lys Asn Thr Asp Leu Glu Ala Leu Met Glu Trp Leu Lys Thr
           35                  40                  45
   Arg Pro Ile Leu Ser Pro Leu Thr Lys Gly Ile Leu Gly Phe Val Phe
       50                  55                  60
   Thr Leu Thr Val Pro Ser Glu Arg Gly Leu Gln Arg Arg Arg Phe Val
   65                  70                  75                  80
   Gln Asn Ala Leu Ser Gly Asn Gly Asp Pro Asn Asn Met Asp Arg Ala
                   85                  90                  95
   Val Lys Leu Tyr Arg Lys Leu Lys Arg Glu Ile Thr Phe His Gly Ala
               100                 105                 110
   Lys Glu Val Ala Leu Ser Tyr Ser Thr Gly Ala Leu Ala Ser Cys Met
           115                 120                 125
   Gly Leu Ile Tyr Asn Arg Met Gly Thr Val Thr Thr Glu Val Ala Phe
       130                 135                 140
   Gly Leu Val Cys Ala Thr Cys Glu Gln Ile Ala Asp Ser Gln His Arg
   145                 150                 155                 160
   Ser His Arg Gln Met Val Thr Thr Thr Asn Pro Leu Ile Arg His Glu
                   165                 170                 175
   Asn Arg Met Val Leu Ala Ser Thr Thr Ala Lys Ala Met Glu Gln Met
               180                 185                 190
   Ala Gly Ser Ser Glu Gln Ala Ala Glu Ala Met Glu Val Ala Ser Arg
           195                 200                 205
   Ala Arg Gln Met Val Gln Ala Met Arg Thr Ile Gly Thr His Pro Ser
       210                 215                 220
   Ser Ser Ala Gly Leu Lys Asp Asp Leu Leu Glu Asn Leu Gln Ala Tyr
   225                 230                 235                 240
   Gln Lys Arg Met Gly Val Gln Met Gln Arg Phe Lys
                   245                 250
<210> 77
   <211> 1698
   <212> DNA
   <213> Influenza virus
<220>
   <221> CDS
   <222> (1)..(1698)
<400> 77
   atg aag aca acc att att tta ata cta ctg acc cat tgg gcc tac agt       48
   Met Lys Thr Thr Ile Ile Leu Ile Leu Leu Thr His Trp Ala Tyr Ser
   1               5                   10                  15
   caa aac cca atc agt ggc aat aac aca gcc aca ctg tgt ctg gga cac       96
   Gln Asn Pro Ile Ser Gly Asn Asn Thr Ala Thr Leu Cys Leu Gly His
               20                  25                  30
   cat gca gta gca aat gga aca ttg gta aaa aca atg agt gat gat caa      144
   His Ala Val Ala Asn Gly Thr Leu Val Lys Thr Met Ser Asp Asp Gln
           35                  40                  45
   att gag gtg aca aat gct aca gaa tta gtt cag agc att tca atg ggg      192
   Ile Glu Val Thr Asn Ala Thr Glu Leu Val Gln Ser Ile Ser Met Gly
       50                  55                  60
   aaa ata tgc aac aaa tca tat aga att cta gat gga aga aat tgc aca      240
   Lys Ile Cys Asn Lys Ser Tyr Arg Ile Leu Asp Gly Arg Asn Cys Thr
   65                  70                  75                  80
   tta ata gat gca atg cta gga gac ccc cac tgt gac gcc ttt cag tat      288
   Leu Ile Asp Ala Met Leu Gly Asp Pro His Cys Asp Ala Phe Gln Tyr
                   85                  90                  95
   gag agt tgg gac ctc ttt ata gaa aga agc agc gct ttc agc aat tgc      336
   Glu Ser Trp Asp Leu Phe Ile Glu Arg Ser Ser Ala Phe Ser Asn Cys
               100                 105                 110
   tac cca tat gac atc cct gac tat gca ccg ctc cga tcc att gta gca      384
   Tyr Pro Tyr Asp Ile Pro Asp Tyr Ala Pro Leu Arg Ser Ile Val Ala
           115                 120                 125
   tcc tca ggg aca gtg gaa ttc aca gca gag gga ttc aca tgg aca ggt        432
   Ser Ser Gly Thr Val Glu Phe Thr Ala Glu Gly Phe Thr Trp Thr Gly
       130                 135                 140
   gta act caa aac gga aga agt gga gcc tgc aaa agg gga tca gcc gat        480
   Val Thr Gln Asn Gly Arg Ser Gly Ala Cys Lys Arg Gly Ser Ala Asp
   145                 150                 155                 160
   agt ttc ttt agc cga ctg aat tgg cta aca aaa tct gga agc tct tac        528
   Ser Phe Phe Ser Arg Leu Asn Trp Leu Thr Lys Ser Gly Ser Ser Tyr
                   165                 170                 175
   ccc aca ttg aat gtg aca atg cct aac aat aaa aat ttc gac aag cta        576
   Pro Thr Leu Asn Val Thr Met Pro Asn Asn Lys Asn Phe Asp Lys Leu
               180                 185                 190
   tac atc tgg ggg att cat cac ccg agc tca aat caa gag cag aca aaa        624
   Tyr Ile Trp Gly Ile His His Pro Ser Ser Asn Gln Glu Gln Thr Lys
           195                 200                 205
   ttg tac atc caa gaa tca gga cga gta aca gtc tca aca aaa aga agt        672
   Leu Tyr Ile Gln Glu Ser Gly Arg Val Thr Val Ser Thr Lys Arg Ser
       210                 215                 220
   caa caa aca ata atc cct aac atc gga tct aga ccg ttg gtc aga ggt        720
   Gln Gln Thr Ile Ile Pro Asn Ile Gly Ser Arg Pro Leu Val Arg Gly
   225                 230                 235                 240
   caa tca ggc agg ata agc ata tac tgg acc att gta aaa cct gga gat        768
   Gln Ser Gly Arg Ile Ser Ile Tyr Trp Thr Ile Val Lys Pro Gly Asp
                   245                 250                 255
   atc cta atg ata aac agt aat ggc aac tta gtt gca ccg cgg gga tat        816
   Ile Leu Met Ile Asn Ser Asn Gly Asn Leu Val Ala Pro Arg Gly Tyr
               260                 265                 270
   ttt aaa ttg aac aca ggg aaa agc tct gta atg aga tcc gat gta ccc        864
   Phe Lys Leu Asn Thr Gly Lys Ser Ser Val Met Arg Ser Asp Val Pro
           275                 280                 285
   ata gac att tgt gtg tct gaa tgt att aca cca aat gga agc atc tcc        912
   Ile Asp Ile Cys Val Ser Glu Cys Ile Thr Pro Asn Gly Ser Ile Ser
       290                 295                 300
   aac gac aag cca ttc caa aat gtg aac aaa gtt aca tat gga aaa tgc        960
   Asn Asp Lys Pro Phe Gln Asn Val Asn Lys Val Thr Tyr Gly Lys Cys
   305                 310                 315                 320
   ccc aag tat atc agg caa aac act tta aag ctg gcc act ggg atg agg       1008
   Pro Lys Tyr Ile Arg Gln Asn Thr Leu Lys Leu Ala Thr Gly Met Arg
                   325                 330                 335
   aat gta cca gaa aag caa acc aga gga atc ttt gga gca ata gcg gga       1056
   Asn Val Pro Glu Lys Gln Thr Arg Gly Ile Phe Gly Ala Ile Ala Gly
               340                 345                 350
   ttc atc gaa aac ggc tgg gaa gga atg gtt gat ggg tgg tat ggg ttc       1104
   Phe Ile Glu Asn Gly Trp Glu Gly Met Val Asp Gly Trp Tyr Gly Phe
           355                 360                 365
   cga tat caa aac tct gaa gga aca ggg caa gct gca gat cta aag agc       1152
   Arg Tyr Gln Asn Ser Glu Gly Thr Gly Gln Ala Ala Asp Leu Lys Ser
       370                 375                 380
   act caa gca gcc atc gac cag att aat gga aag tta aac aga gtg att       1200
   Thr Gln Ala Ala Ile Asp Gln Ile Asn Gly Lys Leu Asn Arg Val Ile
   385                 390                 395                 400
   gaa aga acc aat gag aaa ttc cat caa ata gag aag gaa ttc tca gaa      1248
   Glu Arg Thr Asn Glu Lys Phe His Gln Ile Glu Lys Glu Phe Ser Glu
                   405                 410                 415
   gta gaa gga aga att cag gac ttg gag aaa tat gta gaa gac acc aaa      1296
   Val Glu Gly Arg Ile Gln Asp Leu Glu Lys Tyr Val Glu Asp Thr Lys
               420                 425                 430
   ata gac cta tgg tcc tac aat gca gaa ttg ctg gtg gct cta gaa aat      1344
   Ile Asp Leu Trp Ser Tyr Asn Ala Glu Leu Leu Val Ala Leu Glu Asn
           435                 440                 445
   caa cat aca att gac tta aca gat gca gaa atg aat aaa tta ttt gag      1392
   Gln His Thr Ile Asp Leu Thr Asp Ala Glu Met Asn Lys Leu Phe Glu
       450                 455                 460
   aag act aga cgc cag tta aga gaa aac gca gaa gac atg gga ggt gga      1440
   Lys Thr Arg Arg Gln Leu Arg Glu Asn Ala Glu Asp Met Gly Gly Gly
   465                 470                 475                 480
   tgt ttc aag att tac cac aaa tgt gat aat gca tgc att gaa tca ata      1488
   Cys Phe Lys Ile Tyr His Lys Cys Asp Asn Ala Cys Ile Glu Ser Ile
                   485                 490                 495
   aga act ggg aca tat gac cat tac ata tac aaa gat gaa gca tta aac      1536
   Arg Thr Gly Thr Tyr Asp His Tyr Ile Tyr Lys Asp Glu Ala Leu Asn
               500                 505                 510
   aat cga ttt cag atc aaa ggt gta gag ttg aaa tca ggc tac aaa gat      1584
   Asn Arg Phe Gln Ile Lys Gly Val Glu Leu Lys Ser Gly Tyr Lys Asp
           515                 520                 525
   tgg ata ctg tgg att tca ttc gcc ata tca tgc ttc tta att tgc gtt      1632
   Trp Ile Leu Trp Ile Ser Phe Ala Ile Ser Cys Phe Leu Ile Cys Val
       530                 535                 540
   gtt cta ttg ggt ttc att atg tgg gct tgc caa aaa ggc aac atc aga      1680
   Val Leu Leu Gly Phe Ile Met Trp Ala Cys Gln Lys Gly Asn Ile Arg
   545                 550                 555                 560
   tgc aac att tgc att tga                                              1698
   Cys Asn Ile Cys Ile
                   565
<210> 78
   <211> 565
   <212> PRT
   <213> Influenza virus
<400> 78
   Met Lys Thr Thr Ile Ile Leu Ile Leu Leu Thr His Trp Ala Tyr Ser
   1               5                   10                  15
   Gln Asn Pro Ile Ser Gly Asn Asn Thr Ala Thr Leu Cys Leu Gly His
               20                  25                  30
   His Ala Val Ala Asn Gly Thr Leu Val Lys Thr Met Ser Asp Asp Gln
           35                  40                  45
   Ile Glu Val Thr Asn Ala Thr Glu Leu Val Gln Ser Ile Ser Met Gly
       50                  55                  60
   Lys Ile Cys Asn Lys Ser Tyr Arg Ile Leu Asp Gly Arg Asn Cys Thr
   65                  70                  75                  80
   Leu Ile Asp Ala Met Leu Gly Asp Pro His Cys Asp Ala Phe Gln Tyr
                   85                  90                  95
   Glu Ser Trp Asp Leu Phe Ile Glu Arg Ser Ser Ala Phe Ser Asn Cys
               100                 105                 110
   Tyr Pro Tyr Asp Ile Pro Asp Tyr Ala Pro Leu Arg Ser Ile Val Ala
           115                 120                 125
   Ser Ser Gly Thr Val Glu Phe Thr Ala Glu Gly Phe Thr Trp Thr Gly
       130                 135                 140
   Val Thr Gln Asn Gly Arg Ser Gly Ala Cys Lys Arg Gly Ser Ala Asp
   145                 150                 155                 160
   Ser Phe Phe Ser Arg Leu Asn Trp Leu Thr Lys Ser Gly Ser Ser Tyr
                   165                 170                 175
   Pro Thr Leu Asn Val Thr Met Pro Asn Asn Lys Asn Phe Asp Lys Leu
               180                 185                 190
   Tyr Ile Trp Gly Ile His His Pro Ser Ser Asn Gln Glu Gln Thr Lys
           195                 200                 205
   Leu Tyr Ile Gln Glu Ser Gly Arg Val Thr Val Ser Thr Lys Arg Ser
       210                 215                 220
   Gln Gln Thr Ile Ile Pro Asn Ile Gly Ser Arg Pro Leu Val Arg Gly
   225                 230                 235                 240
   Gln Ser Gly Arg Ile Ser Ile Tyr Trp Thr Ile Val Lys Pro Gly Asp
                   245                 250                 255
   Ile Leu Met Ile Asn Ser Asn Gly Asn Leu Val Ala Pro Arg Gly Tyr
               260                 265                 270
   Phe Lys Leu Asn Thr Gly Lys Ser Ser Val Met Arg Ser Asp Val Pro
           275                 280                 285
   Ile Asp Ile Cys Val Ser Glu Cys Ile Thr Pro Asn Gly Ser Ile Ser
       290                 295                 300
   Asn Asp Lys Pro Phe Gln Asn Val Asn Lys Val Thr Tyr Gly Lys Cys
   305                 310                 315                 320
   Pro Lys Tyr Ile Arg Gln Asn Thr Leu Lys Leu Ala Thr Gly Met Arg
                   325                 330                 335
   Asn Val Pro Glu Lys Gln Thr Arg Gly Ile Phe Gly Ala Ile Ala Gly
   340 345 350
   Phe Ile Glu Asn Gly Trp Glu Gly Met Val Asp Gly Trp Tyr Gly Phe
   355 360 365
   Arg Tyr Gln Asn Ser Glu Gly Thr Gly Gln Ala Ala Asp Leu Lys Ser
   370 375 380
   Thr Gln Ala Ala Ile Asp Gln Ile Asn Gly Lys Leu Asn Arg Val Ile
   385 390 395 400
   Glu Arg Thr Asn Glu Lys Phe His Gln Ile Glu Lys Glu Phe Ser Glu
   405 410 415
   Val Glu Gly Arg Ile Gln Asp Leu Glu Lys Tyr Val Glu Asp Thr Lys
   420 425 430
   Ile Asp Leu Trp Ser Tyr Asn Ala Glu Leu Leu Val Ala Leu Glu Asn
   435 440 445
   Gln His Thr Ile Asp Leu Thr Asp Ala Glu Met Asn Lys Leu Phe Glu
   450 455 460
   Lys Thr Arg Arg Gln Leu Arg Glu Asn Ala Glu Asp Met Gly Gly Gly
   465 470 475 480
   Cys Phe Lys Ile Tyr His Lys Cys Asp Asn Ala Cys Ile Glu Ser Ile
   485 490 495
   Arg Thr Gly Thr Tyr Asp His Tyr Ile Tyr Lys Asp Glu Ala Leu Asn
   500 505 510
   Asn Arg Phe Gln Ile Lys Gly Val Glu Leu Lys Ser Gly Tyr Lys Asp
   515 520 525
   Trp Ile Leu Trp Ile Ser Phe Ala Ile Ser Cys Phe Leu Ile Cys Val
   530 535 540
   Val Leu Leu Gly Phe Ile Met Trp Ala Cys Gln Lys Gly Asn Ile Arg
   545 550 555 560
   Cys Asn Ile Cys Ile
   565
<210> 79
   <211> 20
   <212> DNA
   <213> Influenza virus
<400> 79
   tatgcatcgc tccgatccat 20
<210> 80
   <211> 21
   <212> DNA
   <213> Influenza virus
<400> 80
   gctccacttc ttccgttttg a 21
<210> 81
   <211> 30
   <212> DNA
   <213> Influenza virus
<400> 81
   aattcacagc agagggattc acatggacag 30
<210> 82
   <211> 24
   <212> DNA
   <213> Influenza Virus
<220>
   <221> variation
   <222> (7)..(7)
   <223> r = a or g
<400> 82
   catggartgg ctaaagacaa gacc 24
<210> 83
   <211> 24
   <212> DNA
   <213> Influenza virus
<220>
   <221> variation
   <222> (18)..(18)
   <223> k = g or t
<400> 83
   agggcatttt ggacaaakcg tcta 24
<210> 84
   <211> 18
   <212> DNA
   <213> Influenza virus
<400> 84
   acgctcaccg tgcccagt 18
<210> 85
   <211> 28
   <212> DNA
   <213> Influenza virus
<400> 85
   tattcgtctc agggagcaaa agcagggg 28
<210> 86
   <211> 42
   <212> DNA
   <213> Influenza virus
<400> 86
   tgtaatacga ctcactatag ggctccactt cttccgtttt ga 42
<210> 87
   <211> 30
   <212> DNA
   <213> Influenza virus
<400> 87
   gatcgctctt cagggagcaa aagcaggtag 30
<210> 88
   <211> 40
   <212> DNA
   <213> Influenza virus
<400> 88
   tgtaatacga ctcactatag ggcattttgg acaaagcgtc 40 1